# EUROPEAN PATENT APPLICATION

(11) **EP 2 857 415 A1**
(43) Date of publication of application: **08.04.2015**
(21) Application number: 13306368.5
(22) Date of filing: 02.10.2013
(51) Int. Cl.: C07K 14/16, G06F 19/16

(54) **Crystal structure of a Nef/sdAb19/HckSH3 complex and uses thereof**

(71) Applicant: INSTITUT NATIONAL DE LA SANTE ET DE LA RECHERCHE MEDICALE (INSERM), 75013 Paris (FR); Centre National de la Recherche Scientifique (CNRS), 75016 Paris (FR); Max-Planck-Gesellschaft zur Förderung der Wissenschaften e.V., 80539 München (DE); UNIVERSITE PARIS DESCARTES, 75006 Paris (FR)
(72) Inventor: Benichou, Serge, 75010 Paris (FR); Geyer, Matthias, 53359 Rheinbach (DE); Lülf, Sebastian, 44229 Dortmund (DE)
(74) Representative: Regimbeau

(57) **Abstract**

The present invention provides detailed three-dimensional structural information for the complex formed by the Nef protein and the sdAb19 antibody fragment. In addition, the present invention also provides residues which mediate the interaction between the Nef protein and the sdAb19 antibody fragment. The present invention also provides methods for identifying compounds modulating the interaction of the Nef protein and the sdAb19 antibody.

SEQUENCE LISTING

<110>  INSTITUT NATIONAL DE LA SANTE ET DE LA RECHERCHE MEDICALE
       (INSERM)
       CENTRE NATIONAL DE LA RECHERCHE SCIENTIFIQUE (CNRS)
       UNIVERSITE PARIS DESCARTES
       MAX PLANCK GESELLSCHAFT ZUR FORDERUNG DER WISSENSCHAFTEN E.v

<120>  CRYSTAL STRUCTURE OF A NEF/SdAb19 COMPLEX AND USES THEREOF

<130>  70489D32025

<160>  11

<170>  PatentIn version 3.5

<210>  1
<211>  621
<212>  DNA
<213>  Human immunodeficiency virus

<220>
<221>  misc_feature
<223>  Nef gene

<400>  1
atgggtggca agtggtcaaa acgtagtgca ggtggatggt ctgctgtaag gaaaagaatg      60

gaacaagctg agccagcagc agatggggtg ggagcagtat ctcgagactt ggaaaaatat     120

ggagcaatca caagtagcaa tacagcaact aataatgctg attgtgcctg gctggaagca     180

caagaggagg aggaggtggg gtttccagtc agacctcagg tacctttaag accaatgact     240

tacaaggcag ctgtagatat tagccacttt ttaaaagaaa aggggggact ggaagggcta     300

attcactccc aaagaagaca agatatcctt gatctgtgga tctaccacac acaaggcttc     360

ttccctgatt ggcagaacta cacaccaggg ccagggatca gatatccact gacctttgga     420

tggtgcttca agctagtacc agttgaggca gaacaggtag aagaggccaa tacaggagag     480

aacaacagcc tgttacaccc tatgagcctg catgggatgg aggacgcgga gagagaagta     540

ttacagtgga agtttgacag ccgcctagca tttcatcaca tggcccgaga gctgcatccg     600

gagtactaca aagactgcta g                                               621

<210>  2
<211>  210
<212>  PRT
<213>  Human immunodeficiency virus

<220>
<221>  misc_feature
<223>  Nef protein

<400>  2

Met Gly Gly Lys Trp Ser Lys Arg Ser Met Gly Gly Trp Ser Ala Ile
1               5                   10                  15

Arg Glu Arg Met Arg Arg Ala Glu Pro Arg Ala Glu Pro Ala Ala Asp
            20                  25                  30

Gly Val Gly Ala Val Ser Arg Asp Leu Glu Lys His Gly Ala Ile Thr
        35                  40                  45

Ser Ser Asn Thr Ala Ala Thr Asn Ala Asp Cys Ala Trp Leu Glu Ala
    50                  55                  60

Gln Glu Glu Glu Glu Val Gly Phe Pro Val Arg Pro Gln Val Pro Leu
65                  70                  75                  80

Arg Pro Met Thr Tyr Lys Ala Ala Leu Asp Ile Ser His Phe Leu Lys
                85                  90                  95

Glu Lys Gly Gly Leu Glu Gly Leu Ile Trp Ser Gln Arg Arg Gln Glu
            100                 105                 110

Ile Leu Asp Leu Trp Ile Tyr His Thr Gln Gly Tyr Phe Pro Asp Trp
        115                 120                 125

Gln Asn Tyr Thr Pro Gly Pro Gly Ile Arg Tyr Pro Leu Thr Phe Gly
    130                 135                 140

Trp Cys Phe Lys Leu Val Pro Val Glu Pro Glu Lys Val Glu Glu Ala
145                 150                 155                 160

Asn Glu Gly Glu Asn Asn Ser Leu Leu His Pro Met Ser Leu His Gly
                165                 170                 175

Met Glu Asp Ala Glu Lys Glu Val Leu Val Trp Arg Phe Asp Ser Lys
            180                 185                 190

Leu Ala Phe His His Met Ala Arg Glu Leu His Pro Glu Tyr Tyr Lys
        195                 200                 205

Asp Cys
    210

<210>  3
<211>  444
<212>  DNA
<213>  artificial

<220>

<223>  Nef gene del1-44; 158-178

<400>  3
ggcgccatgg caagtagcaa tacagcagct actaatgctg attgtgcctg gctagaagca      60

caagaggagg aagaggtggg ttttccagtc cgccctcagg tacctttacg tccaatgact     120

tacaaggcag ctttagatat tagccacttt ttaaaagaaa aggggggact ggaagggctg     180

atttggtccc aacgccgtca agagatcctt gatctgtgga tctaccacac acaaggctac     240

ttccctgatt ggcagaatta cacaccaggg ccagggatca gatatccact gacctttgga     300

tggtgcttca agctagtacc agttgagcca gagaaggtcg acgcggagaa agaagtgtta     360

gtgtggcggt ttgacagcaa actggcattt catcacatgg cccgagagct gcatccggag     420

tactacaaag acgcctgaga attc                                            444


<210>  4
<211>  145
<212>  PRT
<213>  artificial

<220>
<223>  Nef protein del1-44; 158-178

<400>  4

Gly Ala Met Ala Ser Ser Asn Thr Ala Ala Thr Asn Ala Asp Cys Ala
1               5                   10                  15


Trp Leu Glu Ala Gln Glu Glu Glu Glu Val Gly Phe Pro Val Arg Pro
            20                  25                  30


Gln Val Pro Leu Arg Pro Met Thr Tyr Lys Ala Ala Leu Asp Ile Ser
        35                  40                  45


His Phe Leu Lys Glu Lys Gly Gly Leu Glu Gly Leu Ile Trp Ser Gln
    50                  55                  60


Arg Arg Gln Glu Ile Leu Asp Leu Trp Ile Tyr His Thr Gln Gly Tyr
65                  70                  75                  80


Phe Pro Asp Trp Gln Asn Tyr Thr Pro Gly Pro Gly Ile Arg Tyr Pro
                85                  90                  95


Leu Thr Phe Gly Trp Cys Phe Lys Leu Val Pro Val Glu Pro Glu Lys
            100                 105                 110


Val Asp Ala Glu Lys Glu Val Leu Val Trp Arg Phe Asp Ser Lys Leu
        115                 120                 125


Ala Phe His His Met Ala Arg Glu Leu His Pro Glu Tyr Tyr Lys Asp

    130                 135                 140

Ala
145

<210> 5
<211> 7
<212> PRT
<213> artificial

<220>
<223> CDR1 sdAb19 (single-chain camelid antibody derivative)

<400> 5

Phe Thr Phe Gly Thr Ser Asn
1               5

<210> 6
<211> 8
<212> PRT
<213> artificial

<220>
<223> CDR2 sdAb19 (single-chain camelid antibody derivative)

<400> 6

Thr Ile Ser Gly Tyr Thr Asp Tyr
1               5

<210> 7
<211> 6
<212> PRT
<213> artificial

<220>
<223> CDR3 sdAb19 (single-chain camelid antibody derivative)

<400> 7

Arg Val Gly Ser Glu Tyr
1               5

<210> 8
<211> 361
<212> DNA
<213> artificial

<220>
<223> sequence encoding sdAb19 (single-chain camelid antibody
derivative)

<400> 8
atggccgagg tgcagctggt ggagtctggg ggaggcttgg tgcaggctgg gggctctctg 60

agactcttct gtgcggcctc tggattcacc ttcggtacca gtaatatggc ctggctccgc 120

caggctccag ggaagcgtcg cgagtgggtc gcacttatta cgattagtgg ctacacagac     180

tatgcagact ccgtgaagga ccgattcacc atatccagag acaacgccaa gaacacggtg     240

tctctgcaaa tgaacagcct gaaacctgag gacacggcca tttatttttg cgcccggcgt     300

gtagggtctg agtatgattt gtggggccag gggacccagg tcactgtctc ctcatgaatt     360

c                                                                     361

<210>  9
<211>  118
<212>  PRT
<213>  artificial

<220>
<223>  sdAb19 (single-chain camelid antibody derivative)

<400>  9

Met Ala Glu Val Gln Leu Val Glu Ser Gly Gly Gly Leu Val Gln Ala
1               5                   10                  15

Gly Gly Ser Leu Arg Leu Phe Cys Ala Ala Ser Gly Phe Thr Phe Gly
            20                  25                  30

Thr Ser Asn Met Ala Trp Leu Arg Gln Ala Pro Gly Lys Arg Arg Glu
        35                  40                  45

Trp Val Ala Leu Ile Thr Ile Ser Gly Tyr Thr Asp Tyr Ala Asp Ser
    50                  55                  60

Val Lys Asp Arg Phe Thr Ile Ser Arg Asp Asn Ala Lys Asn Thr Val
65                  70                  75                  80

Ser Leu Gln Met Asn Ser Leu Lys Pro Glu Asp Thr Ala Ile Tyr Phe
                85                  90                  95

Cys Ala Arg Arg Val Gly Ser Glu Tyr Asp Leu Trp Gly Gln Gly Thr
            100                 105                 110

Gln Val Thr Val Ser Ser
        115

<210>  10
<211>  195
<212>  DNA
<213>  artificial

<220>
<223>  sequence encoding the SH3 domain of human protein Heck (78-138)

<400>  10
ggatccgagg acatcatcgt ggttgccctg tatgattact attccccttt ctcctgggac     60

ctcagcttcc agaaggggga ccagatggtg gtcctagagg aatccgggga gtggtggaag 120

gctcgatccc tggccacccg gaaggagggc tacatcccaa gcaactatgt cgcccgcgtt 180

gactcttgag aattc 195

<210> 11
<211> 62
<212> PRT
<213> artificial

<220>
<223> SH3 domain of human protein Heck (78-138)

<400> 11

Gly Ser Glu Asp Ile Ile Val Val Ala Leu Tyr Asp Tyr Tyr Ser Pro
1               5                   10                  15

Phe Ser Trp Asp Leu Ser Phe Gln Lys Gly Asp Gln Met Val Val Leu
            20                  25                  30

Glu Glu Ser Gly Glu Trp Trp Lys Ala Arg Ser Leu Ala Thr Arg Lys
        35                  40                  45

Glu Gly Tyr Ile Pro Ser Asn Tyr Val Ala Arg Val Asp Ser
    50                  55                  60

## Description

### Introduction

Significant advances in antiretroviral therapy of HIV infection have been made since the introduction of zidovudine (AZT) in 1987. Intensive research on HIV led to the development of highly active antiretroviral therapies (HAART). HAART is defined as treatment with at least three active anti-retroviral medications, commonly reverse transcriptase inhibitors and protease inhibitors. These therapies have been extremely successful in controlling the spread of the disease. Indeed, with the advent of HAART, HIV infection is now manageable as a chronic disease in patients who have access to medication and who achieve durable virological suppression.

However, HAART is unable to lead to virus eradication in infected patients. Moreover, there are serious concerns regarding HAART, such as e.g. the drug resistances, the side-effects associated with the anti-retroviral drugs or the costs of the treatment. Therefore, new anti-HIV drugs are still needed.

Targeting other original viral determinants, such as virus regulatory proteins, directly involved in viral pathogenesis, may have a high beneficial impact. In particular, the HIV Nef protein (Negative Regulatory Factor) has long been known to be required for full virus infectivity and AIDS pathogenesis. More specifically, it has a positive effect on viral infection and replication and promotes dysfunction of the immune target cells of HIV.

It is now clear that this role is linked to the combination of several activities of the Nef protein *in vitro.* Nef interacts with tyrosine and serine/threonine kinases, thus affecting cellular signaling pathways. In addition, Nef increases viral infectivity after entry into the cell. But the best-documented activity of Nef is related to its ability to down-modulate the expression levels of important molecules at the surface of infected cells, such as e.g. CD4, CD28 and MHC class I and class II (MHC-I and MHC-II, respectively) molecules through interfering with the endocytic pathway.

The three-dimensional structure of Nef is available (Grzesiek et al., Nat.Struct.Mol.Biol. 3:340-345, 1996; Arold et al., Structure, 5(10): 1361-1372, 1997; Grzesiek et al., Protein Sci. 6: 1248-1263, 1997; Horenkamp et al., Traffic 12: 867-877, 2011). A flexible, N-terminal region of about 60 residues, which mediates protein attachment to the cell membranes, is followed by a conserved domain of approximately 130 residues having a stable three-dimensional structure. Finally, the C-terminal region comprises another flexible loop, between residues 149 and 178, which projects outside of the central domain.

Several motifs important for protein-protein interactions and Nef functions have been identified and precisely localized on the Nef structure. For example, the di-Leu motif (₁₆₀ENTSLL₁₆₅), within the C-terminal loop, is required for CD4 modulation. On the other hand, MHC-I down-regulation requires several regions localized within the N-terminal part of Nef and two motifs, the acidic (₆₂EEEE₆₅) and poly-proline (₇₂PxxP₇₅). The residues of the Nef protein are numbered by reference to the sequence of UniProtKB/Swiss-Prot accession number P03407 (SEQ ID NO. 2)

Although Nef has a crucial role in HIV-1 pathogenesis, it is currently not targeted by anti-retroviral therapeutic strategies. Small molecules have been described which bind to Nef, but they only do so with relatively low affinity, display high cytotoxicity and/or inhibit only a subset of the Nef functions (Betzi et al., Proc. Natl. Acad. Sci. U.S.A., 104: 19256-19261, 2007; Olszewski et al., Proc. Natl. Acad. Sci. U.S.A., 101: 14079-14084, 2004; Emert-Sedlak et al., ACS Chem Biol, 4: 939-947, 2009; Dikeakos et al., Mol Biol Cell, 21: 3279-3292, 2010).

A camelid single-domain antibody fragment, designated sdAb19, having high affinity for Nef, was previously shown to be capable of inhibiting critical biological activities of Nef (WO 2009/066241; Bouchet et al., Blood, 117(13): 3559-3568, 2011; Bouchet et al., J. Virol., 86: 4856-4867, 2012). This antibody fragment is capable of counteracting the Nef-mediated endocytosis of CD4 from the cell surface, and it also inhibits the positive effect of Nef on viral replication and infectivity. Finally, sdAb19 is also active *in vivo* by rescuing Nef mediated thymic CD4⁺ T-cell maturation defects and peripheral CD4⁺ T-cell activation in a CD4c/HIV-1^{Nef} transgenic mouse model. However, sdAb19 does not inhibit the Nef-mediated down-regulation of MHC-I molecules (WO 2009/066241; Bouchet et al., Blood, 117(13): 3559-3568, 2011).

The sdAb19 antibody fragment shows that it is possible to inhibit most important functions of Nef with a single component. Hence it represents a crucial tool for establishing new anti-HIV therapies. This could lead to isolation of compounds mimicking the action of sdAb19 and counteracting the biological activities of Nef. However, although it is thought that sdAb19 recognizes an epitope in the C-terminal part of Nef, precise mapping of this epitope has thus far been unsuccessful (WO 2009/066241; Bouchet et al., Blood, 117(13): 3559-3568, 2011).

There is thus still a need for identifying the location of the region of the Nef protein which mediates the interaction with sdAb19.

### Description

The sdAb19 antibody fragment is capable of inhibiting critical biological activities of Nef, leading to the possibility that compounds mimicking the binding of sdAb19 onto Nef may possess the same inhibitory activities. However, previous attempts at identifying the binding site of sdAb19 on Nef have failed (WO 2009/066241; Bouchet et al., Blood, 117(13): 3559-3568, 2011; Bouchet et al., J. Virol., 86: 4856-4867, 2012).

In general, it is an object of the present invention to provide detailed three-dimensional structural information for the complex formed by the Nef protein and the sdAb19 antibody fragment. In particular, the present invention provides detailed three-dimensional structural information for the complex formed by the Nef protein, the sdAb19 antibody and the SH3 domain of the Hck tyrosine kinase. In addition, the present invention also provides residues which mediate the interaction between the Nef protein and the sdAb19 antibody fragment.

The determination of the three-dimensional structure of the Nef/sdAb19 complex is useful in the design, identification and screening of new Nef inhibitory compounds which are useful for the inhibition of Nef functions during HIV infection.

In one embodiment, three-dimensional structural information is obtained from a crystal of the complex formed by Nef protein, sdAb19, and the SH3 domain of the Hck kinase.

By "Nef protein", it is herein referred to a ca. 27 kD, N-terminally myristoylated accessory protein. Preferably, the Nef protein is encoded by a lentiviral gene, more preferably an HIV gene. Even more preferably, the Nef protein is encoded by a gene having the sequence represented by SEQ ID No. 1 and which corresponds to the sequence of GenBank accession number: DQ242535.1. Most preferably, the Nef protein has an amino acid sequence represented by SEQ ID No. 2, which corresponds to the sequence of UniProtKB/Swiss-Prot accession number P03407, or a mutated sequence thereof.

Nef was previously shown to comprise an unstructured N-terminal domain and a flexible C-terminal loop (Geyer et al., J.Mol.Biol. 289: 123-138, 1999; Grzesiek et al., Nat.Struct.Mol.Biol. 3: 340-345, 1996; Arold et al., Structure, 5(10): 1361-1372, 1997; Grzesiek et al., Protein Sci. 6: 1248-1263, 1997). It is well known in the art that rigid stable proteins are much more likely to crystallize than proteins that are internally flexible or have dynamic surfaces. It is thus advantageous to delete or remove the flexible regions at the N-terminus and/or within the C-terminal region of Nef. Accordingly, in a preferred embodiment, the Nef protein of the invention lacks residues 1-44 and/or residues 158-178. Most preferably, the Nef protein of the invention lacks both regions (residues 1-44 and residues 158-178). Even more preferably, the Nef protein of the invention has the sequence of SEQ ID No. 4 or a mutated sequence thereof, and is encoded by the gene of SEQ ID No. 3.

By "sdAb19", it is herein referred to the single-chain antibody fragment directed against the Nef protein, which is described in WO 2009/066241 and in Bouchet et al. (Blood, 117(13): 3559-3568, 2011). A "single-chain antibody fragment" according to the invention is a non-conventional antibody, said antibody comprising the variable region of a heavy chain, but no light chain. Examples of single-chain antibodies include the antibodies produced by sharks or camelids (Wesolowski et al., Med Microbiol Immunol, 198(3): 157-174, 2009; de Marco, Microbial Cell Factories 2011, 10:44, 2011).

The sdAb19 antibody fragment of the invention is capable of binding Nef with high affinity and of inhibiting most of the Nef functions thereof. Preferably, the sdAb19 antibody fragment of the invention consists of a fragment of a heavy chain, said heavy chain comprising 3 CDRs having the sequences SEQ ID Nos. 5-7. More preferably, the sdAb19 antibody fragment of the invention consists of a fragment of a heavy chain, said heavy chain fragment comprising the sequence 9 and is encoded by a gene comprising the sequence of SEQ ID NO. 8.

By "Hck SH3 domain" or "Hck SH3 fragment", it is herein referred to a domain of around 60 amino acids of the Hck protein which has homology to a canonical SH3 domain.

The "Hck protein" according to the present invention is a tyrosine protein kinase of the Src family (UniProtKB accession number: UniProtKB P08631) which is encoded by the *hck* gene (accession number: NM_002110).

The Hck SH3 domain binds with high affinity to the poly-proline motif found in the N-terminal region of Nef (Lee et al., EMBO J, 14(20): 5006-5015, 1995). Mutations in the specificity-determining RT-loop region of this SH3 domain resulting in an even higher affinity have been previously described (Hiipakka et al., J Mol Biol, 293: 1097-1106, 1999; Breuer et al., PLoS One, 6(5):e20033, 2011; Järviluoma et al., PLoS One, 7(7):e40331, 2012; Bouchet et al., J Virol, 86(9): 4856-4867, 2012). Such mutants are also encompassed by the term "Hck SH3 domain". Preferably, the Hck SH3 domain of the invention has the sequence of SEQ ID NO. 11 and is encoded by a gene having the sequence of SEQ ID NO. 10, or a mutated sequence thereof.

In preferred embodiment, the invention relates to a crystal of the complex formed by the Nef protein, sdAb19, and the SH3 domain of the Hck kinase, wherein the Nef protein has the sequence of SEQ ID N. 4, the sdAb19 antibody fragment has the sequence of SEQ ID NO. 9 , and the Hck SH3 domain has the sequence of SEQ ID NO. 11.

The protein subunits of the complex of the invention also encompass mutated sequences thereof.

As used herein, by "mutated sequence", it is meant an amino acid sequence which comprises deletion(s), addition(s) or substitution(s) of amino acid(s), but still retains all the essential characteristics of the protein of reference, i.e. in the context of the invention, retains at least the structural characteristics of the crystal (such as atomic coordinates) with acceptable standard variations. In particular, a polypeptide with a mutated sequence retains the ability to interact and form a complex with the two other partner polypeptides. A mutated sequence may be made, for example, by mutagenesis techniques on the nucleic acid encoding the protein of reference, such as site-directed mutagenesis.

Preferably, the present invention provides a crystal of a complex formed between the Nef protein, sdAb19, and the Hck SH3 domain, wherein the space group is P4₁(76), and the unit cell dimension are a = 73.07 Å, b = 73.07 Å, c = 71.25 Å, with α=β=γ=90°.

As used herein, a "unit cell" refers to the smallest repeating unit of a crystal and is defined by three cell edges (a, b, and c in Å), and three angles (α, β and y, in degrees) between each pair of edges. Each unit cell may contain one or more protein molecules related by crystal symmetry. The unique portion of the unit cell, i.e., the portion that is not related to other portions by crystal symmetry, is the "asymetric unit". A "space group", or "three dimensional space group", means herein a particular combination of symmetry elements in a crystal among the 230 existing possible combinations (Hahn (Ed.), International Tables of Crystallography, Volume A, 5th Edition, Space-Group Symmetry, WILEY, 2006).

The complex structure disclosed in the present invention is listed in Table 1 and provides new and surprising insight into the structural arrangement of the sdAb19 interaction with Nef.

Thus in a preferred embodiment, the invention relates to a crystal wherein the atomic coordinates are defined in Table 1.

**Table 1: Atomic coordinates of the Nef/sdAb19/HckSH₃ crystal**

| | | | | | | | | | | |
|---|---|---|---|---|---|---|---|---|---|---|
| REMARK | 3 | REFINEMENT. | | | | | | | | |
| REMARK | 3 | PROGRAM | : PHENIX (phenix.refine: 1.8.1_1168) | | | | | | | |
| REMARK | 3 | AUTHORS | : Adams, Afonine, Chen, Davis, Echols, Gildea, Gopal, | | | | | | | |
| REMARK | 3 | : Grosse-Kunstleve, Headd, Hung, Immormino, Ioerger, McCoy, | | | | | | | | |
| REMARK | 3 | | : McKee, Moriarty, Pai, Read, Richardson, Richardson, Romo, | | | | | | | |
| REMARK | 3 | | : Sacchettini, Sauter, Smith, Storoni, Terwilliger, Zwart | | | | | | | |
| REMARK | 3 | | | | | | | | | |
| REMARK | 3 | REFINEMENT TARGET : MLHL | | | | | | | | |
| REMARK | 3 | DATA USED IN REFINEMENT. | | | | | | | | |
| REMARK | 3 | RESOLUTION RANGE HIGH | | (ANGSTROMS) | : 2.000 | | | | | |
| REMARK | 3 | RESOLUTION RANGE LOW | | (ANGSTROMS) | : 41.751 | | | | | |
| REMARK | 3 | MIN(FOBS/SIGMA_FOBS) | | | : 2.04 | | | | | |
| REMARK | 3 | COMPLETENESS FOR RANGE | | (%) | : 98.94 | | | | | |
| REMARK | 3 | NUMBER OF REFLECTIONS | | | : 25031 | | | | | |
| REMARK | 3 | NUMBER OF REFLECTIONS (NON-ANOMALOUS) | | | | : 25031 | | | | |
| REMARK | 3 | | | | | | | | | |
| REMARK | 3 | FIT TO DATA USED IN REFINEMENT. | | | | | | | | |
| REMARK | 3 | R VALUE | (WORKING + TEST SET) | | : 0.2036 | | | | | |
| REMARK | 3 | R VALUE | (WORKING SET) | | : 0.2018 | | | | | |
| REMARK | 3 | FREE R VALUE | | | : 0.2364 | | | | | |
| REMARK | 3 | FREE R VALUE TEST SET SIZE | | (%) | : 5.00 | | | | | |
| REMARK | 3 | FREE R VALUE TEST SET COUNT | | | | : 1252 | | | | |
| REMARK | 3 | | | | | | | | | |
| REMARK | 3 | FIT TO DATA USED IN REFINEMENT (IN BINS). | | | | | | | | |
| REMARK | 3 | BIN | RESOLUTION | RANGE | COMPL. | NWORK | NFREE | RWORK | RFREE | |
| REMARK | 3 | 1 | 41.7600 | - 4.1590 | 1.00 | 2717 | 144 | 0.1686 | 0.2010 | |
| REMARK | 3 | 2 | 4.1590 | - 3.3015 | 0.99 | 2656 | 139 | 0.1784 | 0.2194 | |
| REMARK | 3 | 3 | 3.3015 | - 2.8843 | 0.99 | 2658 | 140 | 0.2162 | 0.2391 | |
| REMARK | 3 | 4 | 2.8843 | - 2.6206 | 0.99 | 2621 | 138 | 0.2189 | 0.2412 | |
| REMARK | 3 | 5 | 2.6206 | - 2.4328 | 0.99 | 2645 | 139 | 0.2349 | 0.2651 | |
| REMARK | 3 | 6 | 2.4328 | - 2.2894 | 0.99 | 2638 | 139 | 0.2387 | 0.2992 | |
| REMARK | 3 | 7 | 2.2894 | - 2.1748 | 0.98 | 2624 | 138 | 0.2342 | 0.2715 | |
| REMARK | 3 | 8 | 2.1748 | - 2.0801 | 0.99 | 2592 | 137 | 0.2397 | 0.2712 | |
| REMARK | 3 | 9 | 2.0801 | - 2.0000 | 0.98 | 2628 | 138 | 0.2500 | 0.2915 | |
| REMARK | 3 | | | | | | | | | |
| REMARK | 3 | BULK SOLVENT MODELLING. | | | | | | | | |
| REMARK | 3 | METHOD USED | | | : FLAT BULK SOLVENT MODEL | | | | | |
| REMARK | 3 | SOLVENT RADIUS | | | : 1.11 | | | | | |
| REMARK | 3 | SHRINKAGE RADIUS | | | : 0.90 | | | | | |
| REMARK | 3 | GRID STEP FACTOR | | | : 4.00 | | | | | |
| REMARK | 3 | | | | | | | | | |
| REMARK | 3 | ERROR ESTIMATES. | | | | | | | | |
| REMARK | 3 | COORDINATE ERROR (MAXIMUM-LIKELIHOOD BASED) | | | | | | : 0.21 | | |
| REMARK | 3 | PHASE ERROR (DEGREES, MAXIMUM-LIKELIHOOD BASED) | | | | | | : 24.91 | | |
| REMARK | 3 | | | | | | | | | |
| REMARK | 3 | STRUCTURE FACTORS CALCULATION ALGORITHM : | | | | | FFT | | | |
| REMARK | 3 | | | | | | | | | |
| REMARK | 3 | DEVIATIONS FROM IDEAL VALUES. | | | | | | | | |
| REMARK | 3 | | | RMSD | MAX | COUNT | | | | |
| REMARK | 3 | BOND : | | 0.008 | 0.077 | 2399 | | | | |
| REMARK | 3 | ANGLE : | | 1.288 | 19.370 | 3255 | | | | |
| REMARK | 3 | CHIRALITY : | | 0.085 | 0.347 | 336 | | | | |
| REMARK | 3 | PLANARITY : | | 0.006 | 0.051 | 413 | | | | |
| REMARK | 3 | DIHEDRAL : | | 15.518 | 87.623 | 865 | | | | |
| REMARK | 3 | MIN NONBONDED DISTANCE : | | | 1.910 | | | | | |
| REMARK | 3 | | | | | | | | | |
| REMARK | 3 | MOLPROBITY STATISTICS. | | | | | | | | |
| REMARK | 3 | ALL-ATOM CLASHSCORE : | | | 11.09 | | | | | |
| REMARK | 3 | RAMACHANDRAN PLOT: | | | | | | | | |
| REMARK | 3 | | OUTLIERS | : 0.72 % | | | | | | |
| REMARK | 3 | | ALLOWED | : 3.94 % | | | | | | |
| REMARK | 3 | | FAVORED | : 95.34 % | | | | | | |
| REMARK | 3 | ROTAMER OUTLIERS | | | : 6.94 % | | | | | |
| REMARK | 3 | CBETA DEVIATIONS | | | : 1 | | | | | |
| REMARK | 3 | | | | | | | | | |
| REMARK | 3 | ATOMIC DISPLACEMENT PARAMETERS. | | | | | | | | |
| REMARK | 3 | WILSON B : | | 29.90 | | | | | | |
| REMARK | 3 | RMS(B_ISO_OR_EQUIVALENT_BONDED) : | | | | | 3.13 | | | |
| REMARK | 3 | ATOMS | | NUMBER OF ATOMS | | | | | | |
| REMARK | 3 | | | | ISO. | ANISO. | | | | |
| REMARK | 3 | ALL : | | | 2459 | 0 | | | | |
| REMARK | 3 | ALL (NO H) : | | | 2459 | 0 | | | | |
| REMARK | 3 | SOLVENT : | | | 129 | 0 | | | | |
| REMARK | 3 | NON-SOLVENT : | | | 2330 | 0 | | | | |
| REMARK | 3 | HYDROGENS : | | | 0 | 0 | | | | |
| CRYST1 | 73.000 | | 73.000 | | 71.000 | 90.00 | 90.00 | 90.00 | P 41 | |
| SCALE1 | | 0.013699 | | 0.000000 | 0.000000 | | 0.00000 | | | |
| SCALE2 | | 0.000000 | | 0.013699 | 0.000000 | | 0.00000 | | | |
| SCALE3 | | 0.000000 | | 0.000000 | 0.014085 | | 0.00000 | | | |
| ATOM | 1 | N | ILE A | 81 | 25.183 | 21.754 | 30.131 | 1.00 | 55.67 | N |
| ATOM | 2 | CA | ILE A | 81 | 23.796 | 21.643 | 29.702 | 1.00 | 48.33 | C |
| ATOM | 3 | CB | ILE A | 81 | 23.637 | 22.075 | 28.231 | 1.00 | 49.24 | C |
| ATOM | 4 | CG1 | ILE A | 81 | 24.577 | 23.233 | 27.903 | 1.00 | 50.92 | C |
| ATOM | 5 | CD1 | ILE A | 81 | 24.331 | 23.858 | 26.533 | 1.00 | 52.03 | C |
| ATOM | 6 | CG2 | ILE A | 81 | 22.200 | 22.456 | 27.937 | 1.00 | 49.92 | C |
| ATOM | 7 | C | ILE A | 81 | 23.274 | 20.211 | 29.863 | 1.00 | 50.39 | C |
| ATOM | 8 | O | ILE A | 81 | 23.472 | 19.372 | 28.980 | 1.00 | 50.43 | O |
| ATOM | 9 | N | ILE A | 82 | 22.622 | 19.930 | 30.990 | 1.00 | 48.47 | N |
| ATOM | 10 | CA | ILE A | 82 | 22.015 | 18.615 | 31.228 | 1.00 | 46.42 | C |
| ATOM | 11 | CB | ILE A | 82 | 22.108 | 18.166 | 32.707 | 1.00 | 45.99 | C |
| ATOM | 12 | CG1 | ILE A | 82 | 23.562 | 17.891 | 33.099 | 1.00 | 51.09 | C |
| ATOM | 13 | CD1 | ILE A | 82 | 24.343 | 19.126 | 33.520 | 1.00 | 53.80 | C |
| ATOM | 14 | CG2 | ILE A | 82 | 21.279 | 16.907 | 32.952 | 1.00 | 42.68 | C |
| ATOM | 15 | C | ILE A | 82 | 20.549 | 18.627 | 30.788 | 1.00 | 51.22 | C |
| ATOM | 16 | O | ILE A | 82 | 19.831 | 19.617 | 30.996 | 1.00 | 49.05 | O |
| ATOM | 17 | N | VAL A | 83 | 20.114 | 17.537 | 30.154 | 1.00 | 46.05 | N |
| ATOM | 18 | CA | VAL A | 83 | 18.763 | 17.461 | 29.626 | 1.00 | 43.86 | C |
| ATOM | 19 | CB | VAL A | 83 | 18.759 | 17.693 | 28.101 | 1.00 | 46.62 | C |
| ATOM | 20 | CG1 | VAL A | 83 | 17.473 | 18.215 | 27.702 | 1.00 | 48.47 | C |
| ATOM | 21 | CG2 | VAL A | 83 | 19.770 | 18.743 | 27.706 | 1.00 | 48.67 | C |
| ATOM | 22 | C | VAL A | 83 | 18.123 | 16.119 | 29.980 | 1.00 | 43.48 | C |
| ATOM | 23 | O | VAL A | 83 | 18.825 | 15.124 | 30.181 | 1.00 | 43.32 | O |
| ATOM | 24 | N | VAL A | 84 | 16.797 | 16.097 | 30.102 | 1.00 | 43.79 | N |
| ATOM | 25 | CA | VAL A | 84 | 16.074 | 14.840 | 30.289 | 1.00 | 41.63 | C |
| ATOM | 26 | CB | VAL A | 84 | 15.281 | 14.797 | 31.614 | 1.00 | 40.91 | C |
| ATOM | 27 | CG1 | VAL A | 84 | 16.231 | 14.747 | 32.794 | 1.00 | 51.43 | C |
| ATOM | 28 | CG2 | VAL A | 84 | 14.353 | 16.010 | 31.712 | 1.00 | 44.07 | C |
| ATOM | 29 | C | VAL A | 84 | 15.108 | 14.599 | 29.129 | 1.00 | 40.55 | C |
| ATOM | 30 | O | VAL A | 84 | 14.579 | 15.542 | 28.544 | 1.00 | 41.40 | O |
| ATOM | 31 | N | ALA A | 85 | 14.878 | 13.329 | 28.819 | 1.00 | 43.39 | N |
| ATOM | 32 | CA | ALA A | 85 | 14.022 | 12.940 | 27.706 | 1.00 | 42.75 | C |
| ATOM | 33 | CB | ALA A | 85 | 14.385 | 11.547 | 27.253 | 1.00 | 40.78 | C |
| ATOM | 34 | C | ALA A | 85 | 12.546 | 12.996 | 28.083 | 1.00 | 43.34 | C |
| ATOM | 35 | O | ALA A | 85 | 12.132 | 12.382 | 29.075 | 1.00 | 36.58 | O |
| ATOM | 36 | N | LEU A | 86 | 11.761 | 13.719 | 27.281 | 1.00 | 39.59 | N |
| ATOM | 37 | CA | LEU A | 86 | 10.319 | 13.831 | 27.488 | 1.00 | 36.63 | C |
| ATOM | 38 | CB | LEU A | 86 | 9.798 | 15.135 | 26.883 | 1.00 | 34.52 | C |
| ATOM | 39 | CG | LEU A | 86 | 10.525 | 16.393 | 27.365 | 1.00 | 41.12 | C |
| ATOM | 40 | CD1 | LEU A | 86 | 10.176 | 17.618 | 26.528 | 1.00 | 36.67 | C |
| ATOM | 41 | CD2 | LEU A | 86 | 10.239 | 16.655 | 28.843 | 1.00 | 42.79 | C |
| ATOM | 42 | C | LEU A | 86 | 9.574 | 12.646 | 26.894 | 1.00 | 38.82 | C |
| ATOM | 43 | O | LEU A | 86 | 8.448 | 12.343 | 27.298 | 1.00 | 39.02 | O |
| ATOM | 44 | N | TYR A | 87 | 10.203 | 11.984 | 25.922 | 1.00 | 38.75 | N |
| ATOM | 45 | CA | TYR A | 87 | 9.616 | 10.825 | 25.253 | 1.00 | 38.32 | C |
| ATOM | 46 | CB | TYR A | 87 | 8.977 | 11.227 | 23.914 | 1.00 | 37.48 | C |
| ATOM | 47 | CG | TYR A | 87 | 8.186 | 12.523 | 23.904 | 1.00 | 36.15 | C |
| ATOM | 48 | CD2 | TYR A | 87 | 6.798 | 12.519 | 24.006 | 1.00 | 36.18 | C |
| ATOM | 49 | CE2 | TYR A | 87 | 6.074 | 13.699 | 23.979 | 1.00 | 34.59 | C |
| ATOM | 50 | CZ | TYR A | 87 | 6.742 | 14.898 | 23.842 | 1.00 | 37.14 | C |
| ATOM | 51 | OH | TYR A | 87 | 6.047 | 16.086 | 23.816 | 1.00 | 47.52 | O |
| ATOM | 52 | CE1 | TYR A | 87 | 8.113 | 14.927 | 23.729 | 1.00 | 38.43 | C |
| ATOM | 53 | CD1 | TYR A | 87 | 8.826 | 13.744 | 23.747 | 1.00 | 35.35 | C |
| ATOM | 54 | C | TYR A | 87 | 10.724 | 9.808 | 24.972 | 1.00 | 36.19 | C |
| ATOM | 55 | O | TYR A | 87 | 11.911 | 10.140 | 25.046 | 1.00 | 35.56 | O |
| ATOM | 56 | N | ASP A | 88 | 10.344 | 8.582 | 24.634 | 1.00 | 33.13 | N |
| ATOM | 57 | CA | ASP A | 88 | 11.312 | 7.602 | 24.160 | 1.00 | 37.35 | C |
| ATOM | 58 | CB | ASP A | 88 | 10.702 | 6.203 | 24.139 | 1.00 | 36.61 | C |
| ATOM | 59 | CG | ASP A | 88 | 10.496 | 5.624 | 25.541 | 1.00 | 42.74 | C |
| ATOM | 60 | OD1 | ASP A | 88 | 10.931 | 6.254 | 26.527 | 1.00 | 37.86 | O |
| ATOM | 61 | OD2 | ASP A | 88 | 9.915 | 4.523 | 25.647 | 1.00 | 42.08 | O |
| ATOM | 62 | C | ASP A | 88 | 11.739 | 7.967 | 22.744 | 1.00 | 38.55 | C |
| ATOM | 63 | O | ASP A | 88 | 10.911 | 8.419 | 21.953 | 1.00 | 39.12 | O |
| ATOM | 64 | N | TYR A | 89 | 13.015 | 7.772 | 22.420 | 1.00 | 34.96 | N |
| ATOM | 65 | CA | TYR A | 89 | 13.461 | 7.910 | 21.036 | 1.00 | 32.64 | C |
| ATOM | 66 | CB | TYR A | 89 | 14.320 | 9.158 | 20.847 | 1.00 | 31.43 | C |
| ATOM | 67 | CG | TYR A | 89 | 14.833 | 9.323 | 19.430 | 1.00 | 33.08 | C |
| ATOM | 68 | CD1 | TYR A | 89 | 13.951 | 9.438 | 18.356 | 1.00 | 29.12 | C |
| ATOM | 69 | CE1 | TYR A | 89 | 14.421 | 9.592 | 17.045 | 1.00 | 28.69 | C |
| ATOM | 70 | CZ | TYR A | 89 | 15.781 | 9.637 | 16.812 | 1.00 | 29.25 | C |
| ATOM | 71 | OH | TYR A | 89 | 16.271 | 9.789 | 15.529 | 1.00 | 27.49 | O |
| ATOM | 72 | CE2 | TYR A | 89 | 16.675 | 9.536 | 17.871 | 1.00 | 31.52 | C |
| ATOM | 73 | CD2 | TYR A | 89 | 16.197 | 9.373 | 19.168 | 1.00 | 30.43 | C |
| ATOM | 74 | C | TYR A | 89 | 14.240 | 6.690 | 20.595 | 1.00 | 36.31 | C |
| ATOM | 75 | O | TYR A | 89 | 15.346 | 6.432 | 21.090 | 1.00 | 35.03 | O |
| ATOM | 76 | N | TYR A | 90 | 13.677 | 5.927 | 19.665 | 1.00 | 33.01 | N |
| ATOM | 77 | CA | TYR A | 90 | 14.430 | 4.815 | 19.120 | 1.00 | 36.13 | C |
| ATOM | 78 | CB | TYR A | 90 | 13.696 | 3.480 | 19.260 | 1.00 | 37.41 | C |
| ATOM | 79 | CG | TYR A | 90 | 14.465 | 2.396 | 18.546 | 1.00 | 47.05 | C |
| ATOM | 80 | CD1 | TYR A | 90 | 15.654 | 1.893 | 19.079 | 1.00 | 45.99 | C |
| ATOM | 81 | CE1 | TYR A | 90 | 16.383 | 0.917 | 18.411 | 1.00 | 45.71 | C |
| ATOM | 82 | CZ | TYR A | 90 | 15.929 | 0.447 | 17.192 | 1.00 | 48.46 | C |
| ATOM | 83 | OH | TYR A | 90 | 16.631 | -0.517 | 16.503 | 1.00 | 55.88 | O |
| ATOM | 84 | CE2 | TYR A | 90 | 14.763 | 0.943 | 16.642 | 1.00 | 49.04 | C |
| ATOM | 85 | CD2 | TYR A | 90 | 14.043 | 1.915 | 17.313 | 1.00 | 45.53 | C |
| ATOM | 86 | C | TYR A | 90 | 14.806 | 5.082 | 17.660 | 1.00 | 39.09 | C |
| ATOM | 87 | O | TYR A | 90 | 13.947 | 5.417 | 16.838 | 1.00 | 35.97 | O |
| ATOM | 88 | N | SER A | 91 | 16.093 | 4.945 | 17.350 | 1.00 | 35.20 | N |
| ATOM | 89 | CA | SER A | 91 | 16.595 | 5.190 | 15.993 | 1.00 | 30.76 | C |
| ATOM | 90 | CB | SER A | 91 | 17.541 | 6.381 | 16.005 | 1.00 | 27.35 | C |
| ATOM | 91 | OG | SER A | 91 | 18.060 | 6.649 | 14.710 | 1.00 | 26.97 | O |
| ATOM | 92 | C | SER A | 91 | 17.359 | 3.972 | 15.510 | 1.00 | 32.86 | C |
| ATOM | 93 | O | SER A | 91 | 18.159 | 3.415 | 16.257 | 1.00 | 30.31 | O |
| ATOM | 94 | N | PRO A | 92 | 17.134 | 3.558 | 14.253 | 1.00 | 31.66 | N |
| ATOM | 95 | CA | PRO A | 92 | 17.911 | 2.425 | 13.754 | 1.00 | 28.88 | C |
| ATOM | 96 | CB | PRO A | 92 | 17.113 | 1.978 | 12.523 | 1.00 | 30.10 | C |
| ATOM | 97 | CG | PRO A | 92 | 16.486 | 3.248 | 12.018 | 1.00 | 29.52 | C |
| ATOM | 98 | CD | PRO A | 92 | 16.168 | 4.060 | 13.253 | 1.00 | 30.43 | C |
| ATOM | 99 | C | PRO A | 92 | 19.320 | 2.837 | 13.350 | 1.00 | 27.99 | C |
| ATOM | 100 | O | PRO A | 92 | 20.113 | 1.969 | 12.992 | 1.00 | 30.62 | O |
| ATOM | 101 | N | PHE A | 93 | 19.629 | 4.132 | 13.398 | 1.00 | 25.47 | N |
| ATOM | 102 | CA | PHE A | 93 | 20.893 | 4.633 | 12.860 | 1.00 | 25.61 | C |
| ATOM | 103 | CB | PHE A | 93 | 20.676 | 6.003 | 12.225 | 1.00 | 25.05 | C |
| ATOM | 104 | CG | PHE A | 93 | 19.564 | 6.021 | 11.212 | 1.00 | 25.80 | C |
| ATOM | 105 | CD1 | PHE A | 93 | 19.688 | 5.307 | 10.021 | 1.00 | 21.78 | C |
| ATOM | 106 | CE1 | PHE A | 93 | 18.668 | 5.316 | 9.074 | 1.00 | 23.71 | C |
| ATOM | 107 | CZ | PHE A | 93 | 17.506 | 6.021 | 9.330 | 1.00 | 26.22 | C |
| ATOM | 108 | CE2 | PHE A | 93 | 17.367 | 6.734 | 10.531 | 1.00 | 24.53 | C |
| ATOM | 109 | CD2 | PHE A | 93 | 18.391 | 6.729 | 11.462 | 1.00 | 22.89 | C |
| ATOM | 110 | C | PHE A | 93 | 22.021 | 4.738 | 13.876 | 1.00 | 26.10 | C |
| ATOM | 111 | O | PHE A | 93 | 21.806 | 5.217 | 14.984 | 1.00 | 27.45 | O |
| ATOM | 112 | N | SER A | 94 | 23.233 | 4.354 | 13.471 | 1.00 | 26.07 | N |
| ATOM | 113 | CA | SER A | 94 | 24.382 | 4.336 | 14.393 | 1.00 | 32.79 | C |
| ATOM | 114 | CB | SER A | 94 | 25.585 | 3.617 | 13.757 | 1.00 | 32.78 | C |
| ATOM | 115 | OG | SER A | 94 | 26.100 | 4.346 | 12.654 | 1.00 | 34.66 | O |
| ATOM | 116 | C | SER A | 94 | 24.791 | 5.732 | 14.883 | 1.00 | 29.32 | C |
| ATOM | 117 | O | SER A | 94 | 25.300 | 5.881 | 15.987 | 1.00 | 28.40 | O |
| ATOM | 118 | N | TRP A | 95 | 24.562 | 6.758 | 14.067 | 1.00 | 28.71 | N |
| ATOM | 119 | CA | TRP A | 95 | 24.940 | 8.122 | 14.442 | 1.00 | 28.77 | C |
| ATOM | 120 | CB | TRP A | 95 | 25.049 | 9.026 | 13.211 | 1.00 | 26.45 | C |
| ATOM | 121 | CG | TRP A | 95 | 23.824 | 9.006 | 12.322 | 1.00 | 26.08 | C |
| ATOM | 122 | CD1 | TRP A | 95 | 22.695 | 9.790 | 12.427 | 1.00 | 27.44 | C |
| ATOM | 123 | NE1 | TRP A | 95 | 21.803 | 9.473 | 11.421 | 1.00 | 22.50 | N |
| ATOM | 124 | CE2 | TRP A | 95 | 22.361 | 8.493 | 10.635 | 1.00 | 21.67 | C |
| ATOM | 125 | CD2 | TRP A | 95 | 23.629 | 8.177 | 11.175 | 1.00 | 24.64 | C |
| ATOM | 126 | CE3 | TRP A | 95 | 24.403 | 7.192 | 10.552 | 1.00 | 26.10 | C |
| ATOM | 127 | CZ3 | TRP A | 95 | 23.902 | 6.561 | 9.439 | 1.00 | 21.30 | C |
| ATOM | 128 | CH2 | TRP A | 95 | 22.641 | 6.893 | 8.922 | 1.00 | 23.00 | C |
| ATOM | 129 | CZ2 | TRP A | 95 | 21.858 | 7.853 | 9.503 | 1.00 | 24.47 | C |
| ATOM | 130 | C | TRP A | 95 | 24.002 | 8.789 | 15.449 | 1.00 | 28.03 | C |
| ATOM | 131 | O | TRP A | 95 | 24.307 | 9.887 | 15.935 | 1.00 | 30.30 | O |
| ATOM | 132 | N | ASP A | 96 | 22.860 | 8.163 | 15.722 | 1.00 | 27.95 | N |
| ATOM | 133 | CA | ASP A | 96 | 21.888 | 8.700 | 16.686 | 1.00 | 26.03 | C |
| ATOM | 134 | CB | ASP A | 96 | 20.447 | 8.425 | 16.230 | 1.00 | 25.06 | C |
| ATOM | 135 | CG | ASP A | 96 | 19.944 | 9.421 | 15.199 | 1.00 | 27.49 | C |
| ATOM | 136 | OD1 | ASP A | 96 | 20.600 | 10.470 | 14.993 | 1.00 | 24.69 | O |
| ATOM | 137 | OD2 | ASP A | 96 | 18.869 | 9.151 | 14.592 | 1.00 | 27.26 | O |
| ATOM | 138 | C | ASP A | 96 | 22.045 | 8.046 | 18.050 | 1.00 | 27.97 | C |
| ATOM | 139 | O | ASP A | 96 | 22.388 | 6.875 | 18.145 | 1.00 | 30.41 | O |
| ATOM | 140 | N | LEU A | 97 | 21.732 | 8.791 | 19.100 | 1.00 | 27.13 | N |
| ATOM | 141 | CA | LEU A | 97 | 21.609 | 8.203 | 20.427 | 1.00 | 27.74 | C |
| ATOM | 142 | CB | LEU A | 97 | 22.124 | 9.184 | 21.489 | 1.00 | 31.56 | C |
| ATOM | 143 | CG | LEU A | 97 | 22.080 | 8.745 | 22.957 | 1.00 | 33.28 | C |
| ATOM | 144 | CD1 | LEU A | 97 | 22.960 | 7.520 | 23.165 | 1.00 | 32.14 | C |
| ATOM | 145 | CD2 | LEU A | 97 | 22.516 | 9.892 | 23.850 | 1.00 | 37.01 | C |
| ATOM | 146 | C | LEU A | 97 | 20.147 | 7.885 | 20.704 | 1.00 | 28.56 | C |
| ATOM | 147 | O | LEU A | 97 | 19.310 | 8.795 | 20.759 | 1.00 | 31.93 | O |
| ATOM | 148 | N | SER A | 98 | 19.826 | 6.605 | 20.858 | 1.00 | 24.97 | N |
| ATOM | 149 | CA | SER A | 98 | 18.495 | 6.221 | 21.281 | 1.00 | 30.11 | C |
| ATOM | 150 | CB | SER A | 98 | 18.219 | 4.752 | 20.967 | 1.00 | 32.77 | C |
| ATOM | 151 | OG | SER A | 98 | 18.265 | 4.513 | 19.573 | 1.00 | 35.45 | O |
| ATOM | 152 | C | SER A | 98 | 18.405 | 6.461 | 22.783 | 1.00 | 33.52 | C |
| ATOM | 153 | O | SER A | 98 | 19.425 | 6.460 | 23.475 | 1.00 | 31.86 | O |
| ATOM | 154 | N | PHE A | 99 | 17.195 | 6.681 | 23.282 | 1.00 | 35.14 | N |
| ATOM | 155 | CA | PHE A | 99 | 16.993 | 6.882 | 24.718 | 1.00 | 34.45 | C |
| ATOM | 156 | CB | PHE A | 99 | 17.439 | 8.281 | 25.156 | 1.00 | 33.38 | C |
| ATOM | 157 | CG | PHE A | 99 | 16.834 | 9.406 | 24.348 | 1.00 | 35.19 | C |
| ATOM | 158 | CD1 | PHE A | 99 | 15.518 | 9.794 | 24.542 | 1.00 | 35.96 | C |
| ATOM | 159 | CE1 | PHE A | 99 | 14.964 | 10.838 | 23.809 | 1.00 | 35.30 | C |
| ATOM | 160 | CZ | PHE A | 99 | 15.733 | 11.514 | 22.866 | 1.00 | 31.00 | C |
| ATOM | 161 | CE2 | PHE A | 99 | 17.053 | 11.134 | 22.665 | 1.00 | 34.52 | C |
| ATOM | 162 | CD2 | PHE A | 99 | 17.597 | 10.090 | 23.410 | 1.00 | 34.60 | C |
| ATOM | 163 | C | PHE A | 99 | 15.546 | 6.616 | 25.129 | 1.00 | 39.70 | C |
| ATOM | 164 | O | PHE A | 99 | 14.668 | 6.447 | 24.276 | 1.00 | 35.27 | O |
| ATOM | 165 | N | GLN A | 100 | 15.307 | 6.556 | 26.435 | 1.00 | 37.50 | N |
| ATOM | 166 | CA | GLN A | 100 | 13.947 | 6.432 | 26.945 | 1.00 | 40.45 | C |
| ATOM | 167 | CB | GLN A | 100 | 13.791 | 5.171 | 27.797 | 1.00 | 45.43 | C |
| ATOM | 168 | CG | GLN A | 100 | 14.286 | 3.904 | 27.117 | 1.00 | 43.18 | C |
| ATOM | 169 | CD | GLN A | 100 | 15.041 | 3.010 | 28.076 | 1.00 | 53.89 | C |
| ATOM | 170 | OE1 | GLN A | 100 | 15.060 | 3.259 | 29.280 | 1.00 | 56.63 | O |
| ATOM | 171 | NE2 | GLN A | 100 | 15.675 | 1.967 | 27.549 | 1.00 | 57.30 | N |
| ATOM | 172 | C | GLN A | 100 | 13.560 | 7.673 | 27.751 | 1.00 | 41.22 | C |
| ATOM | 173 | O | GLN A | 100 | 14.429 | 8.427 | 28.197 | 1.00 | 39.02 | O |
| ATOM | 174 | N | LYS A | 101 | 12.255 | 7.892 | 27.908 | 1.00 | 39.58 | N |
| ATOM | 175 | CA | LYS A | 101 | 11.753 | 8.974 | 28.748 | 1.00 | 43.54 | C |
| ATOM | 176 | CB | LYS A | 101 | 10.217 | 8.960 | 28.809 | 1.00 | 43.47 | C |
| ATOM | 177 | CG | LYS A | 101 | 9.641 | 9.868 | 29.893 | 1.00 | 42.47 | C |
| ATOM | 178 | CD | LYS A | 101 | 8.198 | 10.267 | 29.613 | 1.00 | 42.37 | C |
| ATOM | 179 | CE | LYS A | 101 | 7.201 | 9.285 | 30.197 | 1.00 | 48.09 | C |
| ATOM | 180 | NZ | LYS A | 101 | 5.796 | 9.712 | 29.931 | 1.00 | 49.21 | N |
| ATOM | 181 | C | LYS A | 101 | 12.340 | 8.850 | 30.153 | 1.00 | 42.40 | C |
| ATOM | 182 | O | LYS A | 101 | 12.241 | 7.800 | 30.793 | 1.00 | 44.15 | O |
| ATOM | 183 | N | GLY A | 102 | 12.979 | 9.915 | 30.616 | 1.00 | 42.94 | N |
| ATOM | 184 | CA | GLY A | 102 | 13.600 | 9.895 | 31.923 | 1.00 | 47.07 | C |
| ATOM | 185 | C | GLY A | 102 | 15.105 | 9.994 | 31.818 | 1.00 | 49.23 | C |
| ATOM | 186 | O | GLY A | 102 | 15.736 | 10.722 | 32.598 | 1.00 | 46.09 | O |
| ATOM | 187 | N | ASP A | 103 | 15.671 | 9.269 | 30.849 | 1.00 | 48.88 | N |
| ATOM | 188 | CA | ASP A | 103 | 17.117 | 9.242 | 30.629 | 1.00 | 44.34 | C |
| ATOM | 189 | CB | ASP A | 103 | 17.453 | 8.493 | 29.338 | 1.00 | 42.41 | C |
| ATOM | 190 | CG | ASP A | 103 | 17.278 | 6.996 | 29.468 | 1.00 | 47.34 | C |
| ATOM | 191 | OD2 | ASP A | 103 | 17.362 | 6.293 | 28.430 | 1.00 | 49.11 | O |
| ATOM | 192 | OD1 | ASP A | 103 | 17.070 | 6.518 | 30.603 | 1.00 | 47.29 | O |
| ATOM | 193 | C | ASP A | 103 | 17.704 | 10.638 | 30.559 | 1.00 | 44.15 | C |
| ATOM | 194 | O | ASP A | 103 | 17.192 | 11.501 | 29.840 | 1.00 | 45.89 | O |
| ATOM | 195 | N | GLN A | 104 | 18.779 | 10.858 | 31.306 | 1.00 | 39.77 | N |
| ATOM | 196 | CA | GLN A | 104 | 19.446 | 12.151 | 31.325 | 1.00 | 41.41 | C |
| ATOM | 197 | CB | GLN A | 104 | 19.880 | 12.510 | 32.750 | 1.00 | 46.00 | C |
| ATOM | 198 | CG | GLN A | 104 | 18.778 | 12.293 | 33.792 | 1.00 | 50.23 | C |
| ATOM | 199 | CD | GLN A | 104 | 18.819 | 13.307 | 34.922 | 1.00 | 55.72 | C |
| ATOM | 200 | OE1 | GLN A | 104 | 19.542 | 14.306 | 34.857 | 1.00 | 56.40 | O |
| ATOM | 201 | NE2 | GLN A | 104 | 18.035 | 13.055 | 35.964 | 1.00 | 51.37 | N |
| ATOM | 202 | C | GLN A | 104 | 20.653 | 12.136 | 30.398 | 1.00 | 36.84 | C |
| ATOM | 203 | O | GLN A | 104 | 21.299 | 11.106 | 30.234 | 1.00 | 37.93 | O |
| ATOM | 204 | N | MET A | 105 | 20.960 | 13.285 | 29.806 | 1.00 | 36.89 | N |
| ATOM | 205 | CA | MET A | 105 | 22.024 | 13.371 | 28.822 | 1.00 | 38.62 | C |
| ATOM | 206 | CB | MET A | 105 | 21.457 | 13.182 | 27.396 | 1.00 | 35.84 | C |
| ATOM | 207 | CG | MET A | 105 | 20.848 | 11.810 | 27.124 | 1.00 | 33.31 | C |
| ATOM | 208 | SD | MET A | 105 | 19.792 | 11.739 | 25.638 | 1.00 | 35.66 | S |
| ATOM | 209 | CE | MET A | 105 | 18.306 | 12.510 | 26.293 | 1.00 | 35.80 | C |
| ATOM | 210 | C | MET A | 105 | 22.730 | 14.711 | 28.940 | 1.00 | 39.26 | C |
| ATOM | 211 | O | MET A | 105 | 22.155 | 15.683 | 29.431 | 1.00 | 44.66 | O |
| ATOM | 212 | N | VAL A | 106 | 23.981 | 14.761 | 28.497 | 1.00 | 38.43 | N |
| ATOM | 213 | CA | VAL A | 106 | 24.731 | 16.009 | 28.455 | 1.00 | 40.68 | C |
| ATOM | 214 | CB | VAL A | 106 | 26.136 | 15.859 | 29.090 | 1.00 | 42.42 | C |
| ATOM | 215 | CG1 | VAL A | 106 | 27.009 | 17.041 | 28.756 | 1.00 | 43.08 | C |
| ATOM | 216 | CG2 | VAL A | 106 | 26.012 | 15.743 | 30.585 | 1.00 | 49.13 | C |
| ATOM | 217 | C | VAL A | 106 | 24.869 | 16.442 | 27.010 | 1.00 | 43.99 | C |
| ATOM | 218 | O | VAL A | 106 | 25.230 | 15.635 | 26.152 | 1.00 | 43.40 | O |
| ATOM | 219 | N | VAL A | 107 | 24.574 | 17.713 | 26.746 | 1.00 | 43.17 | N |
| ATOM | 220 | CA | VAL A | 107 | 24.646 | 18.260 | 25.398 | 1.00 | 43.29 | C |
| ATOM | 221 | CB | VAL A | 107 | 23.692 | 19.450 | 25.237 | 1.00 | 41.21 | C |
| ATOM | 222 | CG1 | VAL A | 107 | 23.905 | 20.151 | 23.900 | 1.00 | 43.03 | C |
| ATOM | 223 | CG2 | VAL A | 107 | 22.269 | 18.973 | 25.365 | 1.00 | 40.65 | C |
| ATOM | 224 | C | VAL A | 107 | 26.062 | 18.700 | 25.096 | 1.00 | 44.42 | C |
| ATOM | 225 | O | VAL A | 107 | 26.640 | 19.491 | 25.841 | 1.00 | 46.20 | O |
| ATOM | 226 | N | LEU A | 108 | 26.615 | 18.181 | 24.004 | 1.00 | 42.50 | N |
| ATOM | 227 | CA | LEU A | 108 | 27.984 | 18.480 | 23.612 | 1.00 | 41.70 | C |
| ATOM | 228 | CB | LEU A | 108 | 28.693 | 17.209 | 23.118 | 1.00 | 42.33 | C |
| ATOM | 229 | CG | LEU A | 108 | 28.504 | 15.902 | 23.904 | 1.00 | 42.31 | C |
| ATOM | 230 | CD1 | LEU A | 108 | 29.344 | 14.774 | 23.315 | 1.00 | 41.29 | C |
| ATOM | 231 | CD2 | LEU A | 108 | 28.843 | 16.086 | 25.364 | 1.00 | 43.61 | C |
| ATOM | 232 | C | LEU A | 108 | 27.993 | 19.526 | 22.507 | 1.00 | 43.77 | C |
| ATOM | 233 | O | LEU A | 108 | 29.010 | 20.171 | 22.260 | 1.00 | 43.67 | O |
| ATOM | 234 | N | GLU A | 109 | 26.852 | 19.700 | 21.843 | 1.00 | 43.99 | N |
| ATOM | 235 | CA | GLU A | 109 | 26.809 | 20.503 | 20.628 | 1.00 | 41.58 | C |
| ATOM | 236 | CB | GLU A | 109 | 27.454 | 19.692 | 19.506 | 1.00 | 44.01 | C |
| ATOM | 237 | CG | GLU A | 109 | 27.483 | 20.320 | 18.128 | 1.00 | 45.46 | C |
| ATOM | 238 | CD | GLU A | 109 | 28.033 | 19.336 | 17.101 | 1.00 | 53.72 | C |
| ATOM | 239 | OE1 | GLU A | 109 | 27.359 | 19.069 | 16.073 | 1.00 | 48.12 | O |
| ATOM | 240 | OE2 | GLU A | 109 | 29.140 | 18.803 | 17.344 | 1.00 | 58.49 | O |
| ATOM | 241 | C | GLU A | 109 | 25.366 | 20.895 | 20.272 | 1.00 | 45.34 | C |
| ATOM | 242 | O | GLU A | 109 | 24.544 | 20.026 | 19.977 | 1.00 | 43.78 | O |
| ATOM | 243 | N | GLU A | 110 | 25.066 | 22.195 | 20.341 | 1.00 | 47.92 | N |
| ATOM | 244 | CA | GLU A | 110 | 23.762 | 22.746 | 19.947 | 1.00 | 46.36 | C |
| ATOM | 245 | CB | GLU A | 110 | 23.404 | 23.964 | 20.798 | 1.00 | 47.90 | C |
| ATOM | 246 | CG | GLU A | 110 | 22.318 | 23.729 | 21.843 | 1.00 | 51.19 | C |
| ATOM | 247 | CD | GLU A | 110 | 21.633 | 25.022 | 22.251 | 1.00 | 54.97 | C |
| ATOM | 248 | OE1 | GLU A | 110 | 20.453 | 25.226 | 21.879 | 1.00 | 55.62 | O |
| ATOM | 249 | OE2 | GLU A | 110 | 22.281 | 25.843 | 22.932 | 1.00 | 62.84 | O |
| ATOM | 250 | C | GLU A | 110 | 23.798 | 23.149 | 18.474 | 1.00 | 46.21 | C |
| ATOM | 251 | O | GLU A | 110 | 23.740 | 24.330 | 18.129 | 1.00 | 46.88 | O |
| ATOM | 252 | N | SER A | 111 | 23.867 | 22.139 | 17.615 | 1.00 | 46.35 | N |
| ATOM | 253 | CA | SER A | 111 | 24.164 | 22.293 | 16.203 | 1.00 | 46.72 | C |
| ATOM | 254 | CB | SER A | 111 | 24.855 | 21.017 | 15.743 | 1.00 | 50.32 | C |
| ATOM | 255 | OG | SER A | 111 | 24.498 | 19.935 | 16.601 | 1.00 | 48.65 | O |
| ATOM | 256 | C | SER A | 111 | 22.920 | 22.520 | 15.342 | 1.00 | 51.96 | C |
| ATOM | 257 | O | SER A | 111 | 22.838 | 22.023 | 14.212 | 1.00 | 48.81 | O |
| ATOM | 258 | N | GLY A | 112 | 21.966 | 23.283 | 15.860 | 1.00 | 48.52 | N |
| ATOM | 259 | CA | GLY A | 112 | 20.681 | 23.414 | 15.206 | 1.00 | 43.62 | C |
| ATOM | 260 | C | GLY A | 112 | 19.676 | 22.716 | 16.088 | 1.00 | 38.67 | C |
| ATOM | 261 | O | GLY A | 112 | 19.769 | 22.811 | 17.309 | 1.00 | 43.29 | O |
| ATOM | 262 | N | GLU A | 113 | 18.733 | 21.992 | 15.495 | 1.00 | 36.27 | N |
| ATOM | 263 | CA | GLU A | 113 | 17.703 | 21.351 | 16.299 | 1.00 | 32.74 | C |
| ATOM | 264 | CB | GLU A | 113 | 16.334 | 21.575 | 15.678 | 1.00 | 36.65 | C |
| ATOM | 265 | CG | GLU A | 113 | 16.036 | 23.030 | 15.377 | 1.00 | 41.91 | C |
| ATOM | 266 | CD | GLU A | 113 | 14.567 | 23.337 | 15.509 | 1.00 | 47.48 | C |
| ATOM | 267 | OE1 | GLU A | 113 | 13.989 | 23.909 | 14.560 | 1.00 | 58.53 | O |
| ATOM | 268 | OE2 | GLU A | 113 | 13.987 | 22.990 | 16.562 | 1.00 | 47.89 | O |
| ATOM | 269 | C | GLU A | 113 | 17.974 | 19.863 | 16.496 | 1.00 | 31.20 | C |
| ATOM | 270 | O | GLU A | 113 | 17.251 | 19.171 | 17.224 | 1.00 | 30.91 | O |
| ATOM | 271 | N | TRP A | 114 | 19.022 | 19.376 | 15.845 | 1.00 | 29.06 | N |
| ATOM | 272 | CA | TRP A | 114 | 19.542 | 18.052 | 16.137 | 1.00 | 30.72 | C |
| ATOM | 273 | CB | TRP A | 114 | 19.691 | 17.218 | 14.865 | 1.00 | 24.69 | C |
| ATOM | 274 | CG | TRP A | 114 | 18.378 | 16.686 | 14.323 | 1.00 | 21.09 | C |
| ATOM | 275 | CD1 | TRP A | 114 | 17.478 | 17.360 | 13.545 | 1.00 | 24.58 | C |
| ATOM | 276 | NE1 | TRP A | 114 | 16.421 | 16.534 | 13.217 | 1.00 | 22.12 | N |
| ATOM | 277 | CE2 | TRP A | 114 | 16.633 | 15.301 | 13.782 | 1.00 | 22.01 | C |
| ATOM | 278 | CD2 | TRP A | 114 | 17.862 | 15.356 | 14.471 | 1.00 | 22.42 | C |
| ATOM | 279 | CE3 | TRP A | 114 | 18.324 | 14.202 | 15.116 | 1.00 | 23.80 | C |
| ATOM | 280 | CZ3 | TRP A | 114 | 17.554 | 13.056 | 15.069 | 1.00 | 23.17 | C |
| ATOM | 281 | CH2 | TRP A | 114 | 16.332 | 13.029 | 14.373 | 1.00 | 20.38 | C |
| ATOM | 282 | CZ2 | TRP A | 114 | 15.861 | 14.139 | 13.724 | 1.00 | 21.02 | C |
| ATOM | 283 | C | TRP A | 114 | 20.887 | 18.223 | 16.834 | 1.00 | 32.36 | C |
| ATOM | 284 | O | TRP A | 114 | 21.849 | 18.730 | 16.252 | 1.00 | 35.16 | O |
| ATOM | 285 | N | TRP A | 115 | 20.931 | 17.798 | 18.088 | 1.00 | 32.59 | N |
| ATOM | 286 | CA | TRP A | 115 | 22.061 | 18.066 | 18.968 | 1.00 | 37.50 | C |
| ATOM | 287 | CB | TRP A | 115 | 21.530 | 18.527 | 20.337 | 1.00 | 33.97 | C |
| ATOM | 288 | CG | TRP A | 115 | 20.831 | 19.860 | 20.301 | 1.00 | 33.93 | C |
| ATOM | 289 | CD1 | TRP A | 115 | 20.845 | 20.764 | 19.280 | 1.00 | 35.62 | C |
| ATOM | 290 | NE1 | TRP A | 115 | 20.096 | 21.872 | 19.614 | 1.00 | 36.49 | N |
| ATOM | 291 | CE2 | TRP A | 115 | 19.589 | 21.700 | 20.878 | 1.00 | 39.65 | C |
| ATOM | 292 | CD2 | TRP A | 115 | 20.035 | 20.446 | 21.345 | 1.00 | 36.24 | C |
| ATOM | 293 | CE3 | TRP A | 115 | 19.651 | 20.027 | 22.622 | 1.00 | 36.32 | C |
| ATOM | 294 | CZ3 | TRP A | 115 | 18.846 | 20.860 | 23.382 | 1.00 | 38.20 | C |
| ATOM | 295 | CH2 | TRP A | 115 | 18.425 | 22.104 | 22.893 | 1.00 | 36.37 | C |
| ATOM | 296 | CZ2 | TRP A | 115 | 18.784 | 22.541 | 21.647 | 1.00 | 36.49 | C |
| ATOM | 297 | C | TRP A | 115 | 22.914 | 16.812 | 19.162 | 1.00 | 35.63 | C |
| ATOM | 298 | O | TRP A | 115 | 22.393 | 15.695 | 19.134 | 1.00 | 34.46 | O |
| ATOM | 299 | N | LYS A | 116 | 24.217 | 16.990 | 19.364 | 1.00 | 36.54 | N |
| ATOM | 300 | CA | LYS A | 116 | 25.054 | 15.864 | 19.767 | 1.00 | 39.76 | C |
| ATOM | 301 | CB | LYS A | 116 | 26.508 | 16.043 | 19.304 | 1.00 | 38.99 | C |
| ATOM | 302 | CG | LYS A | 116 | 27.316 | 14.742 | 19.289 | 1.00 | 41.42 | C |
| ATOM | 303 | CD | LYS A | 116 | 28.811 | 14.973 | 18.979 | 1.00 | 47.95 | C |
| ATOM | 304 | CE | LYS A | 116 | 29.046 | 15.474 | 17.549 | 1.00 | 52.94 | C |
| ATOM | 305 | NZ | LYS A | 116 | 30.432 | 15.203 | 17.056 | 1.00 | 49.06 | N |
| ATOM | 306 | C | LYS A | 116 | 24.972 | 15.776 | 21.282 | 1.00 | 37.88 | C |
| ATOM | 307 | O | LYS A | 116 | 25.179 | 16.774 | 21.977 | 1.00 | 39.88 | O |
| ATOM | 308 | N | ALA A | 117 | 24.635 | 14.599 | 21.795 | 1.00 | 35.72 | N |
| ATOM | 309 | CA | ALA A | 117 | 24.512 | 14.410 | 23.235 | 1.00 | 38.26 | C |
| ATOM | 310 | CB | ALA A | 117 | 23.045 | 14.363 | 23.652 | 1.00 | 35.94 | C |
| ATOM | 311 | C | ALA A | 117 | 25.234 | 13.141 | 23.687 | 1.00 | 42.44 | C |
| ATOM | 312 | O | ALA A | 117 | 25.596 | 12.289 | 22.867 | 1.00 | 39.65 | O |
| ATOM | 313 | N | ARG A | 118 | 25.435 | 13.026 | 24.997 | 1.00 | 42.34 | N |
| ATOM | 314 | CA | ARG A | 118 | 26.029 | 11.836 | 25.601 | 1.00 | 40.14 | C |
| ATOM | 315 | CB | ARG A | 118 | 27.456 | 12.131 | 26.074 | 1.00 | 42.93 | C |
| ATOM | 316 | CG | ARG A | 118 | 28.096 | 11.036 | 26.948 | 1.00 | 45.59 | C |
| ATOM | 317 | CD | ARG A | 118 | 29.372 | 11.548 | 27.614 | 1.00 | 45.06 | C |
| ATOM | 318 | NE | ARG A | 118 | 30.257 | 12.184 | 26.639 | 1.00 | 49.81 | N |
| ATOM | 319 | CZ | ARG A | 118 | 31.234 | 13.027 | 26.953 | 1.00 | 51.99 | C |
| ATOM | 320 | NH1 | ARG A | 118 | 31.455 | 13.347 | 28.225 | 1.00 | 54.63 | N |
| ATOM | 321 | NH2 | ARG A | 118 | 31.986 | 13.559 | 25.997 | 1.00 | 49.89 | N |
| ATOM | 322 | C | ARG A | 118 | 25.167 | 11.401 | 26.777 | 1.00 | 40.74 | C |
| ATOM | 323 | O | ARG A | 118 | 24.783 | 12.232 | 27.608 | 1.00 | 41.01 | O |
| ATOM | 324 | N | SER A | 119 | 24.847 | 10.110 | 26.832 | 1.00 | 34.97 | N |
| ATOM | 325 | CA | SER A | 119 | 24.043 | 9.558 | 27.912 | 1.00 | 38.46 | C |
| ATOM | 326 | CB | SER A | 119 | 23.567 | 8.149 | 27.560 | 1.00 | 41.83 | C |
| ATOM | 327 | OG | SER A | 119 | 23.293 | 7.399 | 28.739 | 1.00 | 46.46 | O |
| ATOM | 328 | C | SER A | 119 | 24.843 | 9.500 | 29.208 | 1.00 | 45.87 | C |
| ATOM | 329 | O | SER A | 119 | 25.987 | 9.032 | 29.222 | 1.00 | 43.64 | O |
| ATOM | 330 | N | LEU A | 120 | 24.236 | 9.968 | 30.295 | 1.00 | 46.06 | N |
| ATOM | 331 | CA | LEU A | 120 | 24.877 | 9.928 | 31.607 | 1.00 | 44.67 | C |
| ATOM | 332 | CB | LEU A | 120 | 24.025 | 10.683 | 32.630 | 1.00 | 45.83 | C |
| ATOM | 333 | CG | LEU A | 120 | 24.540 | 12.068 | 33.022 | 1.00 | 47.68 | C |
| ATOM | 334 | CD1 | LEU A | 120 | 25.352 | 12.661 | 31.903 | 1.00 | 43.91 | C |
| ATOM | 335 | CD2 | LEU A | 120 | 23.391 | 12.991 | 33.391 | 1.00 | 47.95 | C |
| ATOM | 336 | C | LEU A | 120 | 25.086 | 8.483 | 32.032 | 1.00 | 46.10 | C |
| ATOM | 337 | O | LEU A | 120 | 26.155 | 8.116 | 32.513 | 1.00 | 50.16 | O |
| ATOM | 338 | N | ALA A | 121 | 24.061 | 7.665 | 31.810 | 1.00 | 47.04 | N |
| ATOM | 339 | CA | ALA A | 121 | 24.085 | 6.251 | 32.162 | 1.00 | 48.05 | C |
| ATOM | 340 | CB | ALA A | 121 | 22.720 | 5.624 | 31.912 | 1.00 | 45.85 | C |
| ATOM | 341 | C | ALA A | 121 | 25.182 | 5.452 | 31.446 | 1.00 | 51.26 | C |
| ATOM | 342 | O | ALA A | 121 | 26.051 | 4.865 | 32.098 | 1.00 | 52.15 | O |
| ATOM | 343 | N | THR A | 122 | 25.138 | 5.432 | 30.112 | 1.00 | 48.58 | N |
| ATOM | 344 | CA | THR A | 122 | 26.001 | 4.555 | 29.315 | 1.00 | 47.21 | C |
| ATOM | 345 | CB | THR A | 122 | 25.203 | 3.894 | 28.160 | 1.00 | 48.41 | C |
| ATOM | 346 | OG1 | THR A | 122 | 24.642 | 4.917 | 27.326 | 1.00 | 49.90 | O |
| ATOM | 347 | CG2 | THR A | 122 | 24.086 | 3.021 | 28.701 | 1.00 | 46.19 | C |
| ATOM | 348 | C | THR A | 122 | 27.255 | 5.211 | 28.716 | 1.00 | 45.14 | C |
| ATOM | 349 | O | THR A | 122 | 28.173 | 4.507 | 28.297 | 1.00 | 46.08 | O |
| ATOM | 350 | N | ARG A | 123 | 27.275 | 6.541 | 28.658 | 1.00 | 43.66 | N |
| ATOM | 351 | CA | ARG A | 123 | 28.337 | 7.310 | 27.983 | 1.00 | 44.50 | C |
| ATOM | 352 | CB | ARG A | 123 | 29.733 | 6.991 | 28.543 | 1.00 | 48.81 | C |
| ATOM | 353 | CG | ARG A | 123 | 30.659 | 8.195 | 28.634 | 1.00 | 52.25 | C |
| ATOM | 354 | CD | ARG A | 123 | 31.675 | 8.032 | 29.768 | 1.00 | 56.50 | C |
| ATOM | 355 | NE | ARG A | 123 | 32.090 | 9.318 | 30.330 | 1.00 | 59.51 | N |
| ATOM | 356 | CZ | ARG A | 123 | 32.988 | 10.126 | 29.771 | 1.00 | 60.92 | C |
| ATOM | 357 | NH1 | ARG A | 123 | 33.567 | 9.787 | 28.626 | 1.00 | 59.26 | N |
| ATOM | 358 | NH2 | ARG A | 123 | 33.306 | 11.278 | 30.353 | 1.00 | 63.11 | N |
| ATOM | 359 | C | ARG A | 123 | 28.325 | 7.196 | 26.448 | 1.00 | 44.35 | C |
| ATOM | 360 | O | ARG A | 123 | 29.196 | 7.766 | 25.775 | 1.00 | 39.44 | O |
| ATOM | 361 | N | LYS A | 124 | 27.351 | 6.467 | 25.896 | 1.00 | 44.21 | N |
| ATOM | 362 | CA | LYS A | 124 | 27.185 | 6.407 | 24.437 | 1.00 | 41.21 | C |
| ATOM | 363 | CB | LYS A | 124 | 26.054 | 5.463 | 24.067 | 1.00 | 42.29 | C |
| ATOM | 364 | CG | LYS A | 124 | 26.195 | 4.045 | 24.580 | 1.00 | 45.88 | C |
| ATOM | 365 | CD | LYS A | 124 | 24.861 | 3.323 | 24.427 | 1.00 | 48.13 | C |
| ATOM | 366 | CE | LYS A | 124 | 25.031 | 1.813 | 24.373 | 1.00 | 52.03 | C |
| ATOM | 367 | NZ | LYS A | 124 | 23.702 | 1.142 | 24.397 | 1.00 | 53.26 | N |
| ATOM | 368 | C | LYS A | 124 | 26.828 | 7.789 | 23.912 | 1.00 | 40.82 | C |
| ATOM | 369 | O | LYS A | 124 | 26.057 | 8.514 | 24.546 | 1.00 | 41.68 | O |
| ATOM | 370 | N | GLU A | 125 | 27.359 | 8.150 | 22.749 | 1.00 | 40.48 | N |
| ATOM | 371 | CA | GLU A | 125 | 27.075 | 9.448 | 22.167 | 1.00 | 36.48 | C |
| ATOM | 372 | CB | GLU A | 125 | 28.374 | 10.213 | 21.917 | 1.00 | 40.92 | C |
| ATOM | 373 | CG | GLU A | 125 | 29.315 | 10.241 | 23.123 | 1.00 | 43.53 | C |
| ATOM | 374 | CD | GLU A | 125 | 30.422 | 11.268 | 22.974 | 1.00 | 47.48 | C |
| ATOM | 375 | OE1 | GLU A | 125 | 30.727 | 11.658 | 21.827 | 1.00 | 50.31 | O |
| ATOM | 376 | OE2 | GLU A | 125 | 30.979 | 11.701 | 24.009 | 1.00 | 51.52 | O |
| ATOM | 377 | C | GLU A | 125 | 26.290 | 9.309 | 20.865 | 1.00 | 39.88 | C |
| ATOM | 378 | O | GLU A | 125 | 26.269 | 8.245 | 20.249 | 1.00 | 36.81 | O |
| ATOM | 379 | N | GLY A | 126 | 25.641 | 10.387 | 20.448 | 1.00 | 35.90 | N |
| ATOM | 380 | CA | GLY A | 126 | 24.878 | 10.354 | 19.215 | 1.00 | 34.50 | C |
| ATOM | 381 | C | GLY A | 126 | 24.064 | 11.618 | 19.044 | 1.00 | 35.67 | C |
| ATOM | 382 | O | GLY A | 126 | 23.978 | 12.444 | 19.960 | 1.00 | 31.70 | O |
| ATOM | 383 | N | TYR A | 127 | 23.473 | 11.773 | 17.865 | 1.00 | 30.40 | N |
| ATOM | 384 | CA | TYR A | 127 | 22.577 | 12.891 | 17.622 | 1.00 | 30.86 | C |
| ATOM | 385 | CB | TYR A | 127 | 22.549 | 13.238 | 16.127 | 1.00 | 32.58 | C |
| ATOM | 386 | CG | TYR A | 127 | 23.805 | 13.985 | 15.736 | 1.00 | 33.85 | C |
| ATOM | 387 | CD1 | TYR A | 127 | 23.902 | 15.350 | 15.939 | 1.00 | 33.91 | C |
| ATOM | 388 | CE1 | TYR A | 127 | 25.048 | 16.039 | 15.620 | 1.00 | 37.27 | C |
| ATOM | 389 | CZ | TYR A | 127 | 26.126 | 15.368 | 15.093 | 1.00 | 40.22 | C |
| ATOM | 390 | OH | TYR A | 127 | 27.263 | 16.073 | 14.782 | 1.00 | 47.63 | O |
| ATOM | 391 | CE2 | TYR A | 127 | 26.070 | 14.008 | 14.887 | 1.00 | 36.65 | C |
| ATOM | 392 | CD2 | TYR A | 127 | 24.911 | 13.319 | 15.216 | 1.00 | 37.00 | C |
| ATOM | 393 | C | TYR A | 127 | 21.194 | 12.602 | 18.216 | 1.00 | 28.83 | C |
| ATOM | 394 | O | TYR A | 127 | 20.784 | 11.439 | 18.334 | 1.00 | 30.72 | O |
| ATOM | 395 | N | ILE A | 128 | 20.499 | 13.657 | 18.641 | 1.00 | 30.70 | N |
| ATOM | 396 | CA | ILE A | 128 | 19.193 | 13.512 | 19.282 | 1.00 | 25.61 | C |
| ATOM | 397 | CB | ILE A | 128 | 19.295 | 13.573 | 20.838 | 1.00 | 29.99 | C |
| ATOM | 398 | CG1 | ILE A | 128 | 19.733 | 14.963 | 21.319 | 1.00 | 26.02 | C |
| ATOM | 399 | CD1 | ILE A | 128 | 19.536 | 15.164 | 22.848 | 1.00 | 33.92 | C |
| ATOM | 400 | CG2 | ILE A | 128 | 20.237 | 12.494 | 21.373 | 1.00 | 29.20 | C |
| ATOM | 401 | C | ILE A | 128 | 18.272 | 14.624 | 18.824 | 1.00 | 24.21 | C |
| ATOM | 402 | O | ILE A | 128 | 18.725 | 15.742 | 18.564 | 1.00 | 28.39 | O |
| ATOM | 403 | N | PRO A | 129 | 16.970 | 14.332 | 18.718 | 1.00 | 23.96 | N |
| ATOM | 404 | CA | PRO A | 129 | 16.070 | 15.392 | 18.263 | 1.00 | 26.83 | C |
| ATOM | 405 | CB | PRO A | 129 | 14.803 | 14.631 | 17.859 | 1.00 | 25.29 | C |
| ATOM | 406 | CG | PRO A | 129 | 14.840 | 13.358 | 18.677 | 1.00 | 24.65 | C |
| ATOM | 407 | CD | PRO A | 129 | 16.286 | 13.042 | 18.927 | 1.00 | 24.63 | C |
| ATOM | 408 | C | PRO A | 129 | 15.811 | 16.304 | 19.449 | 1.00 | 27.02 | C |
| ATOM | 409 | O | PRO A | 129 | 15.343 | 15.803 | 20.465 | 1.00 | 27.94 | O |
| ATOM | 410 | N | SER A | 130 | 16.126 | 17.593 | 19.338 | 1.00 | 32.57 | N |
| ATOM | 411 | CA | SER A | 130 | 16.074 | 18.479 | 20.509 | 1.00 | 30.97 | C |
| ATOM | 412 | CB | SER A | 130 | 16.750 | 19.829 | 20.235 | 1.00 | 31.52 | C |
| ATOM | 413 | OG | SER A | 130 | 16.093 | 20.561 | 19.223 | 1.00 | 31.77 | O |
| ATOM | 414 | C | SER A | 130 | 14.670 | 18.657 | 21.100 | 1.00 | 34.39 | C |
| ATOM | 415 | O | SER A | 130 | 14.527 | 18.872 | 22.310 | 1.00 | 32.44 | O |
| ATOM | 416 | N | ASN A | 131 | 13.629 | 18.532 | 20.281 | 1.00 | 29.81 | N |
| ATOM | 417 | CA | ASN A | 131 | 12.260 | 18.614 | 20.829 | 1.00 | 30.46 | C |
| ATOM | 418 | CB | ASN A | 131 | 11.224 | 18.924 | 19.736 | 1.00 | 29.39 | C |
| ATOM | 419 | CG | ASN A | 131 | 11.166 | 17.848 | 18.664 | 1.00 | 33.39 | C |
| ATOM | 420 | OD1 | ASN A | 131 | 12.143 | 17.132 | 18.441 | 1.00 | 30.63 | O |
| ATOM | 421 | ND2 | ASN A | 131 | 10.019 | 17.733 | 17.991 | 1.00 | 32.00 | N |
| ATOM | 422 | C | ASN A | 131 | 11.831 | 17.388 | 21.642 | 1.00 | 30.40 | C |
| ATOM | 423 | O | ASN A | 131 | 10.697 | 17.317 | 22.115 | 1.00 | 33.34 | O |
| ATOM | 424 | N | TYR A | 132 | 12.722 | 16.414 | 21.817 | 1.00 | 28.99 | N |
| ATOM | 425 | CA | TYR A | 132 | 12.379 | 15.237 | 22.615 | 1.00 | 29.39 | C |
| ATOM | 426 | CB | TYR A | 132 | 12.940 | 13.953 | 21.989 | 1.00 | 28.32 | C |
| ATOM | 427 | CG | TYR A | 132 | 12.034 | 13.297 | 20.966 | 1.00 | 29.44 | C |
| ATOM | 428 | CD1 | TYR A | 132 | 11.562 | 14.005 | 19.865 | 1.00 | 27.49 | C |
| ATOM | 429 | CE1 | TYR A | 132 | 10.735 | 13.391 | 18.920 | 1.00 | 26.25 | C |
| ATOM | 430 | CZ | TYR A | 132 | 10.383 | 12.068 | 19.091 | 1.00 | 29.30 | C |
| ATOM | 431 | OH | TYR A | 132 | 9.566 | 11.444 | 18.189 | 1.00 | 29.89 | O |
| ATOM | 432 | CE2 | TYR A | 132 | 10.853 | 11.349 | 20.180 | 1.00 | 29.66 | C |
| ATOM | 433 | CD2 | TYR A | 132 | 11.669 | 11.965 | 21.096 | 1.00 | 27.62 | C |
| ATOM | 434 | C | TYR A | 132 | 12.905 | 15.385 | 24.049 | 1.00 | 33.20 | C |
| ATOM | 435 | O | TYR A | 132 | 12.713 | 14.493 | 24.874 | 1.00 | 33.32 | O |
| ATOM | 436 | N | VAL A | 133 | 13.567 | 16.510 | 24.323 | 1.00 | 32.72 | N |
| ATOM | 437 | CA | VAL A | 133 | 14.238 | 16.735 | 25.605 | 1.00 | 38.98 | C |
| ATOM | 438 | CB | VAL A | 133 | 15.771 | 16.540 | 25.473 | 1.00 | 34.63 | C |
| ATOM | 439 | CG1 | VAL A | 133 | 16.127 | 15.125 | 25.069 | 1.00 | 33.98 | C |
| ATOM | 440 | CG2 | VAL A | 133 | 16.368 | 17.557 | 24.507 | 1.00 | 37.20 | C |
| ATOM | 441 | C | VAL A | 133 | 13.957 | 18.135 | 26.193 | 1.00 | 38.66 | C |
| ATOM | 442 | O | VAL A | 133 | 13.596 | 19.064 | 25.463 | 1.00 | 37.45 | O |
| ATOM | 443 | N | ALA A | 134 | 14.141 | 18.283 | 27.508 | 1.00 | 45.17 | N |
| ATOM | 444 | CA | ALA A | 134 | 13.973 | 19.580 | 28.187 | 1.00 | 45.91 | C |
| ATOM | 445 | CB | ALA A | 134 | 12.660 | 19.610 | 28.938 | 1.00 | 49.44 | C |
| ATOM | 446 | C | ALA A | 134 | 15.128 | 19.860 | 29.153 | 1.00 | 47.52 | C |
| ATOM | 447 | O | ALA A | 134 | 15.563 | 18.956 | 29.862 | 1.00 | 49.59 | O |
| ATOM | 448 | N | ARG A | 135 | 15.625 | 21.095 | 29.178 | 1.00 | 49.57 | N |
| ATOM | 449 | CA | ARG A | 135 | 16.721 | 21.480 | 30.080 | 1.00 | 53.26 | C |
| ATOM | 450 | CB | ARG A | 135 | 17.057 | 22.962 | 29.886 | 1.00 | 56.22 | C |
| ATOM | 451 | CG | ARG A | 135 | 18.133 | 23.496 | 30.813 | 1.00 | 59.93 | C |
| ATOM | 452 | CD | ARG A | 135 | 19.499 | 23.411 | 30.179 | 1.00 | 54.03 | C |
| ATOM | 453 | NE | ARG A | 135 | 19.912 | 24.702 | 29.641 | 1.00 | 56.70 | N |
| ATOM | 454 | CZ | ARG A | 135 | 20.967 | 25.384 | 30.074 | 1.00 | 58.75 | C |
| ATOM | 455 | NH1 | ARG A | 135 | 21.720 | 24.888 | 31.045 | 1.00 | 61.34 | N |
| ATOM | 456 | NH2 | ARG A | 135 | 21.274 | 26.558 | 29.535 | 1.00 | 60.46 | N |
| ATOM | 457 | C | ARG A | 135 | 16.387 | 21.213 | 31.552 | 1.00 | 52.86 | C |
| ATOM | 458 | O | ARG A | 135 | 15.217 | 21.266 | 31.939 | 1.00 | 54.79 | O |
| ATOM | 459 | N | VAL A | 136 | 17.402 | 20.909 | 32.366 | 1.00 | 55.28 | N |
| ATOM | 460 | CA | VAL A | 136 | 17.203 | 20.745 | 33.818 | 1.00 | 55.67 | C |
| ATOM | 461 | CB | VAL A | 136 | 17.598 | 19.341 | 34.316 | 1.00 | 52.91 | C |
| ATOM | 462 | CG1 | VAL A | 136 | 17.436 | 19.257 | 35.828 | 1.00 | 55.89 | C |
| ATOM | 463 | CG2 | VAL A | 136 | 16.758 | 18.282 | 33.645 | 1.00 | 50.46 | C |
| ATOM | 464 | C | VAL A | 136 | 17.963 | 21.787 | 34.643 | 1.00 | 54.12 | C |
| ATOM | 465 | O | VAL A | 136 | 19.000 | 22.304 | 34.217 | 1.00 | 53.25 | O |
| TER | 466 | | VAL A | 136 | | | | | | |
| ATOM | 466 | N | GLY B | 71 | 0.417 | 14.143 | 28.066 | 1.00 | 53.05 | N |
| ATOM | 467 | CA | GLY B | 71 | -0.004 | 12.754 | 28.065 | 1.00 | 48.48 | C |
| ATOM | 468 | C | GLY B | 71 | -0.485 | 12.280 | 26.708 | 1.00 | 44.43 | C |
| ATOM | 469 | O | GLY B | 71 | -0.813 | 11.101 | 26.533 | 1.00 | 45.91 | O |
| ATOM | 470 | N | PHE B | 72 | -0.544 | 13.197 | 25.744 | 1.00 | 45.96 | N |
| ATOM | 471 | CA | PHE B | 72 | -0.881 | 12.831 | 24.370 | 1.00 | 43.56 | C |
| ATOM | 472 | CB | PHE B | 72 | -0.855 | 14.075 | 23.464 | 1.00 | 42.80 | C |
| ATOM | 473 | CG | PHE B | 72 | -0.976 | 13.771 | 21.999 | 1.00 | 42.25 | C |
| ATOM | 474 | CD2 | PHE B | 72 | -0.049 | 14.277 | 21.101 | 1.00 | 44.29 | C |
| ATOM | 475 | CE2 | PHE B | 72 | -0.147 | 14.005 | 19.745 | 1.00 | 40.69 | C |
| ATOM | 476 | CZ | PHE B | 72 | -1.188 | 13.221 | 19.262 | 1.00 | 42.59 | C |
| ATOM | 477 | CE1 | PHE B | 72 | -2.123 | 12.708 | 20.144 | 1.00 | 41.61 | C |
| ATOM | 478 | CD1 | PHE B | 72 | -2.020 | 12.996 | 21.511 | 1.00 | 42.89 | C |
| ATOM | 479 | C | PHE B | 72 | 0.114 | 11.758 | 23.927 | 1.00 | 44.98 | C |
| ATOM | 480 | O | PHE B | 72 | 1.328 | 11.987 | 23.953 | 1.00 | 48.63 | O |
| ATOM | 481 | N | PRO B | 73 | -0.398 | 10.566 | 23.571 | 1.00 | 42.74 | N |
| ATOM | 482 | CA | PRO B | 73 | 0.446 | 9.374 | 23.404 | 1.00 | 45.22 | C |
| ATOM | 483 | CB | PRO B | 73 | -0.555 | 8.206 | 23.476 | 1.00 | 44.92 | C |
| ATOM | 484 | CG | PRO B | 73 | -1.865 | 8.799 | 23.905 | 1.00 | 43.47 | C |
| ATOM | 485 | CD | PRO B | 73 | -1.832 | 10.239 | 23.488 | 1.00 | 41.22 | C |
| ATOM | 486 | C | PRO B | 73 | 1.206 | 9.287 | 22.083 | 1.00 | 46.42 | C |
| ATOM | 487 | O | PRO B | 73 | 1.763 | 8.226 | 21.814 | 1.00 | 49.70 | O |
| ATOM | 488 | N | VAL B | 74 | 1.231 | 10.342 | 21.276 | 1.00 | 41.70 | N |
| ATOM | 489 | CA | VAL B | 74 | 2.012 | 10.292 | 20.040 | 1.00 | 43.77 | C |
| ATOM | 490 | C | VAL B | 74 | 3.213 | 11.219 | 20.163 | 1.00 | 39.62 | C |
| ATOM | 491 | O | VAL B | 74 | 3.066 | 12.399 | 20.489 | 1.00 | 45.08 | O |
| ATOM | 492 | CB | VAL B | 74 | 1.201 | 10.691 | 18.789 | 1.00 | 41.05 | C |
| ATOM | 493 | CG1 | VAL B | 74 | 2.010 | 10.383 | 17.528 | 1.00 | 42.94 | C |
| ATOM | 494 | CG2 | VAL B | 74 | -0.131 | 9.953 | 18.738 | 1.00 | 42.30 | C |
| ATOM | 495 | N | ARG B | 75 | 4.397 | 10.683 | 19.902 | 1.00 | 38.54 | N |
| ATOM | 496 | CA | ARG B | 75 | 5.623 | 11.465 | 19.997 | 1.00 | 37.63 | C |
| ATOM | 497 | CB | ARG B | 75 | 6.831 | 10.533 | 19.923 | 1.00 | 38.89 | C |
| ATOM | 498 | CG | ARG B | 75 | 6.981 | 9.659 | 21.150 | 1.00 | 43.45 | C |
| ATOM | 499 | CD | ARG B | 75 | 7.974 | 8.543 | 20.924 | 1.00 | 42.17 | C |
| ATOM | 500 | NE | ARG B | 75 | 7.339 | 7.350 | 20.378 | 1.00 | 51.60 | N |
| ATOM | 501 | CZ | ARG B | 75 | 7.721 | 6.758 | 19.252 | 1.00 | 53.56 | C |
| ATOM | 502 | NH1 | ARG B | 75 | 8.743 | 7.247 | 18.568 | 1.00 | 51.92 | N |
| ATOM | 503 | NH2 | ARG B | 75 | 7.089 | 5.674 | 18.816 | 1.00 | 55.29 | N |
| ATOM | 504 | C | ARG B | 75 | 5.668 | 12.472 | 18.863 | 1.00 | 35.86 | C |
| ATOM | 505 | O | ARG B | 75 | 5.159 | 12.189 | 17.778 | 1.00 | 33.09 | O |
| ATOM | 506 | N | PRO B | 76 | 6.279 | 13.649 | 19.105 | 1.00 | 33.79 | N |
| ATOM | 507 | CA | PRO B | 76 | 6.284 | 14.687 | 18.070 | 1.00 | 31.03 | C |
| ATOM | 508 | CB | PRO B | 76 | 6.875 | 15.908 | 18.788 | 1.00 | 32.53 | C |
| ATOM | 509 | CG | PRO B | 76 | 7.556 | 15.381 | 19.996 | 1.00 | 33.38 | C |
| ATOM | 510 | CD | PRO B | 76 | 6.851 | 14.124 | 20.380 | 1.00 | 34.66 | C |
| ATOM | 511 | C | PRO B | 76 | 7.121 | 14.324 | 16.837 | 1.00 | 30.41 | C |
| ATOM | 512 | O | PRO B | 76 | 7.976 | 13.424 | 16.884 | 1.00 | 25.08 | O |
| ATOM | 513 | N | GLN B | 77 | 6.862 | 15.024 | 15.735 | 1.00 | 25.27 | N |
| ATOM | 514 | CA | GLN B | 77 | 7.692 | 14.892 | 14.548 | 1.00 | 25.22 | C |
| ATOM | 515 | CB | GLN B | 77 | 7.065 | 15.706 | 13.406 | 1.00 | 25.98 | C |
| ATOM | 516 | CG | GLN B | 77 | 7.967 | 15.914 | 12.203 | 1.00 | 25.17 | C |
| ATOM | 517 | CD | GLN B | 77 | 8.408 | 14.612 | 11.564 | 1.00 | 24.99 | C |
| ATOM | 518 | OE1 | GLN B | 77 | 7.631 | 13.657 | 11.460 | 1.00 | 27.67 | O |
| ATOM | 519 | NE2 | GLN B | 77 | 9.669 | 14.567 | 11.127 | 1.00 | 24.16 | N |
| ATOM | 520 | C | GLN B | 77 | 9.095 | 15.403 | 14.900 | 1.00 | 26.80 | C |
| ATOM | 521 | O | GLN B | 77 | 9.229 | 16.472 | 15.519 | 1.00 | 25.53 | O |
| ATOM | 522 | N | VAL B | 78 | 10.135 | 14.630 | 14.561 | 1.00 | 24.88 | N |
| ATOM | 523 | CA | VAL B | 78 | 11.527 | 15.076 | 14.779 | 1.00 | 22.67 | C |
| ATOM | 524 | CB | VAL B | 78 | 12.559 | 13.957 | 14.444 | 1.00 | 25.58 | C |
| ATOM | 525 | CG1 | VAL B | 78 | 12.306 | 12.709 | 15.291 | 1.00 | 24.74 | C |
| ATOM | 526 | CG2 | VAL B | 78 | 12.548 | 13.628 | 12.941 | 1.00 | 22.57 | C |
| ATOM | 527 | C | VAL B | 78 | 11.790 | 16.323 | 13.935 | 1.00 | 23.97 | C |
| ATOM | 528 | O | VAL B | 78 | 11.107 | 16.531 | 12.925 | 1.00 | 26.17 | O |
| ATOM | 529 | N | PRO B | 79 | 12.757 | 17.169 | 14.344 | 1.00 | 23.96 | N |
| ATOM | 530 | CA | PRO B | 79 | 12.985 | 18.456 | 13.673 | 1.00 | 26.13 | C |
| ATOM | 531 | CB | PRO B | 79 | 14.164 | 19.049 | 14.443 | 1.00 | 23.41 | C |
| ATOM | 532 | CG | PRO B | 79 | 14.064 | 18.425 | 15.797 | 1.00 | 25.87 | C |
| ATOM | 533 | CD | PRO B | 79 | 13.592 | 17.031 | 15.554 | 1.00 | 24.59 | C |
| ATOM | 534 | C | PRO B | 79 | 13.321 | 18.362 | 12.178 | 1.00 | 27.29 | C |
| ATOM | 535 | O | PRO B | 79 | 14.076 | 17.480 | 11.733 | 1.00 | 23.18 | O |
| ATOM | 536 | N | LEU B | 80 | 12.766 | 19.299 | 11.416 | 1.00 | 23.53 | N |
| ATOM | 537 | CA | LEU B | 80 | 12.839 | 19.261 | 9.954 | 1.00 | 28.15 | C |
| ATOM | 538 | CB | LEU B | 80 | 11.603 | 19.939 | 9.352 | 1.00 | 27.81 | C |
| ATOM | 539 | CG | LEU B | 80 | 10.399 | 19.051 | 9.024 | 1.00 | 33.69 | C |
| ATOM | 540 | CD1 | LEU B | 80 | 10.420 | 17.769 | 9.826 | 1.00 | 28.73 | C |
| ATOM | 541 | CD2 | LEU B | 80 | 9.064 | 19.786 | 9.206 | 1.00 | 28.86 | C |
| ATOM | 542 | C | LEU B | 80 | 14.104 | 19.950 | 9.465 | 1.00 | 24.56 | C |
| ATOM | 543 | O | LEU B | 80 | 14.570 | 20.872 | 10.091 | 1.00 | 24.47 | O |
| ATOM | 544 | N | ARG B | 81 | 14.646 | 19.508 | 8.334 | 1.00 | 24.11 | N |
| ATOM | 545 | CA | ARG B | 81 | 15.923 | 20.020 | 7.842 | 1.00 | 25.18 | C |
| ATOM | 546 | CB | ARG B | 81 | 17.082 | 19.518 | 8.721 | 1.00 | 23.62 | C |
| ATOM | 547 | CG | ARG B | 81 | 17.058 | 18.022 | 8.970 | 1.00 | 24.48 | C |
| ATOM | 548 | CD | ARG B | 81 | 18.079 | 17.593 | 10.023 | 1.00 | 24.31 | C |
| ATOM | 549 | NE | ARG B | 81 | 17.917 | 16.187 | 10.388 | 1.00 | 20.72 | N |
| ATOM | 550 | CZ | ARG B | 81 | 18.836 | 15.460 | 11.019 | 1.00 | 23.02 | C |
| ATOM | 551 | NH1 | ARG B | 81 | 19.999 | 16.005 | 11.371 | 1.00 | 22.94 | N |
| ATOM | 552 | NH2 | ARG B | 81 | 18.596 | 14.185 | 11.294 | 1.00 | 18.66 | N |
| ATOM | 553 | C | ARG B | 81 | 16.114 | 19.565 | 6.398 | 1.00 | 23.93 | C |
| ATOM | 554 | O | ARG B | 81 | 15.564 | 18.550 | 6.003 | 1.00 | 25.62 | O |
| ATOM | 555 | N | PRO B | 82 | 16.881 | 20.328 | 5.606 | 1.00 | 25.50 | N |
| ATOM | 556 | CA | PRO B | 82 | 17.124 | 19.964 | 4.209 | 1.00 | 25.91 | C |
| ATOM | 557 | CB | PRO B | 82 | 17.557 | 21.292 | 3.585 | 1.00 | 25.60 | C |
| ATOM | 558 | CG | PRO B | 82 | 18.267 | 22.006 | 4.737 | 1.00 | 26.20 | C |
| ATOM | 559 | CD | PRO B | 82 | 17.520 | 21.612 | 5.973 | 1.00 | 29.35 | C |
| ATOM | 560 | C | PRO B | 82 | 18.248 | 18.926 | 4.088 | 1.00 | 23.90 | C |
| ATOM | 561 | O | PRO B | 82 | 19.175 | 18.919 | 4.902 | 1.00 | 24.55 | O |
| ATOM | 562 | N | MET B | 83 | 18.149 | 18.062 | 3.081 | 1.00 | 22.73 | N |
| ATOM | 563 | CA | MET B | 83 | 19.123 | 17.008 | 2.857 | 1.00 | 23.46 | C |
| ATOM | 564 | CB | MET B | 83 | 18.693 | 16.160 | 1.656 | 1.00 | 24.51 | C |
| ATOM | 565 | CG | MET B | 83 | 19.666 | 15.055 | 1.265 | 1.00 | 21.88 | C |
| ATOM | 566 | SD | MET B | 83 | 19.932 | 13.899 | 2.601 | 1.00 | 23.25 | S |
| ATOM | 567 | CE | MET B | 83 | 18.303 | 13.166 | 2.810 | 1.00 | 24.47 | C |
| ATOM | 568 | C | MET B | 83 | 20.500 | 17.624 | 2.619 | 1.00 | 26.55 | C |
| ATOM | 569 | O | MET B | 83 | 20.618 | 18.708 | 2.040 | 1.00 | 26.46 | O |
| ATOM | 570 | N | THR B | 84 | 21.542 | 16.946 | 3.092 | 1.00 | 23.31 | N |
| ATOM | 571 | CA | THR B | 84 | 22.903 | 17.413 | 2.888 | 1.00 | 25.75 | C |
| ATOM | 572 | CB | THR B | 84 | 23.568 | 17.733 | 4.226 | 1.00 | 21.23 | C |
| ATOM | 573 | OG1 | THR B | 84 | 23.587 | 16.544 | 5.017 | 1.00 | 21.29 | O |
| ATOM | 574 | CG2 | THR B | 84 | 22.797 | 18.837 | 4.987 | 1.00 | 24.02 | C |
| ATOM | 575 | C | THR B | 84 | 23.697 | 16.295 | 2.229 | 1.00 | 22.21 | C |
| ATOM | 576 | O | THR B | 84 | 23.222 | 15.169 | 2.157 | 1.00 | 20.23 | O |
| ATOM | 577 | N | TYR B | 85 | 24.903 | 16.594 | 1.761 | 1.00 | 21.22 | N |
| ATOM | 578 | CA | TYR B | 85 | 25.786 | 15.546 | 1.236 | 1.00 | 21.85 | C |
| ATOM | 579 | CB | TYR B | 85 | 27.162 | 16.124 | 0.850 | 1.00 | 22.97 | C |
| ATOM | 580 | CG | TYR B | 85 | 28.219 | 15.044 | 0.619 | 1.00 | 23.71 | C |
| ATOM | 581 | CD2 | TYR B | 85 | 28.463 | 14.560 | -0.654 | 1.00 | 21.53 | C |
| ATOM | 582 | CE2 | TYR B | 85 | 29.415 | 13.570 | -0.881 | 1.00 | 20.80 | C |
| ATOM | 583 | CZ | TYR B | 85 | 30.118 | 13.047 | 0.172 | 1.00 | 21.50 | C |
| ATOM | 584 | OH | TYR B | 85 | 31.056 | 12.071 | -0.054 | 1.00 | 22.71 | O |
| ATOM | 585 | CE1 | TYR B | 85 | 29.896 | 13.506 | 1.463 | 1.00 | 23.83 | C |
| ATOM | 586 | CD1 | TYR B | 85 | 28.960 | 14.507 | 1.679 | 1.00 | 21.37 | C |
| ATOM | 587 | C | TYR B | 85 | 25.995 | 14.464 | 2.287 | 1.00 | 22.75 | C |
| ATOM | 588 | O | TYR B | 85 | 25.914 | 13.270 | 1.993 | 1.00 | 21.34 | O |
| ATOM | 589 | N | LYS B | 86 | 26.300 | 14.879 | 3.515 | 1.00 | 21.40 | N |
| ATOM | 590 | CA | LYS B | 86 | 26.712 | 13.917 | 4.535 | 1.00 | 20.16 | C |
| ATOM | 591 | CB | LYS B | 86 | 27.403 | 14.619 | 5.707 | 1.00 | 23.82 | C |
| ATOM | 592 | CG | LYS B | 86 | 27.962 | 13.654 | 6.752 | 1.00 | 23.16 | C |
| ATOM | 593 | CD | LYS B | 86 | 28.980 | 12.712 | 6.110 | 1.00 | 21.18 | C |
| ATOM | 594 | CE | LYS B | 86 | 29.916 | 12.114 | 7.149 | 1.00 | 21.53 | C |
| ATOM | 595 | NZ | LYS B | 86 | 29.193 | 11.297 | 8.155 | 1.00 | 20.44 | N |
| ATOM | 596 | C | LYS B | 86 | 25.551 | 13.033 | 5.022 | 1.00 | 22.58 | C |
| ATOM | 597 | O | LYS B | 86 | 25.747 | 11.847 | 5.303 | 1.00 | 21.39 | O |
| ATOM | 598 | N | ALA B | 87 | 24.348 | 13.596 | 5.099 | 1.00 | 22.19 | N |
| ATOM | 599 | CA | ALA B | 87 | 23.191 | 12.808 | 5.499 | 1.00 | 20.26 | C |
| ATOM | 600 | CB | ALA B | 87 | 22.009 | 13.700 | 5.769 | 1.00 | 20.34 | C |
| ATOM | 601 | C | ALA B | 87 | 22.863 | 11.821 | 4.391 | 1.00 | 19.05 | C |
| ATOM | 602 | O | ALA B | 87 | 22.488 | 10.677 | 4.648 | 1.00 | 19.88 | O |
| ATOM | 603 | N | ALA B | 88 | 23.010 | 12.248 | 3.149 | 1.00 | 18.27 | N |
| ATOM | 604 | CA | ALA B | 88 | 22.701 | 11.343 | 2.051 | 1.00 | 19.65 | C |
| ATOM | 605 | CB | ALA B | 88 | 22.672 | 12.097 | 0.727 | 1.00 | 18.95 | C |
| ATOM | 606 | C | ALA B | 88 | 23.723 | 10.212 | 2.027 | 1.00 | 18.09 | C |
| ATOM | 607 | O | ALA B | 88 | 23.381 | 9.046 | 1.801 | 1.00 | 18.35 | O |
| ATOM | 608 | N | LEU B | 89 | 24.982 | 10.558 | 2.270 | 1.00 | 16.31 | N |
| ATOM | 609 | CA | LEU B | 89 | 26.050 | 9.566 | 2.331 | 1.00 | 17.82 | C |
| ATOM | 610 | CB | LEU B | 89 | 27.418 | 10.243 | 2.524 | 1.00 | 18.14 | C |
| ATOM | 611 | CG | LEU B | 89 | 28.615 | 9.292 | 2.607 | 1.00 | 20.26 | C |
| ATOM | 612 | CD1 | LEU B | 89 | 28.938 | 8.777 | 1.209 | 1.00 | 20.57 | C |
| ATOM | 613 | CD2 | LEU B | 89 | 29.835 | 9.979 | 3.232 | 1.00 | 18.91 | C |
| ATOM | 614 | C | LEU B | 89 | 25.802 | 8.579 | 3.451 | 1.00 | 19.21 | C |
| ATOM | 615 | O | LEU B | 89 | 25.842 | 7.359 | 3.240 | 1.00 | 18.10 | O |
| ATOM | 616 | N | ASP B | 90 | 25.542 | 9.110 | 4.645 | 1.00 | 19.47 | N |
| ATOM | 617 | CA | ASP B | 90 | 25.303 | 8.283 | 5.814 | 1.00 | 17.86 | C |
| ATOM | 618 | CB | ASP B | 90 | 25.033 | 9.152 | 7.050 | 1.00 | 21.84 | C |
| ATOM | 619 | CG | ASP B | 90 | 26.308 | 9.744 | 7.652 | 1.00 | 22.67 | C |
| ATOM | 620 | OD1 | ASP B | 90 | 27.417 | 9.269 | 7.318 | 1.00 | 20.38 | O |
| ATOM | 621 | OD2 | ASP B | 90 | 26.196 | 10.684 | 8.474 | 1.00 | 19.58 | O |
| ATOM | 622 | C | ASP B | 90 | 24.130 | 7.344 | 5.594 | 1.00 | 18.44 | C |
| ATOM | 623 | O | ASP B | 90 | 24.230 | 6.152 | 5.852 | 1.00 | 19.09 | O |
| ATOM | 624 | N | ILE B | 91 | 23.018 | 7.884 | 5.115 | 1.00 | 17.78 | N |
| ATOM | 625 | CA | ILE B | 91 | 21.832 | 7.063 | 4.921 | 1.00 | 19.27 | C |
| ATOM | 626 | CB | ILE B | 91 | 20.564 | 7.930 | 4.701 | 1.00 | 20.73 | C |
| ATOM | 627 | CG1 | ILE B | 91 | 20.306 | 8.824 | 5.930 | 1.00 | 17.79 | C |
| ATOM | 628 | CD1 | ILE B | 91 | 19.434 | 10.052 | 5.610 | 1.00 | 19.73 | C |
| ATOM | 629 | CG2 | ILE B | 91 | 19.334 | 7.026 | 4.440 | 1.00 | 20.03 | C |
| ATOM | 630 | C | ILE B | 91 | 22.010 | 6.029 | 3.783 | 1.00 | 18.88 | C |
| ATOM | 631 | O | ILE B | 91 | 21.542 | 4.893 | 3.911 | 1.00 | 18.99 | O |
| ATOM | 632 | N | SER B | 92 | 22.695 | 6.411 | 2.697 | 1.00 | 19.49 | N |
| ATOM | 633 | CA | SER B | 92 | 23.020 | 5.468 | 1.607 | 1.00 | 19.19 | C |
| ATOM | 634 | CB | SER B | 92 | 23.966 | 6.088 | 0.565 | 1.00 | 17.73 | C |
| ATOM | 635 | OG | SER B | 92 | 23.417 | 7.222 | -0.007 | 1.00 | 24.41 | O |
| ATOM | 636 | C | SER B | 92 | 23.762 | 4.291 | 2.173 | 1.00 | 19.12 | C |
| ATOM | 637 | O | SER B | 92 | 23.462 | 3.135 | 1.860 | 1.00 | 18.77 | O |
| ATOM | 638 | N | HIS B | 93 | 24.756 | 4.580 | 3.001 | 1.00 | 21.30 | N |
| ATOM | 639 | CA | HIS B | 93 | 25.588 | 3.509 | 3.545 | 1.00 | 20.35 | C |
| ATOM | 640 | CB | HIS B | 93 | 26.884 | 4.051 | 4.138 | 1.00 | 22.22 | C |
| ATOM | 641 | CG | HIS B | 93 | 27.861 | 4.514 | 3.107 | 1.00 | 23.66 | C |
| ATOM | 642 | ND1 | HIS B | 93 | 28.175 | 3.759 | 1.993 | 1.00 | 27.28 | N |
| ATOM | 643 | CE1 | HIS B | 93 | 29.052 | 4.415 | 1.255 | 1.00 | 27.12 | C |
| ATOM | 644 | NE2 | HIS B | 93 | 29.328 | 5.563 | 1.852 | 1.00 | 26.92 | N |
| ATOM | 645 | CD2 | HIS B | 93 | 28.598 | 5.647 | 3.014 | 1.00 | 23.42 | C |
| ATOM | 646 | C | HIS B | 93 | 24.842 | 2.606 | 4.528 | 1.00 | 20.78 | C |
| ATOM | 647 | O | HIS B | 93 | 25.053 | 1.395 | 4.528 | 1.00 | 20.30 | O |
| ATOM | 648 | N | PHE B | 94 | 23.957 | 3.183 | 5.336 | 1.00 | 19.25 | N |
| ATOM | 649 | CA | PHE B | 94 | 23.089 | 2.385 | 6.204 | 1.00 | 21.86 | C |
| ATOM | 650 | CB | PHE B | 94 | 22.149 | 3.307 | 6.979 | 1.00 | 21.98 | C |
| ATOM | 651 | CG | PHE B | 94 | 21.094 | 2.586 | 7.765 | 1.00 | 24.77 | C |
| ATOM | 652 | CD1 | PHE B | 94 | 21.336 | 2.186 | 9.075 | 1.00 | 27.53 | C |
| ATOM | 653 | CE1 | PHE B | 94 | 20.359 | 1.526 | 9.808 | 1.00 | 26.34 | C |
| ATOM | 654 | CZ | PHE B | 94 | 19.124 | 1.273 | 9.237 | 1.00 | 26.22 | C |
| ATOM | 655 | CE2 | PHE B | 94 | 18.874 | 1.661 | 7.930 | 1.00 | 27.17 | C |
| ATOM | 656 | CD2 | PHE B | 94 | 19.847 | 2.329 | 7.206 | 1.00 | 23.05 | C |
| ATOM | 657 | C | PHE B | 94 | 22.266 | 1.375 | 5.385 | 1.00 | 22.76 | C |
| ATOM | 658 | O | PHE B | 94 | 22.206 | 0.181 | 5.717 | 1.00 | 21.45 | O |
| ATOM | 659 | N | LEU B | 95 | 21.620 | 1.862 | 4.328 | 1.00 | 22.06 | N |
| ATOM | 660 | CA | LEU B | 95 | 20.773 | 1.018 | 3.488 | 1.00 | 20.69 | C |
| ATOM | 661 | CB | LEU B | 95 | 20.008 | 1.852 | 2.456 | 1.00 | 20.00 | C |
| ATOM | 662 | CG | LEU B | 95 | 18.917 | 2.778 | 2.999 | 1.00 | 22.29 | C |
| ATOM | 663 | CD1 | LEU B | 95 | 18.521 | 3.798 | 1.947 | 1.00 | 23.77 | C |
| ATOM | 664 | CD2 | LEU B | 95 | 17.714 | 1.994 | 3.470 | 1.00 | 23.46 | C |
| ATOM | 665 | C | LEU B | 95 | 21.612 | -0.038 | 2.792 | 1.00 | 21.22 | C |
| ATOM | 666 | O | LEU B | 95 | 21.183 | -1.169 | 2.651 | 1.00 | 26.25 | O |
| ATOM | 667 | N | LYS B | 96 | 22.811 | 0.330 | 2.355 | 1.00 | 19.63 | N |
| ATOM | 668 | CA | LYS B | 96 | 23.749 | -0.655 | 1.815 | 1.00 | 21.38 | C |
| ATOM | 669 | CB | LYS B | 96 | 25.034 | 0.028 | 1.345 | 1.00 | 22.97 | C |
| ATOM | 670 | CG | LYS B | 96 | 25.776 | -0.756 | 0.275 | 1.00 | 28.30 | C |
| ATOM | 671 | CD | LYS B | 96 | 26.811 | -1.672 | 0.851 | 1.00 | 31.47 | C |
| ATOM | 672 | CE | LYS B | 96 | 27.620 | -2.333 | -0.297 | 1.00 | 37.44 | C |
| ATOM | 673 | NZ | LYS B | 96 | 26.860 | -3.393 | -1.027 | 1.00 | 32.07 | N |
| ATOM | 674 | C | LYS B | 96 | 24.076 | -1.778 | 2.809 | 1.00 | 23.97 | C |
| ATOM | 675 | O | LYS B | 96 | 24.007 | -2.956 | 2.451 | 1.00 | 24.11 | O |
| ATOM | 676 | N | GLU B | 97 | 24.420 | -1.431 | 4.050 | 1.00 | 23.26 | N |
| ATOM | 677 | CA | GLU B | 97 | 24.689 | -2.447 | 5.071 | 1.00 | 25.61 | C |
| ATOM | 678 | CB | GLU B | 97 | 24.988 | -1.805 | 6.427 | 1.00 | 27.53 | C |
| ATOM | 679 | CG | GLU B | 97 | 26.219 | -0.957 | 6.490 | 1.00 | 31.81 | C |
| ATOM | 680 | CD | GLU B | 97 | 26.537 | -0.587 | 7.931 | 1.00 | 37.15 | C |
| ATOM | 681 | OE1 | GLU B | 97 | 26.103 | -1.332 | 8.846 | 1.00 | 32.65 | O |
| ATOM | 682 | OE2 | GLU B | 97 | 27.201 | 0.449 | 8.133 | 1.00 | 39.51 | O |
| ATOM | 683 | C | GLU B | 97 | 23.494 | -3.368 | 5.275 | 1.00 | 26.59 | C |
| ATOM | 684 | O | GLU B | 97 | 23.651 | -4.565 | 5.486 | 1.00 | 27.22 | O |
| ATOM | 685 | N | LYS B | 98 | 22.299 | -2.799 | 5.250 | 1.00 | 26.65 | N |
| ATOM | 686 | CA | LYS B | 98 | 21.083 | -3.583 | 5.476 | 1.00 | 29.06 | C |
| ATOM | 687 | CB | LYS B | 98 | 19.959 | -2.667 | 5.950 | 1.00 | 29.97 | C |
| ATOM | 688 | CG | LYS B | 98 | 20.258 | -1.949 | 7.245 | 1.00 | 32.67 | C |
| ATOM | 689 | CD | LYS B | 98 | 20.068 | -2.852 | 8.437 | 1.00 | 32.63 | C |
| ATOM | 690 | CE | LYS B | 98 | 20.440 | -2.143 | 9.732 | 1.00 | 34.81 | C |
| ATOM | 691 | NZ | LYS B | 98 | 21.862 | -2.383 | 10.141 | 1.00 | 42.37 | N |
| ATOM | 692 | C | LYS B | 98 | 20.637 | -4.311 | 4.213 | 1.00 | 33.28 | C |
| ATOM | 693 | O | LYS B | 98 | 19.755 | -5.164 | 4.267 | 1.00 | 32.93 | O |
| ATOM | 694 | N | GLY B | 99 | 21.238 | -3.962 | 3.078 | 1.00 | 31.65 | N |
| ATOM | 695 | CA | GLY B | 99 | 20.869 | -4.551 | 1.799 | 1.00 | 36.02 | C |
| ATOM | 696 | C | GLY B | 99 | 19.478 | -4.109 | 1.382 | 1.00 | 36.14 | C |
| ATOM | 697 | O | GLY B | 99 | 18.813 | -4.783 | 0.578 | 1.00 | 32.52 | O |
| ATOM | 698 | N | GLY B | 100 | 19.057 | -2.955 | 1.912 | 1.00 | 30.17 | N |
| ATOM | 699 | CA | GLY B | 100 | 17.685 | -2.482 | 1.801 | 1.00 | 31.12 | C |
| ATOM | 700 | C | GLY B | 100 | 17.040 | -2.146 | 0.461 | 1.00 | 38.82 | C |
| ATOM | 701 | O | GLY B | 100 | 15.818 | -2.349 | 0.310 | 1.00 | 44.56 | O |
| ATOM | 702 | N | LEU B | 101 | 17.818 | -1.625 | -0.494 | 1.00 | 32.36 | N |
| ATOM | 703 | CA | LEU B | 101 | 17.284 | -1.123 | -1.771 | 1.00 | 28.97 | C |
| ATOM | 704 | CB | LEU B | 101 | 17.662 | 0.351 | -1.953 | 1.00 | 27.79 | C |
| ATOM | 705 | CG | LEU B | 101 | 16.806 | 1.450 | -1.357 | 1.00 | 28.51 | C |
| ATOM | 706 | CD1 | LEU B | 101 | 17.452 | 2.809 | -1.653 | 1.00 | 24.52 | C |
| ATOM | 707 | CD2 | LEU B | 101 | 15.383 | 1.383 | -1.929 | 1.00 | 25.93 | C |
| ATOM | 708 | C | LEU B | 101 | 17.838 | -1.841 | -2.989 | 1.00 | 28.45 | C |
| ATOM | 709 | O | LEU B | 101 | 17.213 | -1.842 | -4.063 | 1.00 | 27.79 | O |
| ATOM | 710 | N | GLU B | 102 | 19.034 | -2.404 | -2.831 | 1.00 | 25.79 | N |
| ATOM | 711 | CA | GLU B | 102 | 19.765 | -3.023 | -3.933 | 1.00 | 28.66 | C |
| ATOM | 712 | CB | GLU B | 102 | 20.973 | -3.779 | -3.379 | 1.00 | 28.01 | C |
| ATOM | 713 | CG | GLU B | 102 | 21.924 | -4.280 | -4.432 | 1.00 | 30.72 | C |
| ATOM | 714 | CD | GLU B | 102 | 22.665 | -3.144 | -5.117 | 1.00 | 29.94 | C |
| ATOM | 715 | OE1 | GLU B | 102 | 22.635 | -1.999 | -4.619 | 1.00 | 31.94 | O |
| ATOM | 716 | OE2 | GLU B | 102 | 23.291 | -3.391 | -6.148 | 1.00 | 33.32 | O |
| ATOM | 717 | C | GLU B | 102 | 18.881 | -3.981 | -4.738 | 1.00 | 26.64 | C |
| ATOM | 718 | O | GLU B | 102 | 18.245 | -4.854 | -4.165 | 1.00 | 26.11 | O |
| ATOM | 719 | N | GLY B | 103 | 18.807 | -3.781 | -6.053 | 1.00 | 26.66 | N |
| ATOM | 720 | CA | GLY B | 103 | 18.071 | -4.686 | -6.930 | 1.00 | 24.87 | C |
| ATOM | 721 | C | GLY B | 103 | 16.562 | -4.522 | -6.987 | 1.00 | 26.77 | C |
| ATOM | 722 | O | GLY B | 103 | 15.919 | -5.124 | -7.848 | 1.00 | 27.03 | O |
| ATOM | 723 | N | LEU B | 104 | 15.994 | -3.714 | -6.089 | 1.00 | 23.17 | N |
| ATOM | 724 | CA | LEU B | 104 | 14.553 | -3.484 | -6.042 | 1.00 | 27.10 | C |
| ATOM | 725 | CB | LEU B | 104 | 14.205 | -2.677 | -4.806 | 1.00 | 30.14 | C |
| ATOM | 726 | CG | LEU B | 104 | 13.398 | -3.265 | -3.662 | 1.00 | 36.57 | C |
| ATOM | 727 | CD1 | LEU B | 104 | 13.075 | -2.159 | -2.690 | 1.00 | 32.54 | C |
| ATOM | 728 | CD2 | LEU B | 104 | 12.128 | -3.921 | -4.181 | 1.00 | 36.03 | C |
| ATOM | 729 | C | LEU B | 104 | 14.080 | -2.666 | -7.233 | 1.00 | 26.99 | C |
| ATOM | 730 | O | LEU B | 104 | 14.658 | -1.626 | -7.514 | 1.00 | 23.70 | O |
| ATOM | 731 | N | ILE B | 105 | 12.999 | -3.097 | -7.886 | 1.00 | 26.72 | N |
| ATOM | 732 | CA | ILE B | 105 | 12.422 | -2.353 | -9.009 | 1.00 | 25.87 | C |
| ATOM | 733 | CB | ILE B | 105 | 11.249 | -3.130 | -9.662 | 1.00 | 26.54 | C |
| ATOM | 734 | CG1 | ILE B | 105 | 11.781 | -4.298 | -10.493 | 1.00 | 25.50 | C |
| ATOM | 735 | CD1 | ILE B | 105 | 10.680 | -5.304 | -10.856 | 1.00 | 27.86 | C |
| ATOM | 736 | CG2 | ILE B | 105 | 10.401 | -2.213 | -10.552 | 1.00 | 22.83 | C |
| ATOM | 737 | C | ILE B | 105 | 11.907 | -1.011 | -8.515 | 1.00 | 24.36 | C |
| ATOM | 738 | O | ILE B | 105 | 11.157 | -0.958 | -7.542 | 1.00 | 25.27 | O |
| ATOM | 739 | N | TRP B | 106 | 12.323 | 0.069 | -9.175 | 1.00 | 23.91 | N |
| ATOM | 740 | CA | TRP B | 106 | 11.902 | 1.408 | -8.779 | 1.00 | 23.13 | C |
| ATOM | 741 | CB | TRP B | 106 | 12.745 | 2.465 | -9.495 | 1.00 | 23.30 | C |
| ATOM | 742 | CG | TRP B | 106 | 12.353 | 3.875 | -9.127 | 1.00 | 23.58 | C |
| ATOM | 743 | CD1 | TRP B | 106 | 12.889 | 4.639 | -8.120 | 1.00 | 23.19 | C |
| ATOM | 744 | NE1 | TRP B | 106 | 12.273 | 5.871 | -8.086 | 1.00 | 23.04 | N |
| ATOM | 745 | CE2 | TRP B | 106 | 11.324 | 5.925 | -9.073 | 1.00 | 23.84 | C |
| ATOM | 746 | CD2 | TRP B | 106 | 11.346 | 4.686 | -9.752 | 1.00 | 23.48 | C |
| ATOM | 747 | CE3 | TRP B | 106 | 10.460 | 4.484 | -10.821 | 1.00 | 25.98 | C |
| ATOM | 748 | CZ3 | TRP B | 106 | 9.586 | 5.508 | -11.170 | 1.00 | 26.22 | C |
| ATOM | 749 | CH2 | TRP B | 106 | 9.587 | 6.734 | -10.479 | 1.00 | 28.29 | C |
| ATOM | 750 | CZ2 | TRP B | 106 | 10.441 | 6.959 | -9.422 | 1.00 | 29.58 | C |
| ATOM | 751 | C | TRP B | 106 | 10.427 | 1.634 | -9.116 | 1.00 | 27.12 | C |
| ATOM | 752 | O | TRP B | 106 | 9.938 | 1.178 | -10.153 | 1.00 | 26.32 | O |
| ATOM | 753 | N | SER B | 107 | 9.722 | 2.333 | -8.234 | 1.00 | 23.43 | N |
| ATOM | 754 | CA | SER B | 107 | 8.399 | 2.850 | -8.550 | 1.00 | 25.77 | C |
| ATOM | 755 | CB | SER B | 107 | 7.300 | 1.834 | -8.222 | 1.00 | 29.79 | C |
| ATOM | 756 | OG | SER B | 107 | 7.046 | 1.777 | -6.824 | 1.00 | 28.21 | O |
| ATOM | 757 | C | SER B | 107 | 8.196 | 4.145 | -7.761 | 1.00 | 30.76 | C |
| ATOM | 758 | O | SER B | 107 | 8.873 | 4.386 | -6.751 | 1.00 | 25.15 | O |
| ATOM | 759 | N | GLN B | 108 | 7.290 | 4.985 | -8.241 | 1.00 | 28.16 | N |
| ATOM | 760 | CA | GLN B | 108 | 6.939 | 6.210 | -7.538 | 1.00 | 32.64 | C |
| ATOM | 761 | CB | GLN B | 108 | 5.788 | 6.894 | -8.272 | 1.00 | 39.49 | C |
| ATOM | 762 | CG | GLN B | 108 | 5.312 | 8.174 | -7.643 | 1.00 | 41.11 | C |
| ATOM | 763 | CD | GLN B | 108 | 6.012 | 9.380 | -8.206 | 1.00 | 48.05 | C |
| ATOM | 764 | OE1 | GLN B | 108 | 7.237 | 9.396 | -8.349 | 1.00 | 49.81 | O |
| ATOM | 765 | NE2 | GLN B | 108 | 5.235 | 10.406 | -8.540 | 1.00 | 51.66 | N |
| ATOM | 766 | C | GLN B | 108 | 6.551 | 5.917 | -6.083 | 1.00 | 31.48 | C |
| ATOM | 767 | O | GLN B | 108 | 7.033 | 6.572 | -5.151 | 1.00 | 29.85 | O |
| ATOM | 768 | N | ARG B | 109 | 5.712 | 4.906 | -5.883 | 1.00 | 32.18 | N |
| ATOM | 769 | CA | ARG B | 109 | 5.258 | 4.560 | -4.538 | 1.00 | 31.67 | C |
| ATOM | 770 | CB | ARG B | 109 | 4.105 | 3.548 | -4.578 | 1.00 | 31.20 | C |
| ATOM | 771 | CG | ARG B | 109 | 3.570 | 3.174 | -3.195 | 1.00 | 35.21 | C |
| ATOM | 772 | CD | ARG B | 109 | 3.033 | 4.404 | -2.454 | 1.00 | 39.90 | C |
| ATOM | 773 | NE | ARG B | 109 | 2.619 | 4.105 | -1.084 | 1.00 | 45.71 | N |
| ATOM | 774 | CZ | ARG B | 109 | 2.674 | 4.975 | -0.079 | 1.00 | 46.77 | C |
| ATOM | 775 | NH1 | ARG B | 109 | 3.134 | 6.204 | -0.282 | 1.00 | 42.82 | N |
| ATOM | 776 | NH2 | ARG B | 109 | 2.278 | 4.612 | 1.133 | 1.00 | 49.58 | N |
| ATOM | 777 | C | ARG B | 109 | 6.388 | 4.041 | -3.648 | 1.00 | 29.79 | C |
| ATOM | 778 | O | ARG B | 109 | 6.473 | 4.426 | -2.482 | 1.00 | 31.70 | O |
| ATOM | 779 | N | ARG B | 110 | 7.253 | 3.176 | -4.175 | 1.00 | 26.10 | N |
| ATOM | 780 | CA | ARG B | 110 | 8.362 | 2.667 | -3.365 | 1.00 | 26.13 | C |
| ATOM | 781 | CB | ARG B | 110 | 9.151 | 1.570 | -4.075 | 1.00 | 25.71 | C |
| ATOM | 782 | CG | ARG B | 110 | 8.445 | 0.236 | -4.069 | 1.00 | 33.70 | C |
| ATOM | 783 | CD | ARG B | 110 | 9.345 | -0.915 | -4.476 | 1.00 | 32.12 | C |
| ATOM | 784 | NE | ARG B | 110 | 8.537 | -2.120 | -4.619 | 1.00 | 34.15 | N |
| ATOM | 785 | CZ | ARG B | 110 | 8.430 | -2.833 | -5.732 | 1.00 | 40.90 | C |
| ATOM | 786 | NH1 | ARG B | 110 | 9.103 | -2.486 | -6.824 | 1.00 | 39.84 | N |
| ATOM | 787 | NH2 | ARG B | 110 | 7.643 | -3.902 | -5.756 | 1.00 | 41.59 | N |
| ATOM | 788 | C | ARG B | 110 | 9.296 | 3.787 | -2.945 | 1.00 | 25.50 | C |
| ATOM | 789 | O | ARG B | 110 | 9.714 | 3.849 | -1.786 | 1.00 | 26.64 | O |
| ATOM | 790 | N | GLN B | 111 | 9.610 | 4.685 | -3.867 | 1.00 | 24.54 | N |
| ATOM | 791 | CA | GLN B | 111 | 10.498 | 5.770 | -3.504 | 1.00 | 25.12 | C |
| ATOM | 792 | CB | GLN B | 111 | 10.916 | 6.598 | -4.697 | 1.00 | 26.27 | C |
| ATOM | 793 | CG | GLN B | 111 | 11.981 | 7.582 | -4.281 | 1.00 | 26.81 | C |
| ATOM | 794 | CD | GLN B | 111 | 12.316 | 8.573 | -5.339 | 1.00 | 27.76 | C |
| ATOM | 795 | OE1 | GLN B | 111 | 12.550 | 8.217 | -6.497 | 1.00 | 29.05 | O |
| ATOM | 796 | NE2 | GLN B | 111 | 12.356 | 9.844 | -4.953 | 1.00 | 28.07 | N |
| ATOM | 797 | C | GLN B | 111 | 9.851 | 6.689 | -2.470 | 1.00 | 27.56 | C |
| ATOM | 798 | O | GLN B | 111 | 10.535 | 7.242 | -1.614 | 1.00 | 25.40 | O |
| ATOM | 799 | N | GLU B | 112 | 8.532 | 6.850 | -2.561 | 1.00 | 27.88 | N |
| ATOM | 800 | CA | GLU B | 112 | 7.794 | 7.679 | -1.622 | 1.00 | 26.28 | C |
| ATOM | 801 | CB | GLU B | 112 | 6.334 | 7.833 | -2.086 | 1.00 | 28.36 | C |
| ATOM | 802 | CG | GLU B | 112 | 5.507 | 8.788 | -1.228 | 1.00 | 38.25 | C |
| ATOM | 803 | CD | GLU B | 112 | 4.071 | 8.933 | -1.726 | 1.00 | 35.56 | C |
| ATOM | 804 | OE1 | GLU B | 112 | 3.748 | 9.981 | -2.321 | 1.00 | 43.10 | O |
| ATOM | 805 | OE2 | GLU B | 112 | 3.273 | 7.995 | -1.523 | 1.00 | 35.91 | O |
| ATOM | 806 | C | GLU B | 112 | 7.877 | 7.089 | -0.215 | 1.00 | 25.46 | C |
| ATOM | 807 | O | GLU B | 112 | 8.126 | 7.810 | 0.755 | 1.00 | 27.99 | O |
| ATOM | 808 | N | ILE B | 113 | 7.709 | 5.773 | -0.105 | 1.00 | 24.02 | N |
| ATOM | 809 | CA | ILE B | 113 | 7.867 | 5.095 | 1.171 | 1.00 | 23.08 | C |
| ATOM | 810 | CB | ILE B | 113 | 7.582 | 3.586 | 1.042 | 1.00 | 25.19 | C |
| ATOM | 811 | CG1 | ILE B | 113 | 6.108 | 3.361 | 0.676 | 1.00 | 30.45 | C |
| ATOM | 812 | CD1 | ILE B | 113 | 5.737 | 1.903 | 0.510 | 1.00 | 31.44 | C |
| ATOM | 813 | CG2 | ILE B | 113 | 7.898 | 2.854 | 2.344 | 1.00 | 31.63 | C |
| ATOM | 814 | C | ILE B | 113 | 9.280 | 5.319 | 1.698 | 1.00 | 28.43 | C |
| ATOM | 815 | O | ILE B | 113 | 9.487 | 5.549 | 2.889 | 1.00 | 23.36 | O |
| ATOM | 816 | N | LEU B | 114 | 10.260 | 5.268 | 0.804 | 1.00 | 25.88 | N |
| ATOM | 817 | CA | LEU B | 114 | 11.640 | 5.498 | 1.209 | 1.00 | 25.12 | C |
| ATOM | 818 | CB | LEU B | 114 | 12.588 | 5.237 | 0.035 | 1.00 | 21.45 | C |
| ATOM | 819 | CG | LEU B | 114 | 14.046 | 5.657 | 0.193 | 1.00 | 26.67 | C |
| ATOM | 820 | CD1 | LEU B | 114 | 14.679 | 5.009 | 1.395 | 1.00 | 26.45 | C |
| ATOM | 821 | CD2 | LEU B | 114 | 14.821 | 5.319 | -1.087 | 1.00 | 24.71 | C |
| ATOM | 822 | C | LEU B | 114 | 11.786 | 6.925 | 1.729 | 1.00 | 23.51 | C |
| ATOM | 823 | O | LEU B | 114 | 12.258 | 7.128 | 2.858 | 1.00 | 23.40 | O |
| ATOM | 824 | N | ASP B | 115 | 11.338 | 7.897 | 0.935 | 1.00 | 24.81 | N |
| ATOM | 825 | CA | ASP B | 115 | 11.488 | 9.310 | 1.300 | 1.00 | 21.84 | C |
| ATOM | 826 | CB | ASP B | 115 | 11.062 | 10.216 | 0.150 | 1.00 | 25.23 | C |
| ATOM | 827 | CG | ASP B | 115 | 12.048 | 10.185 | -1.010 | 1.00 | 30.40 | C |
| ATOM | 828 | OD1 | ASP B | 115 | 13.260 | 9.958 | -0.764 | 1.00 | 27.72 | O |
| ATOM | 829 | OD2 | ASP B | 115 | 11.616 | 10.396 | -2.163 | 1.00 | 28.48 | O |
| ATOM | 830 | C | ASP B | 115 | 10.752 | 9.701 | 2.576 | 1.00 | 24.23 | C |
| ATOM | 831 | O | ASP B | 115 | 11.256 | 10.516 | 3.358 | 1.00 | 21.21 | O |
| ATOM | 832 | N | LEU B | 116 | 9.576 | 9.117 | 2.786 | 1.00 | 22.97 | N |
| ATOM | 833 | CA | LEU B | 116 | 8.750 | 9.410 | 3.957 | 1.00 | 22.88 | C |
| ATOM | 834 | CB | LEU B | 116 | 7.317 | 8.949 | 3.706 | 1.00 | 22.74 | C |
| ATOM | 835 | CG | LEU B | 116 | 6.267 | 9.992 | 3.301 | 1.00 | 28.75 | C |
| ATOM | 836 | CD1 | LEU B | 116 | 6.829 | 11.150 | 2.536 | 1.00 | 29.35 | C |
| ATOM | 837 | CD2 | LEU B | 116 | 5.198 | 9.306 | 2.486 | 1.00 | 32.54 | C |
| ATOM | 838 | C | LEU B | 116 | 9.276 | 8.729 | 5.207 | 1.00 | 25.27 | C |
| ATOM | 839 | O | LEU B | 116 | 9.071 | 9.218 | 6.318 | 1.00 | 21.91 | O |
| ATOM | 840 | N | TRP B | 117 | 9.929 | 7.580 | 5.034 | 1.00 | 22.82 | N |
| ATOM | 841 | CA | TRP B | 117 | 10.599 | 6.917 | 6.148 | 1.00 | 21.11 | C |
| ATOM | 842 | CB | TRP B | 117 | 11.055 | 5.500 | 5.740 | 1.00 | 25.00 | C |
| ATOM | 843 | CG | TRP B | 117 | 12.002 | 4.857 | 6.730 | 1.00 | 25.97 | C |
| ATOM | 844 | CD1 | TRP B | 117 | 11.695 | 4.367 | 7.966 | 1.00 | 26.41 | C |
| ATOM | 845 | NE1 | TRP B | 117 | 12.834 | 3.859 | 8.573 | 1.00 | 28.16 | N |
| ATOM | 846 | CE2 | TRP B | 117 | 13.896 | 4.014 | 7.717 | 1.00 | 33.80 | C |
| ATOM | 847 | CD2 | TRP B | 117 | 13.409 | 4.634 | 6.545 | 1.00 | 26.39 | C |
| ATOM | 848 | CE3 | TRP B | 117 | 14.302 | 4.899 | 5.504 | 1.00 | 29.48 | C |
| ATOM | 849 | CZ3 | TRP B | 117 | 15.641 | 4.547 | 5.655 | 1.00 | 30.12 | C |
| ATOM | 850 | CH2 | TRP B | 117 | 16.097 | 3.937 | 6.831 | 1.00 | 33.83 | C |
| ATOM | 851 | CZ2 | TRP B | 117 | 15.244 | 3.657 | 7.869 | 1.00 | 32.16 | C |
| ATOM | 852 | C | TRP B | 117 | 11.769 | 7.771 | 6.638 | 1.00 | 21.19 | C |
| ATOM | 853 | O | TRP B | 117 | 11.972 | 7.932 | 7.840 | 1.00 | 23.25 | O |
| ATOM | 854 | N | ILE B | 118 | 12.531 | 8.347 | 5.712 | 1.00 | 22.40 | N |
| ATOM | 855 | CA | ILE B | 118 | 13.592 | 9.301 | 6.076 | 1.00 | 21.04 | C |
| ATOM | 856 | CB | ILE B | 118 | 14.488 | 9.632 | 4.849 | 1.00 | 19.50 | C |
| ATOM | 857 | CG1 | ILE B | 118 | 15.288 | 8.386 | 4.460 | 1.00 | 21.30 | C |
| ATOM | 858 | CD1 | ILE B | 118 | 15.800 | 8.413 | 3.002 | 1.00 | 24.40 | C |
| ATOM | 859 | CG2 | ILE B | 118 | 15.442 | 10.772 | 5.158 | 1.00 | 18.81 | C |
| ATOM | 860 | C | ILE B | 118 | 13.022 | 10.594 | 6.686 | 1.00 | 22.59 | C |
| ATOM | 861 | O | ILE B | 118 | 13.590 | 11.156 | 7.630 | 1.00 | 21.01 | O |
| ATOM | 862 | N | TYR B | 119 | 11.909 | 11.076 | 6.149 | 1.00 | 20.72 | N |
| ATOM | 863 | CA | TYR B | 119 | 11.232 | 12.228 | 6.754 | 1.00 | 23.06 | C |
| ATOM | 864 | CB | TYR B | 119 | 9.982 | 12.603 | 5.949 | 1.00 | 21.97 | C |
| ATOM | 865 | CG | TYR B | 119 | 9.174 | 13.725 | 6.562 | 1.00 | 21.50 | C |
| ATOM | 866 | CD1 | TYR B | 119 | 9.468 | 15.055 | 6.286 | 1.00 | 24.58 | C |
| ATOM | 867 | CE1 | TYR B | 119 | 8.711 | 16.097 | 6.857 | 1.00 | 26.75 | C |
| ATOM | 868 | CZ | TYR B | 119 | 7.664 | 15.789 | 7.716 | 1.00 | 27.22 | C |
| ATOM | 869 | OH | TYR B | 119 | 6.897 | 16.789 | 8.314 | 1.00 | 27.52 | O |
| ATOM | 870 | CE2 | TYR B | 119 | 7.359 | 14.473 | 7.993 | 1.00 | 24.40 | C |
| ATOM | 871 | CD2 | TYR B | 119 | 8.111 | 13.452 | 7.420 | 1.00 | 26.68 | C |
| ATOM | 872 | C | TYR B | 119 | 10.869 | 11.923 | 8.209 | 1.00 | 23.41 | C |
| ATOM | 873 | O | TYR B | 119 | 11.190 | 12.692 | 9.124 | 1.00 | 23.06 | O |
| ATOM | 874 | N | HIS B | 120 | 10.228 | 10.779 | 8.420 | 1.00 | 24.56 | N |
| ATOM | 875 | CA | HIS B | 120 | 9.720 | 10.403 | 9.740 | 1.00 | 25.02 | C |
| ATOM | 876 | CB | HIS B | 120 | 8.834 | 9.160 | 9.613 | 1.00 | 24.94 | C |
| ATOM | 877 | CG | HIS B | 120 | 8.155 | 8.779 | 10.888 | 1.00 | 31.85 | C |
| ATOM | 878 | ND1 | HIS B | 120 | 7.085 | 9.485 | 11.401 | 1.00 | 34.72 | N |
| ATOM | 879 | CE1 | HIS B | 120 | 6.701 | 8.932 | 12.538 | 1.00 | 35.64 | C |
| ATOM | 880 | NE2 | HIS B | 120 | 7.482 | 7.893 | 12.781 | 1.00 | 38.57 | N |
| ATOM | 881 | CD2 | HIS B | 120 | 8.398 | 7.774 | 11.762 | 1.00 | 31.69 | C |
| ATOM | 882 | C | HIS B | 120 | 10.807 | 10.153 | 10.795 | 1.00 | 27.90 | C |
| ATOM | 883 | O | HIS B | 120 | 10.663 | 10.529 | 11.967 | 1.00 | 23.73 | O |
| ATOM | 884 | N | THR B | 121 | 11.882 | 9.485 | 10.388 | 1.00 | 22.33 | N |
| ATOM | 885 | CA | THR B | 121 | 12.921 | 9.067 | 11.326 | 1.00 | 23.12 | C |
| ATOM | 886 | CB | THR B | 121 | 13.544 | 7.729 | 10.867 | 1.00 | 24.94 | C |
| ATOM | 887 | OG1 | THR B | 121 | 14.141 | 7.908 | 9.573 | 1.00 | 22.85 | O |
| ATOM | 888 | CG2 | THR B | 121 | 12.473 | 6.645 | 10.774 | 1.00 | 22.75 | C |
| ATOM | 889 | C | THR B | 121 | 14.023 | 10.116 | 11.516 | 1.00 | 21.92 | C |
| ATOM | 890 | O | THR B | 121 | 14.683 | 10.146 | 12.560 | 1.00 | 24.24 | O |
| ATOM | 891 | N | GLN B | 122 | 14.215 | 10.978 | 10.514 | 1.00 | 21.69 | N |
| ATOM | 892 | CA | GLN B | 122 | 15.369 | 11.890 | 10.501 | 1.00 | 22.47 | C |
| ATOM | 893 | CB | GLN B | 122 | 16.414 | 11.411 | 9.466 | 1.00 | 20.76 | C |
| ATOM | 894 | CG | GLN B | 122 | 17.101 | 10.093 | 9.862 | 1.00 | 20.92 | C |
| ATOM | 895 | CD | GLN B | 122 | 18.046 | 10.253 | 11.048 | 1.00 | 23.81 | C |
| ATOM | 896 | OE1 | GLN B | 122 | 19.227 | 10.579 | 10.873 | 1.00 | 24.02 | O |
| ATOM | 897 | NE2 | GLN B | 122 | 17.532 | 10.035 | 12.260 | 1.00 | 22.18 | N |
| ATOM | 898 | C | GLN B | 122 | 15.022 | 13.353 | 10.242 | 1.00 | 21.49 | C |
| ATOM | 899 | O | GLN B | 122 | 15.854 | 14.232 | 10.461 | 1.00 | 21.71 | O |
| ATOM | 900 | N | GLY B | 123 | 13.825 | 13.610 | 9.712 | 1.00 | 21.85 | N |
| ATOM | 901 | CA | GLY B | 123 | 13.379 | 14.978 | 9.501 | 1.00 | 22.17 | C |
| ATOM | 902 | C | GLY B | 123 | 13.684 | 15.625 | 8.160 | 1.00 | 24.17 | C |
| ATOM | 903 | O | GLY B | 123 | 13.393 | 16.809 | 7.975 | 1.00 | 23.23 | O |
| ATOM | 904 | N | TYR B | 124 | 14.242 | 14.872 | 7.209 | 1.00 | 22.07 | N |
| ATOM | 905 | CA | TYR B | 124 | 14.592 | 15.462 | 5.908 | 1.00 | 20.04 | C |
| ATOM | 906 | CB | TYR B | 124 | 15.723 | 14.671 | 5.208 | 1.00 | 22.09 | C |
| ATOM | 907 | CG | TYR B | 124 | 17.011 | 14.760 | 5.994 | 1.00 | 21.32 | C |
| ATOM | 908 | CD1 | TYR B | 124 | 17.751 | 15.937 | 6.010 | 1.00 | 20.71 | C |
| ATOM | 909 | CE1 | TYR B | 124 | 18.912 | 16.039 | 6.753 | 1.00 | 22.02 | C |
| ATOM | 910 | CZ | TYR B | 124 | 19.337 | 14.967 | 7.510 | 1.00 | 21.65 | C |
| ATOM | 911 | OH | TYR B | 124 | 20.478 | 15.082 | 8.260 | 1.00 | 24.24 | O |
| ATOM | 912 | CE2 | TYR B | 124 | 18.639 | 13.789 | 7.515 | 1.00 | 20.87 | C |
| ATOM | 913 | CD2 | TYR B | 124 | 17.462 | 13.691 | 6.756 | 1.00 | 20.58 | C |
| ATOM | 914 | C | TYR B | 124 | 13.369 | 15.639 | 5.003 | 1.00 | 21.52 | C |
| ATOM | 915 | O | TYR B | 124 | 12.660 | 14.678 | 4.724 | 1.00 | 23.21 | O |
| ATOM | 916 | N | PHE B | 125 | 13.128 | 16.870 | 4.559 | 1.00 | 24.32 | N |
| ATOM | 917 | CA | PHE B | 125 | 12.071 | 17.154 | 3.586 | 1.00 | 27.43 | C |
| ATOM | 918 | CB | PHE B | 125 | 12.165 | 18.595 | 3.077 | 1.00 | 29.23 | C |
| ATOM | 919 | CG | PHE B | 125 | 12.076 | 19.644 | 4.157 | 1.00 | 32.92 | C |
| ATOM | 920 | CD1 | PHE B | 125 | 10.884 | 19.862 | 4.846 | 1.00 | 32.13 | C |
| ATOM | 921 | CE1 | PHE B | 125 | 10.802 | 20.852 | 5.838 | 1.00 | 31.40 | C |
| ATOM | 922 | CZ | PHE B | 125 | 11.907 | 21.629 | 6.127 | 1.00 | 30.18 | C |
| ATOM | 923 | CE2 | PHE B | 125 | 13.099 | 21.432 | 5.428 | 1.00 | 31.56 | C |
| ATOM | 924 | CD2 | PHE B | 125 | 13.176 | 20.445 | 4.450 | 1.00 | 32.58 | C |
| ATOM | 925 | C | PHE B | 125 | 12.297 | 16.248 | 2.393 | 1.00 | 25.67 | C |
| ATOM | 926 | O | PHE B | 125 | 13.428 | 16.141 | 1.930 | 1.00 | 26.35 | O |
| ATOM | 927 | N | PRO B | 126 | 11.230 | 15.604 | 1.894 | 1.00 | 24.85 | N |
| ATOM | 928 | CA | PRO B | 126 | 11.315 | 14.628 | 0.801 | 1.00 | 30.16 | C |
| ATOM | 929 | CB | PRO B | 126 | 10.038 | 13.796 | 0.984 | 1.00 | 31.80 | C |
| ATOM | 930 | CG | PRO B | 126 | 9.061 | 14.769 | 1.602 | 1.00 | 29.16 | C |
| ATOM | 931 | CD | PRO B | 126 | 9.886 | 15.643 | 2.504 | 1.00 | 27.99 | C |
| ATOM | 932 | C | PRO B | 126 | 11.343 | 15.281 | -0.578 | 1.00 | 28.75 | C |
| ATOM | 933 | O | PRO B | 126 | 10.498 | 14.985 | -1.429 | 1.00 | 33.88 | O |
| ATOM | 934 | N | ASP B | 127 | 12.323 | 16.148 | -0.802 | 1.00 | 29.43 | N |
| ATOM | 935 | CA | ASP B | 127 | 12.445 | 16.847 | -2.074 | 1.00 | 32.15 | C |
| ATOM | 936 | CB | ASP B | 127 | 12.184 | 18.354 | -1.891 | 1.00 | 34.07 | C |
| ATOM | 937 | CG | ASP B | 127 | 13.143 | 19.009 | -0.898 | 1.00 | 37.76 | C |
| ATOM | 938 | OD1 | ASP B | 127 | 14.258 | 18.482 | -0.661 | 1.00 | 38.17 | O |
| ATOM | 939 | OD2 | ASP B | 127 | 12.791 | 20.080 | -0.351 | 1.00 | 43.14 | O |
| ATOM | 940 | C | ASP B | 127 | 13.826 | 16.646 | -2.689 | 1.00 | 31.30 | C |
| ATOM | 941 | O | ASP B | 127 | 14.219 | 17.382 | -3.604 | 1.00 | 31.65 | O |
| ATOM | 942 | N | TRP B | 128 | 14.565 | 15.657 | -2.192 | 1.00 | 24.60 | N |
| ATOM | 943 | CA | TRP B | 128 | 15.978 | 15.555 | -2.536 | 1.00 | 24.10 | C |
| ATOM | 944 | CB | TRP B | 128 | 16.801 | 15.385 | -1.261 | 1.00 | 23.03 | C |
| ATOM | 945 | CG | TRP B | 128 | 16.386 | 14.173 | -0.503 | 1.00 | 23.26 | C |
| ATOM | 946 | CD1 | TRP B | 128 | 15.411 | 14.101 | 0.456 | 1.00 | 23.49 | C |
| ATOM | 947 | NE1 | TRP B | 128 | 15.305 | 12.811 | 0.921 | 1.00 | 21.32 | N |
| ATOM | 948 | CE2 | TRP B | 128 | 16.212 | 12.020 | 0.259 | 1.00 | 24.14 | C |
| ATOM | 949 | CD2 | TRP B | 128 | 16.913 | 12.844 | -0.644 | 1.00 | 23.87 | C |
| ATOM | 950 | CE3 | TRP B | 128 | 17.910 | 12.274 | -1.447 | 1.00 | 24.14 | C |
| ATOM | 951 | CZ3 | TRP B | 128 | 18.169 | 10.914 | -1.320 | 1.00 | 23.26 | C |
| ATOM | 952 | CH2 | TRP B | 128 | 17.448 | 10.121 | -0.414 | 1.00 | 22.51 | C |
| ATOM | 953 | CZ2 | TRP B | 128 | 16.469 | 10.651 | 0.383 | 1.00 | 23.95 | C |
| ATOM | 954 | C | TRP B | 128 | 16.292 | 14.400 | -3.477 | 1.00 | 26.23 | C |
| ATOM | 955 | O | TRP B | 128 | 17.293 | 14.444 | -4.201 | 1.00 | 26.03 | O |
| ATOM | 956 | N | GLN B | 129 | 15.461 | 13.358 | -3.465 | 1.00 | 23.34 | N |
| ATOM | 957 | CA | GLN B | 129 | 15.814 | 12.141 | -4.185 | 1.00 | 23.97 | C |
| ATOM | 958 | CB | GLN B | 129 | 15.284 | 10.871 | -3.489 | 1.00 | 23.43 | C |
| ATOM | 959 | CG | GLN B | 129 | 15.914 | 9.605 | -4.089 | 1.00 | 25.47 | C |
| ATOM | 960 | CD | GLN B | 129 | 15.369 | 8.311 | -3.512 | 1.00 | 28.70 | C |
| ATOM | 961 | OE1 | GLN B | 129 | 14.644 | 8.314 | -2.510 | 1.00 | 27.37 | O |
| ATOM | 962 | NE2 | GLN B | 129 | 15.722 | 7.194 | -4.141 | 1.00 | 20.93 | N |
| ATOM | 963 | C | GLN B | 129 | 15.362 | 12.198 | -5.640 | 1.00 | 23.77 | C |
| ATOM | 964 | O | GLN B | 129 | 14.405 | 11.528 | -6.035 | 1.00 | 23.57 | O |
| ATOM | 965 | N | ASN B | 130 | 16.043 | 13.029 | -6.419 | 1.00 | 23.03 | N |
| ATOM | 966 | CA | ASN B | 130 | 15.754 | 13.184 | -7.839 | 1.00 | 23.44 | C |
| ATOM | 967 | CB | ASN B | 130 | 15.570 | 14.667 | -8.177 | 1.00 | 24.89 | C |
| ATOM | 968 | CG | ASN B | 130 | 14.521 | 15.333 | -7.309 | 1.00 | 31.82 | C |
| ATOM | 969 | OD1 | ASN B | 130 | 13.375 | 14.885 | -7.263 | 1.00 | 34.16 | O |
| ATOM | 970 | ND2 | ASN B | 130 | 14.914 | 16.391 | -6.591 | 1.00 | 33.27 | N |
| ATOM | 971 | C | ASN B | 130 | 16.917 | 12.640 | -8.639 | 1.00 | 22.61 | C |
| ATOM | 972 | O | ASN B | 130 | 18.060 | 12.652 | -8.154 | 1.00 | 23.12 | O |
| ATOM | 973 | N | TYR B | 131 | 16.643 | 12.204 | -9.869 | 1.00 | 20.04 | N |
| ATOM | 974 | CA | TYR B | 131 | 17.660 | 11.591 | -10.712 | 1.00 | 21.12 | C |
| ATOM | 975 | CB | TYR B | 131 | 17.390 | 10.084 | -10.847 | 1.00 | 21.17 | C |
| ATOM | 976 | CG | TYR B | 131 | 17.402 | 9.318 | -9.531 | 1.00 | 20.33 | C |
| ATOM | 977 | CD1 | TYR B | 131 | 18.574 | 8.746 | -9.045 | 1.00 | 21.36 | C |
| ATOM | 978 | CE1 | TYR B | 131 | 18.591 | 8.051 | -7.837 | 1.00 | 18.50 | C |
| ATOM | 979 | CZ | TYR B | 131 | 17.423 | 7.913 | -7.107 | 1.00 | 21.86 | C |
| ATOM | 980 | OH | TYR B | 131 | 17.428 | 7.207 | -5.912 | 1.00 | 18.51 | O |
| ATOM | 981 | CE2 | TYR B | 131 | 16.249 | 8.467 | -7.572 | 1.00 | 20.17 | C |
| ATOM | 982 | CD2 | TYR B | 131 | 16.246 | 9.171 | -8.778 | 1.00 | 21.40 | C |
| ATOM | 983 | C | TYR B | 131 | 17.707 | 12.235 | -12.112 | 1.00 | 25.17 | C |
| ATOM | 984 | O | TYR B | 131 | 16.756 | 12.873 | -12.534 | 1.00 | 22.79 | O |
| ATOM | 985 | N | THR B | 132 | 18.816 | 12.060 | -12.820 | 1.00 | 19.27 | N |
| ATOM | 986 | CA | THR B | 132 | 18.943 | 12.573 | -14.181 | 1.00 | 25.54 | C |
| ATOM | 987 | CB | THR B | 132 | 20.362 | 12.311 | -14.741 | 1.00 | 24.19 | C |
| ATOM | 988 | OG1 | THR B | 132 | 20.640 | 10.904 | -14.712 | 1.00 | 24.28 | O |
| ATOM | 989 | CG2 | THR B | 132 | 21.401 | 13.040 | -13.919 | 1.00 | 25.46 | C |
| ATOM | 990 | C | THR B | 132 | 17.883 | 11.893 | -15.053 | 1.00 | 24.55 | C |
| ATOM | 991 | O | THR B | 132 | 17.412 | 10.799 | -14.713 | 1.00 | 23.99 | O |
| ATOM | 992 | N | PRO B | 133 | 17.500 | 12.530 | -16.177 | 1.00 | 29.90 | N |
| ATOM | 993 | CA | PRO B | 133 | 16.303 | 12.069 | -16.895 | 1.00 | 29.92 | C |
| ATOM | 994 | CB | PRO B | 133 | 15.950 | 13.269 | -17.783 | 1.00 | 30.40 | C |
| ATOM | 995 | CG | PRO B | 133 | 17.250 | 13.956 | -18.003 | 1.00 | 27.82 | C |
| ATOM | 996 | CD | PRO B | 133 | 18.008 | 13.803 | -16.721 | 1.00 | 28.15 | C |
| ATOM | 997 | C | PRO B | 133 | 16.449 | 10.808 | -17.759 | 1.00 | 29.71 | C |
| ATOM | 998 | O | PRO B | 133 | 15.427 | 10.261 | -18₋191 | 1.00 | 33.50 | O |
| ATOM | 999 | N | GLY B | 134 | 17.659 | 10.339 | -18.020 | 1.00 | 23.90 | N |
| ATOM | 1000 | CA | GLY B | 134 | 17.768 | 9.166 | -18.870 | 1.00 | 29.78 | C |
| ATOM | 1001 | C | GLY B | 134 | 17.527 | 9.507 | -20.339 | 1.00 | 28.64 | C |
| ATOM | 1002 | O | GLY B | 134 | 17.398 | 10.676 | -20.669 | 1.00 | 30.38 | O |
| ATOM | 1003 | N | PRO B | 135 | 17.428 | 8.500 | -21.220 | 1.00 | 30.09 | N |
| ATOM | 1004 | CA | PRO B | 135 | 17.301 | 7.052 | -20.989 | 1.00 | 32.03 | C |
| ATOM | 1005 | CB | PRO B | 135 | 17.062 | 6.484 | -22.404 | 1.00 | 35.14 | C |
| ATOM | 1006 | CG | PRO B | 135 | 16.794 | 7.673 | -23.285 | 1.00 | 39.82 | C |
| ATOM | 1007 | CD | PRO B | 135 | 17.534 | 8.809 | -22.661 | 1.00 | 36.18 | C |
| ATOM | 1008 | C | PRO B | 135 | 18.573 | 6.446 | -20.432 | 1.00 | 28.63 | C |
| ATOM | 1009 | O | PRO B | 135 | 19.609 | 7.126 | -20.422 | 1.00 | 28.70 | O |
| ATOM | 1010 | N | GLY B | 136 | 18.495 | 5.196 | -19.975 | 1.00 | 26.64 | N |
| ATOM | 1011 | CA | GLY B | 136 | 19.656 | 4.512 | -19.435 | 1.00 | 26.42 | C |
| ATOM | 1012 | C | GLY B | 136 | 19.758 | 4.743 | -17.933 | 1.00 | 27.62 | C |
| ATOM | 1013 | O | GLY B | 136 | 18.747 | 4.923 | -17.253 | 1.00 | 23.06 | O |
| ATOM | 1014 | N | ILE B | 137 | 20.973 | 4.742 | -17.407 | 1.00 | 26.52 | N |
| ATOM | 1015 | CA | ILE B | 137 | 21.152 | 4.871 | -15.964 | 1.00 | 25.08 | C |
| ATOM | 1016 | CB | ILE B | 137 | 22.610 | 4.559 | -15.571 | 1.00 | 26.34 | C |
| ATOM | 1017 | CG1 | ILE B | 137 | 22.919 | 3.077 | -15.864 | 1.00 | 23.49 | C |
| ATOM | 1018 | CD1 | ILE B | 137 | 24.380 | 2.675 | -15.647 | 1.00 | 27.49 | C |
| ATOM | 1019 | CG2 | ILE B | 137 | 22.878 | 4.933 | -14.127 | 1.00 | 22.77 | C |
| ATOM | 1020 | C | ILE B | 137 | 20.737 | 6.275 | -15.531 | 1.00 | 22.22 | C |
| ATOM | 1021 | O | ILE B | 137 | 21.036 | 7.258 | -16.210 | 1.00 | 20.64 | O |
| ATOM | 1022 | N | ARG B | 138 | 20.025 | 6.363 | -14.411 | 1.00 | 21.15 | N |
| ATOM | 1023 | CA | ARG B | 138 | 19.627 | 7.654 | -13.847 | 1.00 | 22.16 | C |
| ATOM | 1024 | CB | ARG B | 138 | 18.136 | 7.631 | -13.475 | 1.00 | 19.88 | C |
| ATOM | 1025 | CG | ARG B | 138 | 17.263 | 6.861 | -14.468 | 1.00 | 20.29 | C |
| ATOM | 1026 | CD | ARG B | 138 | 17.261 | 7.490 | -15.879 | 1.00 | 21.23 | C |
| ATOM | 1027 | NE | ARG B | 138 | 16.512 | 6.655 | -16.825 | 1.00 | 24.68 | N |
| ATOM | 1028 | CZ | ARG B | 138 | 15.187 | 6.682 | -16.965 | 1.00 | 27.88 | C |
| ATOM | 1029 | NH1 | ARG B | 138 | 14.460 | 7.516 | -16.232 | 1.00 | 26.65 | N |
| ATOM | 1030 | NH2 | ARG B | 138 | 14.587 | 5.869 | -17.834 | 1.00 | 26.63 | N |
| ATOM | 1031 | C | ARG B | 138 | 20.491 | 7.964 | -12.625 | 1.00 | 21.80 | C |
| ATOM | 1032 | O | ARG B | 138 | 20.470 | 7.227 | -11.627 | 1.00 | 19.52 | O |
| ATOM | 1033 | N | TYR B | 139 | 21.277 | 9.037 | -12.712 | 1.00 | 19.54 | N |
| ATOM | 1034 | CA | TYR B | 139 | 22.201 | 9.402 | -11.638 | 1.00 | 17.46 | C |
| ATOM | 1035 | CB | TYR B | 139 | 23.504 | 9.955 | -12.216 | 1.00 | 18.69 | C |
| ATOM | 1036 | CG | TYR B | 139 | 24.251 | 8.933 | -13.045 | 1.00 | 23.10 | C |
| ATOM | 1037 | CD1 | TYR B | 139 | 25.062 | 7.979 | -12.445 | 1.00 | 24.59 | C |
| ATOM | 1038 | CE1 | TYR B | 139 | 25.744 | 7.027 | -13.210 | 1.00 | 24.21 | C |
| ATOM | 1039 | CZ | TYR B | 139 | 25.609 | 7.041 | -14.584 | 1.00 | 27.68 | C |
| ATOM | 1040 | OH | TYR B | 139 | 26.280 | 6.113 | -15.350 | 1.00 | 21.79 | O |
| ATOM | 1041 | CE2 | TYR B | 139 | 24.810 | 7.985 | -15.198 | 1.00 | 23.79 | C |
| ATOM | 1042 | CD2 | TYR B | 139 | 24.139 | 8.920 | -14.434 | 1.00 | 25.24 | C |
| ATOM | 1043 | C | TYR B | 139 | 21.581 | 10.398 | -10.646 | 1.00 | 19.87 | C |
| ATOM | 1044 | O | TYR B | 139 | 20.774 | 11.234 | -11.028 | 1.00 | 20.29 | O |
| ATOM | 1045 | N | PRO B | 140 | 21.934 | 10.282 | -9.361 | 1.00 | 17.20 | N |
| ATOM | 1046 | CA | PRO B | 140 | 21.344 | 11.139 | -8.325 | 1.00 | 19.39 | C |
| ATOM | 1047 | CB | PRO B | 140 | 21.859 | 10.522 | -7.019 | 1.00 | 18.37 | C |
| ATOM | 1048 | CG | PRO B | 140 | 23.122 | 9.840 | -7.383 | 1.00 | 19.97 | C |
| ATOM | 1049 | CD | PRO B | 140 | 22.870 | 9.293 | -8.795 | 1.00 | 20.06 | C |
| ATOM | 1050 | C | PRO B | 140 | 21.792 | 12.603 | -8.437 | 1.00 | 24.40 | C |
| ATOM | 1051 | O | PRO B | 140 | 22.997 | 12.891 | -8.540 | 1.00 | 22.56 | O |
| ATOM | 1052 | N | LEU B | 141 | 20.824 | 13.518 | -8.422 | 1.00 | 21.38 | N |
| ATOM | 1053 | CA | LEU B | 141 | 21.119 | 14.951 | -8.408 | 1.00 | 22.72 | C |
| ATOM | 1054 | CB | LEU B | 141 | 19.846 | 15.780 | -8.662 | 1.00 | 25.28 | C |
| ATOM | 1055 | CG | LEU B | 141 | 19.122 | 15.496 | -9.980 | 1.00 | 26.16 | C |
| ATOM | 1056 | CD1 | LEU B | 141 | 18.211 | 16.652 | -10.396 | 1.00 | 29.62 | C |
| ATOM | 1057 | CD2 | LEU B | 141 | 20.127 | 15.220 | -11.049 | 1.00 | 23.21 | C |
| ATOM | 1058 | C | LEU B | 141 | 21.771 | 15.364 | -7.092 | 1.00 | 23.82 | C |
| ATOM | 1059 | O | LEU B | 141 | 22.588 | 16.286 | -7.058 | 1.00 | 23.66 | O |
| ATOM | 1060 | N | THR B | 142 | 21.429 | 14.670 | -6.011 | 1.00 | 23.59 | N |
| ATOM | 1061 | CA | THR B | 142 | 22.015 | 14.976 | -4.702 | 1.00 | 24.72 | C |
| ATOM | 1062 | CB | THR B | 142 | 21.062 | 14.590 | -3.563 | 1.00 | 26.06 | C |
| ATOM | 1063 | OG1 | THR B | 142 | 19.811 | 15.258 | -3.763 | 1.00 | 25.73 | O |
| ATOM | 1064 | CG2 | THR B | 142 | 21.625 | 14.996 | -2.217 | 1.00 | 22.78 | C |
| ATOM | 1065 | C | THR B | 142 | 23.350 | 14.276 | -4.509 | 1.00 | 22.02 | C |
| ATOM | 1066 | O | THR B | 142 | 23.406 | 13.062 | -4.296 | 1.00 | 21.86 | O |
| ATOM | 1067 | N | PHE B | 143 | 24.433 | 15.046 | -4.573 | 1.00 | 20.29 | N |
| ATOM | 1068 | CA | PHE B | 143 | 25.755 | 14.508 | -4.340 | 1.00 | 20.95 | C |
| ATOM | 1069 | CB | PHE B | 143 | 26.809 | 15.616 | -4.489 | 1.00 | 22.14 | C |
| ATOM | 1070 | CG | PHE B | 143 | 28.229 | 15.121 | -4.489 | 1.00 | 21.29 | C |
| ATOM | 1071 | CD1 | PHE B | 143 | 28.543 | 13.844 | -4.938 | 1.00 | 21.74 | C |
| ATOM | 1072 | CE1 | PHE B | 143 | 29.858 | 13.391 | -4.936 | 1.00 | 21.79 | C |
| ATOM | 1073 | CZ | PHE B | 143 | 30.873 | 14.218 | -4.487 | 1.00 | 22.14 | C |
| ATOM | 1074 | CE2 | PHE B | 143 | 30.570 | 15.498 | -4.048 | 1.00 | 24.75 | C |
| ATOM | 1075 | CD2 | PHE B | 143 | 29.259 | 15.942 | -4.046 | 1.00 | 22.38 | C |
| ATOM | 1076 | C | PHE B | 143 | 25.782 | 13.914 | -2.933 | 1.00 | 22.04 | C |
| ATOM | 1077 | O | PHE B | 143 | 25.315 | 14.541 | -1.964 | 1.00 | 22.58 | O |
| ATOM | 1078 | N | GLY B | 144 | 26.301 | 12.696 | -2.815 | 1.00 | 21.35 | N |
| ATOM | 1079 | CA | GLY B | 144 | 26.300 | 12.017 | -1.527 | 1.00 | 23.31 | C |
| ATOM | 1080 | C | GLY B | 144 | 25.380 | 10.810 | -1.476 | 1.00 | 20.52 | C |
| ATOM | 1081 | O | GLY B | 144 | 25.669 | 9.846 | -0.772 | 1.00 | 20.24 | O |
| ATOM | 1082 | N | TRP B | 145 | 24.277 | 10.858 | -2.216 | 1.00 | 18.90 | N |
| ATOM | 1083 | CA | TRP B | 145 | 23.370 | 9.727 | -2.296 | 1.00 | 20.59 | C |
| ATOM | 1084 | CB | TRP B | 145 | 22.043 | 10.201 | -2.879 | 1.00 | 19.97 | C |
| ATOM | 1085 | CG | TRP B | 145 | 20.991 | 9.140 | -3.016 | 1.00 | 21.67 | C |
| ATOM | 1086 | CD1 | TRP B | 145 | 20.381 | 8.753 | -4.175 | 1.00 | 19.30 | C |
| ATOM | 1087 | NE1 | TRP B | 145 | 19.449 | 7.761 | -3.913 | 1.00 | 22.16 | N |
| ATOM | 1088 | CE2 | TRP B | 145 | 19.444 | 7.497 | -2.569 | 1.00 | 18.64 | C |
| ATOM | 1089 | CD2 | TRP B | 145 | 20.399 | 8.349 | -1.967 | 1.00 | 19.05 | C |
| ATOM | 1090 | CE3 | TRP B | 145 | 20.579 | 8.282 | -0.580 | 1.00 | 20.18 | C |
| ATOM | 1091 | CZ3 | TRP B | 145 | 19.817 | 7.376 | 0.147 | 1.00 | 20.33 | C |
| ATOM | 1092 | CH2 | TRP B | 145 | 18.881 | 6.547 | -0.478 | 1.00 | 21.66 | C |
| ATOM | 1093 | CZ2 | TRP B | 145 | 18.673 | 6.596 | -1.837 | 1.00 | 21.46 | C |
| ATOM | 1094 | C | TRP B | 145 | 24.026 | 8.688 | -3.189 | 1.00 | 22.75 | C |
| ATOM | 1095 | O | TRP B | 145 | 24.351 | 8.987 | -4.334 | 1.00 | 21.33 | O |
| ATOM | 1096 | N | CYS B | 146 | 24.260 | 7.487 | -2.669 | 1.00 | 19.49 | N |
| ATOM | 1097 | CA | CYS B | 146 | 25.068 | 6.513 | -3.405 | 1.00 | 21.50 | C |
| ATOM | 1098 | CB | CYS B | 146 | 26.099 | 5.859 | -2.481 | 1.00 | 22.35 | C |
| ATOM | 1099 | SG | CYS B | 146 | 27.408 | 7.028 | -1.960 | 1.00 | 24.08 | S |
| ATOM | 1100 | C | CYS B | 146 | 24.255 | 5.440 | -4.118 | 1.00 | 22.14 | C |
| ATOM | 1101 | O | CYS B | 146 | 24.729 | 4.320 | -4.294 | 1.00 | 20.77 | O |
| ATOM | 1102 | N | PHE B | 147 | 23.032 | 5.777 | -4.507 | 1.00 | 19.02 | N |
| ATOM | 1103 | CA | PHE B | 147 | 22.235 | 4.876 | -5.335 | 1.00 | 19.43 | C |
| ATOM | 1104 | CB | PHE B | 147 | 20.929 | 4.489 | -4.637 | 1.00 | 18.89 | C |
| ATOM | 1105 | CG | PHE B | 147 | 21.120 | 3.484 | -3.537 | 1.00 | 18.57 | C |
| ATOM | 1106 | CD2 | PHE B | 147 | 21.084 | 2.122 | -3.808 | 1.00 | 17.71 | C |
| ATOM | 1107 | CE2 | PHE B | 147 | 21.281 | 1.180 | -2.785 | 1.00 | 21.46 | C |
| ATOM | 1108 | CZ | PHE B | 147 | 21.536 | 1.612 | -1.495 | 1.00 | 22.02 | C |
| ATOM | 1109 | CE1 | PHE B | 147 | 21.582 | 2.972 | -1.214 | 1.00 | 16.93 | C |
| ATOM | 1110 | CD1 | PHE B | 147 | 21.379 | 3.902 | -2.242 | 1.00 | 22.01 | C |
| ATOM | 1111 | C | PHE B | 147 | 21.907 | 5.568 | -6.634 | 1.00 | 21.33 | C |
| ATOM | 1112 | O | PHE B | 147 | 21.669 | 6.780 | -6.660 | 1.00 | 17.74 | O |
| ATOM | 1113 | N | LYS B | 148 | 21.864 | 4.779 | -7.702 | 1.00 | 17.47 | N |
| ATOM | 1114 | CA | LYS B | 148 | 21.483 | 5.273 | -8.997 | 1.00 | 19.70 | C |
| ATOM | 1115 | CB | LYS B | 148 | 22.717 | 5.295 | -9.900 | 1.00 | 17.79 | C |
| ATOM | 1116 | CG | LYS B | 148 | 23.395 | 3.941 | -10.095 | 1.00 | 20.92 | C |
| ATOM | 1117 | CD | LYS B | 148 | 24.722 | 4.105 | -10.846 | 1.00 | 20.73 | C |
| ATOM | 1118 | CE | LYS B | 148 | 25.284 | 2.727 | -11.225 | 1.00 | 25.24 | C |
| ATOM | 1119 | NZ | LYS B | 148 | 26.712 | 2.821 | -11.652 | 1.00 | 23.45 | N |
| ATOM | 1120 | C | LYS B | 148 | 20.427 | 4.289 | -9.500 | 1.00 | 20.48 | C |
| ATOM | 1121 | O | LYS B | 148 | 20.231 | 3.226 | -8.897 | 1.00 | 20.62 | O |
| ATOM | 1122 | N | LEU B | 149 | 19.716 | 4.641 | -10.561 | 1.00 | 16.40 | N |
| ATOM | 1123 | CA | LEU B | 149 | 18.677 | 3.763 | -11.085 | 1.00 | 17.86 | C |
| ATOM | 1124 | CB | LEU B | 149 | 17.377 | 4.543 | -11.281 | 1.00 | 18.69 | C |
| ATOM | 1125 | CG | LEU B | 149 | 16.833 | 5.299 | -10.063 | 1.00 | 19.87 | C |
| ATOM | 1126 | CD1 | LEU B | 149 | 15.539 | 6.006 | -10.425 | 1.00 | 19.44 | C |
| ATOM | 1127 | CD2 | LEU B | 149 | 16.628 | 4.378 | -8.859 | 1.00 | 19.12 | C |
| ATOM | 1128 | C | LEU B | 149 | 19.163 | 3.178 | -12.420 | 1.00 | 22.88 | C |
| ATOM | 1129 | O | LEU B | 149 | 19.571 | 3.925 | -13.304 | 1.00 | 22.83 | O |
| ATOM | 1130 | N | VAL B | 150 | 19.114 | 1.856 | -12.557 | 1.00 | 20.76 | N |
| ATOM | 1131 | CA | VAL B | 150 | 19.678 | 1.175 | -13.728 | 1.00 | 22.72 | C |
| ATOM | 1132 | CB | VAL B | 150 | 20.780 | 0.201 | -13.284 | 1.00 | 24.96 | C |
| ATOM | 1133 | CG1 | VAL B | 150 | 21.322 | -0.592 | -14.475 | 1.00 | 31.62 | C |
| ATOM | 1134 | CG2 | VAL B | 150 | 21.911 | 0.951 | -12.555 | 1.00 | 24.77 | C |
| ATOM | 1135 | C | VAL B | 150 | 18.593 | 0.388 | -14.457 | 1.00 | 22.64 | C |
| ATOM | 1136 | O | VAL B | 150 | 17.784 | -0.292 | -13.817 | 1.00 | 23.11 | O |
| ATOM | 1137 | N | PRO B | 151 | 18.556 | 0.471 | -15.799 | 1.00 | 26.33 | N |
| ATOM | 1138 | CA | PRO B | 151 | 17.555 | -0.318 | -16.531 | 1.00 | 27.96 | C |
| ATOM | 1139 | CB | PRO B | 151 | 17.827 | 0.040 | -17.996 | 1.00 | 29.84 | C |
| ATOM | 1140 | CG | PRO B | 151 | 18.441 | 1.390 | -17.931 | 1.00 | 29.20 | C |
| ATOM | 1141 | CD | PRO B | 151 | 19.302 | 1.371 | -16.695 | 1.00 | 26.43 | C |
| ATOM | 1142 | C | PRO B | 151 | 17.757 | -1.808 | -16.298 | 1.00 | 29.19 | C |
| ATOM | 1143 | O | PRO B | 151 | 18.885 | -2.288 | -16.292 | 1.00 | 30.26 | O |
| ATOM | 1144 | N | VAL B | 152 | 16.663 | -2.521 | -16.072 | 1.00 | 35.42 | N |
| ATOM | 1145 | CA | VAL B | 152 | 16.734 | -3.940 | -15.765 | 1.00 | 38.22 | C |
| ATOM | 1146 | CB | VAL B | 152 | 15.887 | -4.272 | -14.510 | 1.00 | 33.20 | C |
| ATOM | 1147 | CG2 | VAL B | 152 | 16.261 | -5.628 | -13.937 | 1.00 | 38.64 | C |
| ATOM | 1148 | CG1 | VAL B | 152 | 14.409 | -4.200 | -14.816 | 1.00 | 33.56 | C |
| ATOM | 1149 | C | VAL B | 152 | 16.298 | -4.748 | -16.987 | 1.00 | 44.09 | C |
| ATOM | 1150 | O | VAL B | 152 | 16.526 | -5.956 | -17.059 | 1.00 | 42.93 | O |
| ATOM | 1151 | N | GLU B | 153 | 15.696 | -4.057 | -17.954 | 1.00 | 43.08 | N |
| ATOM | 1152 | CA | GLU B | 153 | 15.294 | -4.662 | -19.228 | 1.00 | 47.12 | C |
| ATOM | 1153 | C | GLU B | 153 | 15.969 | -3.999 | -20.427 | 1.00 | 53.69 | C |
| ATOM | 1154 | O | GLU B | 153 | 15.324 | -3.868 | -21.467 | 1.00 | 57.83 | O |
| ATOM | 1155 | CB | GLU B | 153 | 13.778 | -4.494 | -19.451 | 1.00 | 43.39 | C |
| ATOM | 1156 | CG | GLU B | 153 | 12.863 | -5.411 | -18.653 | 1.00 | 43.72 | C |
| ATOM | 1157 | CD | GLU B | 153 | 12.735 | -6.799 | -19.263 | 1.00 | 44.67 | C |
| ATOM | 1158 | OE1 | GLU B | 153 | 11.723 | -7.470 | -18.972 | 1.00 | 43.06 | O |
| ATOM | 1159 | OE2 | GLU B | 153 | 13.641 | -7.229 | -20.014 | 1.00 | 44.56 | O |
| ATOM | 1160 | O | PRO B | 154 | 17.526 | -4.230 | -23.076 | 1.00 | 61.10 | O |
| ATOM | 1161 | N | PRO B | 154 | 17.254 | -3.600 | -20.308 | 1.00 | 53.30 | N |
| ATOM | 1162 | CA | PRO B | 154 | 17.803 | -2.640 | -21.285 | 1.00 | 60.45 | C |
| ATOM | 1163 | C | PRO B | 154 | 17.591 | -3.032 | -22.763 | 1.00 | 63.79 | C |
| ATOM | 1164 | CB | PRO B | 154 | 19.302 | -2.601 | -20.931 | 1.00 | 52.63 | C |
| ATOM | 1165 | CG | PRO B | 154 | 19.580 | -3.923 | -20.320 | 1.00 | 54.76 | C |
| ATOM | 1166 | CD | PRO B | 154 | 18.328 | -4.255 | -19.534 | 1.00 | 54.61 | C |
| ATOM | 1167 | O | GLU B | 155 | 16.577 | -0.143 | -25.497 | 1.00 | 63.95 | O |
| ATOM | 1168 | N | GLU B | 155 | 17.463 | -2.051 | -23.658 | 1.00 | 64.11 | N |
| ATOM | 1169 | CA | GLU B | 155 | 17.526 | -0.625 | -23.341 | 1.00 | 60.63 | C |
| ATOM | 1170 | C | GLU B | 155 | 16.577 | 0.118 | -24.292 | 1.00 | 59.30 | C |
| ATOM | 1171 | CB | GLU B | 155 | 18.960 | -0.115 | -23.528 | 1.00 | 61.82 | C |
| ATOM | 1172 | CG | GLU B | 155 | 19.490 | 0.751 | -22.386 | 1.00 | 60.25 | C |
| ATOM | 1173 | CD | GLU B | 155 | 20.977 | 1.114 | -22.541 | 1.00 | 64.13 | C |
| ATOM | 1174 | OE1 | GLU B | 155 | 21.674 | 0.537 | -23.425 | 1.00 | 56.04 | O |
| ATOM | 1175 | OE2 | GLU B | 155 | 21.444 | 1.990 | -21.770 | 1.00 | 54.15 | O |
| ATOM | 1176 | N | LYS B | 156 | 15.773 | 1.039 | -23.764 | 1.00 | 56.41 | N |
| ATOM | 1177 | CA | LYS B | 156 | 14.730 | 1.687 | -24.569 | 1.00 | 50.72 | C |
| ATOM | 1178 | CB | LYS B | 156 | 13.374 | 1.613 | -23.864 | 1.00 | 51.42 | C |
| ATOM | 1179 | CG | LYS B | 156 | 12.694 | 0.258 | -23.937 | 1.00 | 54.05 | C |
| ATOM | 1180 | CD | LYS B | 156 | 11.712 | 0.095 | -22.781 | 1.00 | 50.26 | C |
| ATOM | 1181 | CE | LYS B | 156 | 11.208 | -1.343 | -22.678 | 1.00 | 54.54 | C |
| ATOM | 1182 | NZ | LYS B | 156 | 10.490 | -1.600 | -21.391 | 1.00 | 49.30 | N |
| ATOM | 1183 | C | LYS B | 156 | 15.002 | 3.139 | -24.967 | 1.00 | 57.63 | C |
| ATOM | 1184 | O | LYS B | 156 | 15.834 | 3.830 | -24.370 | 1.00 | 55.47 | O |
| ATOM | 1185 | N | VAL B | 157 | 14.268 | 3.581 | -25.988 | 1.00 | 59.12 | N |
| ATOM | 1186 | CA | VAL B | 157 | 14.334 | 4.941 | -26.521 | 1.00 | 59.88 | C |
| ATOM | 1187 | CB | VAL B | 157 | 13.759 | 4.980 | -27.965 | 1.00 | 60.17 | C |
| ATOM | 1188 | CG1 | VAL B | 157 | 13.634 | 6.416 | -28.480 | 1.00 | 58.69 | C |
| ATOM | 1189 | CG2 | VAL B | 157 | 14.611 | 4.125 | -28.904 | 1.00 | 58.35 | C |
| ATOM | 1190 | C | VAL B | 157 | 13.525 | 5.891 | -25.643 | 1.00 | 53.93 | C |
| ATOM | 1191 | O | VAL B | 157 | 13.948 | 7.014 | -25.353 | 1.00 | 55.46 | O |
| ATOM | 1192 | N | ASP B | 179 | 12.350 | 5.430 | -25.228 | 1.00 | 54.99 | N |
| ATOM | 1193 | CA | ASP B | 179 | 11.497 | 6.204 | -24.341 | 1.00 | 56.26 | C |
| ATOM | 1194 | CB | ASP B | 179 | 10.025 | 5.901 | -24.623 | 1.00 | 56.44 | C |
| ATOM | 1195 | CG | ASP B | 179 | 9.088 | 6.941 | -24.037 | 1.00 | 61.39 | C |
| ATOM | 1196 | OD1 | ASP B | 179 | 9.558 | 7.832 | -23.293 | 1.00 | 58.32 | O |
| ATOM | 1197 | OD2 | ASP B | 179 | 7.870 | 6.860 | -24.317 | 1.00 | 65.63 | O |
| ATOM | 1198 | C | ASP B | 179 | 11.851 | 5.890 | -22.879 | 1.00 | 55.57 | C |
| ATOM | 1199 | O | ASP B | 179 | 11.586 | 4.791 | -22.376 | 1.00 | 51.79 | O |
| ATOM | 1200 | N | ALA B | 180 | 12.457 | 6.867 | -22.208 | 1.00 | 51.63 | N |
| ATOM | 1201 | CA | ALA B | 180 | 12.878 | 6.711 | -20.823 | 1.00 | 48.32 | C |
| ATOM | 1202 | CB | ALA B | 180 | 13.601 | 7.969 | -20.355 | 1.00 | 39.49 | C |
| ATOM | 1203 | C | ALA B | 180 | 11.718 | 6.347 | -19.872 | 1.00 | 48.00 | C |
| ATOM | 1204 | O | ALA B | 180 | 11.935 | 5.703 | -18.842 | 1.00 | 49.02 | O |
| ATOM | 1205 | N | GLU B | 181 | 10.495 | 6.742 | -20.223 | 1.00 | 52.36 | N |
| ATOM | 1206 | CA | GLU B | 181 | 9.319 | 6.459 | -19.392 | 1.00 | 49.91 | C |
| ATOM | 1207 | CB | GLU B | 181 | 8.153 | 7.373 | -19.780 | 1.00 | 52.98 | C |
| ATOM | 1208 | CG | GLU B | 181 | 8.573 | 8.738 | -20.311 | 1.00 | 62.14 | C |
| ATOM | 1209 | CD | GLU B | 181 | 7.403 | 9.532 | -20.880 | 1.00 | 73.57 | C |
| ATOM | 1210 | OE1 | GLU B | 181 | 6.263 | 9.005 | -20.872 | 1.00 | 73.19 | O |
| ATOM | 1211 | OE2 | GLU B | 181 | 7.623 | 10.680 | -21.335 | 1.00 | 73.65 | O |
| ATOM | 1212 | C | GLU B | 181 | 8.865 | 5.001 | -19.488 | 1.00 | 45.66 | C |
| ATOM | 1213 | O | GLU B | 181 | 8.000 | 4.563 | -18.734 | 1.00 | 48.16 | O |
| ATOM | 1214 | N | LYS B | 182 | 9.432 | 4.259 - | 20.431 | 1.00 | 49.29 | N |
| ATOM | 1215 | CA | LYS B | 182 | 9.070 | 2.859 | -20.602 | 1.00 | 49.86 | C |
| ATOM | 1216 | CB | LYS B | 182 | 8.887 | 2.524 | -22.088 | 1.00 | 50.03 | C |
| ATOM | 1217 | CG | LYS B | 182 | 7.925 | 1.356 | -22.345 | 1.00 | 58.18 | C |
| ATOM | 1218 | CD | LYS B | 182 | 8.266 | 0.581 | -23.622 | 1.00 | 57.29 | C |
| ATOM | 1219 | CE | LYS B | 182 | 8.436 | 1.516 | -24.808 | 1.00 | 60.53 | C |
| ATOM | 1220 | NZ | LYS B | 182 | 7.254 | 2.418 | -24.946 | 1.00 | 68.21 | N |
| ATOM | 1221 | C | LYS B | 182 | 10.138 | 1.948 | -19.999 | 1.00 | 47.78 | C |
| ATOM | 1222 | O | LYS B | 182 | 9.949 | 0.732 | -19.922 | 1.00 | 43.06 | O |
| ATOM | 1223 | N | GLU B | 183 | 11.266 | 2.530 | -19.592 | 1.00 | 42.63 | N |
| ATOM | 1224 | CA | GLU B | 183 | 12.353 | 1.734 | -19.037 | 1.00 | 38.93 | C |
| ATOM | 1225 | CB | GLU B | 183 | 13.668 | 2.514 | -19.025 | 1.00 | 35.80 | C |
| ATOM | 1226 | CG | GLU B | 183 | 14.329 | 2.701 | -20.366 | 1.00 | 41.51 | C |
| ATOM | 1227 | CD | GLU B | 183 | 15.780 | 3.158 | -20.244 | 1.00 | 38.27 | C |
| ATOM | 1228 | OE1 | GLU B | 183 | 16.040 | 4.180 | -19.572 | 1.00 | 31.93 | O |
| ATOM | 1229 | OE2 | GLU B | 183 | 16.666 | 2.495 | -20.828 | 1.00 | 46.19 | O |
| ATOM | 1230 | C | GLU B | 183 | 12.007 | 1.337 | -17.615 | 1.00 | 32.81 | C |
| ATOM | 1231 | O | GLU B | 183 | 11.651 | 2.187 | -16.809 | 1.00 | 31.58 | O |
| ATOM | 1232 | N | VAL B | 184 | 12.132 | 0.052 | -17.303 | 1.00 | 33.75 | N |
| ATOM | 1233 | CA | VAL B | 184 | 11.984 | -0.405 | -15.924 | 1.00 | 29.80 | C |
| ATOM | 1234 | CB | VAL B | 184 | 11.466 | -1.844 | -15.861 | 1.00 | 31.97 | C |
| ATOM | 1235 | CG1 | VAL B | 184 | 11.180 | -2.228 | -14.424 | 1.00 | 25.12 | C |
| ATOM | 1236 | CG2 | VAL B | 184 | 10.196 | -1.967 | -16.693 | 1.00 | 31.51 | C |
| ATOM | 1237 | C | VAL B | 184 | 13.340 | -0.313 | -15.230 | 1.00 | 26.24 | C |
| ATOM | 1238 | O | VAL B | 184 | 14.343 | -0.757 | -15.786 | 1.00 | 27.73 | O |
| ATOM | 1239 | N | LEU B | 185 | 13.371 | 0.266 | -14.028 | 1.00 | 25.80 | N |
| ATOM | 1240 | CA | LEU B | 185 | 14.642 | 0.561 | -13.344 | 1.00 | 24.67 | C |
| ATOM | 1241 | CB | LEU B | 185 | 14.748 | 2.071 | -13.082 | 1.00 | 19.69 | C |
| ATOM | 1242 | CG | LEU B | 185 | 14.457 | 2.920 | -14.327 | 1.00 | 23.03 | C |
| ATOM | 1243 | CD1 | LEU B | 185 | 14.030 | 4.331 | -13.953 | 1.00 | 23.05 | C |
| ATOM | 1244 | CD2 | LEU B | 185 | 15.680 | 2.953 | -15.217 | 1.00 | 20.99 | C |
| ATOM | 1245 | C | LEU B | 185 | 14.748 | -0.189 | -12.027 | 1.00 | 20.15 | C |
| ATOM | 1246 | O | LEU B | 185 | 13.724 | -0.496 | -11.425 | 1.00 | 22.72 | O |
| ATOM | 1247 | N | VAL B | 186 | 15.970 | -0.499 | -11.585 | 1.00 | 17.04 | N |
| ATOM | 1248 | CA | VAL B | 186 | 16.185 | -0.974 | -10.207 | 1.00 | 22.98 | C |
| ATOM | 1249 | CB | VAL B | 186 | 16.785 | -2.398 | -10.145 | 1.00 | 22.19 | C |
| ATOM | 1250 | CG1 | VAL B | 186 | 15.775 | -3.428 | -10.594 | 1.00 | 22.57 | C |
| ATOM | 1251 | CG2 | VAL B | 186 | 18.083 | -2.483 | -10.967 | 1.00 | 22.39 | C |
| ATOM | 1252 | C | VAL B | 186 | 17.152 | -0.025 | -9.503 | 1.00 | 20.43 | C |
| ATOM | 1253 | O | VAL B | 186 | 17.974 | 0.610 | -10.160 | 1.00 | 19.95 | O |
| ATOM | 1254 | N | TRP B | 187 | 17.042 | 0.100 | -8.184 | 1.00 | 21.32 | N |
| ATOM | 1255 | CA | TRP B | 187 | 18.050 | 0.843 | -7.435 | 1.00 | 20.07 | C |
| ATOM | 1256 | CB | TRP B | 187 | 17.644 | 1.022 | -5.973 | 1.00 | 20.21 | C |
| ATOM | 1257 | CG | TRP B | 187 | 16.789 | 2.194 | -5.649 | 1.00 | 18.93 | C |
| ATOM | 1258 | CD1 | TRP B | 187 | 17.206 | 3.489 | -5.435 | 1.00 | 20.71 | C |
| ATOM | 1259 | NE1 | TRP B | 187 | 16.124 | 4.285 | -5.104 | 1.00 | 23.78 | N |
| ATOM | 1260 | CE2 | TRP B | 187 | 14.989 | 3.510 | -5.092 | 1.00 | 23.66 | C |
| ATOM | 1261 | CD2 | TRP B | 187 | 15.373 | 2.186 | -5.428 | 1.00 | 22.46 | C |
| ATOM | 1262 | CE3 | TRP B | 187 | 14.385 | 1.186 | -5.484 | 1.00 | 23.55 | C |
| ATOM | 1263 | CZ3 | TRP B | 187 | 13.067 | 1.539 | -5.212 | 1.00 | 23.72 | C |
| ATOM | 1264 | CH2 | TRP B | 187 | 12.723 | 2.860 | -4.874 | 1.00 | 22.22 | C |
| ATOM | 1265 | CZ2 | TRP B | 187 | 13.665 | 3.860 | -4.822 | 1.00 | 21.55 | C |
| ATOM | 1266 | C | TRP B | 187 | 19.341 | 0.039 | -7.460 | 1.00 | 21.60 | C |
| ATOM | 1267 | O | TRP B | 187 | 19.321 | -1.195 | -7.325 | 1.00 | 20.83 | O |
| ATOM | 1268 | N | ARG B | 188 | 20.467 | 0.738 | -7.548 | 1.00 | 16.37 | N |
| ATOM | 1269 | CA | ARG B | 188 | 21.768 | 0.088 | -7.559 | 1.00 | 20.86 | C |
| ATOM | 1270 | CB | ARG B | 188 | 22.273 | -0.036 | -8.995 | 1.00 | 26.58 | C |
| ATOM | 1271 | CG | ARG B | 188 | 23.711 | -0.544 | -9.094 | 1.00 | 32.98 | C |
| ATOM | 1272 | CD | ARG B | 188 | 23.789 | -2.067 | -9.023 | 1.00 | 35.89 | C |
| ATOM | 1273 | NE | ARG B | 188 | 23.251 | -2.706 | -10.222 | 1.00 | 33.94 | N |
| ATOM | 1274 | CZ | ARG B | 188 | 22.111 | -3.383 | -10.251 | 1.00 | 36.66 | C |
| ATOM | 1275 | NH1 | ARG B | 188 | 21.397 | -3.504 | -9.138 | 1.00 | 40.51 | N |
| ATOM | 1276 | NH2 | ARG B | 188 | 21.694 | -3.940 | -11.382 | 1.00 | 33.39 | N |
| ATOM | 1277 | C | ARG B | 188 | 22.751 | 0.936 | -6.776 | 1.00 | 21.45 | C |
| ATOM | 1278 | O | ARG B | 188 | 22.891 | 2.124 | -7.048 | 1.00 | 19.90 | O |
| ATOM | 1279 | N | PHE B | 189 | 23.429 | 0.333 | -5.807 | 1.00 | 18.41 | N |
| ATOM | 1280 | CA | PHE B | 189 | 24.387 | 1.070 | -4.995 | 1.00 | 21.26 | C |
| ATOM | 1281 | CB | PHE B | 189 | 24.675 | 0.296 | -3.696 | 1.00 | 18.17 | C |
| ATOM | 1282 | CG | PHE B | 189 | 25.733 | 0.931 | -2.812 | 1.00 | 22.84 | C |
| ATOM | 1283 | CD2 | PHE B | 189 | 27.061 | 0.512 | -2.877 | 1.00 | 21.99 | C |
| ATOM | 1284 | CE2 | PHE B | 189 | 28.041 | 1.094 | -2.048 | 1.00 | 25.61 | C |
| ATOM | 1285 | CZ | PHE B | 189 | 27.685 | 2.100 | -1.163 | 1.00 | 21.68 | C |
| ATOM | 1286 | CE1 | PHE B | 189 | 26.367 | 2.516 | -1.090 | 1.00 | 22.75 | C |
| ATOM | 1287 | CD1 | PHE B | 189 | 25.397 | 1.927 | -1.911 | 1.00 | 22.04 | C |
| ATOM | 1288 | C | PHE B | 189 | 25.646 | 1.266 | -5.821 | 1.00 | 21.64 | C |
| ATOM | 1289 | O | PHE B | 189 | 26.031 | 0.385 | -6.590 | 1.00 | 19.75 | O |
| ATOM | 1290 | N | ASP B | 190 | 26.260 | 2.439 | -5.712 | 1.00 | 20.97 | N |
| ATOM | 1291 | CA | ASP B | 190 | 27.540 | 2.688 | -6.381 | 1.00 | 25.16 | C |
| ATOM | 1292 | CB | ASP B | 190 | 27.328 | 3.436 | -7.702 | 1.00 | 20.69 | C |
| ATOM | 1293 | CG | ASP B | 190 | 28.616 | 3.572 | -8.526 | 1.00 | 27.01 | C |
| ATOM | 1294 | OD1 | ASP B | 190 | 29.734 | 3.481 | -7.954 | 1.00 | 22.82 | O |
| ATOM | 1295 | OD2 | ASP B | 190 | 28.507 | 3.781 | -9.763 | 1.00 | 25.91 | O |
| ATOM | 1296 | C | ASP B | 190 | 28.430 | 3.502 | -5.451 | 1.00 | 23.15 | C |
| ATOM | 1297 | O | ASP B | 190 | 28.181 | 4.692 | -5.247 | 1.00 | 19.09 | O |
| ATOM | 1298 | N | SER B | 191 | 29.452 | 2.859 | -4.876 | 1.00 | 23.19 | N |
| ATOM | 1299 | CA | SER B | 191 | 30.325 | 3.526 | -3.908 | 1.00 | 24.65 | C |
| ATOM | 1300 | CB | SER B | 191 | 31.404 | 2.563 | -3.390 | 1.00 | 25.94 | C |
| ATOM | 1301 | OG | SER B | 191 | 32.245 | 2.179 | -4.464 | 1.00 | 27.94 | O |
| ATOM | 1302 | C | SER B | 191 | 30.990 | 4.780 | -4.482 | 1.00 | 23.64 | C |
| ATOM | 1303 | O | SER B | 191 | 31.250 | 5.728 | -3.752 | 1.00 | 24.05 | O |
| ATOM | 1304 | N | LYS B | 192 | 31.251 | 4.793 | -5.787 | 1.00 | 21.90 | N |
| ATOM | 1305 | CA | LYS B | 192 | 31.972 | 5.909 | -6.403 | 1.00 | 22.16 | C |
| ATOM | 1306 | CB | LYS B | 192 | 32.411 | 5.525 | -7.822 | 1.00 | 25.84 | C |
| ATOM | 1307 | CG | LYS B | 192 | 33.483 | 4.446 | -7.828 | 1.00 | 29.14 | C |
| ATOM | 1308 | CD | LYS B | 192 | 33.274 | 3.426 | -8.948 | 1.00 | 37.98 | C |
| ATOM | 1309 | CE | LYS B | 192 | 33.330 | 4.070 | -10.316 | 1.00 | 38.44 | C |
| ATOM | 1310 | NZ | LYS B | 192 | 33.558 | 3.063 | -11.410 | 1.00 | 40.44 | N |
| ATOM | 1311 | C | LYS B | 192 | 31.163 | 7.204 | -6.419 | 1.00 | 22.85 | C |
| ATOM | 1312 | O | LYS B | 192 | 31.728 | 8.300 | -6.509 | 1.00 | 22.69 | O |
| ATOM | 1313 | N | LEU B | 193 | 29.842 | 7.087 | -6.324 | 1.00 | 20.52 | N |
| ATOM | 1314 | CA | LEU B | 193 | 28.979 | 8.271 | -6.237 | 1.00 | 20.61 | C |
| ATOM | 1315 | CB | LEU B | 193 | 27.499 | 7.875 | -6.286 | 1.00 | 21.14 | C |
| ATOM | 1316 | CG | LEU B | 193 | 26.992 | 7.182 | -7.553 | 1.00 | 21.39 | C |
| ATOM | 1317 | CD1 | LEU B | 193 | 25.553 | 6.692 | -7.373 | 1.00 | 21.41 | C |
| ATOM | 1318 | CD2 | LEU B | 193 | 27.109 | 8.092 | -8.769 | 1.00 | 25.32 | C |
| ATOM | 1319 | C | LEU B | 193 | 29.265 | 9.163 | -5.010 | 1.00 | 24.17 | C |
| ATOM | 1320 | O | LEU B | 193 | 28.835 | 10.318 | -4.980 | 1.00 | 23.71 | O |
| ATOM | 1321 | N | ALA B | 194 | 29.994 | 8.642 | -4.018 | 1.00 | 21.59 | N |
| ATOM | 1322 | CA | ALA B | 194 | 30.408 | 9.451 | -2.852 | 1.00 | 22.74 | C |
| ATOM | 1323 | CB | ALA B | 194 | 30.892 | 8.557 | -1.736 | 1.00 | 20.96 | C |
| ATOM | 1324 | C | ALA B | 194 | 31.493 | 10.467 | -3.200 | 1.00 | 24.14 | C |
| ATOM | 1325 | O | ALA B | 194 | 31.671 | 11.462 | -2.489 | 1.00 | 22.93 | O |
| ATOM | 1326 | N | PHE B | 195 | 32.210 | 10.212 | -4.291 | 1.00 | 24.05 | N |
| ATOM | 1327 | CA | PHE B | 195 | 33.381 | 11.012 | -4.677 | 1.00 | 23.49 | C |
| ATOM | 1328 | CB | PHE B | 195 | 34.627 | 10.118 | -4.789 | 1.00 | 23.25 | C |
| ATOM | 1329 | CG | PHE B | 195 | 34.960 | 9.389 | -3.519 | 1.00 | 25.05 | C |
| ATOM | 1330 | CD1 | PHE B | 195 | 35.678 | 10.019 | -2.507 | 1.00 | 27.94 | C |
| ATOM | 1331 | CE1 | PHE B | 195 | 35.967 | 9.346 | -1.324 | 1.00 | 25.66 | C |
| ATOM | 1332 | CZ | PHE B | 195 | 35.536 | 8.038 | -1.159 | 1.00 | 25.06 | C |
| ATOM | 1333 | CE2 | PHE B | 195 | 34.836 | 7.406 | -2.161 | 1.00 | 26.85 | C |
| ATOM | 1334 | CD2 | PHE B | 195 | 34.544 | 8.085 | -3.332 | 1.00 | 25.92 | C |
| ATOM | 1335 | C | PHE B | 195 | 33.165 | 11.705 | -6.013 | 1.00 | 25.63 | C |
| ATOM | 1336 | O | PHE B | 195 | 33.797 | 12.719 | -6.316 | 1.00 | 22.80 | O |
| ATOM | 1337 | N | HIS B | 196 | 32.280 | 11.142 | -6.820 | 1.00 | 22.47 | N |
| ATOM | 1338 | CA | HIS B | 196 | 32.084 | 11.612 | -8.187 | 1.00 | 26.14 | C |
| ATOM | 1339 | CB | HIS B | 196 | 32.373 | 10.471 | -9.166 | 1.00 | 27.60 | C |
| ATOM | 1340 | CG | HIS B | 196 | 33.745 | 9.885 | -9.023 | 1.00 | 30.84 | C |
| ATOM | 1341 | ND1 | HIS B | 196 | 34.091 | 8.653 | -9.536 | 1.00 | 35.66 | N |
| ATOM | 1342 | CE1 | HIS B | 196 | 35.360 | 8.404 | -9.272 | 1.00 | 34.02 | C |
| ATOM | 1343 | NE2 | HIS B | 196 | 35.853 | 9.427 | -8.602 | 1.00 | 33.98 | N |
| ATOM | 1344 | CD2 | HIS B | 196 | 34.861 | 10.361 | -8.421 | 1.00 | 35.63 | C |
| ATOM | 1345 | C | HIS B | 196 | 30.647 | 12.078 | -8.339 | 1.00 | 24.95 | C |
| ATOM | 1346 | O | HIS B | 196 | 29.717 | 11.288 | -8.187 | 1.00 | 21.61 | O |
| ATOM | 1347 | N | HIS B | 197 | 30.456 | 13.364 | -8.614 | 1.00 | 23.19 | N |
| ATOM | 1348 | CA | HIS B | 197 | 29.108 | 13.891 | -8.811 | 1.00 | 21.99 | C |
| ATOM | 1349 | CB | HIS B | 197 | 29.055 | 15.371 | -8.452 | 1.00 | 25.06 | C |
| ATOM | 1350 | CG | HIS B | 197 | 27.669 | 15.885 | -8.207 | 1.00 | 24.97 | C |
| ATOM | 1351 | ND1 | HIS B | 197 | 27.405 | 17.200 | -7.882 | 1.00 | 26.04 | N |
| ATOM | 1352 | CE1 | HIS B | 197 | 26.103 | 17.360 | -7.721 | 1.00 | 26.94 | C |
| ATOM | 1353 | NE2 | HIS B | 197 | 25.513 | 16.194 | -7.904 | 1.00 | 25.66 | N |
| ATOM | 1354 | CD2 | HIS B | 197 | 26.470 | 15.253 | -8.216 | 1.00 | 23.69 | C |
| ATOM | 1355 | C | HIS B | 197 | 28.719 | 13.703 | -10.263 | 1.00 | 25.37 | C |
| ATOM | 1356 | O | HIS B | 197 | 28.779 | 14.654 | -11.059 | 1.00 | 25.16 | O |
| ATOM | 1357 | N | MET B | 198 | 28.330 | 12.479 | -10.613 | 1.00 | 23.95 | N |
| ATOM | 1358 | CA | MET B | 198 | 28.090 | 12.121 | -12.016 | 1.00 | 24.27 | C |
| ATOM | 1359 | CB | MET B | 198 | 27.690 | 10.645 | -12.130 | 1.00 | 24.33 | C |
| ATOM | 1360 | CG | MET B | 198 | 28.785 | 9.703 | -11.593 | 1.00 | 25.94 | C |
| ATOM | 1361 | SD | MET B | 198 | 30.367 | 9.801 | -12.517 | 1.00 | 42.21 | S |
| ATOM | 1362 | CE | MET B | 198 | 30.147 | 8.462 | -13.697 | 1.00 | 33.34 | C |
| ATOM | 1363 | C | MET B | 198 | 27.029 | 12.999 | -12.650 | 1.00 | 24.69 | C |
| ATOM | 1364 | O | MET B | 198 | 27.169 | 13.435 | -13.791 | 1.00 | 26.82 | O |
| ATOM | 1365 | N | ALA B | 199 | 25.972 | 13.277 | -11.903 | 1.00 | 23.83 | N |
| ATOM | 1366 | CA | ALA B | 199 | 24.878 | 14.090 | -12.425 | 1.00 | 24.95 | C |
| ATOM | 1367 | CB | ALA B | 199 | 23.794 | 14.217 | -11.392 | 1.00 | 23.64 | C |
| ATOM | 1368 | C | ALA B | 199 | 25.354 | 15.476 | -12.871 | 1.00 | 26.01 | C |
| ATOM | 1369 | O | ALA B | 199 | 24.897 | 16.012 | -13.886 | 1.00 | 27.02 | O |
| ATOM | 1370 | N | ARG B | 200 | 26.276 | 16.046 | -12.109 | 1.00 | 24.32 | N |
| ATOM | 1371 | CA | ARG B | 200 | 26.749 | 17.399 | -12.360 | 1.00 | 29.28 | C |
| ATOM | 1372 | CB | ARG B | 200 | 27.484 | 17.920 | -11.136 | 1.00 | 30.68 | C |
| ATOM | 1373 | CG | ARG B | 200 | 27.825 | 19.394 | -11.202 | 1.00 | 35.27 | C |
| ATOM | 1374 | CD | ARG B | 200 | 28.950 | 19.699 | -10.233 | 1.00 | 42.65 | C |
| ATOM | 1375 | NE | ARG B | 200 | 28.651 | 20.843 | -9.384 | 1.00 | 48.88 | N |
| ATOM | 1376 | CZ | ARG B | 200 | 28.646 | 22.099 | -9.807 | 1.00 | 45.62 | C |
| ATOM | 1377 | NH1 | ARG B | 200 | 28.911 | 22.373 | -11.078 | 1.00 | 51.91 | N |
| ATOM | 1378 | NH2 | ARG B | 200 | 28.371 | 23.081 | -8.957 | 1.00 | 47.84 | N |
| ATOM | 1379 | C | ARG B | 200 | 27.685 | 17.410 | -13.556 | 1.00 | 33.26 | C |
| ATOM | 1380 | O | ARG B | 200 | 27.814 | 18.413 | -14.264 | 1.00 | 29.97 | O |
| ATOM | 1381 | N | GLU B | 201 | 28.349 | 16.289 | -13.791 | 1.00 | 28.49 | N |
| ATOM | 1382 | CA | GLU B | 201 | 29.183 | 16.221 | -14.976 | 1.00 | 32.34 | C |
| ATOM | 1383 | CB | GLU B | 201 | 30.280 | 15.219 | -14.767 | 1.00 | 32.08 | C |
| ATOM | 1384 | CG | GLU B | 201 | 31.358 | 15.885 | -14.010 | 1.00 | 39.64 | C |
| ATOM | 1385 | CD | GLU B | 201 | 32.333 | 14.913 | -13.568 | 1.00 | 44.42 | C |
| ATOM | 1386 | OE1 | GLU B | 201 | 33.503 | 15.052 | -13.970 | 1.00 | 44.10 | O |
| ATOM | 1387 | OE2 | GLU B | 201 | 31.897 | 13.990 | -12.848 | 1.00 | 44.79 | O |
| ATOM | 1388 | C | GLU B | 201 | 28.446 | 15.959 | -16.266 | 1.00 | 30.30 | C |
| ATOM | 1389 | O | GLU B | 201 | 28.881 | 16.399 | -17.329 | 1.00 | 31.03 | O |
| ATOM | 1390 | N | LEU B | 202 | 27.335 | 15.243 | -16.181 | 1.00 | 26.35 | N |
| ATOM | 1391 | CA | LEU B | 202 | 26.541 | 14.980 | -17.368 | 1.00 | 30.20 | C |
| ATOM | 1392 | CB | LEU B | 202 | 25.742 | 13.694 | -17.175 | 1.00 | 28.76 | C |
| ATOM | 1393 | CG | LEU B | 202 | 26.506 | 12.381 | -17.061 | 1.00 | 31.54 | C |
| ATOM | 1394 | CD1 | LEU B | 202 | 25.544 | 11.302 | -16.632 | 1.00 | 31.03 | C |
| ATOM | 1395 | CD2 | LEU B | 202 | 27.138 | 12.018 | -18.399 | 1.00 | 34.77 | C |
| ATOM | 1396 | C | LEU B | 202 | 25.584 | 16.135 | -17.629 | 1.00 | 32.35 | C |
| ATOM | 1397 | O | LEU B | 202 | 25.250 | 16.431 | -18.780 | 1.00 | 29.62 | O |
| ATOM | 1398 | N | HIS B | 203 | 25.133 | 16.777 | -16.550 | 1.00 | 29.53 | N |
| ATOM | 1399 | CA | HIS B | 203 | 24.137 | 17.841 | -16.652 | 1.00 | 28.69 | C |
| ATOM | 1400 | CB | HIS B | 203 | 22.761 | 17.315 | -16.236 | 1.00 | 31.57 | C |
| ATOM | 1401 | CG | HIS B | 203 | 22.212 | 16.259 | -17.148 | 1.00 | 32.04 | C |
| ATOM | 1402 | ND1 | HIS B | 203 | 21.489 | 16.562 | -18.286 | 1.00 | 32.80 | N |
| ATOM | 1403 | CE1 | HIS B | 203 | 21.130 | 15.443 | -18.888 | 1.00 | 33.94 | C |
| ATOM | 1404 | NE2 | HIS B | 203 | 21.589 | 14.423 | -18.184 | 1.00 | 33.39 | N |
| ATOM | 1405 | CD2 | HIS B | 203 | 22.265 | 14.907 | -17.087 | 1.00 | 29.87 | C |
| ATOM | 1406 | C | HIS B | 203 | 24.506 | 19.051 | -15.800 | 1.00 | 32.53 | C |
| ATOM | 1407 | O | HIS B | 203 | 23.827 | 19.360 | -14.830 | 1.00 | 33.46 | O |
| ATOM | 1408 | N | PRO B | 204 | 25.579 | 19.758 | -16.177 | 1.00 | 35.26 | N |
| ATOM | 1409 | CA | PRO B | 204 | 26.040 | 20.904 | -15.385 | 1.00 | 35.70 | C |
| ATOM | 1410 | CB | PRO B | 204 | 27.312 | 21.350 | -16.117 | 1.00 | 35.44 | C |
| ATOM | 1411 | CG | PRO B | 204 | 27.176 | 20.801 | -17.506 | 1.00 | 36.65 | C |
| ATOM | 1412 | CD | PRO B | 204 | 26.400 | 19.531 | -17.378 | 1.00 | 30.83 | C |
| ATOM | 1413 | C | PRO B | 204 | 25.003 | 22.041 | -15.317 | 1.00 | 39.02 | C |
| ATOM | 1414 | O | PRO B | 204 | 25.048 | 22.845 | -14.386 | 1.00 | 40.83 | O |
| ATOM | 1415 | N | GLU B | 205 | 24.080 | 22.084 | -16.275 | 1.00 | 40.37 | N |
| ATOM | 1416 | CA | GLU B | 205 | 23.028 | 23.098 | -16.304 | 1.00 | 40.09 | C |
| ATOM | 1417 | CB | GLU B | 205 | 22.212 | 22.992 | -17.601 | 1.00 | 39.54 | C |
| ATOM | 1418 | CG | GLU B | 205 | 21.278 | 21.782 | -17.709 | 1.00 | 39.82 | C |
| ATOM | 1419 | CD | GLU B | 205 | 21.931 | 20.560 | -18.360 | 1.00 | 41.02 | C |
| ATOM | 1420 | OE1 | GLU B | 205 | 23.172 | 20.556 | -18.550 | 1.00 | 40.42 | O |
| ATOM | 1421 | OE2 | GLU B | 205 | 21.199 | 19.593 | -18.668 | 1.00 | 38.49 | O |
| ATOM | 1422 | C | GLU B | 205 | 22.107 | 23.060 | -15.078 | 1.00 | 41.27 | C |
| ATOM | 1423 | O | GLU B | 205 | 21.477 | 24.066 | -14.739 | 1.00 | 44.45 | O |
| ATOM | 1424 | N | TYR B | 206 | 22.037 | 21.915 | -14.402 | 1.00 | 39.43 | N |
| ATOM | 1425 | CA | TYR B | 206 | 21.213 | 21.789 | -13.201 | 1.00 | 40.57 | C |
| ATOM | 1426 | CB | TYR B | 206 | 21.031 | 20.321 | -12.798 | 1.00 | 36.72 | C |
| ATOM | 1427 | CG | TYR B | 206 | 20.303 | 19.466 | -13.803 | 1.00 | 41.02 | C |
| ATOM | 1428 | CD1 | TYR B | 206 | 19.609 | 20.034 | -14.863 | 1.00 | 39.07 | C |
| ATOM | 1429 | CE1 | TYR B | 206 | 18.943 | 19.245 | -15.779 | 1.00 | 39.47 | C |
| ATOM | 1430 | CZ | TYR B | 206 | 18.971 | 17.873 | -15.639 | 1.00 | 38.15 | C |
| ATOM | 1431 | OH | TYR B | 206 | 18.319 | 17.077 | -16.549 | 1.00 | 38.80 | O |
| ATOM | 1432 | CE2 | TYR B | 206 | 19.648 | 17.292 | -14.598 | 1.00 | 32.11 | C |
| ATOM | 1433 | CD2 | TYR B | 206 | 20.310 | 18.082 | -13.689 | 1.00 | 35.85 | C |
| ATOM | 1434 | C | TYR B | 206 | 21.853 | 22.513 | -12.031 | 1.00 | 39.72 | C |
| ATOM | 1435 | O | TYR B | 206 | 21.232 | 22.685 | -10.987 | 1.00 | 40.04 | O |
| ATOM | 1436 | N | TYR B | 207 | 23.110 | 22.906 | -12.200 | 1.00 | 42.29 | N |
| ATOM | 1437 | CA | TYR B | 207 | 23.911 | 23.389 | -11.085 | 1.00 | 40.84 | C |
| ATOM | 1438 | CB | TYR B | 207 | 25.026 | 22.377 | -10.785 | 1.00 | 39.54 | C |
| ATOM | 1439 | CG | TYR B | 207 | 24.487 | 20.991 | -10.451 | 1.00 | 36.50 | C |
| ATOM | 1440 | CD1 | TYR B | 207 | 24.170 | 20.639 | -9.137 | 1.00 | 36.63 | C |
| ATOM | 1441 | CE1 | TYR B | 207 | 23.662 | 19.368 | -8.825 | 1.00 | 32.57 | C |
| ATOM | 1442 | CZ | TYR B | 207 | 23.469 | 18.447 | -9.839 | 1.00 | 32.58 | C |
| ATOM | 1443 | OH | TYR B | 207 | 22.963 | 17.194 | -9.555 | 1.00 | 26.79 | O |
| ATOM | 1444 | CE2 | TYR B | 207 | 23.769 | 18.776 | -11.152 | 1.00 | 32.86 | C |
| ATOM | 1445 | CD2 | TYR B | 207 | 24.274 | 20.045 | -11.451 | 1.00 | 36.51 | C |
| ATOM | 1446 | C | TYR B | 207 | 24.471 | 24.787 | -11.359 | 1.00 | 45.15 | C |
| ATOM | 1447 | O | TYR B | 207 | 25.471 | 24.945 | -12.061 | 1.00 | 49.69 | O |
| ATOM | 1448 | N | LYS B | 208 | 23.814 | 25.803 | -10.811 | 1.00 | 52.52 | N |
| ATOM | 1449 | CA | LYS B | 208 | 24.229 | 27.191 | -11.030 | 1.00 | 54.34 | C |
| ATOM | 1450 | CB | LYS B | 208 | 23.403 | 27.837 | -12.153 | 1.00 | 54.88 | C |
| ATOM | 1451 | CG | LYS B | 208 | 23.600 | 27.188 | -13.513 | 1.00 | 51.86 | C |
| ATOM | 1452 | CD | LYS B | 208 | 23.084 | 28.065 | -14.633 | 1.00 | 58.50 | C |
| ATOM | 1453 | CE | LYS B | 208 | 21.570 | 28.019 | -14.766 | 1.00 | 59.98 | C |
| ATOM | 1454 | NZ | LYS B | 208 | 21.112 | 28.830 | -15.940 | 1.00 | 59.54 | N |
| ATOM | 1455 | C | LYS B | 208 | 24.114 | 28.012 | -9.751 | 1.00 | 55.64 | C |
| ATOM | 1456 | O | LYS B | 208 | 24.964 | 27.922 | -8.864 | 1.00 | 61.14 | O |
| TER | 1458 | | LYS B | 208 | | | | | | |
| ATOM | 1457 | N | GLN C | 5 | 36.332 | -13.536 | -0.869 | 1.00 | 63.11 | N |
| ATOM | 1458 | CA | GLN C | 5 | 36.491 | -12.097 | -1.068 | 1.00 | 62.48 | C |
| ATOM | 1459 | CB | GLN C | 5 | 35.570 | -11.601 | -2.187 | 1.00 | 64.32 | C |
| ATOM | 1460 | CG | GLN C | 5 | 35.878 | -12.160 | -3.565 | 1.00 | 63.75 | C |
| ATOM | 1461 | CD | GLN C | 5 | 34.959 | -11.589 | -4.633 | 1.00 | 71.76 | C |
| ATOM | 1462 | OE1 | GLN C | 5 | 34.068 | -10.787 | -4.341 | 1.00 | 72.50 | O |
| ATOM | 1463 | NE2 | GLN C | 5 | 35.169 | -12.003 | -5.878 | 1.00 | 71.08 | N |
| ATOM | 1464 | C | GLN C | 5 | 36.222 | -11.279 | 0.200 | 1.00 | 61.11 | C |
| ATOM | 1465 | O | GLN C | 5 | 35.479 | -11.699 | 1.095 | 1.00 | 56.25 | O |
| ATOM | 1466 | N | LEU C | 6 | 36.831 | -10.099 | 0.256 | 1.00 | 56.42 | N |
| ATOM | 1467 | CA | LEU C | 6 | 36.595 | -9.149 | 1.338 | 1.00 | 48.94 | C |
| ATOM | 1468 | CB | LEU C | 6 | 37.851 | -9.006 | 2.204 | 1.00 | 43.68 | C |
| ATOM | 1469 | CG | LEU C | 6 | 38.152 | -10.139 | 3.194 | 1.00 | 46.68 | C |
| ATOM | 1470 | CD1 | LEU C | 6 | 39.559 | -10.022 | 3.770 | 1.00 | 41.19 | C |
| ATOM | 1471 | CD2 | LEU C | 6 | 37.123 | -10.141 | 4.312 | 1.00 | 41.80 | C |
| ATOM | 1472 | C | LEU C | 6 | 36.227 | -7.809 | 0.704 | 1.00 | 43.48 | C |
| ATOM | 1473 | O | LEU C | 6 | 37.072 | -7.172 | 0.067 | 1.00 | 45.84 | O |
| ATOM | 1474 | N | VAL C | 7 | 34.970 | -7.393 | 0.845 | 1.00 | 37.90 | N |
| ATOM | 1475 | CA | VAL C | 7 | 34.541 | -6.144 | 0.203 | 1.00 | 44.07 | C |
| ATOM | 1476 | CB | VAL C | 7 | 33.525 | -6.380 | -0.951 | 1.00 | 42.22 | C |
| ATOM | 1477 | CG1 | VAL C | 7 | 33.180 | -5.058 | -1.626 | 1.00 | 39.56 | C |
| ATOM | 1478 | CG2 | VAL C | 7 | 34.116 | -7.343 | -1.972 | 1.00 | 41.42 | C |
| ATOM | 1479 | C | VAL C | 7 | 34.043 | -5.091 | 1.197 | 1.00 | 33.24 | C |
| ATOM | 1480 | O | VAL C | 7 | 32.980 | -5.227 | 1.803 | 1.00 | 33.13 | O |
| ATOM | 1481 | N | GLU C | 8 | 34.845 | -4.047 | 1.356 | 1.00 | 33.99 | N |
| ATOM | 1482 | CA | GLU C | 8 | 34.548 | -2.990 | 2.308 | 1.00 | 32.20 | C |
| ATOM | 1483 | CB | GLU C | 8 | 35.852 | -2.406 | 2.853 | 1.00 | 32.64 | C |
| ATOM | 1484 | CG | GLU C | 8 | 36.820 | -3.445 | 3.404 | 1.00 | 29.81 | C |
| ATOM | 1485 | CD | GLU C | 8 | 37.746 | -4.014 | 2.333 | 1.00 | 35.57 | C |
| ATOM | 1486 | OE1 | GLU C | 8 | 37.448 | -3.876 | 1.120 | 1.00 | 36.44 | O |
| ATOM | 1487 | OE2 | GLU C | 8 | 38.785 | -4.595 | 2.707 | 1.00 | 36.76 | O |
| ATOM | 1488 | C | GLU C | 8 | 33.751 | -1.883 | 1.643 | 1.00 | 31.51 | C |
| ATOM | 1489 | O | GLU C | 8 | 33.920 | -1.621 | 0.450 | 1.00 | 28.02 | O |
| ATOM | 1490 | N | SER C | 9 | 32.885 | -1.230 | 2.410 | 1.00 | 27.24 | N |
| ATOM | 1491 | CA | SER C | 9 | 32.234 | -0.017 | 1.924 | 1.00 | 27.13 | C |
| ATOM | 1492 | CB | SER C | 9 | 30.907 | -0.357 | 1.244 | 1.00 | 29.84 | C |
| ATOM | 1493 | OG | SER C | 9 | 30.026 | -1.025 | 2.129 | 1.00 | 30.96 | O |
| ATOM | 1494 | C | SER C | 9 | 32.017 | 0.983 | 3.062 | 1.00 | 30.00 | C |
| ATOM | 1495 | O | SER C | 9 | 32.259 | 0.668 | 4.241 | 1.00 | 25.10 | O |
| ATOM | 1496 | N | GLY C | 10 | 31.565 | 2.183 | 2.712 | 1.00 | 22.76 | N |
| ATOM | 1497 | CA | GLY C | 10 | 31.321 | 3.207 | 3.715 | 1.00 | 23.61 | C |
| ATOM | 1498 | C | GLY C | 10 | 32.153 | 4.452 | 3.487 | 1.00 | 24.19 | C |
| ATOM | 1499 | O | GLY C | 10 | 31.937 | 5.478 | 4.133 | 1.00 | 25.39 | O |
| ATOM | 1500 | N | GLY C | 11 | 33.095 | 4.382 | 2.555 | 1.00 | 24.12 | N |
| ATOM | 1501 | CA | GLY C | 11 | 33.993 | 5.504 | 2.326 | 1.00 | 25.42 | C |
| ATOM | 1502 | C | GLY C | 11 | 33.286 | 6.725 | 1.745 | 1.00 | 27.21 | C |
| ATOM | 1503 | O | GLY C | 11 | 32.195 | 6.608 | 1.173 | 1.00 | 27.69 | O |
| ATOM | 1504 | N | GLY C | 12 | 33.894 | 7.901 | 1.895 | 1.00 | 26.94 | N |
| ATOM | 1505 | CA | GLY C | 12 | 33.331 | 9.124 | 1.333 | 1.00 | 25.58 | C |
| ATOM | 1506 | C | GLY C | 12 | 34.126 | 10.355 | 1.742 | 1.00 | 29.04 | C |
| ATOM | 1507 | O | GLY C | 12 | 35.249 | 10.226 | 2.238 | 1.00 | 28.49 | O |
| ATOM | 1508 | N | LEU C | 13 | 33.542 | 11.539 | 1.540 | 1.00 | 24.28 | N |
| ATOM | 1509 | CA | LEU C | 13 | 34.169 | 12.814 | 1.892 | 1.00 | 25.75 | C |
| ATOM | 1510 | CB | LEU C | 13 | 33.830 | 13.863 | 0.832 | 1.00 | 27.66 | C |
| ATOM | 1511 | CG | LEU C | 13 | 34.197 | 13.493 | -0.611 | 1.00 | 29.14 | C |
| ATOM | 1512 | CD1 | LEU C | 13 | 33.657 | 14.511 | -1.591 | 1.00 | 25.68 | C |
| ATOM | 1513 | CD2 | LEU C | 13 | 35.715 | 13.377 | -0.771 | 1.00 | 31.90 | C |
| ATOM | 1514 | C | LEU C | 13 | 33.675 | 13.270 | 3.265 | 1.00 | 28.69 | C |
| ATOM | 1515 | O | LEU C | 13 | 32.472 | 13.276 | 3.528 | 1.00 | 26.08 | O |
| ATOM | 1516 | N | VAL C | 14 | 34.596 | 13.613 | 4.163 | 1.00 | 28.30 | N |
| ATOM | 1517 | CA | VAL C | 14 | 34.222 | 13.942 | 5.536 | 1.00 | 30.27 | C |
| ATOM | 1518 | CB | VAL C | 14 | 34.725 | 12.891 | 6.535 | 1.00 | 32.55 | C |
| ATOM | 1519 | CG1 | VAL C | 14 | 34.085 | 13.125 | 7.897 | 1.00 | 31.40 | C |
| ATOM | 1520 | CG2 | VAL C | 14 | 34.436 | 11.479 | 6.045 | 1.00 | 32.61 | C |
| ATOM | 1521 | C | VAL C | 14 | 34.860 | 15.259 | 5.957 | 1.00 | 37.86 | C |
| ATOM | 1522 | O | VAL C | 14 | 36.020 | 15.511 | 5.638 | 1.00 | 35.44 | O |
| ATOM | 1523 | N | GLN C | 15 | 34.116 | 16.090 | 6.682 | 1.00 | 34.12 | N |
| ATOM | 1524 | CA | GLN C | 15 | 34.693 | 17.310 | 7.230 | 1.00 | 39.82 | C |
| ATOM | 1525 | CB | GLN C | 15 | 33.606 | 18.366 | 7.460 | 1.00 | 43.45 | C |
| ATOM | 1526 | CG | GLN C | 15 | 33.355 | 19.268 | 6.245 | 1.00 | 47.50 | C |
| ATOM | 1527 | CD | GLN C | 15 | 34.168 | 20.564 | 6.280 | 1.00 | 55.35 | C |
| ATOM | 1528 | OE1 | GLN C | 15 | 34.750 | 20.920 | 7.309 | 1.00 | 49.48 | O |
| ATOM | 1529 | NE2 | GLN C | 15 | 34.205 | 21.275 | 5.151 | 1.00 | 53.55 | N |
| ATOM | 1530 | C | GLN C | 15 | 35.427 | 16.989 | 8.532 | 1.00 | 36.42 | C |
| ATOM | 1531 | O | GLN C | 15 | 35.012 | 16.094 | 9.269 | 1.00 | 35.51 | O |
| ATOM | 1532 | N | ALA C | 16 | 36.520 | 17.704 | 8.804 | 1.00 | 40.38 | N |
| ATOM | 1533 | CA | ALA C | 16 | 37.285 | 17.489 | 10.036 | 1.00 | 41.70 | C |
| ATOM | 1534 | CB | ALA C | 16 | 38.397 | 18.522 | 10.166 | 1.00 | 45.06 | C |
| ATOM | 1535 | C | ALA C | 16 | 36.361 | 17.537 | 11.245 | 1.00 | 39.93 | C |
| ATOM | 1536 | O | ALA C | 16 | 35.552 | 18.449 | 11.375 | 1.00 | 47.43 | O |
| ATOM | 1537 | N | GLY C | 17 | 36.451 | 16.532 | 12.106 | 1.00 | 35.35 | N |
| ATOM | 1538 | CA | GLY C | 17 | 35.569 | 16.442 | 13.256 | 1.00 | 34.83 | C |
| ATOM | 1539 | C | GLY C | 17 | 34.341 | 15.585 | 13.010 | 1.00 | 37.19 | C |
| ATOM | 1540 | O | GLY C | 17 | 33.601 | 15.260 | 13.948 | 1.00 | 36.57 | O |
| ATOM | 1541 | N | GLY C | 18 | 34.110 | 15.216 | 11.749 | 1.00 | 35.45 | N |
| ATOM | 1542 | CA | GLY C | 18 | 32.962 | 14.393 | 11.408 | 1.00 | 28.79 | C |
| ATOM | 1543 | C | GLY C | 18 | 33.176 | 12.911 | 11.645 | 1.00 | 32.77 | C |
| ATOM | 1544 | O | GLY C | 18 | 34.260 | 12.492 | 12.053 | 1.00 | 35.17 | O |
| ATOM | 1545 | N | SER C | 19 | 32.142 | 12.116 | 11.377 | 1.00 | 27.68 | N |
| ATOM | 1546 | CA | SER C | 19 | 32.171 | 10.678 | 11.621 | 1.00 | 28.88 | C |
| ATOM | 1547 | CB | SER C | 19 | 31.100 | 10.293 | 12.639 | 1.00 | 32.25 | C |
| ATOM | 1548 | OG | SER C | 19 | 31.507 | 10.612 | 13.958 | 1.00 | 40.77 | O |
| ATOM | 1549 | C | SER C | 19 | 31.914 | 9.890 | 10.347 | 1.00 | 29.55 | C |
| ATOM | 1550 | O | SER C | 19 | 31.388 | 10.425 | 9.369 | 1.00 | 24.62 | O |
| ATOM | 1551 | N | LEU C | 20 | 32.254 | 8.607 | 10.388 | 1.00 | 27.78 | N |
| ATOM | 1552 | CA | LEU C | 20 | 31.993 | 7.702 | 9.273 | 1.00 | 28.54 | C |
| ATOM | 1553 | CB | LEU C | 20 | 33.076 | 7.862 | 8.207 | 1.00 | 26.87 | C |
| ATOM | 1554 | CG | LEU C | 20 | 32.780 | 7.561 | 6.744 | 1.00 | 32.72 | C |
| ATOM | 1555 | CD1 | LEU C | 20 | 31.681 | 8.478 | 6.189 | 1.00 | 27.00 | C |
| ATOM | 1556 | CD2 | LEU C | 20 | 34.069 | 7.710 | 5.944 | 1.00 | 28.05 | C |
| ATOM | 1557 | C | LEU C | 20 | 31.961 | 6.275 | 9.811 | 1.00 | 30.06 | C |
| ATOM | 1558 | O | LEU C | 20 | 32.701 | 5.933 | 10.749 | 1.00 | 29.86 | O |
| ATOM | 1559 | N | ARG C | 21 | 31.094 | 5.446 | 9.240 | 1.00 | 24.57 | N |
| ATOM | 1560 | CA | ARG C | 21 | 31.025 | 4.057 | 9.634 | 1.00 | 23.43 | C |
| ATOM | 1561 | CB | ARG C | 21 | 29.662 | 3.740 | 10.260 | 1.00 | 31.87 | C |
| ATOM | 1562 | CG | ARG C | 21 | 29.519 | 2.292 | 10.715 | 1.00 | 33.77 | C |
| ATOM | 1563 | CD | ARG C | 21 | 28.202 | 2.082 | 11.433 | 1.00 | 38.32 | C |
| ATOM | 1564 | NE | ARG C | 21 | 28.052 | 0.719 | 11.938 | 1.00 | 41.61 | N |
| ATOM | 1565 | CZ | ARG C | 21 | 28.210 | 0.366 | 13.209 | 1.00 | 37.11 | C |
| ATOM | 1566 | NH1 | ARG C | 21 | 28.534 | 1.275 | 14.129 | 1.00 | 35.42 | N |
| ATOM | 1567 | NH2 | ARG C | 21 | 28.041 | -0.903 | 13.557 | 1.00 | 43.60 | N |
| ATOM | 1568 | C | ARG C | 21 | 31.307 | 3.138 | 8.460 | 1.00 | 29.37 | C |
| ATOM | 1569 | O | ARG C | 21 | 30.651 | 3.216 | 7.407 | 1.00 | 26.72 | O |
| ATOM | 1570 | N | LEU C | 22 | 32.303 | 2.277 | 8.646 | 1.00 | 23.97 | N |
| ATOM | 1571 | CA | LEU C | 22 | 32.768 | 1.384 | 7.598 | 1.00 | 25.61 | C |
| ATOM | 1572 | CB | LEU C | 22 | 34.305 | 1.376 | 7.564 | 1.00 | 24.94 | C |
| ATOM | 1573 | CG | LEU C | 22 | 35.046 | 2.311 | 6.607 | 1.00 | 32.22 | C |
| ATOM | 1574 | CD1 | LEU C | 22 | 34.318 | 3.624 | 6.349 | 1.00 | 24.83 | C |
| ATOM | 1575 | CD2 | LEU C | 22 | 36.474 | 2.540 | 7.087 | 1.00 | 24.41 | C |
| ATOM | 1576 | C | LEU C | 22 | 32.250 | -0.022 | 7.849 | 1.00 | 26.37 | C |
| ATOM | 1577 | O | LEU C | 22 | 31.959 | -0.412 | 8.984 | 1.00 | 26.96 | O |
| ATOM | 1578 | N | PHE C | 23 | 32.202 | -0.811 | 6.790 | 1.00 | 25.76 | N |
| ATOM | 1579 | CA | PHE C | 23 | 31.528 | -2.095 | 6.840 | 1.00 | 30.92 | C |
| ATOM | 1580 | CB | PHE C | 23 | 30.117 | -1.848 | 6.322 | 1.00 | 37.95 | C |
| ATOM | 1581 | CG | PHE C | 23 | 29.274 | -3.061 | 6.227 | 1.00 | 37.21 | C |
| ATOM | 1582 | CD1 | PHE C | 23 | 28.715 | -3.623 | 7.366 | 1.00 | 43.43 | C |
| ATOM | 1583 | CE1 | PHE C | 23 | 27.892 | -4.735 | 7.273 | 1.00 | 43.38 | C |
| ATOM | 1584 | CZ | PHE C | 23 | 27.622 | -5.283 | 6.028 | 1.00 | 39.47 | C |
| ATOM | 1585 | CE2 | PHE C | 23 | 28.172 | -4.714 | 4.886 | 1.00 | 40.77 | C |
| ATOM | 1586 | CD2 | PHE C | 23 | 28.979 | -3.604 | 4.994 | 1.00 | 38.96 | C |
| ATOM | 1587 | C | PHE C | 23 | 32.258 | -3.070 | 5.931 | 1.00 | 31.26 | C |
| ATOM | 1588 | O | PHE C | 23 | 32.707 | -2.677 | 4.861 | 1.00 | 29.69 | O |
| ATOM | 1589 | N | CYS C | 24 | 32.398 | -4.332 | 6.336 | 1.00 | 31.44 | N |
| ATOM | 1590 | CA | CYS C | 24 | 32.990 | -5.317 | 5.427 | 1.00 | 34.47 | C |
| ATOM | 1591 | CB | CYS C | 24 | 34.422 | -5.690 | 5.825 | 1.00 | 30.83 | C |
| ATOM | 1592 | SG | CYS C | 24 | 35.243 | -6.877 | 4.690 | 1.00 | 39.50 | S |
| ATOM | 1593 | C | CYS C | 24 | 32.139 | -6.570 | 5.356 | 1.00 | 38.46 | C |
| ATOM | 1594 | O | CYS C | 24 | 31.676 | -7.066 | 6.378 | 1.00 | 37.54 | O |
| ATOM | 1595 | N | ALA C | 25 | 31.925 | -7.053 | 4.136 | 1.00 | 43.70 | N |
| ATOM | 1596 | CA | ALA C | 25 | 31.244 | -8.325 | 3.892 | 1.00 | 44.94 | C |
| ATOM | 1597 | CB | ALA C | 25 | 30.140 | -8.147 | 2.859 | 1.00 | 49.56 | C |
| ATOM | 1598 | C | ALA C | 25 | 32.269 | -9.343 | 3.404 | 1.00 | 42.98 | C |
| ATOM | 1599 | O | ALA C | 25 | 32.961 | -9.113 | 2.397 | 1.00 | 46.32 | O |
| ATOM | 1600 | N | ALA C | 26 | 32.354 | -10.470 | 4.111 | 1.00 | 54.41 | N |
| ATOM | 1601 | CA | ALA C | 26 | 33.442 | -11.434 | 3.922 | 1.00 | 55.47 | C |
| ATOM | 1602 | CB | ALA C | 26 | 34.106 | -11.723 | 5.256 | 1.00 | 57.80 | C |
| ATOM | 1603 | C | ALA C | 26 | 33.006 | -12.748 | 3.263 | 1.00 | 58.65 | C |
| ATOM | 1604 | O | ALA C | 26 | 32.048 | -13.389 | 3.709 | 1.00 | 56.00 | O |
| ATOM | 1605 | O | SER C | 27 | 35.794 | -14.905 | 1.359 | 1.00 | 62.45 | O |
| ATOM | 1606 | N | SER C | 27 | 33.736 | -13.163 | 2.225 | 1.00 | 62.23 | N |
| ATOM | 1607 | CA | SER C | 27 | 33.424 | -14.412 | 1.516 | 1.00 | 65.04 | C |
| ATOM | 1608 | C | SER C | 27 | 34.639 | -15.332 | 1.308 | 1.00 | 65.63 | C |
| ATOM | 1609 | CB | SER C | 27 | 32.697 | -14.143 | 0.190 | 1.00 | 61.67 | C |
| ATOM | 1610 | OG | SER C | 27 | 33.515 | -13.423 | -0.718 | 1.00 | 67.44 | O |
| ATOM | 1611 | O | GLY C | 28 | 33.166 | -18.188 | 2.419 | 1.00 | 68.25 | O |
| ATOM | 1612 | N | GLY C | 28 | 34.348 | -16.589 | 1.005 | 1.00 | 66.44 | N |
| ATOM | 1613 | CA | GLY C | 28 | 35.219 | -17.683 | 1.377 | 1.00 | 67.66 | C |
| ATOM | 1614 | C | GLY C | 28 | 34.397 | -18.204 | 2.544 | 1.00 | 69.45 | C |
| ATOM | 1615 | O | PHE C | 29 | 34.493 | -18.782 | 5.672 | 1.00 | 67.60 | O |
| ATOM | 1616 | N | PHE C | 29 | 34.920 | -18.472 | 3.747 | 1.00 | 68.11 | N |
| ATOM | 1617 | CA | PHE C | 29 | 36.313 | -18.624 | 4.153 | 1.00 | 66.86 | C |
| ATOM | 1618 | C | PHE C | 29 | 35.655 | -19.014 | 5.483 | 1.00 | 63.40 | C |
| ATOM | 1619 | CB | PHE C | 29 | 37.157 | -17.382 | 3.878 | 1.00 | 68.24 | C |
| ATOM | 1620 | CG | PHE C | 29 | 37.184 | -16.364 | 4.992 | 1.00 | 64.97 | C |
| ATOM | 1621 | CD2 | PHE C | 29 | 36.384 | -15.256 | 4.943 | 1.00 | 62.98 | C |
| ATOM | 1622 | CD1 | PHE C | 29 | 38.051 | -16.490 | 6.033 | 1.00 | 59.48 | C |
| ATOM | 1623 | CE2 | PHE C | 29 | 36.428 | -14.312 | 5.941 | 1.00 | 58.83 | C |
| ATOM | 1624 | CE1 | PHE C | 29 | 38.104 | -15.556 | 7.029 | 1.00 | 59.20 | C |
| ATOM | 1625 | CZ | PHE C | 29 | 37.287 | -14.465 | 6.991 | 1.00 | 60.43 | C |
| ATOM | 1626 | N | THR C | 30 | 36.391 | -19.522 | 6.443 | 1.00 | 65.43 | N |
| ATOM | 1627 | CA | THR C | 30 | 35.661 | -19.685 | 7.683 | 1.00 | 61.96 | C |
| ATOM | 1628 | CB | THR C | 30 | 35.909 | -20.994 | 8.404 | 1.00 | 63.73 | C |
| ATOM | 1629 | OG1 | THR C | 30 | 34.929 | -21.124 | 9.440 | 1.00 | 60.00 | O |
| ATOM | 1630 | CG2 | THR C | 30 | 37.290 | -21.013 | 8.981 | 1.00 | 61.44 | C |
| ATOM | 1631 | C | THR C | 30 | 35.850 | -18.499 | 8.566 | 1.00 | 61.13 | C |
| ATOM | 1632 | O | THR C | 30 | 36.921 | -18.244 | 9.044 | 1.00 | 58.94 | O |
| ATOM | 1633 | N | PHE C | 31 | 34.763 | -17.781 | 8.750 | 1.00 | 61.80 | N |
| ATOM | 1634 | CA | PHE C | 31 | 34.755 | -16.464 | 9.380 | 1.00 | 58.41 | C |
| ATOM | 1635 | CB | PHE C | 31 | 33.518 | -15.685 | 8.919 | 1.00 | 57.94 | C |
| ATOM | 1636 | CG | PHE C | 31 | 33.482 | -14.253 | 9.390 | 1.00 | 57.20 | C |
| ATOM | 1637 | CD1 | PHE C | 31 | 34.183 | -13.263 | 8.701 | 1.00 | 56.56 | C |
| ATOM | 1638 | CE1 | PHE C | 31 | 34.147 | -11.935 | 9.120 | 1.00 | 50.13 | C |
| ATOM | 1639 | CZ | PHE C | 31 | 33.404 | -11.589 | 10.231 | 1.00 | 49.22 | C |
| ATOM | 1640 | CE2 | PHE C | 31 | 32.699 | -12.566 | 10.929 | 1.00 | 57.05 | C |
| ATOM | 1641 | CD2 | PHE C | 31 | 32.738 | -13.890 | 10.503 | 1.00 | 53.67 | C |
| ATOM | 1642 | C | PHE C | 31 | 34.777 | -16.514 | 10.902 | 1.00 | 56.03 | C |
| ATOM | 1643 | O | PHE C | 31 | 35.436 | -15.697 | 11.544 | 1.00 | 56.21 | O |
| ATOM | 1644 | N | GLY C | 32 | 34.045 | -17.461 | 11.479 | 1.00 | 58.10 | N |
| ATOM | 1645 | CA | GLY C | 32 | 33.827 | -17.468 | 12.915 | 1.00 | 54.70 | C |
| ATOM | 1646 | C | GLY C | 32 | 35.071 | -17.816 | 13.703 | 1.00 | 53.30 | C |
| ATOM | 1647 | O | GLY C | 32 | 35.185 | -17.496 | 14.888 | 1.00 | 53.94 | O |
| ATOM | 1648 | N | THR C | 33 | 36.002 | -18.490 | 13.045 | 1.00 | 54.99 | N |
| ATOM | 1649 | CA | THR C | 33 | 37.275 | -18.805 | 13.671 | 1.00 | 55.63 | C |
| ATOM | 1650 | CB | THR C | 33 | 37.599 | -20.309 | 13.610 | 1.00 | 54.70 | C |
| ATOM | 1651 | OG1 | THR C | 33 | 37.357 | -20.807 | 12.287 | 1.00 | 57.81 | O |
| ATOM | 1652 | CG2 | THR C | 33 | 36.737 | -21.071 | 14.605 | 1.00 | 55.99 | C |
| ATOM | 1653 | C | THR C | 33 | 38.360 | -17.973 | 13.010 | 1.00 | 55.11 | C |
| ATOM | 1654 | O | THR C | 33 | 39.480 | -18.445 | 12.771 | 1.00 | 55.44 | O |
| ATOM | 1655 | N | SER C | 34 | 38.007 | -16.722 | 12.720 | 1.00 | 51.15 | N |
| ATOM | 1656 | CA | SER C | 34 | 38.956 | -15.772 | 12.167 | 1.00 | 44.94 | C |
| ATOM | 1657 | CB | SER C | 34 | 38.552 | -15.365 | 10.749 | 1.00 | 45.18 | C |
| ATOM | 1658 | OG | SER C | 34 | 38.789 | -16.412 | 9.828 | 1.00 | 50.62 | O |
| ATOM | 1659 | C | SER C | 34 | 39.067 | -14.542 | 13.049 | 1.00 | 38.64 | C |
| ATOM | 1660 | O | SER C | 34 | 38.055 | -13.955 | 13.442 | 1.00 | 39.25 | O |
| ATOM | 1661 | N | ASN C | 35 | 40.304 | -14.172 | 13.373 | 1.00 | 33.82 | N |
| ATOM | 1662 | CA | ASN C | 35 | 40.579 | -12.879 | 13.974 | 1.00 | 32.78 | C |
| ATOM | 1663 | CB | ASN C | 35 | 41.964 | -12.887 | 14.604 | 1.00 | 29.76 | C |
| ATOM | 1664 | CG | ASN C | 35 | 42.042 | -13.805 | 15.790 | 1.00 | 27.99 | C |
| ATOM | 1665 | OD1 | ASN C | 35 | 41.157 | -13.799 | 16.644 | 1.00 | 29.26 | O |
| ATOM | 1666 | ND2 | ASN C | 35 | 43.077 | -14.626 | 15.834 | 1.00 | 30.98 | N |
| ATOM | 1667 | C | ASN C | 35 | 40.546 | -11.860 | 12.856 | 1.00 | 30.13 | C |
| ATOM | 1668 | O | ASN C | 35 | 41.202 | -12.063 | 11.844 | 1.00 | 32.36 | O |
| ATOM | 1669 | N | MET C | 36 | 39.790 | -10.779 | 13.027 | 1.00 | 29.46 | N |
| ATOM | 1670 | CA | MET C | 36 | 39.617 | -9.803 | 11.945 | 1.00 | 30.81 | C |
| ATOM | 1671 | CB | MET C | 36 | 38.132 | -9.659 | 11.577 | 1.00 | 28.53 | C |
| ATOM | 1672 | CG | MET C | 36 | 37.506 | -10.952 | 11.018 | 1.00 | 28.45 | C |
| ATOM | 1673 | SD | MET C | 36 | 38.351 | -11.656 | 9.583 | 1.00 | 33.20 | S |
| ATOM | 1674 | CE | MET C | 36 | 38.144 | -10.352 | 8.356 | 1.00 | 37.05 | C |
| ATOM | 1675 | C | MET C | 36 | 40.228 | -8.442 | 12.297 | 1.00 | 29.50 | C |
| ATOM | 1676 | O | MET C | 36 | 40.350 | -8.091 | 13.463 | 1.00 | 26.20 | O |
| ATOM | 1677 | N | ALA C | 37 | 40.624 | -7.674 | 11.289 | 1.00 | 25.56 | N |
| ATOM | 1678 | CA | ALA C | 37 | 41.233 | -6.377 | 11.558 | 1.00 | 25.56 | C |
| ATOM | 1679 | CB | ALA C | 37 | 42.726 | -6.528 | 11.679 | 1.00 | 25.20 | C |
| ATOM | 1680 | C | ALA C | 37 | 40.891 | -5.356 | 10.494 | 1.00 | 25.54 | C |
| ATOM | 1681 | O | ALA C | 37 | 40.500 | -5.726 | 9.377 | 1.00 | 21.62 | O |
| ATOM | 1682 | N | TRP C | 38 | 41.020 | -4.077 | 10.846 | 1.00 | 19.62 | N |
| ATOM | 1683 | CA | TRP C | 38 | 40.992 | -3.001 | 9.854 | 1.00 | 22.89 | C |
| ATOM | 1684 | CB | TRP C | 38 | 39.950 | -1.938 | 10.194 | 1.00 | 22.75 | C |
| ATOM | 1685 | CG | TRP C | 38 | 38.520 | -2.311 | 9.878 | 1.00 | 24.90 | C |
| ATOM | 1686 | CD1 | TRP C | 38 | 37.621 | -2.907 | 10.724 | 1.00 | 25.91 | C |
| ATOM | 1687 | NE1 | TRP C | 38 | 36.414 | -3.071 | 10.092 | 1.00 | 27.22 | N |
| ATOM | 1688 | CE2 | TRP C | 38 | 36.510 | -2.564 | 8.816 | 1.00 | 25.35 | C |
| ATOM | 1689 | CD2 | TRP C | 38 | 37.822 | -2.075 | 8.651 | 1.00 | 22.86 | C |
| ATOM | 1690 | CE3 | TRP C | 38 | 38.178 | -1.498 | 7.422 | 1.00 | 24.35 | C |
| ATOM | 1691 | CZ3 | TRP C | 38 | 37.221 | -1.428 | 6.414 | 1.00 | 23.66 | C |
| ATOM | 1692 | CH2 | TRP C | 38 | 35.925 | -1.932 | 6.611 | 1.00 | 23.43 | C |
| ATOM | 1693 | CZ2 | TRP C | 38 | 35.553 | -2.497 | 7.803 | 1.00 | 25.20 | C |
| ATOM | 1694 | C | TRP C | 38 | 42.364 | -2.346 | 9.807 | 1.00 | 25.86 | C |
| ATOM | 1695 | O | TRP C | 38 | 42.988 | -2.112 | 10.851 | 1.00 | 26.05 | O |
| ATOM | 1696 | N | LEU C | 39 | 42.825 | -2.062 | 8.599 | 1.00 | 25.49 | N |
| ATOM | 1697 | CA | LEU C | 39 | 44.103 | -1.399 | 8.384 | 1.00 | 33.17 | C |
| ATOM | 1698 | CB | LEU C | 39 | 45.082 | -2.318 | 7.647 | 1.00 | 30.92 | C |
| ATOM | 1699 | CG | LEU C | 39 | 45.631 | -3.699 | 8.057 | 1.00 | 37.65 | C |
| ATOM | 1700 | CD1 | LEU C | 39 | 46.686 | -3.618 | 9.127 | 1.00 | 37.35 | C |
| ATOM | 1701 | CD2 | LEU C | 39 | 44.563 | -4.707 | 8.436 | 1.00 | 32.05 | C |
| ATOM | 1702 | C | LEU C | 39 | 43.808 | -0.210 | 7.486 | 1.00 | 29.90 | C |
| ATOM | 1703 | O | LEU C | 39 | 42.710 | -0.108 | 6.922 | 1.00 | 28.77 | O |
| ATOM | 1704 | N | ARG C | 40 | 44.775 | 0.686 | 7.331 | 1.00 | 29.92 | N |
| ATOM | 1705 | CA | ARG C | 40 | 44.622 | 1.775 | 6.359 | 1.00 | 31.07 | C |
| ATOM | 1706 | CB | ARG C | 40 | 44.060 | 3.030 | 7.026 | 1.00 | 30.48 | C |
| ATOM | 1707 | CG | ARG C | 40 | 45.043 | 3.713 | 7.955 | 1.00 | 31.85 | C |
| ATOM | 1708 | CD | ARG C | 40 | 44.403 | 4.835 | 8.714 | 1.00 | 35.51 | C |
| ATOM | 1709 | NE | ARG C | 40 | 45.301 | 5.357 | 9.740 | 1.00 | 37.43 | N |
| ATOM | 1710 | CZ | ARG C | 40 | 44.973 | 6.313 | 10.603 | 1.00 | 41.27 | C |
| ATOM | 1711 | NH1 | ARG C | 40 | 43.756 | 6.848 | 10.583 | 1.00 | 35.74 | N |
| ATOM | 1712 | NH2 | ARG C | 40 | 45.863 | 6.726 | 11.496 | 1.00 | 43.08 | N |
| ATOM | 1713 | C | ARG C | 40 | 45.951 | 2.107 | 5.683 | 1.00 | 37.10 | C |
| ATOM | 1714 | O | ARG C | 40 | 47.026 | 1.914 | 6.265 | 1.00 | 35.87 | O |
| ATOM | 1715 | N | GLN C | 41 | 45.869 | 2.603 | 4.454 | 1.00 | 36.95 | N |
| ATOM | 1716 | CA | GLN C | 41 | 47.051 | 3.022 | 3.712 | 1.00 | 42.19 | C |
| ATOM | 1717 | CB | GLN C | 41 | 47.434 | 1.995 | 2.631 | 1.00 | 42.65 | C |
| ATOM | 1718 | CG | GLN C | 41 | 48.807 | 2.265 | 2.008 | 1.00 | 47.20 | C |
| ATOM | 1719 | CD | GLN C | 41 | 49.455 | 1.033 | 1.393 | 1.00 | 50.23 | C |
| ATOM | 1720 | OE1 | GLN C | 41 | 48.819 | -0.015 | 1.232 | 1.00 | 48.70 | O |
| ATOM | 1721 | NE2 | GLN C | 41 | 50.735 | 1.154 | 1.050 | 1.00 | 50.14 | N |
| ATOM | 1722 | C | GLN C | 41 | 46.849 | 4.401 | 3.087 | 1.00 | 41.01 | C |
| ATOM | 1723 | O | GLN C | 41 | 45.968 | 4.597 | 2.241 | 1.00 | 36.77 | O |
| ATOM | 1724 | N | ALA C | 42 | 47.671 | 5.344 | 3.536 | 1.00 | 47.79 | N |
| ATOM | 1725 | CA | ALA C | 42 | 47.752 | 6.689 | 2.978 | 1.00 | 50.64 | C |
| ATOM | 1726 | CB | ALA C | 42 | 48.346 | 7.630 | 4.018 | 1.00 | 53.41 | C |
| ATOM | 1727 | C | ALA C | 42 | 48.610 | 6.650 | 1.704 | 1.00 | 57.03 | C |
| ATOM | 1728 | O | ALA C | 42 | 49.263 | 5.639 | 1.431 | 1.00 | 59.24 | O |
| ATOM | 1729 | N | PRO C | 43 | 48.613 | 7.738 | 0.908 | 1.00 | 60.58 | N |
| ATOM | 1730 | CA | PRO C | 43 | 49.367 | 7.633 | -0.350 | 1.00 | 61.97 | C |
| ATOM | 1731 | CB | PRO C | 43 | 48.897 | 8.856 | -1.136 | 1.00 | 63.20 | C |
| ATOM | 1732 | CG | PRO C | 43 | 48.565 | 9.859 | -0.080 | 1.00 | 63.96 | C |
| ATOM | 1733 | CD | PRO C | 43 | 47.980 | 9.063 | 1.060 | 1.00 | 61.46 | C |
| ATOM | 1734 | C | PRO C | 43 | 50.877 | 7.698 | -0.126 | 1.00 | 62.03 | C |
| ATOM | 1735 | O | PRO C | 43 | 51.341 | 8.512 | 0.678 | 1.00 | 61.02 | O |
| ATOM | 1736 | N | GLY C | 44 | 51.629 | 6.844 | -0.817 | 1.00 | 62.16 | N |
| ATOM | 1737 | CA | GLY C | 44 | 53.066 | 6.758 | -0.614 | 1.00 | 61.46 | C |
| ATOM | 1738 | C | GLY C | 44 | 53.477 | 6.053 | 0.674 | 1.00 | 65.68 | C |
| ATOM | 1739 | O | GLY C | 44 | 54.415 | 5.251 | 0.677 | 1.00 | 63.82 | O |
| ATOM | 1740 | N | LYS C | 45 | 52.786 | 6.354 | 1.772 | 1.00 | 63.11 | N |
| ATOM | 1741 | CA | LYS C | 45 | 53.098 | 5.760 | 3.069 | 1.00 | 60.78 | C |
| ATOM | 1742 | CB | LYS C | 45 | 52.332 | 6.472 | 4.183 | 1.00 | 60.57 | C |
| ATOM | 1743 | CG | LYS C | 45 | 53.131 | 7.551 | 4.895 | 1.00 | 64.27 | C |
| ATOM | 1744 | CD | LYS C | 45 | 52.307 | 8.209 | 5.998 | 1.00 | 61.41 | C |
| ATOM | 1745 | CE | LYS C | 45 | 51.692 | 7.173 | 6.926 | 1.00 | 62.37 | C |
| ATOM | 1746 | NZ | LYS C | 45 | 52.709 | 6.379 | 7.674 | 1.00 | 65.05 | N |
| ATOM | 1747 | C | LYS C | 45 | 52.840 | 4.254 | 3.145 | 1.00 | 58.76 | C |
| ATOM | 1748 | O | LYS C | 45 | 52.295 | 3.644 | 2.217 | 1.00 | 58.76 | O |
| ATOM | 1749 | N | ARG C | 46 | 53.245 | 3.665 | 4.266 | 1.00 | 55.87 | N |
| ATOM | 1750 | CA | ARG C | 46 | 53.109 | 2.228 | 4.483 | 1.00 | 57.75 | C |
| ATOM | 1751 | CB | ARG C | 46 | 54.315 | 1.705 | 5.261 | 1.00 | 58.08 | C |
| ATOM | 1752 | CG | ARG C | 46 | 54.917 | 2.747 | 6.199 | 1.00 | 62.94 | C |
| ATOM | 1753 | CD | ARG C | 46 | 56.187 | 2.234 | 6.867 | 1.00 | 66.75 | C |
| ATOM | 1754 | NE | ARG C | 46 | 57.012 | 1.445 | 5.951 | 1.00 | 67.73 | N |
| ATOM | 1755 | CZ | ARG C | 46 | 58.306 | 1.192 | 6.134 | 1.00 | 70.55 | C |
| ATOM | 1756 | NH1 | ARG C | 46 | 58.939 | 1.677 | 7.198 | 1.00 | 70.40 | N |
| ATOM | 1757 | NH2 | ARG C | 46 | 58.969 | 0.460 | 5.248 | 1.00 | 64.14 | N |
| ATOM | 1758 | C | ARG C | 46 | 51.837 | 1.927 | 5.258 | 1.00 | 52.81 | C |
| ATOM | 1759 | O | ARG C | 46 | 51.447 | 2.707 | 6.130 | 1.00 | 53.24 | O |
| ATOM | 1760 | N | ARG C | 47 | 51.206 | 0.797 | 4.938 | 1.00 | 53.40 | N |
| ATOM | 1761 | CA | ARG C | 47 | 49.965 | 0.377 | 5.594 | 1.00 | 48.56 | C |
| ATOM | 1762 | CB | ARG C | 47 | 49.521 | -1.014 | 5.109 | 1.00 | 48.62 | C |
| ATOM | 1763 | CG | ARG C | 47 | 49.362 | -2.060 | 6.208 | 1.00 | 50.94 | C |
| ATOM | 1764 | CD | ARG C | 47 | 50.431 | -3.157 | 6.124 | 1.00 | 53.99 | C |
| ATOM | 1765 | NE | ARG C | 47 | 50.408 | -4.056 | 7.282 | 1.00 | 52.20 | N |
| ATOM | 1766 | CZ | ARG C | 47 | 50.918 | -3.757 | 8.479 | 1.00 | 56.26 | C |
| ATOM | 1767 | NH1 | ARG C | 47 | 51.486 | -2.574 | 8.690 | 1.00 | 58.63 | N |
| ATOM | 1768 | NH2 | ARG C | 47 | 50.855 | -4.635 | 9.477 | 1.00 | 52.99 | N |
| ATOM | 1769 | C | ARG C | 47 | 50.121 | 0.423 | 7.105 | 1.00 | 49.71 | C |
| ATOM | 1770 | O | ARG C | 47 | 51.208 | 0.173 | 7.637 | 1.00 | 49.68 | O |
| ATOM | 1771 | N | GLU C | 48 | 49.046 | 0.801 | 7.788 | 1.00 | 39.52 | N |
| ATOM | 1772 | CA | GLU C | 48 | 49.082 | 0.992 | 9.223 | 1.00 | 40.68 | C |
| ATOM | 1773 | CB | GLU C | 48 | 48.957 | 2.481 | 9.546 | 1.00 | 41.10 | C |
| ATOM | 1774 | CG | GLU C | 48 | 48.951 | 2.824 | 11.019 | 1.00 | 42.28 | C |
| ATOM | 1775 | CD | GLU C | 48 | 48.584 | 4.280 | 11.277 | 1.00 | 47.26 | C |
| ATOM | 1776 | OE1 | GLU C | 48 | 48.499 | 4.670 | 12.464 | 1.00 | 48.00 | O |
| ATOM | 1777 | OE2 | GLU C | 48 | 48.371 | 5.029 | 10.289 | 1.00 | 52.78 | O |
| ATOM | 1778 | C | GLU C | 48 | 47.923 | 0.221 | 9.837 | 1.00 | 40.61 | C |
| ATOM | 1779 | O | GLU C | 48 | 46.778 | 0.367 | 9.400 | 1.00 | 36.78 | O |
| ATOM | 1780 | N | TRP C | 49 | 48.217 | -0.607 | 10.834 | 1.00 | 34.79 | N |
| ATOM | 1781 | CA | TRP C | 49 | 47.173 | -1.315 | 11.562 | 1.00 | 35.65 | C |
| ATOM | 1782 | CB | TRP C | 49 | 47.770 | -2.413 | 12.448 | 1.00 | 33.90 | C |
| ATOM | 1783 | CG | TRP C | 49 | 46.774 | -2.983 | 13.379 | 1.00 | 30.83 | C |
| ATOM | 1784 | CD1 | TRP C | 49 | 45.800 | -3.897 | 13.083 | 1.00 | 31.41 | C |
| ATOM | 1785 | NE1 | TRP C | 49 | 45.049 | -4.167 | 14.209 | 1.00 | 32.75 | N |
| ATOM | 1786 | CE2 | TRP C | 49 | 45.534 | -3.423 | 15.254 | 1.00 | 31.08 | C |
| ATOM | 1787 | CD2 | TRP C | 49 | 46.617 | -2.656 | 14.762 | 1.00 | 28.90 | C |
| ATOM | 1788 | CE3 | TRP C | 49 | 47.300 | -1.806 | 15.640 | 1.00 | 29.31 | C |
| ATOM | 1789 | CZ3 | TRP C | 49 | 46.873 | -1.735 | 16.964 | 1.00 | 28.14 | C |
| ATOM | 1790 | CH2 | TRP C | 49 | 45.798 | -2.508 | 17.422 | 1.00 | 28.24 | C |
| ATOM | 1791 | CZ2 | TRP C | 49 | 45.116 | -3.361 | 16.585 | 1.00 | 27.43 | C |
| ATOM | 1792 | C | TRP C | 49 | 46.333 | -0.350 | 12.394 | 1.00 | 33.03 | C |
| ATOM | 1793 | O | TRP C | 49 | 46.871 | 0.550 | 13.041 | 1.00 | 32.39 | O |
| ATOM | 1794 | N | VAL C | 50 | 45.012 | -0.542 | 12.378 | 1.00 | 31.51 | N |
| ATOM | 1795 | CA | VAL C | 50 | 44.105 | 0.405 | 13.022 | 1.00 | 31.38 | C |
| ATOM | 1796 | CB | VAL C | 50 | 43.175 | 1.087 | 11.982 | 1.00 | 32.09 | C |
| ATOM | 1797 | CG1 | VAL C | 50 | 41.937 | 1.624 | 12.650 | 1.00 | 34.55 | C |
| ATOM | 1798 | CG2 | VAL C | 50 | 43.907 | 2.204 | 11.296 | 1.00 | 40.02 | C |
| ATOM | 1799 | C | VAL C | 50 | 43.290 | -0.225 | 14.147 | 1.00 | 29.42 | C |
| ATOM | 1800 | O | VAL C | 50 | 43.222 | 0.312 | 15.253 | 1.00 | 29.62 | O |
| ATOM | 1801 | N | ALA C | 51 | 42.675 | -1.370 | 13.872 | 1.00 | 24.27 | N |
| ATOM | 1802 | CA | ALA C | 51 | 41.854 | -2.038 | 14.874 | 1.00 | 26.18 | C |
| ATOM | 1803 | CB | ALA C | 51 | 40.437 | -1.459 | 14.889 | 1.00 | 23.86 | C |
| ATOM | 1804 | C | ALA C | 51 | 41.805 | -3.535 | 14.660 | 1.00 | 27.14 | C |
| ATOM | 1805 | O | ALA C | 51 | 41.945 | -4.025 | 13.529 | 1.00 | 24.09 | O |
| ATOM | 1806 | N | LEU C | 52 | 41.574 | -4.244 | 15.756 | 1.00 | 23.46 | N |
| ATOM | 1807 | CA | LEU C | 52 | 41.536 | -5.697 | 15.787 | 1.00 | 25.88 | C |
| ATOM | 1808 | CB | LEU C | 52 | 42.793 | -6.236 | 16.482 | 1.00 | 27.36 | C |
| ATOM | 1809 | CG | LEU C | 52 | 42.809 | -7.712 | 16.890 | 1.00 | 30.81 | C |
| ATOM | 1810 | CD1 | LEU C | 52 | 42.914 | -8.584 | 15.647 | 1.00 | 27.62 | C |
| ATOM | 1811 | CD2 | LEU C | 52 | 43.969 | -8.006 | 17.821 | 1.00 | 28.83 | C |
| ATOM | 1812 | C | LEU C | 52 | 40.309 | -6.182 | 16.544 | 1.00 | 26.97 | C |
| ATOM | 1813 | O | LEU C | 52 | 40.017 | -5.695 | 17.634 | 1.00 | 30.37 | O |
| ATOM | 1814 | N | ILE C | 53 | 39.599 | -7.154 | 15.983 | 1.00 | 26.55 | N |
| ATOM | 1815 | CA | ILE C | 53 | 38.577 | -7.863 | 16.746 | 1.00 | 30.34 | C |
| ATOM | 1816 | CB | ILE C | 53 | 37.103 | -7.495 | 16.375 | 1.00 | 32.76 | C |
| ATOM | 1817 | CG1 | ILE C | 53 | 36.146 | -8.163 | 17.379 | 1.00 | 38.72 | C |
| ATOM | 1818 | CD1 | ILE C | 53 | 34.724 | -7.624 | 17.374 | 1.00 | 35.87 | C |
| ATOM | 1819 | CG2 | ILE C | 53 | 36.764 | -7.934 | 14.961 | 1.00 | 31.94 | C |
| ATOM | 1820 | C | ILE C | 53 | 38.818 | -9.360 | 16.663 | 1.00 | 29.63 | C |
| ATOM | 1821 | O | ILE C | 53 | 38.909 | -9.966 | 15.590 | 1.00 | 28.27 | O |
| ATOM | 1822 | N | THR C | 54 | 38.945 | -9.934 | 17.844 | 1.00 | 31.66 | N |
| ATOM | 1823 | CA | THR C | 54 | 39.443 | -11.271 | 18.056 | 1.00 | 32.63 | C |
| ATOM | 1824 | CB | THR C | 54 | 40.046 | -11.254 | 19.483 | 1.00 | 40.14 | C |
| ATOM | 1825 | OG1 | THR C | 54 | 41.484 | -11.239 | 19.424 | 1.00 | 36.32 | O |
| ATOM | 1826 | CG2 | THR C | 54 | 39.522 | -12.351 | 20.307 | 1.00 | 36.07 | C |
| ATOM | 1827 | C | THR C | 54 | 38.316 | -12.315 | 17.923 | 1.00 | 36.63 | C |
| ATOM | 1828 | O | THR C | 54 | 37.147 | -11.975 | 18.116 | 1.00 | 39.98 | O |
| ATOM | 1829 | N | ILE C | 55 | 38.670 | -13.563 | 17.589 | 1.00 | 37.33 | N |
| ATOM | 1830 | CA | ILE C | 55 | 37.715 | -14.683 | 17.450 | 1.00 | 43.41 | C |
| ATOM | 1831 | CB | ILE C | 55 | 38.431 | -16.063 | 17.435 | 1.00 | 42.39 | C |
| ATOM | 1832 | CG1 | ILE C | 55 | 39.053 | -16.332 | 16.074 | 1.00 | 43.35 | C |
| ATOM | 1833 | CD1 | ILE C | 55 | 39.899 | -17.593 | 16.041 | 1.00 | 47.01 | C |
| ATOM | 1834 | CG2 | ILE C | 55 | 37.466 | -17.190 | 17.717 | 1.00 | 46.62 | C |
| ATOM | 1835 | C | ILE C | 55 | 36.604 | -14.716 | 18.498 | 1.00 | 42.19 | C |
| ATOM | 1836 | O | ILE C | 55 | 35.448 | -14.998 | 18.174 | 1.00 | 46.67 | O |
| ATOM | 1837 | N | SER C | 56 | 36.948 | -14.415 | 19.743 | 1.00 | 42.81 | N |
| ATOM | 1838 | CA | SER C | 56 | 35.962 | -14.335 | 20.815 | 1.00 | 43.11 | C |
| ATOM | 1839 | CB | SER C | 56 | 36.534 | -14.895 | 22.122 | 1.00 | 44.47 | C |
| ATOM | 1840 | OG | SER C | 56 | 36.955 | -16.241 | 21.982 | 1.00 | 42.82 | O |
| ATOM | 1841 | C | SER C | 56 | 35.526 | -12.893 | 21.052 | 1.00 | 48.00 | C |
| ATOM | 1842 | O | SER C | 56 | 35.275 | -12.498 | 22.190 | 1.00 | 49.95 | O |
| ATOM | 1843 | N | GLY C | 57 | 35.459 | -12.099 | 19.988 | 1.00 | 45.61 | N |
| ATOM | 1844 | CA | GLY C | 57 | 34.959 | -10.737 | 20.090 | 1.00 | 41.58 | C |
| ATOM | 1845 | C | GLY C | 57 | 35.744 | -9.729 | 20.922 | 1.00 | 38.18 | C |
| ATOM | 1846 | O | GLY C | 57 | 35.220 | -8.665 | 21.241 | 1.00 | 39.75 | O |
| ATOM | 1847 | N | TYR C | 58 | 36.997 | -10.026 | 21.255 | 1.00 | 38.01 | N |
| ATOM | 1848 | CA | TYR C | 58 | 37.839 | -9.070 | 21.996 | 1.00 | 34.84 | C |
| ATOM | 1849 | CB | TYR C | 58 | 38.904 | -9.832 | 22.785 | 1.00 | 37.65 | C |
| ATOM | 1850 | CG | TYR C | 58 | 38.415 | -11.057 | 23.557 | 1.00 | 40.27 | C |
| ATOM | 1851 | CD1 | TYR C | 58 | 37.156 | -11.103 | 24.148 | 1.00 | 42.11 | C |
| ATOM | 1852 | CE1 | TYR C | 58 | 36.743 | -12.225 | 24.861 | 1.00 | 41.82 | C |
| ATOM | 1853 | CZ | TYR C | 58 | 37.595 | -13.302 | 24.992 | 1.00 | 42.80 | C |
| ATOM | 1854 | OH | TYR C | 58 | 37.224 | -14.428 | 25.698 | 1.00 | 49.48 | O |
| ATOM | 1855 | CE2 | TYR C | 58 | 38.835 | -13.270 | 24.414 | 1.00 | 41.91 | C |
| ATOM | 1856 | CD2 | TYR C | 58 | 39.235 | -12.157 | 23.711 | 1.00 | 41.12 | C |
| ATOM | 1857 | C | TYR C | 58 | 38.541 | -8.046 | 21.071 | 1.00 | 37.07 | C |
| ATOM | 1858 | O | TYR C | 58 | 38.991 | -8.412 | 19.976 | 1.00 | 35.75 | O |
| ATOM | 1859 | N | THR C | 59 | 38.679 | -6.793 | 21.521 | 1.00 | 35.79 | N |
| ATOM | 1860 | CA | THR C | 59 | 39.125 | -5.680 | 20.647 | 1.00 | 34.17 | C |
| ATOM | 1861 | CB | THR C | 59 | 38.045 | -4.557 | 20.537 | 1.00 | 34.59 | C |
| ATOM | 1862 | OG1 | THR C | 59 | 38.009 | -3.789 | 21.751 | 1.00 | 33.24 | O |
| ATOM | 1863 | CG2 | THR C | 59 | 36.666 | -5.150 | 20.267 | 1.00 | 33.90 | C |
| ATOM | 1864 | C | THR C | 59 | 40.431 | -4.989 | 21.037 | 1.00 | 31.86 | C |
| ATOM | 1865 | O | THR C | 59 | 40.824 | -5.001 | 22.207 | 1.00 | 37.58 | O |
| ATOM | 1866 | N | ASP C | 60 | 41.099 | -4.385 | 20.053 | 1.00 | 30.16 | N |
| ATOM | 1867 | CA | ASP C | 60 | 42.275 | -3.546 | 20.307 | 1.00 | 28.08 | C |
| ATOM | 1868 | CB | ASP C | 60 | 43.544 | -4.397 | 20.425 | 1.00 | 30.73 | C |
| ATOM | 1869 | CG | ASP C | 60 | 44.640 | -3.699 | 21.218 | 1.00 | 33.43 | C |
| ATOM | 1870 | OD1 | ASP C | 60 | 44.430 | -2.527 | 21.631 | 1.00 | 34.53 | O |
| ATOM | 1871 | OD2 | ASP C | 60 | 45.710 | -4.317 | 21.427 | 1.00 | 29.37 | O |
| ATOM | 1872 | C | ASP C | 60 | 42.440 | -2.484 | 19.233 | 1.00 | 29.78 | C |
| ATOM | 1873 | O | ASP C | 60 | 41.938 | -2.652 | 18.105 | 1.00 | 26.77 | O |
| ATOM | 1874 | N | TYR C | 61 | 43.146 | -1.400 | 19.564 | 1.00 | 28.97 | N |
| ATOM | 1875 | CA | TYR C | 61 | 43.266 | -0.250 | 18.655 | 1.00 | 30.09 | C |
| ATOM | 1876 | CB | TYR C | 61 | 42.276 | 0.852 | 19.067 | 1.00 | 29.44 | C |
| ATOM | 1877 | CG | TYR C | 61 | 40.858 | 0.368 | 19.125 | 1.00 | 31.38 | C |
| ATOM | 1878 | CD2 | TYR C | 61 | 40.043 | 0.435 | 18.007 | 1.00 | 31.80 | C |
| ATOM | 1879 | CE2 | TYR C | 61 | 38.746 | -0.019 | 18.045 | 1.00 | 29.62 | C |
| ATOM | 1880 | CZ | TYR C | 61 | 38.247 | -0.551 | 19.195 | 1.00 | 28.72 | C |
| ATOM | 1881 | OH | TYR C | 61 | 36.949 | -1.020 | 19.221 | 1.00 | 30.46 | O |
| ATOM | 1882 | CE1 | TYR C | 61 | 39.038 | -0.637 | 20.325 | 1.00 | 31.80 | C |
| ATOM | 1883 | CD1 | TYR C | 61 | 40.332 | -0.175 | 20.286 | 1.00 | 29.48 | C |
| ATOM | 1884 | C | TYR C | 61 | 44.665 | 0.341 | 18.593 | 1.00 | 31.75 | C |
| ATOM | 1885 | O | TYR C | 61 | 45.405 | 0.270 | 19.558 | 1.00 | 40.21 | O |
| ATOM | 1886 | N | ALA C | 62 | 45.024 | 0.937 | 17.463 | 1.00 | 32.06 | N |
| ATOM | 1887 | CA | ALA C | 62 | 46.262 | 1.701 | 17.377 | 1.00 | 37.26 | C |
| ATOM | 1888 | CB | ALA C | 62 | 46.517 | 2.163 | 15.972 | 1.00 | 34.18 | C |
| ATOM | 1889 | C | ALA C | 62 | 46.189 | 2.900 | 18.316 | 1.00 | 47.53 | C |
| ATOM | 1890 | O | ALA C | 62 | 45.115 | 3.212 | 18.862 | 1.00 | 44.28 | O |
| ATOM | 1891 | N | ASP C | 63 | 47.332 | 3.565 | 18.500 | 1.00 | 47.41 | N |
| ATOM | 1892 | CA | ASP C | 63 | 47.418 | 4.729 | 19.380 | 1.00 | 47.99 | C |
| ATOM | 1893 | CB | ASP C | 63 | 48.882 | 5.108 | 19.642 | 1.00 | 44.88 | C |
| ATOM | 1894 | CG | ASP C | 63 | 49.466 | 4.389 | 20.841 | 1.00 | 43.62 | C |
| ATOM | 1895 | OD2 | ASP C | 63 | 50.614 | 4.700 | 21.233 | 1.00 | 48.45 | O |
| ATOM | 1896 | OD1 | ASP C | 63 | 48.785 | 3.504 | 21.386 | 1.00 | 42.39 | O |
| ATOM | 1897 | C | ASP C | 63 | 46.702 | 5.905 | 18.746 | 1.00 | 43.83 | C |
| ATOM | 1898 | O | ASP C | 63 | 45.855 | 6.537 | 19.373 | 1.00 | 45.36 | O |
| ATOM | 1899 | N | SER C | 64 | 47.036 | 6.173 | 17.486 | 1.00 | 42.56 | N |
| ATOM | 1900 | CA | SER C | 64 | 46.475 | 7.297 | 16.741 | 1.00 | 47.61 | C |
| ATOM | 1901 | CB | SER C | 64 | 47.098 | 7.351 | 15.344 | 1.00 | 49.66 | C |
| ATOM | 1902 | OG | SER C | 64 | 46.907 | 6.127 | 14.648 | 1.00 | 51.07 | O |
| ATOM | 1903 | C | SER C | 64 | 44.942 | 7.273 | 16.623 | 1.00 | 47.45 | C |
| ATOM | 1904 | O | SER C | 64 | 44.351 | 8.176 | 16.031 | 1.00 | 48.49 | O |
| ATOM | 1905 | N | VAL C | 65 | 44.312 | 6.272 | 17.236 | 1.00 | 44.45 | N |
| ATOM | 1906 | CA | VAL C | 65 | 42.926 | 5.918 | 16.946 | 1.00 | 46.12 | C |
| ATOM | 1907 | CB | VAL C | 65 | 42.910 | 4.685 | 16.020 | 1.00 | 42.44 | C |
| ATOM | 1908 | CG1 | VAL C | 65 | 41.841 | 3.688 | 16.410 | 1.00 | 37.16 | C |
| ATOM | 1909 | CG2 | VAL C | 65 | 42.801 | 5.140 | 14.585 | 1.00 | 36.40 | C |
| ATOM | 1910 | C | VAL C | 65 | 42.096 | 5.750 | 18.223 | 1.00 | 44.70 | C |
| ATOM | 1911 | O | VAL C | 65 | 40.862 | 5.823 | 18.204 | 1.00 | 39.23 | O |
| ATOM | 1912 | N | LYS C | 66 | 42.801 | 5.564 | 19.337 | 1.00 | 45.86 | N |
| ATOM | 1913 | CA | LYS C | 66 | 42.319 | 5.942 | 20.676 | 1.00 | 48.40 | C |
| ATOM | 1914 | CB | LYS C | 66 | 42.535 | 7.454 | 20.901 | 1.00 | 50.52 | C |
| ATOM | 1915 | CG | LYS C | 66 | 42.033 | 8.392 | 19.781 | 1.00 | 44.14 | C |
| ATOM | 1916 | CD | LYS C | 66 | 42.803 | 9.710 | 19.690 | 1.00 | 51.48 | C |
| ATOM | 1917 | CE | LYS C | 66 | 44.320 | 9.504 | 19.748 | 1.00 | 51.80 | C |
| ATOM | 1918 | NZ | LYS C | 66 | 44.833 | 9.511 | 21.150 | 1.00 | 54.05 | N |
| ATOM | 1919 | C | LYS C | 66 | 40.892 | 5.577 | 21.102 | 1.00 | 45.60 | C |
| ATOM | 1920 | O | LYS C | 66 | 40.504 | 4.408 | 21.170 | 1.00 | 52.31 | O |
| ATOM | 1921 | N | ASP C | 67 | 40.135 | 6.606 | 21.437 | 1.00 | 43.03 | N |
| ATOM | 1922 | CA | ASP C | 67 | 38.774 | 6.438 | 21.897 | 1.00 | 52.06 | C |
| ATOM | 1923 | CB | ASP C | 67 | 38.606 | 7.132 | 23.250 | 1.00 | 53.88 | C |
| ATOM | 1924 | CG | ASP C | 67 | 39.677 | 6.713 | 24.248 | 1.00 | 54.42 | C |
| ATOM | 1925 | OD1 | ASP C | 67 | 39.478 | 5.675 | 24.914 | 1.00 | 51.21 | O |
| ATOM | 1926 | OD2 | ASP C | 67 | 40.720 | 7.405 | 24.344 | 1.00 | 51.60 | O |
| ATOM | 1927 | C | ASP C | 67 | 37.880 | 7.060 | 20.843 | 1.00 | 47.96 | C |
| ATOM | 1928 | O | ASP C | 67 | 36.780 | 7.533 | 21.137 | 1.00 | 52.51 | O |
| ATOM | 1929 | N | ARG C | 68 | 38.387 | 7.062 | 19.611 | 1.00 | 47.91 | N |
| ATOM | 1930 | CA | ARG C | 68 | 37.667 | 7.567 | 18.441 | 1.00 | 38.24 | C |
| ATOM | 1931 | CB | ARG C | 68 | 38.609 | 8.367 | 17.537 | 1.00 | 41.26 | C |
| ATOM | 1932 | CG | ARG C | 68 | 39.212 | 9.600 | 18.180 | 1.00 | 43.18 | C |
| ATOM | 1933 | CD | ARG C | 68 | 39.929 | 10.487 | 17.175 | 1.00 | 43.40 | C |
| ATOM | 1934 | NE | ARG C | 68 | 41.108 | 9.877 | 16.559 | 1.00 | 43.27 | N |
| ATOM | 1935 | CZ | ARG C | 68 | 41.383 | 9.932 | 15.254 | 1.00 | 44.48 | C |
| ATOM | 1936 | NH1 | ARG C | 68 | 40.562 | 10.566 | 14.424 | 1.00 | 41.00 | N |
| ATOM | 1937 | NH2 | ARG C | 68 | 42.477 | 9.360 | 14.771 | 1.00 | 36.02 | N |
| ATOM | 1938 | C | ARG C | 68 | 37.035 | 6.421 | 17.636 | 1.00 | 44.24 | C |
| ATOM | 1939 | O | ARG C | 68 | 35.842 | 6.462 | 17.311 | 1.00 | 42.74 | O |
| ATOM | 1940 | N | PHE C | 69 | 37.824 | 5.393 | 17.325 | 1.00 | 39.83 | N |
| ATOM | 1941 | CA | PHE C | 69 | 37.328 | 4.299 | 16.492 | 1.00 | 35.69 | C |
| ATOM | 1942 | CB | PHE C | 69 | 38.439 | 3.712 | 15.604 | 1.00 | 32.69 | C |
| ATOM | 1943 | CG | PHE C | 69 | 38.937 | 4.646 | 14.544 | 1.00 | 30.53 | C |
| ATOM | 1944 | CD1 | PHE C | 69 | 38.589 | 5.987 | 14.544 | 1.00 | 32.25 | C |
| ATOM | 1945 | CE1 | PHE C | 69 | 39.047 | 6.832 | 13.574 | 1.00 | 32.80 | C |
| ATOM | 1946 | CZ | PHE C | 69 | 39.847 | 6.347 | 12.563 | 1.00 | 30.76 | C |
| ATOM | 1947 | CE2 | PHE C | 69 | 40.182 | 5.017 | 12.541 | 1.00 | 30.64 | C |
| ATOM | 1948 | CD2 | PHE C | 69 | 39.729 | 4.175 | 13.524 | 1.00 | 30.99 | C |
| ATOM | 1949 | C | PHE C | 69 | 36.740 | 3.191 | 17.327 | 1.00 | 34.03 | C |
| ATOM | 1950 | O | PHE C | 69 | 37.153 | 2.980 | 18.463 | 1.00 | 37.22 | O |
| ATOM | 1951 | N | THR C | 70 | 35.773 | 2.475 | 16.764 | 1.00 | 30.60 | N |
| ATOM | 1952 | CA | THR C | 70 | 35.231 | 1.292 | 17.403 | 1.00 | 27.85 | C |
| ATOM | 1953 | CB | THR C | 70 | 33.906 | 1.605 | 18.106 | 1.00 | 37.48 | C |
| ATOM | 1954 | OG1 | THR C | 70 | 34.095 | 2.713 | 18.992 | 1.00 | 40.14 | O |
| ATOM | 1955 | CG2 | THR C | 70 | 33.442 | 0.418 | 18.893 | 1.00 | 29.94 | C |
| ATOM | 1956 | C | THR C | 70 | 35.012 | 0.161 | 16.402 | 1.00 | 32.97 | C |
| ATOM | 1957 | O | THR C | 70 | 34.272 | 0.312 | 15.424 | 1.00 | 30.39 | O |
| ATOM | 1958 | N | ILE C | 71 | 35.642 | -0.982 | 16.648 | 1.00 | 28.95 | N |
| ATOM | 1959 | CA | ILE C | 71 | 35.484 | -2.125 | 15.763 | 1.00 | 29.51 | C |
| ATOM | 1960 | CB | ILE C | 71 | 36.847 | -2.810 | 15.471 | 1.00 | 29.19 | C |
| ATOM | 1961 | CG1 | ILE C | 71 | 36.695 | -3.920 | 14.434 | 1.00 | 29.45 | C |
| ATOM | 1962 | CD1 | ILE C | 71 | 38.021 | -4.548 | 14.020 | 1.00 | 26.69 | C |
| ATOM | 1963 | CG2 | ILE C | 71 | 37.514 | -3.345 | 16.780 | 1.00 | 25.74 | C |
| ATOM | 1964 | C | ILE C | 71 | 34.496 | -3.117 | 16.370 | 1.00 | 32.40 | C |
| ATOM | 1965 | O | ILE C | 71 | 34.500 | -3.345 | 17.574 | 1.00 | 35.42 | O |
| ATOM | 1966 | N | SER C | 72 | 33.649 | -3.699 | 15.535 | 1.00 | 30.88 | N |
| ATOM | 1967 | CA | SER C | 72 | 32.637 | -4.632 | 16.013 | 1.00 | 39.22 | C |
| ATOM | 1968 | CB | SER C | 72 | 31.308 | -3.904 | 16.241 | 1.00 | 41.51 | C |
| ATOM | 1969 | OG | SER C | 72 | 30.633 | -3.711 | 15.009 | 1.00 | 39.69 | O |
| ATOM | 1970 | C | SER C | 72 | 32.453 | -5.704 | 14.969 | 1.00 | 36.89 | C |
| ATOM | 1971 | O | SER C | 72 | 32.828 | -5.512 | 13.808 | 1.00 | 34.66 | O |
| ATOM | 1972 | N | ARG C | 73 | 31.890 | -6.839 | 15.371 | 1.00 | 40.75 | N |
| ATOM | 1973 | CA | ARG C | 73 | 31.615 | -7.916 | 14.430 | 1.00 | 40.72 | C |
| ATOM | 1974 | CB | ARG C | 73 | 32.646 | -9.060 | 14.541 | 1.00 | 44.56 | C |
| ATOM | 1975 | CG | ARG C | 73 | 32.482 | -9.991 | 15.755 | 1.00 | 46.81 | C |
| ATOM | 1976 | CD | ARG C | 73 | 33.678 | -10.955 | 15.933 | 1.00 | 41.14 | C |
| ATOM | 1977 | NE | ARG C | 73 | 33.817 | -11.913 | 14.835 | 1.00 | 45.63 | N |
| ATOM | 1978 | CZ | ARG C | 73 | 34.978 | -12.405 | 14.407 | 1.00 | 42.35 | C |
| ATOM | 1979 | NH1 | ARG C | 73 | 36.109 | -12.023 | 14.979 | 1.00 | 41.27 | N |
| ATOM | 1980 | NH2 | ARG C | 73 | 35.016 | -13.265 | 13.392 | 1.00 | 48.58 | N |
| ATOM | 1981 | C | ARG C | 73 | 30.195 | -8.441 | 14.622 | 1.00 | 46.33 | C |
| ATOM | 1982 | O | ARG C | 73 | 29.604 | -8.287 | 15.689 | 1.00 | 51.71 | O |
| ATOM | 1983 | N | ASP C | 74 | 29.649 | -9.024 | 13.562 | 1.00 | 50.40 | N |
| ATOM | 1984 | CA | ASP C | 74 | 28.338 | -9.659 | 13.609 | 1.00 | 60.48 | C |
| ATOM | 1985 | CB | ASP C | 74 | 27.269 | -8.778 | 12.963 | 1.00 | 60.24 | C |
| ATOM | 1986 | CG | ASP C | 74 | 25.855 | -9.235 | 13.295 | 1.00 | 65.96 | C |
| ATOM | 1987 | OD1 | ASP C | 74 | 25.699 | -10.138 | 14.150 | 1.00 | 67.18 | O |
| ATOM | 1988 | OD2 | ASP C | 74 | 24.901 | -8.680 | 12.709 | 1.00 | 65.60 | O |
| ATOM | 1989 | C | ASP C | 74 | 28.447 | -10.995 | 12.893 | 1.00 | 55.80 | C |
| ATOM | 1990 | O | ASP C | 74 | 28.656 | -11.057 | 11.678 | 1.00 | 62.03 | O |
| ATOM | 1991 | N | ASN C | 75 | 28.306 | -12.060 | 13.670 | 1.00 | 63.99 | N |
| ATOM | 1992 | CA | ASN C | 75 | 28.658 | -13.406 | 13.241 | 1.00 | 63.93 | C |
| ATOM | 1993 | CB | ASN C | 75 | 28.907 | -14.252 | 14.487 | 1.00 | 62.16 | C |
| ATOM | 1994 | CG | ASN C | 75 | 29.510 | -13.438 | 15.617 | 1.00 | 59.09 | C |
| ATOM | 1995 | OD1 | ASN C | 75 | 28.814 | -12.656 | 16.262 | 1.00 | 64.40 | O |
| ATOM | 1996 | ND2 | ASN C | 75 | 30.808 | -13.606 | 15.854 | 1.00 | 54.63 | N |
| ATOM | 1997 | C | ASN C | 75 | 27.623 | -14.061 | 12.323 | 1.00 | 65.41 | C |
| ATOM | 1998 | O | ASN C | 75 | 27.943 | -14.979 | 11.560 | 1.00 | 67.89 | O |
| ATOM | 1999 | N | ALA C | 76 | 26.387 | -13.573 | 12.392 | 1.00 | 66.48 | N |
| ATOM | 2000 | CA | ALA C | 76 | 25.303 | -14.061 | 11.540 | 1.00 | 63.29 | C |
| ATOM | 2001 | CB | ALA C | 76 | 23.973 | -13.498 | 12.017 | 1.00 | 57.63 | C |
| ATOM | 2002 | C | ALA C | 76 | 25.513 | -13.729 | 10.055 | 1.00 | 66.58 | C |
| ATOM | 2003 | O | ALA C | 76 | 24.984 | -14.421 | 9.176 | 1.00 | 66.00 | O |
| ATOM | 2004 | N | LYS C | 77 | 26.285 | -12.680 | 9.776 | 1.00 | 62.57 | N |
| ATOM | 2005 | CA | LYS C | 77 | 26.386 | -12.170 | 8.412 | 1.00 | 58.01 | C |
| ATOM | 2006 | CB | LYS C | 77 | 25.881 | -10.731 | 8.363 | 1.00 | 57.84 | C |
| ATOM | 2007 | CG | LYS C | 77 | 24.386 | -10.599 | 8.578 | 1.00 | 58.80 | C |
| ATOM | 2008 | CD | LYS C | 77 | 24.063 | -9.875 | 9.874 | 1.00 | 58.22 | C |
| ATOM | 2009 | CE | LYS C | 77 | 22.579 | -9.530 | 9.935 | 1.00 | 59.82 | C |
| ATOM | 2010 | NZ | LYS C | 77 | 22.246 | -8.563 | 11.014 | 1.00 | 57.29 | N |
| ATOM | 2011 | C | LYS C | 77 | 27.769 | -12.260 | 7.765 | 1.00 | 59.24 | C |
| ATOM | 2012 | O | LYS C | 77 | 27.942 | -11.841 | 6.609 | 1.00 | 58.32 | O |
| ATOM | 2013 | N | ASN C | 78 | 28.732 | -12.839 | 8.482 | 1.00 | 56.18 | N |
| ATOM | 2014 | CA | ASN C | 78 | 30.132 | -12.733 | 8.090 | 1.00 | 56.59 | C |
| ATOM | 2015 | CB | ASN C | 78 | 30.451 | -13.611 | 6.873 | 1.00 | 55.38 | C |
| ATOM | 2016 | CG | ASN C | 78 | 30.534 | -15.085 | 7.221 | 1.00 | 57.68 | C |
| ATOM | 2017 | OD1 | ASN C | 78 | 30.124 | -15.508 | 8.305 | 1.00 | 58.48 | O |
| ATOM | 2018 | ND2 | ASN C | 78 | 31.072 | -15.877 | 6.301 | 1.00 | 57.38 | N |
| ATOM | 2019 | C | ASN C | 78 | 30.469 | -11.264 | 7.812 | 1.00 | 55.29 | C |
| ATOM | 2020 | O | ASN C | 78 | 30.808 | -10.890 | 6.682 | 1.00 | 53.27 | O |
| ATOM | 2021 | N | THR C | 79 | 30.348 | -10.437 | 8.848 | 1.00 | 53.69 | N |
| ATOM | 2022 | CA | THR C | 79 | 30.529 | -8.998 | 8.688 | 1.00 | 53.18 | C |
| ATOM | 2023 | CB | THR C | 79 | 29.179 | -8.294 | 8.418 | 1.00 | 50.64 | C |
| ATOM | 2024 | OG1 | THR C | 79 | 29.405 | -7.169 | 7.568 | 1.00 | 52.19 | O |
| ATOM | 2025 | CG2 | THR C | 79 | 28.523 | -7.832 | 9.694 | 1.00 | 45.48 | C |
| ATOM | 2026 | C | THR C | 79 | 31.274 | -8.304 | 9.841 | 1.00 | 45.83 | C |
| ATOM | 2027 | O | THR C | 79 | 31.111 | -8.652 | 11.014 | 1.00 | 46.91 | O |
| ATOM | 2028 | N | VAL C | 80 | 32.113 | -7.332 | 9.487 | 1.00 | 41.36 | N |
| ATOM | 2029 | CA | VAL C | 80 | 32.814 | -6.531 | 10.491 | 1.00 | 37.61 | C |
| ATOM | 2030 | CB | VAL C | 80 | 34.263 | -7.068 | 10.783 | 1.00 | 38.60 | C |
| ATOM | 2031 | CG1 | VAL C | 80 | 34.840 | -7.802 | 9.598 | 1.00 | 36.67 | C |
| ATOM | 2032 | CG2 | VAL C | 80 | 35.210 | -5.957 | 11.316 | 1.00 | 30.23 | C |
| ATOM | 2033 | C | VAL C | 80 | 32.757 | -5.040 | 10.148 | 1.00 | 35.20 | C |
| ATOM | 2034 | O | VAL C | 80 | 32.872 | -4.650 | 8.978 | 1.00 | 33.20 | O |
| ATOM | 2035 | N | SER C | 81 | 32.514 | -4.231 | 11.174 | 1.00 | 31.95 | N |
| ATOM | 2036 | CA | SER C | 81 | 32.291 | -2.799 | 11.033 | 1.00 | 31.22 | C |
| ATOM | 2037 | CB | SER C | 81 | 30.924 | -2.436 | 11.611 | 1.00 | 32.98 | C |
| ATOM | 2038 | OG | SER C | 81 | 29.924 | -3.238 | 11.020 | 1.00 | 39.45 | O |
| ATOM | 2039 | C | SER C | 81 | 33.356 | -1.983 | 11.757 | 1.00 | 29.94 | C |
| ATOM | 2040 | O | SER C | 81 | 33.993 | -2.475 | 12.695 | 1.00 | 28.22 | O |
| ATOM | 2041 | N | LEU C | 82 | 33.527 | -0.733 | 11.327 | 1.00 | 25.85 | N |
| ATOM | 2042 | CA | LEU C | 82 | 34.423 | 0.206 | 11.992 | 1.00 | 26.23 | C |
| ATOM | 2043 | CB | LEU C | 82 | 35.754 | 0.344 | 11.239 | 1.00 | 24.30 | C |
| ATOM | 2044 | CG | LEU C | 82 | 36.808 | 1.202 | 11.958 | 1.00 | 25.80 | C |
| ATOM | 2045 | CD1 | LEU C | 82 | 37.273 | 0.500 | 13.232 | 1.00 | 25.68 | C |
| ATOM | 2046 | CD2 | LEU C | 82 | 37.988 | 1.497 | 11.035 | 1.00 | 24.62 | C |
| ATOM | 2047 | C | LEU C | 82 | 33.746 | 1.559 | 12.111 | 1.00 | 28.53 | C |
| ATOM | 2048 | O | LEU C | 82 | 33.503 | 2.240 | 11.103 | 1.00 | 27.40 | O |
| ATOM | 2049 | N | GLN C | 83 | 33.413 | 1.943 | 13.341 | 1.00 | 26.38 | N |
| ATOM | 2050 | CA | GLN C | 83 | 32.876 | 3.282 | 13.587 | 1.00 | 29.56 | C |
| ATOM | 2051 | CB | GLN C | 83 | 31.949 | 3.292 | 14.810 | 1.00 | 31.21 | C |
| ATOM | 2052 | CG | GLN C | 83 | 31.455 | 4.698 | 15.192 | 1.00 | 34.53 | C |
| ATOM | 2053 | CD | GLN C | 83 | 30.491 | 5.277 | 14.162 | 1.00 | 34.95 | C |
| ATOM | 2054 | OE1 | GLN C | 83 | 29.713 | 4.547 | 13.547 | 1.00 | 37.98 | O |
| ATOM | 2055 | NE2 | GLN C | 83 | 30.548 | 6.590 | 13.962 | 1.00 | 35.88 | N |
| ATOM | 2056 | C | GLN C | 83 | 34.027 | 4.231 | 13.830 | 1.00 | 29.06 | C |
| ATOM | 2057 | O | GLN C | 83 | 34.885 | 3.961 | 14.662 | 1.00 | 32.09 | O |
| ATOM | 2058 | N | MET C | 84 | 34.058 | 5.343 | 13.107 | 1.00 | 24.78 | N |
| ATOM | 2059 | CA | MET C | 84 | 35.144 | 6.301 | 13.232 | 1.00 | 32.94 | C |
| ATOM | 2060 | CB | MET C | 84 | 35.932 | 6.389 | 11.913 | 1.00 | 28.36 | C |
| ATOM | 2061 | CG | MET C | 84 | 36.461 | 5.050 | 11.437 | 1.00 | 33.37 | C |
| ATOM | 2062 | SD | MET C | 84 | 37.441 | 5.162 | 9.929 | 1.00 | 37.57 | S |
| ATOM | 2063 | CE | MET C | 84 | 36.267 | 5.815 | 8.763 | 1.00 | 27.06 | C |
| ATOM | 2064 | C | MET C | 84 | 34.588 | 7.671 | 13.619 | 1.00 | 36.69 | C |
| ATOM | 2065 | O | MET C | 84 | 33.838 | 8.277 | 12.851 | 1.00 | 33.65 | O |
| ATOM | 2066 | N | ASN C | 85 | 34.948 | 8.153 | 14.808 | 1.00 | 38.07 | N |
| ATOM | 2067 | CA | ASN C | 85 | 34.494 | 9.463 | 15.285 | 1.00 | 37.40 | C |
| ATOM | 2068 | CB | ASN C | 85 | 33.911 | 9.344 | 16.694 | 1.00 | 33.78 | C |
| ATOM | 2069 | CG | ASN C | 85 | 32.625 | 8.562 | 16.729 | 1.00 | 33.84 | C |
| ATOM | 2070 | OD1 | ASN C | 85 | 31.774 | 8.703 | 15.852 | 1.00 | 40.54 | O |
| ATOM | 2071 | ND2 | ASN C | 85 | 32.464 | 7.740 | 17.752 | 1.00 | 42.04 | N |
| ATOM | 2072 | C | ASN C | 85 | 35.619 | 10.490 | 15.298 | 1.00 | 38.41 | C |
| ATOM | 2073 | O | ASN C | 85 | 36.796 | 10.124 | 15.264 | 1.00 | 41.95 | O |
| ATOM | 2074 | N | SER C | 86 | 35.256 | 11.770 | 15.347 | 1.00 | 34.52 | N |
| ATOM | 2075 | CA | SER C | 86 | 36.232 | 12.858 | 15.429 | 1.00 | 41.73 | C |
| ATOM | 2076 | CB | SER C | 86 | 36.831 | 12.922 | 16.847 | 1.00 | 40.86 | C |
| ATOM | 2077 | OG | SER C | 86 | 35.808 | 12.935 | 17.829 | 1.00 | 44.52 | O |
| ATOM | 2078 | C | SER C | 86 | 37.356 | 12.768 | 14.391 | 1.00 | 40.72 | C |
| ATOM | 2079 | O | SER C | 86 | 38.540 | 12.852 | 14.743 | 1.00 | 38.37 | O |
| ATOM | 2080 | N | LEU C | 87 | 36.990 | 12.618 | 13.119 | 1.00 | 38.69 | N |
| ATOM | 2081 | CA | LEU C | 87 | 37.973 | 12.392 | 12.063 | 1.00 | 36.28 | C |
| ATOM | 2082 | CB | LEU C | 87 | 37.290 | 11.907 | 10.776 | 1.00 | 34.65 | C |
| ATOM | 2083 | CG | LEU C | 87 | 36.818 | 10.452 | 10.794 | 1.00 | 35.74 | C |
| ATOM | 2084 | CD1 | LEU C | 87 | 35.864 | 10.155 | 9.618 | 1.00 | 30.68 | C |
| ATOM | 2085 | CD2 | LEU C | 87 | 38.031 | 9.522 | 10.764 | 1.00 | 35.72 | C |
| ATOM | 2086 | C | LEU C | 87 | 38.843 | 13.611 | 11.770 | 1.00 | 41.64 | C |
| ATOM | 2087 | O | LEU C | 87 | 38.381 | 14.760 | 11.829 | 1.00 | 41.82 | O |
| ATOM | 2088 | N | LYS C | 88 | 40.102 | 13.345 | 11.436 | 1.00 | 37.57 | N |
| ATOM | 2089 | CA | LYS C | 88 | 41.085 | 14.387 | 11.202 | 1.00 | 43.01 | C |
| ATOM | 2090 | CB | LYS C | 88 | 42.175 | 14.316 | 12.277 | 1.00 | 45.83 | C |
| ATOM | 2091 | CG | LYS C | 88 | 41.654 | 14.496 | 13.704 | 1.00 | 44.72 | C |
| ATOM | 2092 | CD | LYS C | 88 | 42.767 | 14.340 | 14.730 | 1.00 | 47.75 | C |
| ATOM | 2093 | CE | LYS C | 88 | 43.063 | 12.879 | 15.010 | 1.00 | 48.80 | C |
| ATOM | 2094 | NZ | LYS C | 88 | 44.519 | 12.590 | 15.025 | 1.00 | 51.23 | N |
| ATOM | 2095 | C | LYS C | 88 | 41.676 | 14.170 | 9.823 | 1.00 | 42.36 | C |
| ATOM | 2096 | O | LYS C | 88 | 41.592 | 13.063 | 9.289 | 1.00 | 40.01 | O |
| ATOM | 2097 | N | PRO C | 89 | 42.264 | 15.223 | 9.224 | 1.00 | 40.05 | N |
| ATOM | 2098 | CA | PRO C | 89 | 42.823 | 15.073 | 7.876 | 1.00 | 40.55 | C |
| ATOM | 2099 | CB | PRO C | 89 | 43.469 | 16.438 | 7.610 | 1.00 | 41.51 | C |
| ATOM | 2100 | CG | PRO C | 89 | 42.661 | 17.383 | 8.423 | 1.00 | 41.45 | C |
| ATOM | 2101 | CD | PRO C | 89 | 42.293 | 16.625 | 9.675 | 1.00 | 42.85 | C |
| ATOM | 2102 | C | PRO C | 89 | 43.862 | 13.962 | 7.770 | 1.00 | 42.15 | C |
| ATOM | 2103 | O | PRO C | 89 | 43.984 | 13.356 | 6.710 | 1.00 | 40.72 | O |
| ATOM | 2104 | N | GLU C | 90 | 44.597 | 13.686 | 8.844 | 1.00 | 44.53 | N |
| ATOM | 2105 | CA | GLU C | 90 | 45.607 | 12.633 | 8.774 | 1.00 | 48.39 | C |
| ATOM | 2106 | CB | GLU C | 90 | 46.713 | 12.839 | 9.819 | 1.00 | 46.85 | C |
| ATOM | 2107 | CG | GLU C | 90 | 46.231 | 13.142 | 11.229 | 1.00 | 47.85 | C |
| ATOM | 2108 | CD | GLU C | 90 | 45.882 | 14.607 | 11.426 | 1.00 | 53.51 | C |
| ATOM | 2109 | OE1 | GLU C | 90 | 46.060 | 15.406 | 10.473 | 1.00 | 51.83 | O |
| ATOM | 2110 | OE2 | GLU C | 90 | 45.415 | 14.953 | 12.534 | 1.00 | 57.26 | O |
| ATOM | 2111 | C | GLU C | 90 | 45.005 | 11.221 | 8.844 | 1.00 | 45.68 | C |
| ATOM | 2112 | O | GLU C | 90 | 45.724 | 10.225 | 8.703 | 1.00 | 43.49 | O |
| ATOM | 2113 | N | ASP C | 91 | 43.687 | 11.141 | 9.044 | 1.00 | 44.99 | N |
| ATOM | 2114 | CA | ASP C | 91 | 42.973 | 9.865 | 8.970 | 1.00 | 40.79 | C |
| ATOM | 2115 | CB | ASP C | 91 | 41.662 | 9.931 | 9.745 | 1.00 | 38.93 | C |
| ATOM | 2116 | CG | ASP C | 91 | 41.866 | 10.150 | 11.227 | 1.00 | 40.20 | C |
| ATOM | 2117 | OD1 | ASP C | 91 | 42.686 | 9.433 | 11.845 | 1.00 | 39.88 | O |
| ATOM | 2118 | OD2 | ASP C | 91 | 41.186 | 11.036 | 11.778 | 1.00 | 40.54 | O |
| ATOM | 2119 | C | ASP C | 91 | 42.678 | 9.512 | 7.516 | 1.00 | 38.61 | C |
| ATOM | 2120 | O | ASP C | 91 | 42.231 | 8.404 | 7.207 | 1.00 | 38.01 | O |
| ATOM | 2121 | N | THR C | 92 | 42.905 | 10.465 | 6.621 | 1.00 | 39.03 | N |
| ATOM | 2122 | CA | THR C | 92 | 42.687 | 10.231 | 5.199 | 1.00 | 34.51 | C |
| ATOM | 2123 | CB | THR C | 92 | 43.047 | 11.475 | 4.365 | 1.00 | 37.95 | C |
| ATOM | 2124 | OG1 | THR C | 92 | 42.137 | 12.539 | 4.674 | 1.00 | 35.54 | O |
| ATOM | 2125 | CG2 | THR C | 92 | 42.993 | 11.169 | 2.882 | 1.00 | 29.43 | C |
| ATOM | 2126 | C | THR C | 92 | 43.538 | 9.059 | 4.739 | 1.00 | 39.79 | C |
| ATOM | 2127 | O | THR C | 92 | 44.753 | 9.048 | 4.956 | 1.00 | 40.20 | O |
| ATOM | 2128 | N | ALA C | 93 | 42.888 | 8.083 | 4.102 | 1.00 | 35.20 | N |
| ATOM | 2129 | CA | ALA C | 93 | 43.526 | 6.857 | 3.639 | 1.00 | 32.82 | C |
| ATOM | 2130 | CB | ALA C | 93 | 44.176 | 6.126 | 4.787 | 1.00 | 34.79 | C |
| ATOM | 2131 | C | ALA C | 93 | 42.502 | 5.952 | 3.000 | 1.00 | 32.17 | C |
| ATOM | 2132 | O | ALA C | 93 | 41.289 | 6.215 | 3.058 | 1.00 | 32.06 | O |
| ATOM | 2133 | N | ILE C | 94 | 42.993 | 4.878 | 2.396 | 1.00 | 28.99 | N |
| ATOM | 2134 | CA | ILE C | 94 | 42.143 | 3.778 | 1.989 | 1.00 | 27.27 | C |
| ATOM | 2135 | CB | ILE C | 94 | 42.694 | 3.058 | 0.759 | 1.00 | 30.61 | C |
| ATOM | 2136 | CG1 | ILE C | 94 | 42.712 | 4.021 | -0.440 | 1.00 | 34.46 | C |
| ATOM | 2137 | CD1 | ILE C | 94 | 43.375 | 3.461 | -1.687 | 1.00 | 39.84 | C |
| ATOM | 2138 | CG2 | ILE C | 94 | 41.849 | 1.813 | 0.444 | 1.00 | 28.96 | C |
| ATOM | 2139 | C | ILE C | 94 | 42.091 | 2.811 | 3.166 | 1.00 | 30.50 | C |
| ATOM | 2140 | O | ILE C | 94 | 43.136 | 2.421 | 3.706 | 1.00 | 29.90 | O |
| ATOM | 2141 | N | TYR C | 95 | 40.885 | 2.451 | 3.589 | 1.00 | 28.91 | N |
| ATOM | 2142 | CA | TYR C | 95 | 40.723 | 1.574 | 4.746 | 1.00 | 26.51 | C |
| ATOM | 2143 | CB | TYR C | 95 | 39.650 | 2.112 | 5.690 | 1.00 | 28.70 | C |
| ATOM | 2144 | CG | TYR C | 95 | 40.129 | 3.280 | 6.501 | 1.00 | 28.19 | C |
| ATOM | 2145 | CD2 | TYR C | 95 | 40.439 | 3.133 | 7.839 | 1.00 | 28.60 | C |
| ATOM | 2146 | CE2 | TYR C | 95 | 40.892 | 4.196 | 8.587 | 1.00 | 29.89 | C |
| ATOM | 2147 | CZ | TYR C | 95 | 41.053 | 5.423 | 7.993 | 1.00 | 29.18 | C |
| ATOM | 2148 | OH | TYR C | 95 | 41.508 | 6.489 | 8.735 | 1.00 | 32.98 | O |
| ATOM | 2149 | CE1 | TYR C | 95 | 40.757 | 5.600 | 6.668 | 1.00 | 29.02 | C |
| ATOM | 2150 | CD1 | TYR C | 95 | 40.300 | 4.528 | 5.922 | 1.00 | 28.90 | C |
| ATOM | 2151 | C | TYR C | 95 | 40.328 | 0.208 | 4.264 | 1.00 | 29.02 | C |
| ATOM | 2152 | O | TYR C | 95 | 39.423 | 0.080 | 3.441 | 1.00 | 26.79 | O |
| ATOM | 2153 | N | PHE C | 96 | 40.989 | -0.826 | 4.767 | 1.00 | 26.37 | N |
| ATOM | 2154 | CA | PHE C | 96 | 40.622 | -2.165 | 4.333 | 1.00 | 27.67 | C |
| ATOM | 2155 | CB | PHE C | 96 | 41.552 | -2.698 | 3.225 | 1.00 | 31.60 | C |
| ATOM | 2156 | CG | PHE C | 96 | 42.996 | -2.671 | 3.583 | 1.00 | 34.69 | C |
| ATOM | 2157 | CD2 | PHE C | 96 | 43.625 | -3.811 | 4.076 | 1.00 | 37.06 | C |
| ATOM | 2158 | CE2 | PHE C | 96 | 44.984 | -3.790 | 4.413 | 1.00 | 38.02 | C |
| ATOM | 2159 | CZ | PHE C | 96 | 45.717 | -2.616 | 4.264 | 1.00 | 39.68 | C |
| ATOM | 2160 | CE1 | PHE C | 96 | 45.092 | -1.466 | 3.772 | 1.00 | 41.12 | C |
| ATOM | 2161 | CD1 | PHE C | 96 | 43.741 | -1.506 | 3.424 | 1.00 | 37.42 | C |
| ATOM | 2162 | C | PHE C | 96 | 40.547 | -3.118 | 5.501 | 1.00 | 28.03 | C |
| ATOM | 2163 | O | PHE C | 96 | 41.126 | -2.876 | 6.569 | 1.00 | 26.12 | O |
| ATOM | 2164 | N | CYS C | 97 | 39.782 | -4.177 | 5.292 | 1.00 | 24.72 | N |
| ATOM | 2165 | CA | CYS C | 97 | 39.572 | -5.203 | 6.289 | 1.00 | 28.55 | C |
| ATOM | 2166 | CB | CYS C | 97 | 38.113 | -5.641 | 6.234 | 1.00 | 30.73 | C |
| ATOM | 2167 | SG | CYS C | 97 | 37.535 | -6.685 | 7.581 | 1.00 | 34.65 | S |
| ATOM | 2168 | C | CYS C | 97 | 40.510 | -6.365 | 5.959 | 1.00 | 32.84 | C |
| ATOM | 2169 | O | CYS C | 97 | 40.827 | -6.611 | 4.785 | 1.00 | 31.92 | O |
| ATOM | 2170 | N | ALA C | 98 | 40.973 | -7.065 | 6.987 | 1.00 | 28.14 | N |
| ATOM | 2171 | CA | ALA C | 98 | 41.874 | -8.194 | 6.796 | 1.00 | 29.78 | C |
| ATOM | 2172 | CB | ALA C | 98 | 43.322 | -7.732 | 6.811 | 1.00 | 29.47 | C |
| ATOM | 2173 | C | ALA C | 98 | 41.658 | -9.251 | 7.864 | 1.00 | 30.96 | C |
| ATOM | 2174 | O | ALA C | 98 | 41.139 | -8.957 | 8.941 | 1.00 | 29.29 | O |
| ATOM | 2175 | N | ARG C | 99 | 42.060 | -10.479 | 7.550 | 1.00 | 29.19 | N |
| ATOM | 2176 | CA | ARG C | 99 | 42.070 | -11.562 | 8.514 | 1.00 | 34.39 | C |
| ATOM | 2177 | CB | ARG C | 99 | 41.683 | -12.876 | 7.848 | 1.00 | 37.20 | C |
| ATOM | 2178 | CG | ARG C | 99 | 41.698 | -14.078 | 8.789 | 1.00 | 41.12 | C |
| ATOM | 2179 | CD | ARG C | 99 | 42.220 | -15.284 | 8.041 | 1.00 | 45.24 | C |
| ATOM | 2180 | NE | ARG C | 99 | 41.433 | -16.488 | 8.233 | 1.00 | 50.54 | N |
| ATOM | 2181 | CZ | ARG C | 99 | 41.553 | -17.566 | 7.465 | 1.00 | 49.25 | C |
| ATOM | 2182 | NH1 | ARG C | 99 | 42.423 | -17.572 | 6.465 | 1.00 | 44.89 | N |
| ATOM | 2183 | NH2 | ARG C | 99 | 40.801 | -18.632 | 7.689 | 1.00 | 52.63 | N |
| ATOM | 2184 | C | ARG C | 99 | 43.476 | -11.668 | 9.084 | 1.00 | 34.29 | C |
| ATOM | 2185 | O | ARG C | 99 | 44.463 | -11.689 | 8.342 | 1.00 | 31.79 | O |
| ATOM | 2186 | N | ARG C | 100 | 43.568 | -11.694 | 10.408 | 1.00 | 32.91 | N |
| ATOM | 2187 | CA | ARG C | 100 | 44.862 | -11.833 | 11.058 | 1.00 | 31.67 | C |
| ATOM | 2188 | CB | ARG C | 100 | 44.976 | -10.911 | 12.278 | 1.00 | 28.80 | C |
| ATOM | 2189 | CG | ARG C | 100 | 46.301 | -11.101 | 12.992 | 1.00 | 31.23 | C |
| ATOM | 2190 | CD | ARG C | 100 | 46.599 | -10.039 | 14.026 | 1.00 | 30.81 | C |
| ATOM | 2191 | NE | ARG C | 100 | 47.856 | -10.353 | 14.709 | 1.00 | 36.35 | N |
| ATOM | 2192 | CZ | ARG C | 100 | 48.522 | -9.514 | 15.497 | 1.00 | 37.67 | C |
| ATOM | 2193 | NH1 | ARG C | 100 | 48.062 | -8.287 | 15.701 | 1.00 | 31.21 | N |
| ATOM | 2194 | NH2 | ARG C | 100 | 49.657 | -9.907 | 16.073 | 1.00 | 35.23 | N |
| ATOM | 2195 | C | ARG C | 100 | 45.102 | -13.277 | 11.479 | 1.00 | 32.50 | C |
| ATOM | 2196 | O | ARG C | 100 | 44.272 | -13.882 | 12.170 | 1.00 | 30.92 | O |
| ATOM | 2197 | N | VAL C | 101 | 46.227 | -13.836 | 11.045 | 1.00 | 32.11 | N |
| ATOM | 2198 | CA | VAL C | 101 | 46.621 | -15.162 | 11.509 | 1.00 | 36.26 | C |
| ATOM | 2199 | CB | VAL C | 101 | 46.565 | -16.217 | 10.404 | 1.00 | 34.16 | C |
| ATOM | 2200 | CG1 | VAL C | 101 | 46.934 | -17.602 | 10.980 | 1.00 | 36.37 | C |
| ATOM | 2201 | CG2 | VAL C | 101 | 45.177 | -16.251 | 9.793 | 1.00 | 32.42 | C |
| ATOM | 2202 | C | VAL C | 101 | 48.014 | -15.113 | 12.129 | 1.00 | 35.28 | C |
| ATOM | 2203 | O | VAL C | 101 | 49.026 | -14.886 | 11.448 | 1.00 | 30.99 | O |
| ATOM | 2204 | N | GLY C | 102 | 48.055 | -15.317 | 13.438 | 1.00 | 32.60 | N |
| ATOM | 2205 | CA | GLY C | 102 | 49.306 | -15.220 | 14.155 | 1.00 | 36.44 | C |
| ATOM | 2206 | C | GLY C | 102 | 49.780 | -13.786 | 14.112 | 1.00 | 35.93 | C |
| ATOM | 2207 | O | GLY C | 102 | 49.136 | -12.895 | 14.669 | 1.00 | 38.64 | O |
| ATOM | 2208 | N | SER C | 103 | 50.893 | -13.559 | 13.429 | 1.00 | 36.61 | N |
| ATOM | 2209 | CA | SER C | 103 | 51.469 | -12.228 | 13.345 | 1.00 | 40.30 | C |
| ATOM | 2210 | CB | SER C | 103 | 52.973 | -12.294 | 13.615 | 1.00 | 43.92 | C |
| ATOM | 2211 | OG | SER C | 103 | 53.238 | -12.777 | 14.925 | 1.00 | 47.13 | O |
| ATOM | 2212 | C | SER C | 103 | 51.226 | -11.601 | 11.986 | 1.00 | 39.48 | C |
| ATOM | 2213 | O | SER C | 103 | 51.518 | -10.425 | 11.781 | 1.00 | 45.34 | O |
| ATOM | 2214 | N | GLU C | 104 | 50.687 | -12.382 | 11.058 | 1.00 | 39.98 | N |
| ATOM | 2215 | CA | GLU C | 104 | 50.536 | -11.922 | 9.685 | 1.00 | 38.79 | C |
| ATOM | 2216 | CB | GLU C | 104 | 51.108 | -12.955 | 8.718 | 1.00 | 45.20 | C |
| ATOM | 2217 | CG | GLU C | 104 | 52.596 | -13.184 | 8.865 | 1.00 | 50.95 | C |
| ATOM | 2218 | CD | GLU C | 104 | 53.122 | -14.167 | 7.836 | 1.00 | 61.20 | C |
| ATOM | 2219 | OE1 | GLU C | 104 | 53.467 | -13.727 | 6.718 | 1.00 | 65.55 | O |
| ATOM | 2220 | OE2 | GLU C | 104 | 53.181 | -15.380 | 8.144 | 1.00 | 62.46 | O |
| ATOM | 2221 | C | GLU C | 104 | 49.088 | -11.668 | 9.319 | 1.00 | 39.75 | C |
| ATOM | 2222 | O | GLU C | 104 | 48.169 | -12.135 | 10.003 | 1.00 | 37.59 | O |
| ATOM | 2223 | N | TYR C | 105 | 48.883 | -10.925 | 8.235 | 1.00 | 35.29 | N |
| ATOM | 2224 | CA | TYR C | 105 | 47.535 | -10.708 | 7.735 | 1.00 | 36.13 | C |
| ATOM | 2225 | CB | TYR C | 105 | 47.258 | -9.211 | 7.492 | 1.00 | 37.36 | C |
| ATOM | 2226 | CG | TYR C | 105 | 47.282 | -8.439 | 8.794 | 1.00 | 32.94 | C |
| ATOM | 2227 | CD1 | TYR C | 105 | 46.170 | -8.414 | 9.623 | 1.00 | 31.61 | C |
| ATOM | 2228 | CE1 | TYR C | 105 | 46.196 | -7.742 | 10.842 | 1.00 | 27.23 | C |
| ATOM | 2229 | CZ | TYR C | 105 | 47.345 | -7.103 | 11.250 | 1.00 | 32.26 | C |
| ATOM | 2230 | OH | TYR C | 105 | 47.363 | -6.444 | 12.470 | 1.00 | 33.39 | O |
| ATOM | 2231 | CE2 | TYR C | 105 | 48.473 | -7.123 | 10.450 | 1.00 | 37.47 | C |
| ATOM | 2232 | CD2 | TYR C | 105 | 48.437 | -7.795 | 9.228 | 1.00 | 35.36 | C |
| ATOM | 2233 | C | TYR C | 105 | 47.282 | -11.587 | 6.517 | 1.00 | 44.68 | C |
| ATOM | 2234 | O | TYR C | 105 | 47.776 | -11.325 | 5.420 | 1.00 | 45.87 | O |
| ATOM | 2235 | N | ASP C | 106 | 46.525 | -12.653 | 6.762 | 1.00 | 45.96 | N |
| ATOM | 2236 | CA | ASP C | 106 | 46.192 | -13.687 | 5.788 | 1.00 | 45.60 | C |
| ATOM | 2237 | CB | ASP C | 106 | 45.172 | -14.628 | 6.433 | 1.00 | 42.63 | C |
| ATOM | 2238 | CG | ASP C | 106 | 45.337 | -16.059 | 5.998 | 1.00 | 48.30 | C |
| ATOM | 2239 | OD1 | ASP C | 106 | 46.487 | -16.474 | 5.750 | 1.00 | 52.76 | O |
| ATOM | 2240 | OD2 | ASP C | 106 | 44.315 | -16.779 | 5.920 | 1.00 | 53.44 | O |
| ATOM | 2241 | C | ASP C | 106 | 45.576 | -13.110 | 4.517 | 1.00 | 48.47 | C |
| ATOM | 2242 | O | ASP C | 106 | 46.187 | -13.123 | 3.445 | 1.00 | 47.20 | O |
| ATOM | 2243 | N | LEU C | 107 | 44.352 | -12.611 | 4.664 | 1.00 | 45.63 | N |
| ATOM | 2244 | CA | LEU C | 107 | 43.549 | -12.117 | 3.557 | 1.00 | 43.09 | C |
| ATOM | 2245 | CB | LEU C | 107 | 42.205 | -12.837 | 3.554 | 1.00 | 38.46 | C |
| ATOM | 2246 | CG | LEU C | 107 | 42.275 | -14.299 | 3.987 | 1.00 | 45.72 | C |
| ATOM | 2247 | CD1 | LEU C | 107 | 40.894 | -14.868 | 4.252 | 1.00 | 45.42 | C |
| ATOM | 2248 | CD2 | LEU C | 107 | 42.995 | -15.092 | 2.914 | 1.00 | 49.15 | C |
| ATOM | 2249 | C | LEU C | 107 | 43.293 | -10.642 | 3.794 | 1.00 | 44.87 | C |
| ATOM | 2250 | O | LEU C | 107 | 43.275 | -10.206 | 4.946 | 1.00 | 38.82 | O |
| ATOM | 2251 | N | TRP C | 108 | 43.070 | -9.884 | 2.723 | 1.00 | 41.13 | N |
| ATOM | 2252 | CA | TRP C | 108 | 42.688 | -8.480 | 2.851 | 1.00 | 43.19 | C |
| ATOM | 2253 | CB | TRP C | 108 | 43.912 | -7.610 | 3.122 | 1.00 | 43.19 | C |
| ATOM | 2254 | CG | TRP C | 108 | 44.951 | -7.658 | 2.053 | 1.00 | 47.34 | C |
| ATOM | 2255 | CD1 | TRP C | 108 | 44.977 | -6.925 | 0.897 | 1.00 | 48.46 | C |
| ATOM | 2256 | NE1 | TRP C | 108 | 46.106 | -7.226 | 0.172 | 1.00 | 51.93 | N |
| ATOM | 2257 | CE2 | TRP C | 108 | 46.840 | -8.162 | 0.857 | 1.00 | 52.19 | C |
| ATOM | 2258 | CD2 | TRP C | 108 | 46.143 | -8.454 | 2.051 | 1.00 | 49.43 | C |
| ATOM | 2259 | CE3 | TRP C | 108 | 46.688 | -9.388 | 2.941 | 1.00 | 49.37 | C |
| ATOM | 2260 | CZ3 | TRP C | 108 | 47.895 | -9.999 | 2.612 | 1.00 | 53.39 | C |
| ATOM | 2261 | CH2 | TRP C | 108 | 48.564 | -9.690 | 1.414 | 1.00 | 48.48 | C |
| ATOM | 2262 | CZ2 | TRP C | 108 | 48.056 | -8.776 | 0.529 | 1.00 | 51.10 | C |
| ATOM | 2263 | C | TRP C | 108 | 41.954 | -7.982 | 1.611 | 1.00 | 44.00 | C |
| ATOM | 2264 | O | TRP C | 108 | 42.216 | -8.450 | 0.508 | 1.00 | 40.88 | O |
| ATOM | 2265 | N | GLY C | 109 | 41.033 | -7.038 | 1.795 | 1.00 | 39.53 | N |
| ATOM | 2266 | CA | GLY C | 109 | 40.280 | -6.487 | 0.679 | 1.00 | 41.27 | C |
| ATOM | 2267 | C | GLY C | 109 | 40.979 | -5.318 | 0.011 | 1.00 | 41.44 | C |
| ATOM | 2268 | O | GLY C | 109 | 42.050 | -4.871 | 0.453 | 1.00 | 41.84 | O |
| ATOM | 2269 | N | GLN C | 110 | 40.377 | -4.817 | -1.063 | 1.00 | 40.67 | N |
| ATOM | 2270 | CA | GLN C | 110 | 40.922 | -3.648 | -1.752 | 1.00 | 45.45 | C |
| ATOM | 2271 | CB | GLN C | 110 | 40.403 | -3.553 | -3.186 | 1.00 | 46.48 | C |
| ATOM | 2272 | CG | GLN C | 110 | 41.126 | -4.485 | -4.155 | 1.00 | 48.25 | C |
| ATOM | 2273 | CD | GLN C | 110 | 42.642 | -4.349 | -4.102 | 1.00 | 47.56 | C |
| ATOM | 2274 | OE1 | GLN C | 110 | 43.189 | -3.243 | -4.128 | 1.00 | 51.67 | O |
| ATOM | 2275 | NE2 | GLN C | 110 | 43.329 | -5.482 | -4.030 | 1.00 | 53.72 | N |
| ATOM | 2276 | C | GLN C | 110 | 40.648 | -2.355 | -0.991 | 1.00 | 43.12 | C |
| ATOM | 2277 | O | GLN C | 110 | 41.409 | -1.390 | -1.102 | 1.00 | 48.13 | O |
| ATOM | 2278 | N | GLY C | 111 | 39.577 | -2.335 | -0.202 | 1.00 | 39.94 | N |
| ATOM | 2279 | CA | GLY C | 111 | 39.380 | -1.230 | 0.713 | 1.00 | 32.76 | C |
| ATOM | 2280 | C | GLY C | 111 | 38.408 | -0.168 | 0.254 | 1.00 | 32.23 | C |
| ATOM | 2281 | O | GLY C | 111 | 37.826 | -0.249 | -0.834 | 1.00 | 30.79 | O |
| ATOM | 2282 | N | THR C | 112 | 38.232 | 0.850 | 1.091 | 1.00 | 28.87 | N |
| ATOM | 2283 | CA | THR C | 112 | 37.300 | 1.924 | 0.775 | 1.00 | 28.97 | C |
| ATOM | 2284 | CB | THR C | 112 | 35.924 | 1.680 | 1.483 | 1.00 | 28.62 | C |
| ATOM | 2285 | OG1 | THR C | 112 | 34.968 | 2.664 | 1.072 | 1.00 | 25.58 | O |
| ATOM | 2286 | CG2 | THR C | 112 | 36.074 | 1.702 | 2.998 | 1.00 | 26.01 | C |
| ATOM | 2287 | C | THR C | 112 | 37.954 | 3.253 | 1.146 | 1.00 | 28.93 | C |
| ATOM | 2288 | O | THR C | 112 | 38.678 | 3.348 | 2.155 | 1.00 | 25.48 | O |
| ATOM | 2289 | N | GLN C | 113 | 37.731 | 4.272 | 0.317 | 1.00 | 23.64 | N |
| ATOM | 2290 | CA | GLN C | 113 | 38.471 | 5.526 | 0.446 | 1.00 | 26.56 | C |
| ATOM | 2291 | CB | GLN C | 113 | 38.582 | 6.238 | -0.907 | 1.00 | 27.67 | C |
| ATOM | 2292 | CG | GLN C | 113 | 39.193 | 7.638 | -0.804 | 1.00 | 31.10 | C |
| ATOM | 2293 | CD | GLN C | 113 | 40.684 | 7.583 | -0.555 | 1.00 | 33.34 | C |
| ATOM | 2294 | OE1 | GLN C | 113 | 41.391 | 6.808 | -1.201 | 1.00 | 33.25 | O |
| ATOM | 2295 | NE2 | GLN C | 113 | 41.173 | 8.402 | 0.377 | 1.00 | 31.16 | N |
| ATOM | 2296 | C | GLN C | 113 | 37.834 | 6.472 | 1.453 | 1.00 | 28.68 | C |
| ATOM | 2297 | O | GLN C | 113 | 36.632 | 6.754 | 1.379 | 1.00 | 25.49 | O |
| ATOM | 2298 | N | VAL C | 114 | 38.635 | 6.965 | 2.396 | 1.00 | 24.77 | N |
| ATOM | 2299 | CA | VAL C | 114 | 38.147 | 7.956 | 3.344 | 1.00 | 27.62 | C |
| ATOM | 2300 | CB | VAL C | 114 | 38.252 | 7.469 | 4.796 | 1.00 | 23.79 | C |
| ATOM | 2301 | CG1 | VAL C | 114 | 37.855 | 8.594 | 5.742 | 1.00 | 26.22 | C |
| ATOM | 2302 | CG2 | VAL C | 114 | 37.365 | 6.236 | 5.029 | 1.00 | 24.85 | C |
| ATOM | 2303 | C | VAL C | 114 | 38.978 | 9.218 | 3.186 | 1.00 | 29.79 | C |
| ATOM | 2304 | O | VAL C | 114 | 40.198 | 9.179 | 3.345 | 1.00 | 30.20 | O |
| ATOM | 2305 | N | THR C | 115 | 38.321 | 10.330 | 2.866 | 1.00 | 27.60 | N |
| ATOM | 2306 | CA | THR C | 115 | 39.019 | 11.595 | 2.632 | 1.00 | 29.49 | C |
| ATOM | 2307 | CB | THR C | 115 | 38.860 | 12.069 | 1.184 | 1.00 | 30.66 | C |
| ATOM | 2308 | OG1 | THR C | 115 | 39.400 | 11.081 | 0.308 | 1.00 | 30.76 | O |
| ATOM | 2309 | CG2 | THR C | 115 | 39.596 | 13.370 | 0.965 | 1.00 | 33.64 | C |
| ATOM | 2310 | C | THR C | 115 | 38.481 | 12.664 | 3.554 | 1.00 | 33.16 | C |
| ATOM | 2311 | O | THR C | 115 | 37.328 | 13.076 | 3.430 | 1.00 | 31.14 | O |
| ATOM | 2312 | N | VAL C | 116 | 39.322 | 13.104 | 4.486 | 1.00 | 30.64 | N |
| ATOM | 2313 | CA | VAL C | 116 | 38.920 | 14.053 | 5.507 | 1.00 | 32.59 | C |
| ATOM | 2314 | CB | VAL C | 116 | 39.359 | 13.573 | 6.892 | 1.00 | 34.59 | C |
| ATOM | 2315 | CG1 | VAL C | 116 | 38.812 | 14.479 | 7.971 | 1.00 | 39.90 | C |
| ATOM | 2316 | CG2 | VAL C | 116 | 38.887 | 12.140 | 7.121 | 1.00 | 32.74 | C |
| ATOM | 2317 | C | VAL C | 116 | 39.556 | 15.410 | 5.204 | 1.00 | 43.50 | C |
| ATOM | 2318 | O | VAL C | 116 | 40.778 | 15.515 | 5.020 | 1.00 | 43.07 | O |
| ATOM | 2319 | N | SER C | 117 | 38.723 | 16.442 | 5.127 | 1.00 | 40.68 | N |
| ATOM | 2320 | CA | SER C | 117 | 39.200 | 17.795 | 4.856 | 1.00 | 43.90 | C |
| ATOM | 2321 | CB | SER C | 117 | 38.486 | 18.362 | 3.636 | 1.00 | 42.37 | C |
| ATOM | 2322 | OG | SER C | 117 | 37.105 | 18.524 | 3.914 | 1.00 | 42.46 | O |
| ATOM | 2323 | C | SER C | 117 | 38.913 | 18.680 | 6.061 | 1.00 | 48.45 | C |
| ATOM | 2324 | O | SER C | 117 | 38.133 | 18.310 | 6.944 | 1.00 | 49.40 | O |
| ATOM | 2325 | N | SER C | 118 | 39.540 | 19.851 | 6.101 | 1.00 | 55.77 | N |
| ATOM | 2326 | CA | SER C | 118 | 39.199 | 20.839 | 7.115 | 1.00 | 50.09 | C |
| ATOM | 2327 | CB | SER C | 118 | 40.341 | 21.020 | 8.114 | 1.00 | 48.71 | C |
| ATOM | 2328 | OG | SER C | 118 | 39.901 | 21.745 | 9.245 | 1.00 | 53.21 | O |
| ATOM | 2329 | C | SER C | 118 | 38.854 | 22.161 | 6.444 | 1.00 | 56.01 | C |
| ATOM | 2330 | O | SER C | 118 | 38.179 | 22.185 | 5.412 | 1.00 | 54.58 | O |
| TER | 2333 | | SER C | 118 | | | | | | |
| ATOM | 2331 | O | HOH S | 2 | 15.485 | 17.826 | 1.824 | 1.00 | 29.83 | O |
| ATOM | 2332 | O | HOH S | 5 | 29.782 | -0.029 | -5.294 | 1.00 | 28.46 | O |
| ATOM | 2333 | O | HOH S | 7 | 27.444 | 10.428 | 10.712 | 1.00 | 34.98 | O |
| ATOM | 2334 | O | HOH S | 11 | 20.303 | 19.947 | 7.075 | 1.00 | 29.16 | O |
| ATOM | 2335 | O | HOH S | 12 | 20.816 | 9.997 | -16.960 | 1.00 | 32.47 | O |
| ATOM | 2336 | O | HOH S | 17 | 27.244 | 0.371 | -9.261 | 1.00 | 34.07 | O |
| ATOM | 2337 | O | HOH S | 19 | 11.304 | 21.382 | 12.623 | 1.00 | 36.20 | O |
| ATOM | 2338 | O | HOH S | 20 | 5.081 | 17.064 | 15.977 | 1.00 | 30.12 | O |
| ATOM | 2339 | O | HOH S | 21 | 20.308 | 12.222 | 12.916 | 1.00 | 26.83 | O |
| ATOM | 2340 | O | HOH S | 30 | 10.985 | 1.292 | -12.748 | 1.00 | 24.34 | O |
| ATOM | 2341 | O | HOH S | 31 | 21.579 | 7.623 | -22.579 | 1.00 | 41.16 | O |
| ATOM | 2342 | O | HOH S | 32 | 19.222 | 12.707 | -5.631 | 1.00 | 23.82 | O |
| ATOM | 2343 | O | HOH S | 33 | 14.978 | 9.610 | -14.403 | 1.00 | 28.01 | O |
| ATOM | 2344 | O | HOH S | 35 | 26.988 | 11.513 | -7.493 | 1.00 | 25.77 | O |
| ATOM | 2345 | O | HOH S | 37 | 29.244 | 18.902 | -6.815 | 1.00 | 38.00 | O |
| ATOM | 2346 | O | HOH S | 38 | 49.087 | 4.765 | 5.834 | 1.00 | 49.62 | O |
| ATOM | 2347 | O | HOH S | 39 | 12.418 | 10.130 | -8.474 | 1.00 | 33.33 | O |
| ATOM | 2348 | O | HOH S | 43 | 28.668 | 7.961 | 10.834 | 1.00 | 36.46 | O |
| ATOM | 2349 | O | HOH S | 44 | 24.106 | 18.054 | -4.663 | 1.00 | 27.87 | O |
| ATOM | 2350 | O | HOH S | 45 | 27.422 | 16.147 | -20.676 | 1.00 | 33.80 | O |
| ATOM | 2351 | O | HOH S | 46 | 28.357 | 6.850 | 8.064 | 1.00 | 32.22 | O |
| ATOM | 2352 | O | HOH S | 57 | 24.667 | 17.323 | -1.813 | 1.00 | 32.44 | O |
| ATOM | 2353 | O | HOH S | 61 | 27.227 | 10.266 | 16.227 | 1.00 | 41.91 | O |
| ATOM | 2354 | O | HOH S | 62 | 25.801 | 11.059 | -5.119 | 1.00 | 25.13 | O |
| ATOM | 2355 | O | HOH S | 63 | 28.755 | 1.474 | 6.111 | 1.00 | 32.94 | O |
| ATOM | 2356 | O | HOH S | 65 | 25.886 | 19.380 | 1.893 | 1.00 | 32.42 | O |
| TER | 2360 | | HOH S | 65 | | | | | | |
| HETATM | 2357 | O | HOH S | 70 | 21.776 | 4.083 | -22.570 | 1.00 | 49.15 | O |
| TER | 2362 | | HOH S | 70 | | | | | | |
| HETATM | 2358 | O | HOH S | 71 | 33.146 | 13.376 | 14.992 | 1.00 | 42.36 | O |
| TER | 2364 | | HOH S | 71 | | | | | | |
| ATOM | 2359 | O | HOH D | 1 | 29.872 | 5.422 | 6.167 | 1.00 | 31.04 | O |
| TER | 2366 | | HOH D | 1 | | | | | | |
| ATOM | 2360 | O | HOH E | 2 | 28.396 | 5.868 | -11.498 | 1.00 | 35.57 | O |
| ATOM | 2361 | O | HOH E | 4 | 25.351 | 11.804 | -9.606 | 1.00 | 24.49 | O |
| ATOM | 2362 | O | HOH E | 8 | 30.161 | 18.358 | 0.354 | 1.00 | 45.44 | O |
| ATOM | 2363 | O | HOH E | 11 | 32.440 | 15.306 | -8.847 | 1.00 | 37.72 | O |
| TER | 2371 | | HOH E | 11 | | | | | | |
| ATOM | 2364 | O | HOH F | 1 | 31.255 | 2.691 | -0.119 | 1.00 | 28.29 | O |
| ATOM | 2365 | O | HOH F | 2 | 30.316 | 6.097 | -11.047 | 1.00 | 38.68 | O |
| ATOM | 2366 | O | HOH F | 3 | 35.717 | 5.527 | -11.542 | 1.00 | 40.42 | O |
| ATOM | 2367 | O | HOH F | 5 | 13.080 | 12.466 | 2.875 | 1.00 | 22.80 | O |
| ATOM | 2368 | O | HOH F | 7 | 31.190 | 15.677 | 7.389 | 1.00 | 25.57 | O |
| ATOM | 2369 | O | HOH F | 8 | 31.971 | 17.350 | -0.021 | 1.00 | 42.10 | O |
| ATOM | 2370 | O | HOH F | 9 | 27.244 | 17.681 | 4.225 | 1.00 | 26.03 | O |
| ATOM | 2371 | O | HOH F | 10 | 5.788 | 11.952 | 10.362 | 1.00 | 31.85 | O |
| ATOM | 2372 | O | HOH F | 12 | 30.319 | -3.587 | 1.910 | 1.00 | 42.56 | O |
| ATOM | 2373 | O | HOH F | 13 | 29.575 | -5.659 | 11.601 | 1.00 | 42.45 | O |
| ATOM | 2374 | O | HOH F | 14 | 21.662 | 12.419 | 9.268 | 1.00 | 29.58 | O |
| ATOM | 2375 | O | HOH F | 15 | 23.754 | 11.934 | 8.793 | 1.00 | 28.48 | O |
| ATOM | 2376 | O | HOH F | 16 | 25.580 | 5.610 | -18.213 | 1.00 | 28.55 | O |
| ATOM | 2377 | O | HOH F | 17 | 23.468 | 4.844 | -19.256 | 1.00 | 33.22 | O |
| ATOM | 2378 | O | HOH F | 18 | 10.504 | 9.282 | 16.624 | 1.00 | 39.94 | O |
| TER | 2387 | | HOH F | 18 | | | | | | |
| ATOM | 2379 | O | HOH G | 1 | 22.240 | 14.042 | 12.461 | 1.00 | 34.14 | O |
| ATOM | 2380 | O | HOH G | 2 | 24.741 | 14.808 | 8.904 | 1.00 | 33.90 | O |
| ATOM | 2381 | O | HOH G | 4 | 48.654 | 0.924 | 19.881 | 1.00 | 38.95 | O |
| ATOM | 2382 | O | HOH G | 5 | 23.972 | 2.829 | 10.991 | 1.00 | 37.37 | O |
| ATOM | 2383 | O | HOH G | 6 | 25.558 | 2.933 | 8.907 | 1.00 | 30.37 | O |
| ATOM | 2384 | O | HOH G | 7 | 26.431 | 5.055 | 7.481 | 1.00 | 32.10 | O |
| ATOM | 2385 | O | HOH G | 8 | 28.469 | 0.534 | 3.553 | 1.00 | 33.77 | O |
| ATOM | 2386 | O | HOH G | 9 | 7.127 | -5.048 | -9.046 | 1.00 | 39.35 | O |
| ATOM | 2387 | O | HOH G | 10 | 5.390 | -0.115 | -6.095 | 1.00 | 39.67 | O |
| ATOM | 2388 | O | HOH G | 12 | 3.975 | 3.922 | -8.152 | 1.00 | 35.12 | O |
| ATOM | 2389 | O | HOH G | 13 | 5.868 | 4.445 | -10.525 | 1.00 | 38.82 | O |
| TER | 2399 | | HOH G | 13 | | | | | | |
| ATOM | 2390 | O | HOH H | 1 | 35.372 | 3.780 | -1.975 | 1.00 | 33.48 | O |
| TER | 2401 | | HOH H | 1 | | | | | | |
| HETATM | 2391 | O | HOH J | 1 | 9.124 | 11.909 | 13.306 | 1.00 | 31.33 | O |
| HETATM | 2392 | O | HOH J | 2 | 14.459 | 7.974 | 14.137 | 1.00 | 33.80 | O |
| HETATM | 2393 | O | HOH J | 4 | 13.888 | 11.780 | -10.507 | 1.00 | 26.36 | O |
| HETATM | 2394 | O | HOH J | 5 | 22.175 | 4.405 | 20.818 | 1.00 | 37.49 | O |
| TER | 2406 | | HOH J | 5 | | | | | | |
| HETATM | 2395 | O | HOH J | 7 | 7.821 | 10.767 | 15.465 | 1.00 | 40.89 | O |
| HETATM | 2396 | O | HOH J | 8 | 27.726 | 6.609 | 12.986 | 1.00 | 36.36 | O |
| HETATM | 2397 | O | HOH J | 9 | 8.197 | 19.069 | 14.881 | 1.00 | 40.18 | O |
| HETATM | 2398 | O | HOH J | 10 | 7.570 | 19.898 | 12.658 | 1.00 | 34.88 | O |
| HETATM | 2399 | O | HOH J | 11 | 21.538 | 21.444 | 2.684 | 1.00 | 36.32 | O |
| HETATM | 2400 | O | HOH J | 12 | 22.328 | 18.315 | -0.627 | 1.00 | 34.80 | O |
| HETATM | 2401 | O | HOH J | 13 | 25.284 | 19.882 | -5.693 | 1.00 | 37.97 | O |
| HETATM | 2402 | O | HOH J | 14 | 29.993 | 17.646 | 4.099 | 1.00 | 37.26 | O |
| HETATM | 2403 | O | HOH J | 15 | 26.591 | 18.199 | 6.581 | 1.00 | 37.86 | O |
| HETATM | 2404 | O | HOH J | 16 | 24.099 | 16.863 | 7.678 | 1.00 | 37.62 | O |
| HETATM | 2405 | O | HOH J | 17 | 21.268 | 18.802 | 11.144 | 1.00 | 32.43 | O |
| HETATM | 2406 | O | HOH J | 18 | 18.796 | 20.820 | 12.192 | 1.00 | 39.81 | O |
| HETATM | 2407 | O | HOH J | 19 | 27.211 | 18.710 | -1.958 | 1.00 | 39.03 | O |
| HETATM | 2408 | O | HOH J | 21 | 34.513 | 17.450 | 2.765 | 1.00 | 43.09 | O |
| HETATM | 2409 | O | HOH J | 22 | 21.969 | 17.920 | 8.448 | 1.00 | 36.82 | O |
| HETATM | 2410 | O | HOH J | 24 | 21.477 | -6.378 | -7.460 | 1.00 | 41.28 | O |
| HETATM | 2411 | O | HOH J | 25 | 25.817 | 0.253 | -13.387 | 1.00 | 36.98 | O |
| HETATM | 2412 | O | HOH J | 26 | 20.140 | -7.076 | -0.793 | 1.00 | 48.59 | O |
| HETATM | 2413 | O | HOH J | 28 | 13.514 | 9.331 | -11.832 | 1.00 | 31.85 | O |
| HETATM | 2414 | O | HOH J | 29 | 12.212 | 7.798 | -13.087 | 1.00 | 34.79 | O |
| HETATM | 2415 | O | HOH J | 30 | 13.235 | 10.627 | -17.473 | 1.00 | 41.26 | O |
| HETATM | 2416 | O | HOH J | 31 | 9.781 | 3.439 | -14.218 | 1.00 | 41.71 | O |
| HETATM | 2417 | O | HOH J | 32 | 11.529 | 13.455 | -4.400 | 1.00 | 37.47 | O |
| HETATM | 2418 | O | HOH J | 33 | 12.712 | 3.236 | 13.939 | 1.00 | 42.87 | O |
| HETATM | 2419 | O | HOH J | 34 | 17.874 | 17.178 | -5.879 | 1.00 | 38.55 | O |
| HETATM | 2420 | O | HOH J | 35 | 11.175 | -9.284 | -20.466 | 1.00 | 38.56 | O |
| HETATM | 2421 | O | HOH J | 36 | 13.174 | -12.948 | -19.859 | 1.00 | 32.10 | O |
| HETATM | 2422 | O | HOH J | 38 | 42.264 | -15.680 | 12.424 | 1.00 | 40.41 | O |
| HETATM | 2423 | O | HOH J | 39 | 31.378 | -7.046 | 18.228 | 1.00 | 46.23 | O |
| HETATM | 2424 | O | HOH J | 40 | 31.356 | 5.105 | -0.948 | 1.00 | 31.79 | O |
| TER | 2437 | | HOH J | 40 | | | | | | |
| HETATM | 2425 | O | HOH J | 43 | 19.910 | -0.699 | 12.877 | 1.00 | 39.78 | O |
| HETATM | 2426 | O | HOH J | 44 | 21.748 | 7.550 | -18.677 | 1.00 | 36.35 | O |
| HETATM | 2427 | O | HOH J | 45 | 23.051 | 5.822 | -21.773 | 1.00 | 40.77 | O |
| TER | 2441 | | HOH J | 45 | | | | | | |
| HETATM | 2428 | O | HOH J | 47 | 7.708 | 7.902 | 24.911 | 1.00 | 43.82 | O |
| HETATM | 2429 | O | HOH J | 48 | 23.871 | 21.749 | -1.662 | 1.00 | 46.23 | O |
| HETATM | 2430 | O | HOH J | 49 | 30.579 | 2.280 | -11.681 | 1.00 | 41.70 | O |
| HETATM | 2431 | O | HOH J | 50 | 38.148 | -5.367 | -1.616 | 1.00 | 42.21 | O |
| HETATM | 2432 | O | HOH J | 54 | 51.894 | -12.014 | 16.548 | 1.00 | 38.18 | O |
| TER | 2447 | | HOH J | 54 | | | | | | |
| HETATM | 2433 | O | HOH J | 55 | 26.610 | 7.705 | 17.822 | 1.00 | 40.50 | O |
| HETATM | 2434 | O | HOH J | 56 | 23.513 | 20.174 | -0.233 | 1.00 | 46.57 | O |
| HETATM | 2435 | O | HOH J | 57 | 27.504 | 19.708 | -4.867 | 1.00 | 46.51 | O |
| TER | 2451 | | HOH J | 57 | | | | | | |
| HETATM | 2436 | O | HOH J | 60 | 43.848 | -1.424 | 0.272 | 1.00 | 48.13 | O |
| HETATM | 2437 | O | HOH J | 61 | 34.403 | 3.921 | -4.464 | 1.00 | 37.85 | O |
| TER | 2454 | | HOH J | 61 | | | | | | |
| HETATM | 2438 | O | HOH J | 62 | 30.366 | 19.445 | 6.173 | 1.00 | 51.92 | O |
| HETATM | 2439 | O | HOH J | 63 | 31.129 | 15.444 | 4.295 | 1.00 | 37.69 | O |
| HETATM | 2440 | O | HOH J | 64 | 18.737 | 20.098 | 0.195 | 1.00 | 41.40 | O |
| HETATM | 2441 | O | HOH J | 65 | 14.199 | 19.657 | -5.548 | 1.00 | 52.43 | O |
| HETATM | 2442 | O | HOH J | 66 | 28.142 | 0.403 | -12.034 | 1.00 | 44.03 | O |
| HETATM | 2443 | O | HOH J | 67 | 9.523 | 20.204 | 13.271 | 1.00 | 43.38 | O |
| HETATM | 2444 | O | HOH J | 68 | 5.442 | 10.503 | 16.364 | 1.00 | 42.05 | O |
| HETATM | 2445 | O | HOH J | 69 | 37.250 | 6.665 | -11.572 | 1.00 | 47.30 | O |
| HETATM | 2446 | O | HOH J | 70 | 31.859 | 17.229 | 2.237 | 1.00 | 48.87 | O |
| HETATM | 2447 | O | HOH J | 71 | 32.197 | 7.389 | -11.177 | 1.00 | 38.34 | O |
| HETATM | 2448 | O | HOH J | 72 | 38.767 | 7.143 | -4.676 | 1.00 | 45.60 | O |
| HETATM | 2449 | O | HOH J | 73 | 23.590 | 15.739 | 11.583 | 1.00 | 40.67 | O |
| HETATM | 2450 | O | HOH J | 74 | 20.106 | -1.690 | -0.362 | 1.00 | 35.36 | O |
| HETATM | 2451 | O | HOH J | 75 | 24.202 | -7.340 | -3.012 | 1.00 | 46.93 | O |
| HETATM | 2452 | O | HOH J | 76 | 22.635 | -6.073 | -1.428 | 1.00 | 42.50 | O |
| HETATM | 2453 | O | HOH J | 77 | 12.917 | 11.198 | -14.957 | 1.00 | 42.21 | O |
| HETATM | 2454 | O | HOH J | 78 | 10.631 | 5.424 | -14.823 | 1.00 | 44.70 | O |
| HETATM | 2455 | O | HOH J | 79 | 11.624 | 2.331 | 12.108 | 1.00 | 52.03 | O |
| HETATM | 2456 | O | HOH J | 80 | 32.981 | -3.838 | 20.344 | 1.00 | 45.02 | O |
| HETATM | 2457 | O | HOH J | 81 | 35.445 | -1.991 | 21.515 | 1.00 | 42.53 | O |
| HETATM | 2458 | O | HOH J | 82 | 22.295 | 2.291 | -19.292 | 1.00 | 41.37 | O |
| HETATM | 2459 | O | HOH J | 83 | 23.909 | -2.357 | -12.302 | 1.00 | 45.61 | O |
| TER | 2477 | | HOH J | 83 | | | | | | |

The term "atomic coordinates" as used herein, refers to Cartesian coordinates derived from mathematical equations and corresponding to an atom's spatial relationship to other atoms in a molecule (such as a protein) or molecular complex. These atomic coordinates thus provide information of the three-dimensional (3D) molecular structure of a given molecule, and are preferably reported in a pdb ("protein data bank") format for macromolecules such as proteins (see http://www.wwpdb.org/docs.html). Atomic coordinates can also be used to obtain structural information about another crystallized molecule or molecular complex, such as by molecular replacement, or to obtain structural information about another non-crystallized molecule or molecular complex, such as by homology modeling. Those skilled in the art will understand that the set of atomic coordinates defines the overall three-dimensional structure of the Nef/sdAb19/Hck SH3 domain complex of the invention.

In particular, the present structure enables the identification of the Nef residues which mediate the interaction with sdAb19. These residues map to the C-terminal domain of Nef, but do not define a linear epitope. Rather, the said residues are scattered along the C-terminal domain of Nef and thus define a conformational epitope at the accessible surface of the viral protein. Indeed, the present inventors have shown that the Nef residues involved in sdAb19 interaction comprise any one of the following residues: Y139, K148, E155, R188, K192, M198, E201, L202, and H203, within a shell of 3.8 Å, wherein the residues of the Nef protein are numbered by reference to the sequence of accession number P03407 (SEQ ID NO. 2). More specifically, the inventors have been able to identify the residues which form direct interaction with sdAB19; the said residues include Y139, K148, E155, R188, K192, E201, and H203.

Therefore, in another aspect, the present invention provides a conformational epitope on the HIV-1 Nef protein which is bound by the sdAb19 antibody fragment, said conformational epitope comprising any one of the following residues: Y139, K148, E155, R188, K192, M198, E201, L202, and H203, and all those residues in close proximity that affect the surface structure of this conformational epitope. Such residues which affect the surface structure of the conformational epitope contribute to the binding of sdAb19 to Nef and comprise the following residues: T132, P133, G134, P135, 1137, Y139, K148, V150, P151, V152, P154, E155, K156, V157, R188, Asp190, K192, F195, H196, H197, M198, E201, L202, H203, E205. Preferably, the said conformational epitope comprises all of the above residues. Even more preferably, the said conformational epitope consists of all the above residues.

In another aspect, the present invention provides a set of Nef residues which mediate the interaction with the sdAb19 antibody, wherein the said set comprises any one of the following residues of the Nef protein: Y139, K148, E155, R188, K192, M198, E201, L202, and H203. Preferably, the said set of Nef residues comprises all of the above residues. Even more preferably, the said set of Nef residues consists of all the above residues.

The SH3 domain of Hck seems to be an important stabilization factor of Nef for protein crystallization. Without using the SH3 domain only thin, small, sensitive and bad diffracting crystals could be generated. Therefore, the addition of the Hck SH3 domain as stabilizing agent helped significantly and was the resolution for success.

Covalent fusions of the said SH3 domain to the sdAb19 antibody fragment are known from the prior art (Järviluoma et al., PLoS One, 7(7):e40331, 2012; Bouchet et al., J Virol, 86(9): 4856-4867, 2012). They have been shown to bind to several binding surfaces of Nef, resulting in highly potent *in vitro* inhibition of all the Nef functions (Bouchet et al., J Virol, 86(9): 4856-4867, 2012). Surprisingly, these fusion proteins, called Neffins, are totally unable to generate any crystal when bound to Nef. It is only when the SH3 domain is added as a separate, independent molecule that Nef/sdAb19/Hck SH3 domain crystals can be recovered.

It is also an object of the invention to provide a computer-readable data storage material encoded with computer-readable data comprising the atomic structure coordinates of the crystal according to the present invention, preferably the three-dimensional structure of the Nef/sdAb19/Hck SH3 domain which substantially conforms to the atomic coordinates represented by Table 1.

In yet another aspect, the present invention relates to a method for producing a crystal of the protein complex of the invention, said method comprising the following steps:
a) providing a protein solution comprising the complex of the Nef, sdAb19 and Hck SH3 domain polypeptides of the invention,
b) subjecting said polypeptide solution to conditions which promote optimal crystallization.

In a preferred embodiment of the present invention, the Nef, sdAb19 and Hck SH3 domain polypeptides are first expressed as recombinant proteins and then purified to greater than 80% total protein, more preferably greater than 90%, and even more preferably purified to greater than 95% total protein. For expression and purification purposes, the gene encoding each polypeptide may be subcloned into a convenient vector.

In a further preferred embodiment, the step a) of the method of the present invention comprises the further steps of:
i. constructing a recombinant vector by cloning the gene encoding each polypeptide into an expression vector;
ii. transforming the recombinant vector obtained in step i) into a host cell;
iii. expressing the polypeptide encoded by the said recombinant vector; and
iv. purifying the said polypeptide.

Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art (see, for example, the techniques described in Sambrook et al., 1990, Molecular Cloning, A Laboratory Manual, 2d Ed., Cold Spring Harbor Laboratory, Cold Spring Harbor, N.Y. and Ausubel et al., eds., 1998, Current Protocols in Molecular Biology, John Wiley & Sons, NY). Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan. Thus, it is within the scope of the invention to provide a vector for expressing any of the above Nef, sdAb19 and Hck SH3 domain polypeptides.

In order to express the polypeptides of the invention, the polynucleotides encoding said polypeptides are inserted into expression vectors such that the genes are operatively linked to transcriptional and translational sequences. Expression vectors include plasmids, YACs, cosmids, retrovirus, adenovirus, EBV-derived episomes, and all the other vectors that the skilled man will know to be convenient for ensuring the expression of the protein of interest. Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

In addition, it may be useful, e.g. for ease of purification, to use expression vectors containing tags. According to this embodiment, subcloning of a nucleic acid of the present invention into the said expression vector results in an expression construct encoding a fusion protein wherein the protein of the invention is fused to the said tag. As referred to herein, a "tag" is any additional amino acids which are provided in a protein either C-terminally, N-terminally or internally for the ease of purification, for the improvement of production or for any other purpose which may facilitate the goals of the present invention (e.g, to achieve higher levels of production and/or purification). Such tags include tags known to those skilled in the art to be useful in purification such as, but not limited to, His tag, glutathione-S-transferase tag, flag tag, mbp (maltose binding protein) tag, etc. In a preferred embodiment, the wild-type and mutant sdAB19 protein of the present invention are tagged with glutathione-S-transferase (see example 1 below). In another preferred embodiment, the wild-type and mutant HIV-1 Nef proteins are His-tagged (see example 1 below). Finally, in yet another preferred embodiment, the Hck SH3 domain of the present invention is tagged with glutathione-S-transferase (see example 1 below). Such tagged proteins may also be engineered to comprise a cleavage site, such as a thrombin, TEV (tobacco etch virus), enterokinase or factor X cleavage site, for ease of removal of the tag before, during or after purification. Vector systems which provide a tag and a cleavage site for removal of the tag are particularly useful to make the expression constructs of the present invention.

A large number of vector-host systems known in the art may be used. Examples of vectors include *E. coli* bacteriophages such as lambda derivatives, or plasmids such as pBR322 derivatives or pUC plasmid derivatives, e.g., pGEX vectors (GE Healthcare Life Sciences), pET vectors (Novagen, Madison, WI), pMal vectors (New England Biolabs), pFLAG vectors (Sigma Aldrich), pProEx (Invitrogen), baculovirus vectors (Invitrogen, Pharmingen), etc. The insertion into a cloning vector can, for example, be accomplished by ligating the DNA fragment into a cloning vector which has complementary cohesive termini, by blunt end ligation if no complementary cohesive termini are available or through nucleotide linkers using techniques standard in the art, e.g., Ausubel et al. (eds.), Current Protocols in Molecular Biology, (1992).

In a preferred embodiment, the gene encoding the Nef protein of the invention (SEQ ID NO. 3) is subcloned into pProEx-HTa (Invitrogen). In another preferred embodiment, the polynucleotide encoding sdAb19 (SEQ ID NO. 8) is subcloned into pGEX-4T1 tev (GE Healthcare Life Sciences). In yet another preferred embodiment, the gene encoding the SH3 domain of the Hck protein (SEQ ID NO. 10) is subcloned into pGEX-2T tev (as described in Horenkamp et al., Traffic, 12(7): 867-877, 2011). More preferably, the genes encoding the Nef protein of the invention, the sdAb19 antibody fragment, and the SH3 domain of Hck are subcloned into the pProEx-HTa, pGEX-4T1 tev, and pGEX-2T tev vectors, respectively.

Polynucleotides of the invention and vectors comprising these molecules can be used for the transformation of a suitable host cell. Transformation can be performed by any known method for introducing polynucleotides into a cell host. Such methods are well known of the man skilled in the art and include dextran-mediated transformation, calcium phosphate precipitation, polybrene-mediated transfection, protoplast fusion, electroporation, encapsulation of the polynucleotide into liposomes, biolistic injection and direct microinjection of DNA into nuclei. Therefore, the invention also encompasses a host cell comprising a vector of the invention.

The nature of the host cell will be dictated by the intended use of the vector of the invention. For example, a cloning vector will usually be maintained and propagated in bacterial cells. On the other hand, an expression vector may be transformed either in a bacterial cell, a yeast cell or a mammalian cell in order to express the polypeptides of the invention. In this case, for long-term, high-yield production of recombinant proteins, stable expression is preferred. Host cells can be transformed with a recombinant vector comprising DNA controlled by appropriate expression control elements (e.g., promoter, enhancer, sequences, transcription terminators, polyadenylation sites, etc.), and a selectable marker. The vector may also contain an origin of replication for maintaining the said vector in the said host cell. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the polypeptides of the invention.

Preferably, the said host cell is a eukaryotic cell; more preferably, it is a prokaryotic cell; even more preferably it is a bacterial cell. Most preferably, the host cell is *Escherichia coli.*

The protein of the invention may be prepared by growing a culture of transformed host cells under culture conditions necessary to express the desired protein. The resulting expressed protein may then be purified from the culture medium or cell extracts. Soluble forms of the protein of the invention can be purified from conditioned media.

The protein can be purified using methods known to those skilled in the art. For example, the protein of the invention can be concentrated using a commercially available protein concentration filter, for example, an Amicon or Millipore Pellicon ultrafiltration unit. Following the concentration step, the concentrate can be applied to a purification matrix such as a gel filtration medium. Alternatively, an anion exchange resin can be employed, for example, a matrix or substrate having pendant diethylaminoethyl (DEAE) or polyetheyleneimine (PEI) groups. The matrices can be acrylamide, agarose, dextran, cellulose or other types commonly employed in protein purification.

Alternatively, a cation exchange step can be employed. Suitable cation exchangers include various insoluble matrices comprising sulfopropyl or carboxymethyl groups. Sulfopropyl groups are preferred (e.g., S-Sepharose B columns). The purification of the protein may also include one or more column steps over such affinity resins as concanavalin A-agarose, heparin-Toyopeart or Cibacrom blue 3GA Sepharose B; or by hydrophobic interaction chromatography using such resins as phenyl ether, butyl ether, or propyl ether; or by immunoaffinity chromatography, such as glutathione-sepharose or an equivalent resin, nickel or cobalt-purification resins, antiflag resin, amylose resin, etc. Finally, one or more reverse-phase high performance liquid chromatography (RP-HPLC) steps employing hydrophobic RP-HPLC media, e.g., silica gel having pendant methyl or other aliphatic groups, can be employed to further purify the protein of the invention.

Some or all of the foregoing purification steps, in various combinations or with other known methods, can also be employed to provide a substantially purified isolated recombinant protein. Preferably, the isolated protein of the invention is purified so that it is substantially free of other proteins. By "substantially free of other proteins", it is herein meant that the protein solution resulting from the said purification contains, as a percent of its total protein, no more than 10%, preferably no more than 5%, more preferably no more than 1%, and even more preferably no more than 0.1%, of non-recombinant proteins of the invention. Proteins that are shown to crystallize are typically of a homogeneity that refers to more than 90 % purity and more preferably to 95% purity.

Once each of the polypeptides of the invention is purified, it may be advantageous to assembly a tripartite recombinant complex.

In a further preferred embodiment, the step a) of the method of the present invention thus comprises a further step v) of assembling the complex of the three polypeptides.

By "assembling a protein complex", it is herein meant that the proteins constituting the said complex are mixed in a solution until the said protein complex forms. In the present case, the inventors have observed that the said individually purified proteins can be added at the same time or sequentially, without any marked effect on crystallization. Thus in one embodiment, the three polypeptides are added at the same time, and the ternary complex is allowed to form. In another embodiment, the three proteins are added sequentially. According to this embodiment, either the sdAb19 or the SH3 domain is first added to the Nef protein. After the binary complex has formed, the third protein is added and the tripartite complex is allowed to form.

In a preferred embodiment, each of the polypeptides of the invention is added at a molar ratio comprised between 0.1 and 1; preferably the molar ratio of the said polypeptide is comprised between 0.5 and 1; more preferably the molar ratio of each polypeptide is around 1, i.e. the three purified proteins are added in a molar ratio of nearly 1:1:1.

In a yet further step vi) of the step a) of the method of the present invention, the tripartite protein complex is then purified.

Any of the methods known in the art for purifying multiprotein complexes can be used, for example, chromatography (e.g., ion exchange, affinity, size exclusion, and so on), centrifugation, differential solubility or by any other standard technique for the purification of proteins. Suitable methods of purification will be apparent to a person of ordinary skills in the art. Preferably, the said three-polypeptide complex is purified by size-exclusion chromatography.

After purification, the complex of the polypeptides of the invention may be concentrated to greater than 1 mg/ml for crystallization purposes. In a preferred embodiment the said polypeptides complex is concentrated to greater than 5 mg/ml for crystallization; in a more preferred embodiment, the said complex is concentrated to greater than 15 mg/ml; in the most preferred embodiment, the said complex is concentrated to greater than 20 mg/ml.

Protein complexes containing mutants of at least one of the polypeptides of the invention may alternatively also be crystallized to again yield structural data and insight into the complex structure and/or the binding of Nef ligands to Nef. Thus one embodiment of the present invention relates to a crystallized molecular complex of Nef/sdAb19/Hck SH3 domain, wherein at least one of the said proteins is mutated before being crystallized.

In a preferred embodiment, the crystals of the present invention comprise purified wild-type Nef/sdAb19/Hck SH3 domain and are grown at room temperature by the hanging-drop vapor-diffusion method.

Crystals may be prepared using commercially available screening kits such as, polyethylene glycol (PEG)/ion screens, PEG grid, ammonium sulfate grid, PEG/ammonium sulfate grid or the like purchased from Hampton Research, Emerald Biostructure, Molecular Dimension and from others.

Typically the vapor diffusion buffer comprises 0-27.5 %, preferably 2.5-27.5 % PEG 1 K-20 K, preferably 1-8K or PEG 2000 MME-5000 MME, preferably PEG 2000 MME, or 0-10 % Jeffamine M-600 and/or 5-20 %, e. g. 10-20 % propanol or 15-20 % ethanol or about 15 %-30 %, e. g. about 15 % 2-methyl-2,4-pentanediol (MPD), optionally with 0.01 M-1.6 M salt or salts and/or 0-0.15, e. g. 0-0.1, M of a solution buffer and/or 0-35 %, such as 0-1 5%, glycerol and/or 0- 35 % PEG 300-400; but preferably: 10-25 % PEG 1 K-8K or PEG 2000 MME or 0-10 % Jeffamine M-600 and/or 5-15 %, e. g. 10-15 %, propanol or ethanol, optionally with 0.1 M-0.2 M salt or salts and/or 0-0.15, e. g. 0-0.1 M solution buffer and/or PEG 400, but more preferably: 15-20 % PEG 3350 or PEG 4000 or PEG 2000 MME or 0-10 % Jeffamine M-600 or 5-15 %, e. g. 10-15 % propanol or ethanol, optionally with 0.1 M-0.2 M salt or salts and/or 0-0.15 M solution buffer.

The salt may be an alkali metal (particularly lithium, sodium and potassium), alkaline earth metal (e.g. magnesium or calcium), ammonium, ferric, ferrous or transition metal salt (e.g. zinc) of a halide (e.g. bromide, chloride or fluoride), acetate, formate, nitrate, sulfate, tartrate, citrate or phosphate. This includes sodium fluoride, potassium fluoride, ammonium fluoride, ammonium acetate, lithium acetate, magnesium acetate, sodium acetate, potassium acetate, calcium acetate, zinc acetate, ammonium chloride, lithium chloride, magnesium chloride, potassium chloride, sodium chloride, potassium bromide, magnesium formate, sodium formate, potassium formate, ammonium formate, ammonium nitrate, lithium nitrate, potassium nitrate, sodium nitrate, ammonium sulfate, potassium sulfate, lithium sulfate, sodium sulfate, di-sodium tartrate, potassium sodium tartrate, di-ammonium tartrate, potassium dihydrogen phosphate, tri-sodium citrate, tri-potassium citrate, zinc acetate, ferric chloride, calcium chloride, magnesium nitrate, magnesium sulfate, sodium dihydrogen phosphate, di-sodium hydrogen phosphate, di-potassium hydrogen phosphate, ammonium dihydrogen phosphate, di-ammonium hydrogen phosphate, tri-lithium citrate, nickel chloride, ammonium iodide, di-ammonium hydrogen citrate.

Solution buffers if present include, for example, Hepes, Tris, imidazole, cacodylate, tri-sodium citrate/citric acid, tri-sodium citrate/HCl, acetic acid/sodium acetate, phosphate-citrate, sodium potassium phosphate, 2- (N-morpholino)-ethane sulphonic acid/NaOH (MES), CHES, bis- trispropane, CAPS, potassium dihydrogen phosphate, sodium dihydrogen phosphate, dipotassium hydrogen phosphate or disodium hydrogen phosphate.

Specifically preferred crystallization conditions for the protein complex described herein are: 0.2 M CHKO₂, 17.5% PEG 3350, 0.35 M NH₄Cl.

The pH range is desirably maintained at pH 4.2-10.5, preferably 4.2-9.5, more preferably 4.7-9.5, even more preferably 6.5-9.5, and most preferably 9.0.

According to another preferred embodiment, in step b) as described above, the crystallization temperature is of 5°C to 30°C, more preferably of 10° to 25°C, even more preferably of 20°C.

Crystals may be prepared using a Hampton Research Screening kit, Poly-ethylene glycol (PEG) /ion screens, PEG grid, Ammonium sulfate grid, PEG/ammonium sulfate grid or the like.

In a further aspect, the present invention provides a method for determining the three-dimensional structure of the crystal of the present invention, said method comprising the steps of:
a) providing at least one crystal comprising or consisting of the tripartite polypeptide complex of the invention, said crystal being preferably obtained according to the method for producing a crystal as described above;
b) flash-cooling said crystal into liquid nitrogen after a brief transfer to a cryo-protecting buffer; and
c) analyzing said crystal to determine its three-dimensional structure.

Said method can thus enable the determination of the atomic coordinates which define the three-dimensional structure of the crystal according to the invention.

A cryo-protection buffer according to the invention is a buffer which prevents the formation of ice crystals in the protein crystal during the flash-cooling step. Such buffers are well known in the art (see e.g. Rodgers, Structure, 2(12): 1135-1140, 1994) and include e.g. glycerol, perfluoropolyether oil, ethylene glycol, polyethylene glycol 400, xylitol, (2R, 3R)-(-)-butane-2,3-diol, erythritol, glucose, and 2-methyl-2,4-pentanediol.

According to another preferred embodiment, the analysis of step c) is carried out by X-ray diffraction. Data sets generated from the diffraction analysis can be analyzed by using any appropriate well known software, including, without limitation, Xia2, XDS, MOSFLM and SCALA from the CCP4 program suite (http://www.ccp4.ac.uk), AutoSol from Phenix (http://www.phenix-online.org), MOLREP, REFMAC, TLS, MolProbity, DALI and combinations thereof.

By analyzing the three-dimensional structure of the crystal according to the invention, one skilled in the art can determine critical sites involved in the interaction of sdAb19 with Nef, and thus identify compounds capable of mimicking said processes. Said compounds may then be useful for therapeutic strategies, wherein it might be desired to inhibit partially or completely the various functions of Nef. Indeed, until the elucidation of the three-dimensional structure of the crystal according to the invention, no information was available for structure-based development of therapeutic compounds based on the identification of the Nef/sdAb19 binding interface. It should be noted that mutations in the corresponding Nef-binding region of sdAb19 abolish the antibody ability to inhibit Nef functions, thus emphasizing the functional relevance of the domain identified herein.

In a first aspect, the identification of the Nef residues involved in the interaction with sdAb19 enables to directly assess whether a candidate compound interacts with Nef via the sdAb19 binding region.

For example, the skilled person can introduce mutations in this region by site-directed mutagenesis, and thus generate mutants of Nef unable to bind sdAb19. Any Nef/sdAb19 binding interface-mediated interaction will thus be abrogated as well. Preferably, the binding of a compound which interacts with Nef via the sdAb19 binding region is reduced or abolished when the said region is mutated.

According to this embodiment, the invention provides a method for determining whether a compound interacts with Nef via the sdAb19 binding region, said method comprising the steps of:
a) measuring the binding of said compound to wild-type Nef;
b) measuring the binding of said compound to said Nef protein mutated in the sdAb19-binding region; and
c) comparing the binding of step a) and the binding of step b).

In a preferred embodiment, the sdAb19 binding region of Nef protein comprises any one of the following residues: Y139, K148, E155, R188, K192, M198, E201, L202, and H203, wherein the residues of the Nef protein are numbered by reference to the sequence of UniProtKB/Swiss-Prot accession number P03407 (SEQ ID NO. 2). More preferably, said region comprises all of the above residues. Even more preferably, the said region consists of the group of the above residues.

In another aspect, the present invention relates to a method of screening a library for a compound capable of inhibiting Nef functions, said method comprising the steps of:
a) determining whether each compound of the said library is capable of interacting with Nef via the sdAb19 binding region, according to the method described above; and
b) selecting the compound of step a), if said compound interacts with Nef via the sdAb19 binding region.

In another aspect, the present invention enables the design, selection and synthesis of chemical compounds, including inhibitory compounds, capable of binding to Nef, including binding at the intermolecular interfaces between Nef and sdAb19. The invention can also be used to identify and characterize accessory binding sites By "accessory binding sites", it is herein referred to binding sites, such as e.g. patches for small molecules, at the sides of the interface which interfere with the Nef-sdAb19 binding and thus result in changes. Furthermore, this invention can be used to rationally and semi-rationally design mutants of Nef with altered or improved characteristics and to theoretically model and facilitate experimental determination by X-ray crystallography of the structures of homologous proteins, including related Nef from other species.

According to the present invention, the crystal of the Nef/sdAb19/Hck SH3 domain complex can be used to determine the ability of a compound to bind to the Nef protein via its sdAb19-binding interface, wherein this ability is predicted by a structure-based, drug-design method that is based on the three-dimensional structure of the complex of the present invention. The predicted binding of the chemical compound to the crystal of the present invention is determined by methods standard in the art.

Potential compounds which can bind Nef by interacting with the same residues as sdAb19, and so can inhibit critical biological activities of the protein, can be designed using structure-based drug design. In particular, said potential compounds can be designed to interact with the side chain of one or more Nef amino acid residues of the Nef/sdAb19 binding interface.

Until the discovery of the three-dimensional structure of the present invention, no information was available for structure-based development of therapeutic compounds based on the structure of the binding interface between Nef and sdAb19. Such rational development could not be executed *de novo* from available linear amino acid sequence information, since the epitope recognized by sdAb19 is a conformational epitope.

Structure-based drug design refers to the use of computer simulation to predict a conformational interaction between the three-dimensional sdAb19-binding interface on the Nef protein structure of the present invention and a potential therapeutic compound. For example, generally, for a protein to effectively interact with a therapeutic compound, it is necessary that the three-dimensional structure of the therapeutic compound assume a compatible conformation that allows the compound to bind to the protein in such a manner that a desired result is obtained upon binding. Knowledge of the three-dimensional structure of the protein enables a skilled artisan to design a therapeutic compound having such a compatible conformation.

Thus, the present invention also relates to a method for identifying, screening, characterising or designing compounds interacting with the Nef protein via the sdAb19 binding region of the said protein. According to this embodiment, the method of the invention comprises using the three-dimensional structure of the Nef protein in determining whether said compound interacts with the said region. Advantageously, the compound thus identified is capable of inhibiting at least one Nef function.

Well-known atomic structures of the Nef protein can be used in the invention. In particular, the structural model of Nef protein can be generated according to known atomic coordinates of Nef protein (Grzesiek et al., Nat.Struct.Mol.Biol. 3: 340-345, 1996; Arold et al., Structure, 5(10): 1361-1372, 1997; Grzesiek et al., Protein Sci. 6: 1248-1263, 1997) or according to the atomic coordinates of the tripartite protein complex of the invention (Table 1).

In a preferred embodiment, the present invention relates to a method of identifying, screening, characterising or designing a compound capable of inhibiting Nef functions, said method comprising the steps of:
a) modeling the atomic structure of the Nef protein with a test compound;
b) determining if the test compound interacts with the sdAb19 binding region of the three-dimensional structure of Nef.

In a more preferred embodiment, determining if the test compound interacts with the Nef protein comprises the steps of:
i) determining a predicted minimum interaction energy, a predicted binding constant, a predicted dissociation constant, or a combination thereof, for the test compound in the model of Nef protein;
ii) analysing the data obtained in step i) to determine the characteristics of the association between the compound and the Nef protein or the complex thereof.

In an aspect of the invention, the sdAb19 binding region of Nef protein is obtained from the detailed map of interactions between sdAb19 and Nef protein of Table 2.

In a preferred embodiment, the sdAb19 binding region of Nef protein comprises at least one of the following residues: Y139, K148, E155, R188, K192, M198, E201, L202, and H203, wherein the residues of the Nef protein are numbered by reference to the sequence of UniProtKB/Swiss-Prot accession number P03407 (SEQ ID NO. 2). More preferably, said region comprises all of the above residues. Even more preferably, the said region consists of the group of the above residues. Yet still more preferably, the said region consists of the group of the following residues: Y139, K148, E155, R188, K192, E201, and H203.It will be immediately obvious to the person of skills in the art that the method of the invention enables the identification of structural analogs of compounds interacting with the sdAb19-binding region of the Nef protein. Analogs thus identified can then be tested for interaction with Nef.

**Table 2: Interaction distances between sdAb19 and Nef within a shell of 3.8 Å:**

| **sdAb19** | | | | | | **NefSF2** | |
|---|---|---|---|---|---|---|---|
| ND2 | ASN C | 35 - | O | GLU B | 201 | Distance: | 3.11 |
| CD | ARG C | 46 - | O | GLU B | 155 | Distance: | 3.78 |
| | | | | | | | |
| N | TRP C | 49 - | OE1 | GLU B | 155 | Distance: | 3.35 |
| CZ3 | TRP C | 49 - | CD1 | ILE B | 137 | Distance: | 3.50 |
| CH2 | TRP C | 49 - | CD1 | ILE B | 137 | Distance: | 3.73 |
| | | | | | | | |
| CG2 | THR C | 54 - | CD | GLU B | 201 | Distance: | 3.24 |
| CG2 | THR C | 54 - | OE1 | GLU B | 201 | Distance: | 2.97 |
| CG2 | THR C | 54 - | OE2 | GLU B | 201 | Distance: | 3.27 |
| | | | | | | | |
| N | SER C | 56 - | OE1 | GLU B | 201 | Distance: | 3.18 |
| CA | SER C | 56 - | OE1 | GLU B | 201 | Distance: | 3.68 |
| CB | SER C | 56 - | OE1 | GLU B | 201 | Distance: | 3.03 |
| OG | SER C | 56 - | OE1 | GLU B | 201 | Distance: | 2.84 |
| | | | | | | | |
| CB | TYR C | 58 - | CG | HIS B | 196 | Distance: | 3.71 |
| CB | TYR C | 58 - | ND1 | HIS B | 196 | Distance: | 3.39 |
| CB | TYR C | 58 - | SD | MET B | 198 | Distance: | 3.73 |
| CG | TYR C | 58 - | CB | HIS B | 196 | Distance: | 3.60 |
| CG | TYR C | 58 - | CG | HIS B | 196 | Distance: | 3.26 |
| CG | TYR C | 58 - | CD2 | HIS B | 196 | Distance: | 3.60 |
| CG | TYR C | 58 - | ND1 | HIS B | 196 | Distance: | 3.44 |
| CD1 | TYR C | 58 - | CG | HIS B | 196 | Distance: | 3.36 |
| CD1 | TYR C | 58 - | CD2 | HIS B | 196 | Distance: | 3.18 |
| CD1 | TYR C | 58 - | ND1 | HIS B | 196 | Distance: | 3.52 |
| CD1 | TYR C | 58 - | CE1 | HIS B | 196 | Distance: | 3.44 |
| CD1 | TYR C | 58 - | NE2 | HIS B | 196 | Distance: | 3.22 |
| CD2 | TYR C | 58 - | CB | HIS B | 196 | Distance: | 3.41 |
| CD2 | TYR C | 58 - | CG | HIS B | 196 | Distance: | 3.58 |
| CD2 | TYR C | 58 - | CD | GLU B | 201 | Distance: | 3.58 |
| CD2 | TYR C | 58 - | OE1 | GLU B | 201 | Distance: | 3.63 |
| CD2 | TYR C | 58 - | OE2 | GLU B | 201 | Distance: | 2.82 |
| CE1 | TYR C | 58 - | CG | HIS B | 196 | Distance: | 3.79 |
| CE1 | TYR C | 58 - | CD2 | HIS B | 196 | Distance: | 3.22 |
| CE1 | TYR C | 58 - | NE2 | HIS B | 196 | Distance: | 3.48 |
| CE2 | TYR C | 58 - | CD | GLU B | 201 | Distance: | 3.50 |
| CE2 | TYR C | 58 - | OE1 | GLU B | 201 | Distance: | 3.45 |
| CE2 | TYR C | 58 - | OE2 | GLU B | 201 | Distance: | 2.96 |
| CZ | TYR C | 58 - | CD2 | HIS B | 196 | Distance: | 3.65 |
| | | | | | | | |
| C | THR C | 59 - | NZ | LYS B | 192 | Distance: | 3.74 |
| O | THR C | 59 - | CE | LYS B | 192 | Distance: | 3.33 |
| O | THR C | 59 - | NZ | LYS B | 192 | Distance: | 3.04 |
| OG1 | THR C | 59 - | NZ | LYS B | 192 | Distance: | 2.84 |
| | | | | | | | |
| CB | ASP C | 60 - | OH | TYR B | 139 | Distance: | 3.62 |
| CG | ASP C | 60 - | OH | TYR B | 139 | Distance: | 3.36 |
| CG | ASP C | 60 - | NZ | LYS B | 148 | Distance: | 3.23 |
| OD1 | ASP C | 60 - | NZ | LYS B | 148 | Distance: | 2.91 |
| OD1 | ASP C | 60 - | O | HOH J | 25 | Distance: | 3.60 |
| OD2 | ASP C | 60 - | CD1 | ILE B | 137 | Distance: | 3.69 |
| OD2 | ASP C | 60 - | CE1 | TYR B | 139 | Distance: | 3.24 |
| OD2 | ASP C | 60 - | CZ | TYR B | 139 | Distance: | 3.24 |
| OD2 | ASP C | 60 - | OH | TYR B | 139 | Distance: | 2.42 |
| OD2 | ASP C | 60 - | NZ | LYS B | 148 | Distance: | 2.90 |
| | | | | | | | |
| O | TYR C | 61 - | O | HOH J | 25 | Distance: | 3.16 |
| | | | | | | | |
| CG | ASP C | 63 - | NH2 | ARG B | 188 | Distance: | 3.78 |
| OD1 | ASP C | 63 - | NH2 | ARG B | 188 | Distance: | 3.74 |
| OD2 | ASP C | 63 - | NH2 | ARG B | 188 | Distance: | 3.06 |
| | | | | | | | |
| CD | ARG C | 100 - | CD2 | LEU B | 202 | Distance: | 3.73 |
| NE | ARG C | 100 - | CD2 | LEU B | 202 | Distance: | 3.53 |
| | | | | | | | |
| N | GLY C | 102 - | O | LEU B | 202 | Distance: | 3.48 |
| CA | GLY C | 102 - | O | LEU B | 202 | Distance: | 3.24 |
| CA | GLY C | 102 - | CE1 | HIS B | 203 | Distance: | 3.56 |
| C | GLY C | 102 - | CE1 | HIS B | 203 | Distance: | 3.66 |
| C | GLY C | 102 - | NE2 | HIS B | 203 | Distance: | 3.65 |
| | | | | | | | |
| CB | SER C | 103 - | O | GLY B | 134 | Distance: | 3.32 |
| OG | SER C | 103 - | CG | PRO B | 133 | Distance: | 3.78 |
| OG | SER C | 103 - | O | GLY B | 134 | Distance: | 3.16 |
| OG | SER C | 103 - | CE1 | HIS B | 203 | Distance: | 3.44 |
| OG | SER C | 103 - | NE2 | HIS B | 203 | Distance: | 3.43 |

The identification of the residues which form the interface between Nef and sdAb19 enables the skilled person to identify compounds capable of modulating interaction of Nef and sdAb19, said method comprising the steps of:
a) providing the atomic coordinates of the Nef protein, thereby defining a three-dimensional structure of the Nef protein, and
b) using said three-dimensional structure to design or select a compound capable of interacting with the conformational epitope identified above.

In another aspect, the present invention relates to a method for identifying a compound capable of modulating interaction of Nef and sdAb19, said method comprising the steps of:
a) providing the atomic coordinates of Table 1, thereby defining a three-dimensional structure of the tripartite protein complex of the invention ;
b) using said three-dimensional structure to design or select a compound modulating the interaction of Nef and sdAb19 by computer modeling;
c) providing said compound.

As used herein, the terms "modulating the interaction of Nef and sdAb19" mean to inhibit or enhance the formation of an interaction of Nef and sdAb19 in a sufficient manner such that the assembly of the Nef/sdAb19 complex is prevented or stimulated, respectively.

The identification of such compounds maybe particularly useful for identifying potential inhibitors of Nef functions. The functions of Nef are largely related to perturbations of intracellular trafficking and signalling pathways. They include in particular, down-regulation of cell surface receptors including CD4, interaction with the endocytic and signalling pathways, and increase of HIV infectivity. By "Nef functions", it is thus herein referred to the CD4 cell surface down-regulation activity, interaction with the intracellular endocytic and signalling pathways, and increase of HIV infectivity. Tests for assaying each of these functions are well-known in the art and are thus easily available to the person of skills in the art (WO 2009/066241; Bouchet et al., Blood, 117(13): 3559-3568, 2011; Breuer et al., PLoS One, 6(5):e20033, 2011; Järviluoma et al., PLoS One, 7(7):e40331, 2012; Bouchet et al., J Virol, 86(9): 4856-4867, 2012). These functions are required for Nef contribution to HIV pathogenesis. The said compounds identified by the method of the invention are therefore particularly useful for treating HIV-infected patients.

Examples of modulating compounds as described above include, but are not limited to, peptides, antibodies and small molecules, that would be expected to interfere with the interaction of Nef and sdAb19, and, as a consequence, with the assembly of the Nef/sdAb19 complex.

As described above, nine residues located in the C-terminal half of Nef are comprised within the said sdAb19-binding interface, i.e. they mediate the interaction between Nef and the sdAb19 antibody fragment. These residues correspond to positions 139, 148, 155, 188, 192, 198, 201, 202, and 203. The residues of the Nef protein are numbered by reference to the sequence of UniProtKB/Swiss-Prot accession number P03407 (SEQ I D NO. 2).

Thus, according to a further preferred embodiment, said compound modulating the interaction of Nef and sdAb19 binds to at least one of the amino acid residue selected from the group of amino acid residues at positions 139, 148, 155, 188, 192, 198, 201, 202 and 203 of sequence SEQ ID N°2. Even more preferably, the compounds binding to the said amino acids inhibit the interaction of Nef and sdAb19.

In yet another preferred embodiment, the method for identifying a compound modulating the interaction of Nef and sdAb19 further comprises the step of:
d) physically contacting said compound with the complex between the Nef, and sdAb19 polypeptides to determine the ability of said compound to modulate the interaction of Nef and sdAb19.

In the method(s) described above for identifying compound(s) modulating the interaction of Nef and sdAb19, said compound(s) may be designed *de novo,* using the three-dimensional structure of the crystal according to the invention alone or in combination with a portion of a known compound.

For *de novo* design, one skilled in the art may use any suitable computer modeling program well known in the art. Design in these modeling programs is generally based upon the prediction of a conformational interaction between the three-dimensional structure of a protein (e.g. of the crystal of the present invention) and a candidate compound. For example, generally, for a protein to effectively interact with a compound, it is necessary that the three-dimensional structure of the compound assumes a compatible conformation that allows the compound to bind to the protein in such a manner that a desired result is obtained upon binding. The knowledge of the three-dimensional structure of the protein thus enables a skilled artisan to design or select a compound having such compatible conformation. Examples of such program include, but are not limited to, LUDI (Böhm et al., Comp. Aid. Molec. Design, 6: 61-78, 1992), LeapFrog (Tripos), CAVEAT (Lauri and Bartlett, J. Comp. Aided Mol. Design., 8: 51-66, 1994), GRID (Goodford et al., Med. Chem., 28: 849-857, 1985), MCSS and HOOK (Miranker et al., Proteins, 11: 29-34, 1991; Eisen et al., Proteins, 19(3): 199-221, 1994), BREED (Pierce et al., Med Chem., 47(11): 2768-2775, 2004) and combinations thereof. Other computer modeling techniques known in the art may also be used (Schneider et al., Nat. Rev. Drug Discov., 4: 649-663, 2005; Höltje et al., in Molecular Modeling. Basic Principles and Applications, 3rd edition; Wiley-VCH: Weinheim, 2008; Schneider et al., in Molecular Design - Concepts and Applications, Wiley-VCH, Weinheim, 2008).

For *de novo* design, one skilled in the art may also generate candidate compounds by screening random peptide libraries produced for example in recombinant bacteriophage (Scott et al., Science, 249: 386-390, 1990; Cwirla et al., Proc. Natl. Acad. Sci. USA, 87: 6378-6382, 1990), or a combinatorial chemical library. Candidate compounds selected in this manner can be systematically modified by computer modeling programs until one or more promising candidate compounds are identified.

In the method(s) described above for identifying compound(s) modulating the interaction of Nef and sdAb19, said compound(s) may also be selected from a known compound. For example, one skilled in the art may select a candidate compound by electronic screening of well-known large compound libraries, such as the Available Chemical Directory (ACD; http://www.organicworldwide.net/content/available-chemical-directory). Compounds of such libraries may be analyzed by docking programs. In particular, to evaluate the quality of fit and strength of interactions between ligands or potential ligands and Nef ligand binding sites, docking programs such as Autodock (available from Oxford Molecular, Oxford, UK), Dock (available from Molecular Design Institute, University of California San Francisco, CA), Gold (available from Cambridge Crystallographic Data Centre, Cambridge, UK) and FlexX and FlexiDock (both available from Tripos, St. Louis, MO) may be used. These programs and the program Affinity (available from Molecular Simulations, San Diego, CA) may also be used in further development and optimization of ligands. Standard molecular mechanics force fields such as CHARMm and AMBER may be used in energy minimization and molecular dynamics.

Once candidate compounds are identified, they can be chemically synthetized, and their biological activity tested as follows.

The capacity of a compound to modulate the interaction between Nef and sdAb19 may be tested by any method which is known to the person of skills in the art to be suitable for assessing the interaction between two proteins. These methods include such technique as e.g. immunoblotting, immunoprecipitation analyzes, Radio-Immuno Assays, ELISAs, assays of antibodies by immunofluorescence microscopy, FRET, BRET, surface plasmon resonance (BiaCORE), isothermal titration calorimetry (ITC).

The present invention thus provides the means to test and/or identify new or improved binding compounds to Nef which are capable of modulating the interaction between Nef and sdAb19.

Since sdAb19 inhibits HIV functions *in vivo* in transgenic mice expressing Nef (Bouchet et al., Blood, 117(13): 3559-3568, 2011), potential compounds which can bind Nef through the sdAb19-binding interface are expected to be capable of inhibiting the functions of Nef in infected cells such as promoting HIV replication. Such therapeutic compounds which can inhibit HIV infection can thus be designed using structure-based drug design. Until the discovery of the three-dimensional structure of the present invention, no information was available for structure-based development of therapeutic compounds based on the structure of the domain of Nef mediating the interaction with sdAb19. Such rational development could not be executed *de novo* from available linear amino acid sequence information.

As explained above, structure-based drug design refers to the use of computer simulation to predict a conformational interaction between the three-dimensional sdAb19-interacting Nef domain structure of the present invention and a potential therapeutic compound. For example, generally, for a protein to effectively interact with a therapeutic compound, it is necessary that the three-dimensional structure of the therapeutic compound assume a compatible conformation that allows the compound to bind to the protein in such a manner that a desired result is obtained upon binding. Knowledge of the three-dimensional structure of the protein enables a skilled artisan to design a therapeutic compound having such a compatible conformation.

Thus, it is also an aspect of the invention to provide a method for identifying a potential inhibitor of HIV infection in a human, said method comprising:
a) selecting a compound capable of modulating the interaction between Nef and sdAb19 by the method described above, wherein the said compound is a potential inhibitor.

In a preferred embodiment, the method of the present invention further comprises the steps of:
b) growing a supplemental crystal comprising a protein-ligand complex formed between the Nef protein from HIV-1 and said potential inhibitor from step (a), wherein the supplemental crystal effectively diffracts X-rays for the determination of the atomic coordinates of the protein-ligand complex to a suitable resolution; and
c) determining the three-dimensional structure of the supplemental crystal.

In a preferred embodiment, the method of the present invention further comprises the step of:
d) determining from the three-dimensional structure of the supplemental crystal obtained in step c) whether this potential inhibitor is bound to the said Nef protein via the sdAb19-binding interface.

In a more preferred embodiment, the method for identifying a potential inhibitor according to the present invention, further comprises a step e), wherein it is determined whether the said potential inhibitor interacts with at least one of the residues of the Nef protein selected from the group consisting of the amino acid residues at positions 139, 148, 155, 188, 192, 198, 201, 202, and 203 of the Nef protein.

In another more preferred embodiment, the method of the invention further comprises a step of testing whether the said potential inhibitor is capable of inhibiting Nef biological activities.

In a first aspect, the said potential inhibitor is capable of inhibiting CD4 cell surface down-regulation by Nef. In another aspect, the said potential inhibitor is capable of inhibiting the interaction of Nef with the endocytic and signalling pathways. In yet another aspect, the said potential inhibitor is capable of reducing the infectivity of HIV. Preferably, the said potential inhibitor is capable of inhibiting at least two of the said activities. More preferably, the said inhibitor is capable of inhibiting all three activities. Assays for CD4 cell surface down-regulation, or Nef interaction with the endocytic and signalling pathways, or Nef-mediated enhancement of HIV infectivity are well known in the art (WO 2009/066241; Bouchet et al., Blood, 117(13): 3559-3568, 2011; Breuer et al., PLoS One, 6(5):e20033, 2011; Järviluoma et al., PLoS One, 7(7):e40331, 2012; Bouchet et al., J Virol, 86(9): 4856-4867, 2012) and can thus be easily practiced by the skilled person.

The determined structure co-ordinates, or partial structure co-ordinates, of the complex formed by HIV Nef, sdAb19 and Hck SH3 domain can be used, directly or indirectly, by persons skilled in the art, to model the structures of complex comprising sdAb19 and Hck SH3 domain, and homologous Nef proteins, for example Nef from other HIV strains, in particular other primate lentiviruses, and mutant forms of Nef. Knowledge of the structure of the complex comprising the HIV Nef protein represents a unique and essential basis for modeling of structures of complexes comprising such Nef homologues and Nef mutants.

Preferably, models of sdAb19 binding sites of other Nef variants from other HIV strains, for which the structures are not known, are built by homology modeling and generally comprise the steps of:
1) Aligning the amino acid sequence of the protein to be modeled with the sequence of HIV Nef. A preferred program for aligning two or more homologous amino acid sequences is Clustal W 1.8 (Thompson et al. (1994) Nucleic Acids Res. 22, 4673-4680);
2) An initial model is built on a suitable computer with molecular modeling software by incorporating the protein sequence into the structure of HIV Nef in accordance with the alignment;
3) The modeled structure may then be subjected to energy minimization using standard force fields such as CHARMm or AMBER;
4) The energy-minimized model is remodeled in regions where stereochemistry restraints are violated and to correct bad contacts, bond distances, bond angles and torsion. Information from side chain rotamer and structure libraries may be used in modeling of low homology and/or flexible regions such as loop regions;
5) Optionally, molecular dynamics and more rounds of energy minimization may be performed. Specialized computer programs such as Modeler and Homology (available from Molecular Simulations, San Diego, CA) are used by persons skilled in the art to perform automatic or semi-automatic homology model construction. A review on homology modeling can be found in Rodriguez et al. (Bioinformatics, 14(6): 523-528 1998).

Therefore, a method is provided in the present invention for selecting, testing and/or rationally or semi-rationally designing a modified mature Nef molecule, characterized by applying any of the atomic co-ordinates as shown in Table 1, and/or the atomic co-ordinates of a crystal structure modeled after said co-ordinates.

The present invention furthermore relates to the use of any of the atomic co-ordinates shown in Table 1 and/or the atomic co-ordinates of a crystal structure modeled after said co-ordinates for the identification of a potential inhibitor of a Nef or Nef-like protein, such that it can inhibit the functions of the protein, including receptor cell surface down-regulation, interactions with intracellular endocytic and signalling pathways, and HIV infectivity and replication increase.

Following homology modeling, the quality of the model structure can be estimated using specialized computer programs such as PROCHECK (Laskowski et al., J. Appl. Cryst. 26: 283-291, 1993) and Verify3D (Luthy et al., Nature, 356: 83-85, 1992).

In addition to the modeling of Nef homologs, the present invention provides a method for designing mutants of Nef.

On basis of the detailed atomic and functional description of the sdAb19-interaction Nef domain enabled by this invention, a rational or semi-rational selection of desirable amino acid residues for mutation is enabled. Such mutants can be used to further characterize the role and importance of specific residues and regions within the said protein domain.

Also, knowledge of the structure co-ordinates of the sdAb19/Nef interface aids in selecting amino acid residues for mutagenesis with the purpose of altering the properties of Nef. For example, it could be desirable to increase e.g. the thermostability, the stability towards chaotropic agents and detergents, or the stability at alkaline pH. For example, it could be desirable to alter the intramolecular interactions between the Nef protein and the sdAb19 antibody or the Neffins, or to produce mutants of Nef with reduced or increased sensitivity to inhibitors. Furthermore it could be desirable to design mutants of Nef which are specifically affected in one and only one function. For example, it could be desirable to design mutants which are only affected in CD4 cell surface down-regulation, but whose other activities would be kept intact. Alternatively, it could desirable to design mutants which are only affected in MHC-I cell surface down-regulation, but not in any other function. Likewise, it could be desirable to design Nef mutants which have lost any effect on HIV infectivity. Such mutants would be powerful research tools for understanding Nef functions and regulations.

A number of methods are available for a person skilled in the art for preparing random or site-directed mutants in the sdAb19-interaction domain of Nef. For example, mutations can be introduced by use of oligonucleotide-directed mutagenesis, by error-prone PCR, by UV-light radiation, by chemical agents or by substituting some of the coding region with a different nucleotide sequence either produced by chemical synthesis or of biological origin, e.g. a nucleotide sequence encoding a fragment of Nef from different lentiviral strains and species.

Random and site-directed mutants of Nef can typically be expressed and purified by the same methods as described for expression and purification of wild type Nef.

Once the mutant forms of Nef are obtained, the mutants can be characterized or screened for one or more properties of interest. The interaction between Nef and sdAb19 or the Neffins can be tested by any method known to the person of skills in the arts described above. Likewise, assays for cell surface receptor down-regulation, or Nef interaction with endocytic and signalling pathways, or Nef-mediated enhancement of HIV infectivity are well known in the art (WO 2009/066241; Bouchet et al., Blood, 117(13): 3559-3568, 2011; Breuer et al., PLoS One, 6(5):e20033, 2011; Järviluoma et al., PLoS One, 7(7):e40331, 2012; Bouchet et al., J Virol, 86(9): 4856-4867, 2012).

The practice of the invention employs, unless other otherwise indicated, conventional techniques or protein chemistry, molecular virology, microbiology, recombinant DNA technology, and pharmacology, which are within the skill of the art. Such techniques are explained fully in the literature. (See Ausubel et al., Current Protocols in Molecular Biology, Eds., John Wiley & Sons, Inc. New York, 1995; Remington's Pharmaceutical Sciences, 17th ed., Mack Publishing Co., Easton, Pa., 1985; and Sambrook et al., Molecular cloning: A laboratory manual 2nd edition, Cold Spring Harbor Laboratory Press - Cold Spring Harbor, NY, USA, 1989; Introduction to Glycobiology, Maureen E. Taylor, Kurt Drickamer, Oxford Univ. Press (2003); Worthington Enzyme Manual, Worthington Biochemical Corp. Freehold, NJ; Handbook of Biochemistry: Section A Proteins, Vol I 1976 CRC Press; Handbook of Biochemistry: Section A Proteins, Vol II 1976 CRC Press; Essentials of Glycobiology, Cold Spring Harbor Laboratory Press (1999)). The nomenclatures used in connection with, and the laboratory procedures and techniques of, molecular and cellular biology, protein biochemistry, enzymology and medicinal and pharmaceutical chemistry described herein are those well-known and commonly used in the art.

Unless defined otherwise, all technical and scientific terms used herein have the same meaning as is commonly understood by one of the skill in the art to which this invention belongs.

Other characteristics and advantages of the invention appear further in the description with the examples and figures whose legends are presented below.

### Figure legends:

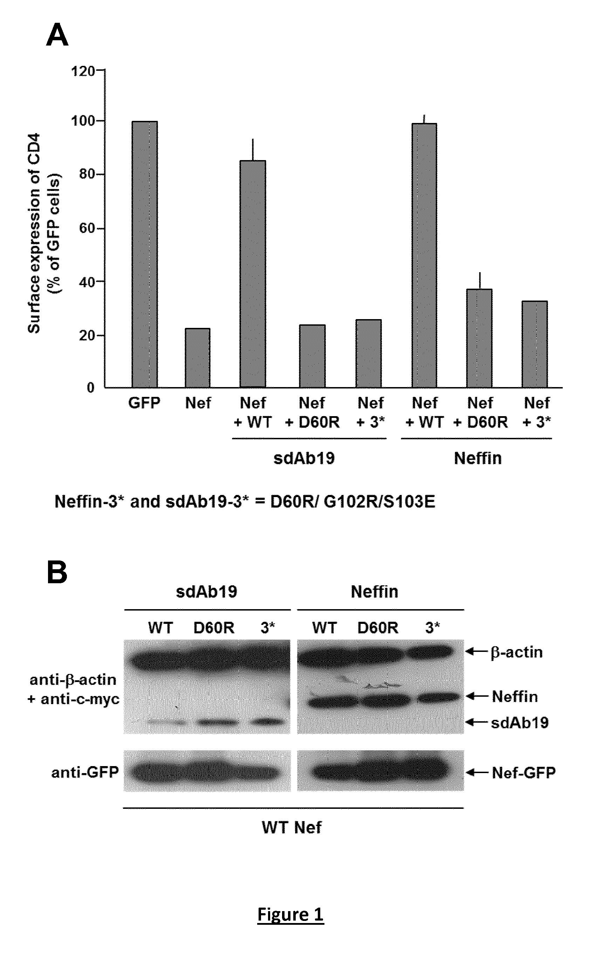

**Figure 1****:** Cell surface CD4 down-regulation induced by the Nef protein of the NL43 HIV-1 strain in cells expressing wild-type or mutated sdAb19 and Neffin. HeLa cells stably expressing the CD4 receptor (HeLa-CD4 cells) were transfected with the plasmid for expression of either Nef-GFP (Nef, NL43 allele) or GFP in combination with the plasmid (1:4 plasmid ratio) for expression of wild-type (WT) or mutated (D60R, and 3* mutants) sdAb19 or Neffin as indicated. A) Transfected cells were stained 24 h later with phycoerytrin(PE)-conjugated anti-CD4 (clone SK3, Becton Dickinson) at 4°C, and the cell surface expression of CD4 in Nef-GFP- or GFP-expressing cells was measured by flow cytometry. Results are expressed as the percentage of the mean fluorescence intensity (MFI) determined in GFP-positive cells relative to that determined in GFP-negative cells. Values are the means from 3 independent experiments. Error bars represent 1 standard deviation (SD) from the means. sdAb19-3* and Neffin-3* mutants contain 3 amino acid substitutions at positions D60, G102 and S103 within the sdAb19 coding sequence (D60R, G102R and S103E, respectively). B) Transfected cells were lysed 24 h later and cell lysates were analyzed by Western blotting (WB) using anti-c-Myc (clone 9E10, Roche) and anti-B-actin (clone A5441, Sigma) (top) or anti-GFP (Roche) antibodies (bottom).

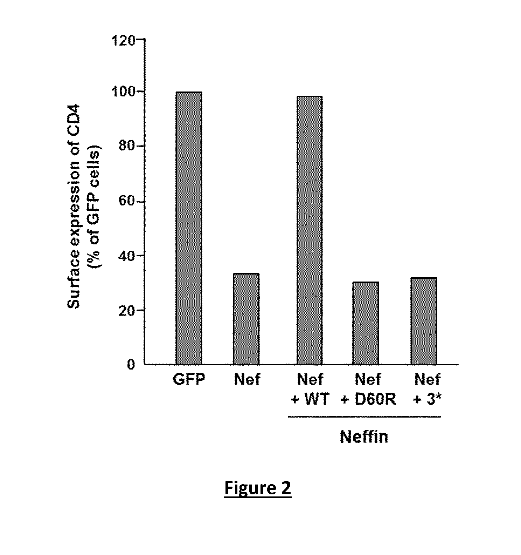

**Figure 2****:** Cell surface CD4 down-regulation induced by the Nef protein of the SF2 HIV-1 strain in cells expressing wild-type or mutated sdAb19 and Neffin. HeLa cells stably expressing the CD4 receptor (HeLa-CD4 cells) were transfected with the plasmid for expression of either Nef-GFP (Nef, SF2 allele) or GFP in combination with the plasmid (1:4 plasmid ratio) for expression of wild-type (WT) or mutated (D60R, and 3* mutants) Neffin as indicated. Transfected cells were stained 24 h later with phycoerytrin(PE)-conjugated anti-CD4 (clone SK3, Becton Dickinson) at 4°C, and the cell surface expression of CD4 in Nef-GFP- or GFP-expressing cells was measured by flow cytometry. Results are expressed as the percentage of the mean fluorescence intensity (MFI) determined in GFP-positive cells relative to that determined in GFP-negative cells. Values are the means from 3 independent experiments. Error bars represent 1 standard deviation (SD) from the means. Neffin-3* mutant contain 3 amino acid substitutions at positions D60, G102 and S103 within the sdAb19 coding sequence (D60R, G102R and S103E, respectively).

### Experimental examples

### Crystallization of the Nef-sdAb19 complex:

Key issues for the successful crystallization of the HIV-1 Nef protein with the sdAb19 antibody fragment were:
- the N-terminal truncated Nef (Nef 45-210)
- deletion of the C-terminal flexible loop in Nef (delta 158-178)
- using the sdAb19 construct 1-118
- addition of an SH3 domain to Nef upon protein crystallization. The SH3 domain of Hck seemed to be an important stabilization factor of Nef for protein crystallization. Without using the SH3 domain only thin, small, sensitive and bad diffracting crystals could be generate.
- Improvement of the initial crystals were performed with the Opti-Salt Additive Screen (a commercial screen from Hampton Research)

### Protein Expression:

**HIV-1 Nef_{SF2}** (accession code K02007)

### Plasmid:

pProEx-HTa Nef (45-210, I47M, T48A, C59S, C210A, delta 158-178, codon optimized for expression in *E.coli,* N-terminal His-tag cleavable with TEV protease)

The deletion 158-178 encompasses the C-terminal flexible loop of Nef, which is required for protein crystallization

### Expression:

LB-Medium, IPTG Induction (0.3 mM)

37°C until OD₆₀₀ of 0.6, then 23°C for 14h

### Purification:

Affinity Chromatography (Nickel column)

Size Exclusion Chromatography: Sd 75 16/60

Buffer: 20 mM Tris, pH 8.0, 100 mM NaCl

### sdAb19:

### Plasmid:

pGEX-4T1 tev sdAb19 (1-118, N-term. GST-tag cleavable with TEV protease)

### Expression:

Fermenter, 27 liter, TB-Medium, Lactose Induction (0.2%)

37°C for 4h, then 23C for 14h

### Purification:

Affinity Chromatography (GSH column)

Size Exclusion Chromatography: Sd 75 16/60

Buffer: 20 mM Tris, pH 9.0, 100 mM NaCl

### SH3-B6:

### Plasmid:

pGEX-2T tev Hck-SH3 B6 (wt) (described in Horenkamp et al., Traffic 2011)

### Expression:

TB-Medium, Lactose Induction (0.2%)

37 °C for 4h, then 23C for 14h

### Purification:

Affinity Chromatography (GSH column)

Size Exclusion Chromatography: Sd 75 16/60

Buffer: 20 mM Tris, pH 9.0, 100 mM NaCl

### Formation of the recombinant protein complex

### Tripartite assembly of three individually purified proteins:

*In vitro:* mixing Nef (45-210, Δf.l.) with sdAb19t

Purification: Size Exclusion Chromatography: Sd 75 16/60

### then adding SH3-B6

### Purification of the tripartite complex:

Size Exclusion Chromatography: Sd 75 16/60

Buffer: 20 mM Tris, pH 9.0, 100 mM NaCl

### Crystallization of the tripartite complex Nef (45-210, Δf.l.)-sdAb19-SH3-B6

big single crystals with
- 0.2 M potassium formate
- 17.5% PEG 3350
- 0.35 M ammonium chloride (additive),
(without additive = sea urchin like crystal clustering)

temperature: 20° C

crystal growth: 0 days - 21 days

size: 500-600 µm

resolution: 2.1 Å

space group: P4₁ (76)

The technical problem that we were facing was how to assembly the complex in a right way to get a perfect sample for crystallization. Here, the addition of the SH3-B6 Hck domain as stabilizing agent helped significantly and was the resolution for success.

### Binding interface between HIV-1 Nef and sdAb19

### a) Shell of 3.8 Å

Residues of sdAb19 involved in binding to HIV-1 Nef:
Asn35, Arg46, Trp49, Thr54, Ser56, Tyr58, Thr59, Asp60, Asp63, Arg100, Gly102, Ser103

Residues of HIV-1 Nef_{SF2} involved in binding to sdAb19:
Tyr139, Lys148, Glu155, Arg188, Lys192, Met198, Glu201, Leu202, His203

*The interacting residues were determined within a distance of 0.38 nm (3.8 Å) between the two molecules.*

### b) Direct interactions

Direct interactions of side chains between sdAb19 and Nef:
Trp49-Glul55; (Asn35/Ser56)-Glu210; Thr59-Lys192; Asp60-(Tyr139/Lys148); Asp63-Arg188; Ser103-His203.

### c) Residues identified in PDBePISA to contribute to the binding

sdAb19:
**Asn35,** Leu39, Arg46, Arg47, Glu48, **Trp49,** Leu52, Thr54, **Ile55, Ser56,** Tyr58, **Thr59, Asp60,** Tyr61, Ala62, **Asp63,** Arg100, Gly102, Ser103.
*CDR1: 29-35; CDR2: 54-61; CDR3: 100-105.*

Nef:
Thr132, Pro133, Gly134, Pro135, Ile137, **Tyr139, Lys148,** Val150, Pro151, Val152, Pro154, **Glu155,** Lys156, Val157, **Arg188,** Asp190, **Lys192,** Phe195, His196, His197, Met198, **Glu201,** Leu202, **His203,** Glu205.

### Bold residues indicate residues that form intermolecular hydrogen bonds

### Cell surface CD4 down-regulation in cells expressing wild-type or mutated sdAb19 and Neffin

In order to assess the functional relevance of the amino acid residues identified above, the effect of sdAb19 or Neffins mutants on Nef-induced cell surface CD4 down-regulation activity was evaluated.

As shown in Figures 1 and 2, expression of Nef proteins from the NL43 or SF2 HIV-1 starins, respectively, in HeLa cells stably expressing the CD4 receptor led to a decrease of CD4 levels at the cell surface. This reduction was reversed by the concomitant expression of wild-type sdAb19 or wild-type Neffin, as previously shown (Bouchet et al., J. Virol., 86: 4856-4867, 2012). However, normal cell surface levels of the CD4 receptor were not recovered when variants of sdAb19 or Neffins mutated in the domain of interaction with Nef (D60R; 3* = D60R/G120R/S103E) were co-expressed with Nef. Thus the domain of sdAb19 mediating the interaction with Nef is required for sdAb19 function.

## Claims

1. A crystal of a protein complex comprising a Nef protein, the sdAb19 antibody fragment, and the SH3 domain of the Hck kinase, wherein the said Nef protein is represented by the sequence SEQ ID No.4 and mutated sequences thereof, the said sdAb19 is represented by the sequence SEQ ID No. 9, and the said SH3 domain of the Hck kinase is represented by the sequence SEQ ID No. 11.

2. The crystal of claim 1, wherein the space group is P4₁(76), and the unit cell dimension are a = 73.07 Å, b = 73.07 Å, c = 71.25 Å, with α = B = γ = 90°.

3. The crystal of claim 2, wherein the atomic coordinates of the said crystal are defined in Table 1.

4. A conformational epitope on the HIV Nef protein which is bound by the sdAb19 antibody fragment, said conformational epitope comprising any one of the following residues: Y139, K148, E155, R188, K192, M198, E201, L202, and H203.

5. The conformational epitope of claim 4, wherein said conformational epitope consists of the following residues: Y139, K148, E155, R188, K192, M198, E201, L202, and H203.

6. A method for producing the crystal of a protein complex comprising a Nef protein, sdAb19, and the SH3 domain of the Hck kinase, comprising the following steps:
a) providing a protein solution comprising the complex of the Nef, sdAb19 and Hck SH3 domain polypeptides, wherein the said Nef protein is represented by the sequence SEQ ID No. 4 and mutated sequences thereof, the said sdAb19 is represented by the sequence SEQ ID No. 9, and the said SH3 domain of the Hck kinase is represented by the sequence SEQ ID No. 11 ;
b) subjecting said protein solution to conditions which promote optimal crystallization.

7. The method of claim 6, wherein step a) comprises the further steps of:
i. constructing a recombinant vector by cloning the gene encoding each polypeptide into an expression vector;
ii. transforming the recombinant vector obtained in step i) into a host cell;
iii. expressing the polypeptide encoded by the said recombinant vector; and
iv. purifying the said polypeptide
v. assembling the complex of the three polypeptides, and
vi. purifying the resulting tripartite complex.

8. The method of anyone of claims 6 or 7, wherein each of the polypeptide is added at a molar ratio comprised between 0.1 and 1, preferably between 0.5 and 1, more preferably of 1.

9. A method for determining the three-dimensional structure of a crystal obtained by the method of anyone of claims 6 to 8, said method comprising the steps of:
a) providing at least one crystal comprising or consisting of the tripartite polypeptide complex of the invention, said crystal being preferably obtained according to the method for producing a crystal as described above ;
b) flash-cooling said crystal into liquid nitrogen after a brief transfer to a cryo-protecting buffer; and
c) analyzing said crystal to determine its three-dimensional structure.

10. A method for determining whether a compound interacts with Nef via the sdAb19 binding region, said method comprising the steps of:
a) measuring the binding of said compound to wild-type Nef;
b) measuring the binding of said compound to said Nef protein mutated in the sdAb19-binding region; and
c) comparing the binding of step a) and the binding of step b).

11. A method of screening a library for a compound capable of inhibiting Nef functions, said method comprising the steps of:
a) determining whether each compound of the said library is capable of interacting with Nef via the sdAb19 binding region by the method of claim 10; and
b) selecting the compound of step a), if said compound interacts with Nef via the sdAb19 binding region.

12. A method for identifying a compound capable of modulating interaction of Nef and sdAb19, said method comprising the steps of:
c) providing the atomic coordinates of the Nef protein;
d) using said three-dimensional structure to design or select a compound capable of interacting with the conformational epitope of claim 4;
e) providing said compound; and
f) optionally physically contacting said compound with the Nef, sdAb19 and Hck SH3 domain polypeptides, to determine the ability of said compound to modulate the interaction of Nef and sdAb19.

13. A method for identifying a compound capable of modulating interaction of Nef and sdAb19, said method comprising the steps of:
g) providing the atomic coordinates of Table 1, thereby defining a three-dimensional structure of the complex of the Nef, sdAb19 and Hck SH3 domain polypeptides ;
h) using said three-dimensional structure to design or select a compound modulating the interaction of Nef and sdAb19 by computer modeling;
i) providing said compound; and
j) optionally physically contacting said compound with the complex of the Nef, sdAb19 and Hck SH3 domain polypeptides, to determine the ability of said compound to modulate the interaction of Nef and sdAb19.

14. A method for identifying a potential inhibitor of HIV infection in a human, said method comprising:
a) selecting a compound capable of modulating the interaction between Nef and sdAb19 by the method of claim 12 or 13, wherein the said compound is a potential inhibitor.

15. The method of claim 14, further comprising the step of:
b) growing a supplemental crystal comprising a protein-ligand complex formed between the Nef protein from HIV-1 and said potential inhibitor from step (a), wherein the supplemental crystal effectively diffracts X-rays for the determination of the atomic coordinates of the protein-ligand complex to a suitable resolution; and
c) determining the three-dimensional structure of the supplemental crystal; and
d) optionally determining from the three-dimensional structure of the supplemental crystal obtained in step c) whether this potential inhibitor is bound to the said Nef protein via the sdAb19-binding interface, wherein the said interface is the conformational epitope of claims 4 or 5.

16. The method of any one of claims 14 or 15, wherein the potential inhibitor is capable of inhibiting at least one the Nef function selected in the group consisting of:
- membrane receptor cell surface down-regulation,
- interaction with endocytic and signaling pathways, and
- increase of HIV infectivity and replication.
